# EUROPEAN PATENT APPLICATION

(11) **EP 0 987 238 A1**
(43) Date of publication of application: **22.03.2000**
(21) Application number: 98902222.3
(22) Date of filing: 13.02.1998
(51) Int. Cl.: C07C 13/36, C07C 25/22, C07C 25/24, C07C 43/192, C07C 43/225, C07C 49/115, C07C 49/215, C07C 69/753, C07C 69/757, C07C 69/76, C07C 69/773, C07C 255/45, C07C 255/50, C07D 239/26, C07F 7/12, C09K 19/32, C09K 19/42, G02F 1/13

(54) **LIQUID-CRYSTALLINE COMPOUNDS HAVING BICYCLO 1.1.1]PENTANE STRUCTURE, LIQUID-CRYSTAL COMPOSITION, AND LIQUID-CRYSTAL DISPLAY ELEMENT**

(30) Priority: 13.02.1997 JP 4461597
(71) Applicant: CHISSO CORPORATION, Osaka-shi, Osaka-fu 530-0005 (JP)
(72) Inventor: DE MEIJERE, Armin, D-37077 Göttingen (DE); MESSNER, Matthias, D-37077 Göttingen (DE); KAZHUSHKOV, Sergei, D-37077 Göttingen (DE); DEMUS, Dietrich, D-06118 Halle (DE); KOBAYASHI, Katsuhiko, Ichihara-shi Chiba 290-0003 (JP); MIYAZAWA, Kazutoshi, Ichihara-shi Chiba 290-0158 (JP); MATSUI, Shuichi, Ichihara-shi Chiba 290-0003 (JP); TAKEUCHI, Hiroyuki, Ichihara-shi Chiba 290-0022 (JP); TAKESHITA, Fusayuki, Kimitsu-shi Chiba 299-1132 (JP); NAKAGAWA, Etsuo, Ichihara-shi Chiba 290-0056 (JP)
(74) Representative: HOFFMANN - EITLE
(86) International application number: JP9800602
(87) International publication number: WO9835924

(57) **Abstract**

An object of the present invention is to provide novel liquid crystalline compounds which have bicyclo[1.1.1]pentane structure and make liquid crystal compositions principally for TFT display mode, STN display mode, or TN display mode develop preferable various physical properties, particularly liquid crystalline compounds from which liquid crystal compositions by which driving at a low voltage and a high speed response are possible and which have a high reliability can be produced;
to provide liquid crystal compositions comprising the compound; and
to provide liquid crystal display devices fabricated by using the composition: the liquid crystalline compounds can be expressed by the general formula (1) 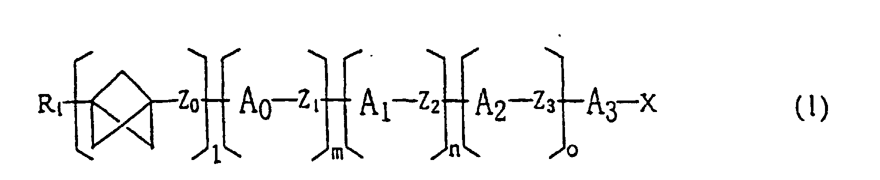 wherein R₁, A₀ to A₃, Z₀ to Z₃, X, ℓ, m, n, and 0 are defined hereinbefore.

## Description

### TECHNICAL FIELD

The present invention relates to novel liquid crystalline compounds which make liquid crystal compositions principally for TFT display mode, STN display mode, or TN display mode develop various preferable physical properties; liquid crystal compositions having various preferable physical properties and comprising the liquid crystalline compound; and liquid crystal display devices fabricated by using the liquid crystal composition. More specifically, the invention relates to novel liquid crystalline compounds which exhibit a positive and large dielectric anisotropy (Δε) or negative and large dielectric anisotropy, and show a high voltage holding ratio (V. H. R.) and excellent miscibility with other liquid crystal compounds by selecting various substituents, bonding groups, and ring structures in the compounds, and to novel liquid crystal compositions having favorable characteristics and produced by using the novel liquid crystalline compound.

### BACKGROUND ART

Display devices fabricated by using liquid crystalline compounds (the term "liquid crystalline compounds" is used in this specification as a general term for the compounds which exhibit a liquid crystal phase and for the compounds which do not exhibit a liquid crystal phase but are useful as component of liquid crystal compositions) have widely been utilized for displays such as that of watches, tabletop calculators, and word processors.

Incidentally, active matrix mode, particularly thin film transistor (TFT) mode is increasingly used as display mode for televisions, viewfinders, and others from the aspect of display performance such as contrast, display capacity, and response time in recent years. Also, STN mode display devices which are simple in structure and can be manufactured at a low production cost compared with the display devices of active matrix mode, while having a large display capacity, is frequently adopted as display for personal computers and the likes.

Recent development in these fields is being progressed while placing stress on downsizing of liquid crystal display devices, portable devices, small power consumption, and high speed response. In order to respond to such requirements, development work is being conducted with a particular emphasis on liquid crystalline compounds and liquid crystal compositions having a low threshold voltage and a low viscosity from the aspect of liquid crystal materials.

In more detail, it is known that the threshold voltage (Vth) can be expressed by the following equation (H. J. Deuling et al., Mol. Cryst. Liq. Cryst., 27 (1975) 81):${\text{Vth = π (K/ε}}_{\text{0}} {\text{· Δε)}}^{\text{1/2}}$

In the equation described above, K is an elastic constant and ε₀ is a dielectric constant in vacuum. As will readily be seen from this equation, two methods, that is, a method of increasing dielectric anisotropy (Δε) and a method of decreasing elastic constant can be considered to lower the threshold voltage. However, since many portions have been unknown with respect to the relation between the elastic constant and the structure of compound, and actual control of elastic constant is impossible in the present technology, it is a current situation that the requirements are usually being coped by using liquid crystal materials having a high dielectric anisotropy (Δε).

Pretilt angle of liquid crystalline compounds or liquid crystal compositions is one of the factors which control threshold voltage. That is, when two compounds having about the same dielectric anisotropy and elastic constant and being different only in pretilt angle were imagined, it is known that the compound having a large pretilt angle is low in threshold voltage. Also, since liquid crystal materials having a large pretilt angle have a large suppressing effect against occurrence of an orientation defect caused by dielectric field, liquid crystalline compounds or liquid crystal composition having a large pretilt angle are required.

It is already known that viscosity is a factor which controls the response speed of liquid crystal molecule oriented in a liquid crystal panel to electric field (Phys. Lett., 39A, 69 (1972)). That is, in order to prepare liquid crystal compositions exhibiting a high speed responsibility, it is preferable to use a large quantity of liquid crystalline compounds having a low viscosity.

Further, in order to make the use at a wide temperature range possible, liquid crystal compositions are required to exhibit nematic phase particularly even at low temperatures, and liquid crystal compositions which do not deposit crystals or develop smectic phase even at low temperatures are required. Liquid crystal compositions to be commercially used are produced by mixing several or thirty-odd liquid crystalline compounds in order to satisfy the characteristics required of individual display devices. Accordingly, it is important that liquid crystalline compounds to be used have a high miscibility to one another even at low temperatures.

Further, since liquid crystal display devices are often used under such severe conditions as high temperatures, high humidities, and outdoors, liquid crystalline compounds used for liquid crystal compositions must have a sufficiently high chemical stability.

Besides, liquid crystal display devices actually used are necessary to maintain a constant level of display quality in an extremely wide temperature range under various environment. Accordingly, in order to achieve this purpose, it is preferable that each physical property of liquid crystal materials is small in its dependency on temperature.

In TFT mode, liquid crystal materials having a high voltage holding ratio (hereinafter abbreviated to V. H. R.) and its small dependency on temperature are necessary from the aspect of structure of the devices. In order to satisfy these requirements, almost all liquid crystal compositions currently used in display devices for TFT mode are composed of fluorine type liquid crystalline compounds.

As such fluorine type liquid crystalline compounds, compounds of the following formula (14) are disclosed in Japanese Patent Publication No. Sho 63-44132. 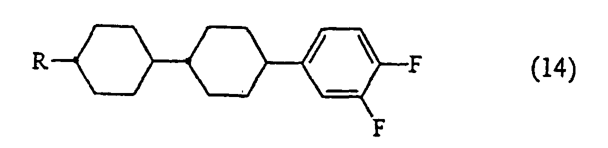 wherein R represents an alkyl group.

Compounds of the formula (14) are liquid crystalline compounds which exhibit a medium extent of dielectric anisotropy (Δε = 5∼6) and have been used as liquid crystal material for various display modes including TFT mode.

On the other hand, development of liquid crystal materials with which driving at a low voltage (3 V or lower) and a high speed response are possible, that is, liquid crystal materials having a high dielectric anisotropy value, exhibiting a low threshold voltage, and having a low viscosity is actively being conducted, and as one of liquid crystalline compounds satisfying such requirements, the compounds of the following formulas are disclosed in Laid-open Japanese Patent Publication No. Hei 2-233626.

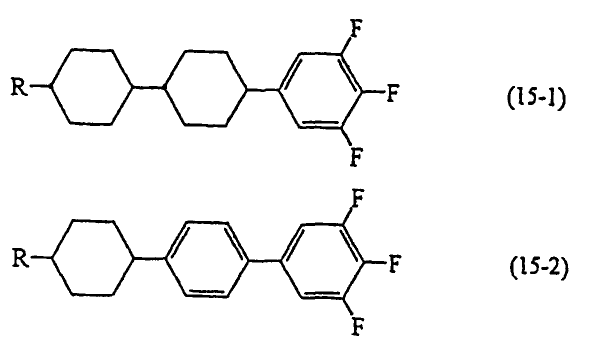

Any compound of the formula (15-1) or (15-2) has 3,4,5-trifluorophenyl group as terminal structure. Compounds of either formula exhibit a large dielectric anisotropy value of Δε = 8∼11 compared with that of the compounds of the formula (14), and are expected as liquid crystal material for driving at a low voltage. However, it a fact well known in the art that in liquid crystalline compounds, the temperature range in which the compounds exhibit nematic phase generally becomes narrow as the number of substituted fluorine atom increases. For instance, whereas nematic phase is noticed in the region of 44.2 to 118.0°C with the compound of the formula (14) in which R = n-C₃H₇, the region in which the compounds exhibit nematic phase is remarkably as narrow as 64.4 to 93.7°C with the compounds of the formula (15-1).

Further, in the case of the compound of the following formula (16) disclosed in DE-19528085 A1, the compound does not exhibit nematic phase at all.

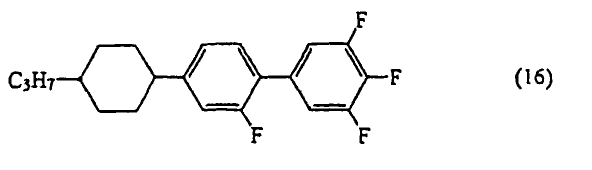

When a liquid crystalline compound in which such many hydrogen atoms are replaced by fluorine atoms was added as component of liquid crystal compositions, driving at a low voltage can be achieved due to its high dielectric anisotropy, but it is extremely difficult to employ the compound for liquid crystal compositions of which the region of a liquid crystal phase is required particularly at high temperature side, since the compound greatly lowers the clearing point of liquid crystal compositions.

Then, as one of the compounds developed for the purpose of solving the problems described above, the compounds of the following formula (17) are disclosed in Laid-open Japanese Patent Publication No. Hei 2-233626.

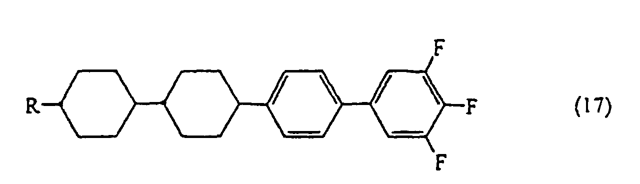

Compounds of the formula (17) have a skeleton structure of four rings, have 3,4,5-trifluorophenyl group as terminal structure of compound just like the compounds of the formula (15-1) or (15-2), exhibit a high dielectric anisotropy. In addition, the compound of the formula (17) in which R = n-C₃H₇ exhibits remarkably excellent characteristics such that the transition temperature of nematic-isotropic liquid is 250°C or higher and the temperature range showing nematic phase is as extremely wide as about 150°C, as liquid crystal material of which driving at a low voltage and the range of liquid crystal phase at high temperature side are required. However, the compounds of the formula (17) are poor in miscibility with other liquid crystalline compounds compared with the three-ring compounds of the formula (14), (15-1), (15-2), or (16), and have a restriction in the amount to be used when they are employed as liquid crystal composition. Additionally, the compounds of the formula (17) have such a problem as deposition of crystals or development of smectic phase in a part of liquid crystal compositions particularly at low temperatures.

In recent years, In-Plane Switching (IPS) mode and Optically Compensated Birefringence (OCB) mode employing TFT display mode were reported in an academic meeting (Asia Display '95 Hamamatsu, Twenty First Panel Discussion on Liquid Crystals) and have attracted public attention as a new driving mode to overcome the narrowness of viewing angle which is a defect of liquid crystal display devices. Also, Vertical Alignment (VA) mode employing TFT mode for the same purpose has been proposed in which mode viewing angle is considerably wider than that in conventional methods (Laid-open Japanese Patent Publication No. Hei 2-176625 and others).

Structure of the IPS mode are characterized in that whereas the panels of a conventional liquid crystal have an electrode on the upper and the lower substrates, the panels driven by the IPS mode have a comb-shaped electrode on a substrate of only one side, and that liquid crystal molecules in the panels are preferably oriented with their major axis being directed in parallel with that of the substrate. As advantages of IPS driving, the following can be mentioned in addition to the widening of viewing angle:
1) Cell thickness can be made smaller than conventional ones since an electrode is provided on a substrate of only one side.
2) Reduction of production cost is possible.
3) Electrodes can be maintained at a constant distance.

As characteristics of liquid crystalline compounds required of the IPS mode and VA mode employing the TFT display mode described above, the following can be mentioned:
1) The compounds exhibit a high voltage holding ratio and are small in their dependency on temperature.
2) The compounds have a high dielectric anisotropy in the direction of minor axis (high negative dielectric anisotropy).

Compounds of the following formula are disclosed in Laid-open Japanese Patent Publication No. Hei 7-501850, Laid-open Japanese Patent Publication No. Sho 56-2952, and Laid-open Japanese patent Publication No. Hei 7-501850 as ones having a negative and high dielectric anisotropy. 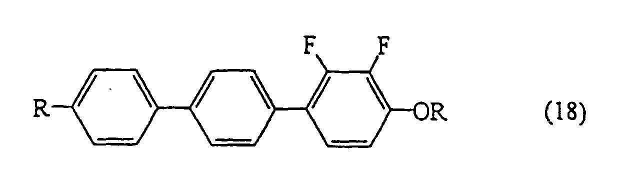 wherein R represents an alkyl group.

It is reported that the compounds of the formula (18) have 2,3-difluoro-4-alkoxyphenyl group as terminal structure and exhibit a negative and comparatively high dielectric anisotropy. However, the compounds of the formula (18) are unpreferably about 10 % at maximum in the miscibility with other known liquid crystalline compounds, and thus have a restriction in the amount to be used when they are employed as component of liquid crystal compositions, since they have a skeleton structure of terphenyl.

As described above, it is a current situation that liquid crystalline compounds have not been found which exhibit a remarkably excellent miscibility at low temperatures as well as exhibiting a high voltage holding ratio and showing a low threshold voltage while having a wide temperature range of nematic phase, that is, liquid crystalline compounds with which liquid crystal compositions the temperature range of which can be widened, which can be driven at a low temperature, and which have a high reliability can be prepared have not been found. Thus, novel liquid crystalline compounds and liquid crystal compositions having improved characteristics to solve the problems described above have long been awaited.

### DISCLOSURE OF THE INVENTION

An object of the present invention is to provide liquid crystalline compounds which are excellent in miscibility, particularly are excellent in miscibility at low temperatures, and from which liquid crystal compositions with which driving at a low voltage and high speed response are possible in various display modes can be produced by proper selection of their substituents; to provide liquid crystal compositions comprising the compound; and to provide liquid crystal display devices fabricated by using the composition.

Liquid crystalline compounds having bicyclo[1.1.1]pentane-1,3-diyl group as their partial structure are already disclosed in patent publications, for example, DE-4118278 A1. In the patent publications, however, only structural formulas of few compounds are specifically described, and physical properties (for example, phase transition points, dielectric anisotropy values, optical anisotropy values, and viscosity of compounds) through which characteristics of the compounds can be grasped, and spectrum data peculiar to the compounds are not described at all. Accordingly, their characteristics as liquid crystalline compound were not known in the least.

As a result of the synthesis of the compound of the following formula (Compound No. 733) which is prepared by inserting bicyclo[1.1.1]pentane-1,3-diyl group between the alkyl chain (R) and cyclohexylene group in the compound expressed by the formula (17) described above and investigations on the physical properties of the compound as liquid crystal by the present inventors for the purpose of clarifying the characteristics of the liquid crystalline compounds having bicyclo[1.1.1]pentane-1,3-diyl group in their partial structure, it has been found out that whereas the compound has a slightly increased viscosity compared with the compounds of the formula (17), it shows about the same values in typical physical properties as liquid crystal as those of the compounds of the formula (17) and exhibits remarkably more excellent characteristics in miscibility, particularly miscibility at low temperatures than the compounds of the formula (17), and that the compound of No. 733 exhibits an extremely high voltage holding ratio and a small its dependency on temperature.

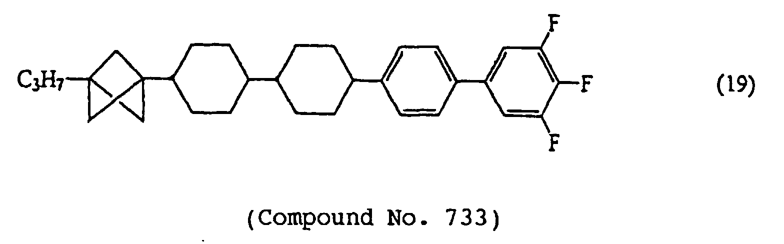

As a result of further investigations by the present inventors, based on the findings described above, for comparing compounds in which bicyclo[1.1.1]pentane-1,3-diyl group is inserted in the structure of known liquid crystalline compounds, it has been found out that miscibility at low temperatures is remarkably improved, that voltage holding ratio is outstandingly increased in the case of fluorine type liquid crystal materials, and that elastic constant ratio (K33/K11) which is also a factor controlling the steepness of V-T curve is lowered by the insertion of bicyclo(1.1.1]pentane-1,3-diyl group, leading to the accomplishment of the present invention.

That is, the present invention is summarized in the aspects (1) to (24) as follows:
(1) The first aspect of the present invention is concerned with a liquid crystalline compound having bicyclo[1.1.1]pentane structure and expressed by the general formula (1) 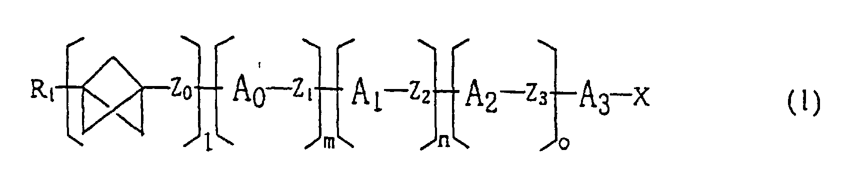 wherein R₁ represents an alkyl group having 1 to 10 carbon atoms in which group not-adjacent any methylene group may be replaced by oxygen atom, sulfur atom, -CH=CH-, -C≡C-, -CO-, -COO-, -OCO-, or SiH₂, and any hydrogen atom in the group may be replaced by a halogen atom; ring A₀, ring A₁, ring A₂, and ring A₃ each independently represent 1,4-cyclohexylene group, 1,3-dioxane-2,5-diyl group, bicyclo[1.1.1]pentane-1,3-diyl group, pyridine-2,5-diyl group, or pyrimidine-2,5-diyl group, or 1,4-phenylene group in which any hydrogen atom on the ring may be replaced by a halogen atom, but at least one of ring A₀, ring A₁, and ring A₂ represents bicyclo[1.1.1]pentane-1,3-diyl group; Z₀, Z₁, Z₂, and Z₃ each independently represent -(CH₂)₂-, -(CH₂)₄-, -CH=CH-, -CH=CH-(CH₂)₂, -(CH₂)₂-CH=CH-, -C≡C-, -CH₂O-, -OCH₂-, -COO-, -OCO-, -CF₂O-, -OCF₂-, -CF=CF-, or single bond; X represents a halogen atom, cyano group, or an alkyl group having 1 to 10 carbon atoms in which alkyl group not-adjacent any methylene group may be replaced by oxygen atom, sulfur atom, -CH=CH-, -C≡C-, -CO-, -COO-, -OCO-, or -SiH₂-, and any hydrogen atom in this group may be replaced by a halogen atom; ℓ, m, n, and o are each independently an integer of 0 to 4, but $\text{ℓ + m + n + o ≦ 4}$, and when ℓ = 0, then $\text{m + n + o ≧ 1}$; and any atom which constitutes this compound may be replaced by its isotope.
(2) The second aspect of the present invention is concerned with the liquid crystalline compound recited in the aspect (1) above wherein $\text{ℓ = m = n = o = 1}$ in the general formula (1).
(3) The third aspect of the present invention is concerned with the liquid crystalline compound recited in the aspect (1) above wherein ℓ = m = 1, $\text{n = o = 0}$, and both ring A₀ and ring A₃ are 1,4-cyclohexylene group in the general formula (1).
(4) The fourth aspect of the present invention is concerned with the liquid crystalline compound recited in the aspect (3) above wherein Z₁ is -CH=CH- in the general formula (1).
(5) The fifth aspect of the present invention is concerned with the liquid crystalline compound recited in the aspect (3) above wherein X is an alkenyl group in the general formula (1).
(6) The sixth aspect of the present invention is concerned with the liquid crystalline compound recited in the aspect (4) above wherein X is an alkenyl group in the general formula (1).
(7) The seventh aspect of the present invention is concerned with the liquid crystalline compound recited in the aspect (1) above wherein ℓ = m = 1, $\text{n = o = 0}$, ring A₃ is 1,4-phenylene group in which any hydrogen atom on the ring may be replaced by a halogen atom, and X is a halogen atom, cyano group, -CF₃, -CF₂H, -OCF₃, or -OCHF₂ in the general formula (1).
(8) The eighth aspect of the present invention is concerned with the liquid crystalline compound recited in the aspect (7) above wherein Z₁ is -COO- or -CF₂O- in the general formula (1).
(9) The ninth aspect of the present invention is concerned with the liquid crystalline compound recited in the aspect (7) above wherein R₁ is an alkenyl group in the general formula (1).
(10) The tenth aspect of the present invention is concerned with the liquid crystalline compound recited in the aspect (1) above wherein X is an alkyl group having 3 to 10 carbon atoms in which group any hydrogen atom may be replaced by a halogen atom in the general formula (1).
(11) The eleventh aspect of the present invention is concerned with the liquid crystalline compound recited in the aspect (1) above wherein at least one of ring A₀, ring A₁, ring A₂, and ring A₃ is 2,3-difluoro-1,4-phenylene group in the general formula (1).
(12) The twelfth aspect of the present invention is concerned with the liquid crystalline compound recited in the aspect (1) above wherein the compound expressed by the general formula (1) is also expressed by the following general formula (1-1), (1-2), (1-3), or (1-4) 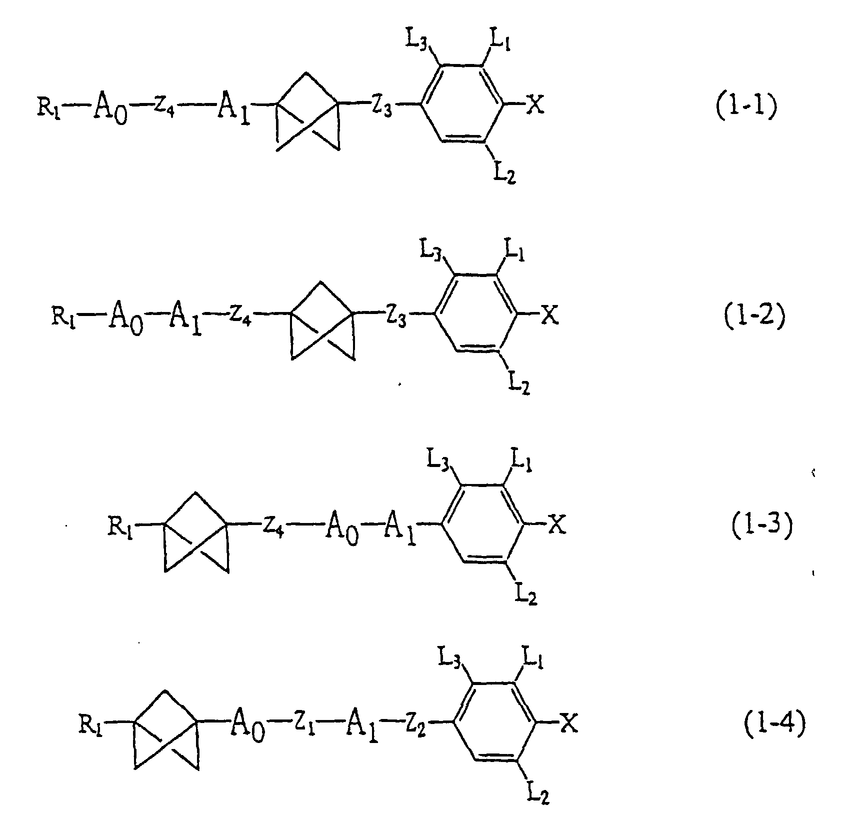 wherein R₁ represents an alkyl group having 1 to 10 carbon atoms in which group not-adjacent any methylene group may be replaced by oxygen atom, sulfur atom, -CH=CH-, -C≡C-, -CO-, -COO-, -OCO-, or -SiH₂-, and any hydrogen atom in this group may be replaced by a halogen atom; ring A₀ and ring A₁ each independently represent 1,4-cyclohexylene group, bicyclo[1.1.1]-pentane-1,3-diyl group, 1,3-dioxane-2,5-diyl group, pyridine-2,5-diyl group, or pyrimidine-2,5-diyl group, or 1,4-phenylene group in which any hydrogen atom on the ring may be replaced by a halogen atom; Z₁, Z₂, and Z₃ each independently represent -(CH₂)₂-, -(CH₂)₄-, -CH=CH-, -CH=CH-(CH₂)₂-, -(CH₂)₂CH=CH-, -C≡C-, -CH₂O-, -OCH₂-, -COO-, -OCO-, -CF₂O-, -OCF₂-, -CF=CF-, or single bond; Z₄ represents -CH₂CH₂-, -(CH₂)₄-, -CH=CH-, -CH=CH-(CH₂)₂-, -(CH₂)₂CH=CH-, -C≡C-, -CH₂O-, -OCH₂-, -COO-, -OCO-, -OF₂O-, -OCF₂-, or -CF=CF-; L₁, L₂, and L₃ each independently represent hydrogen atom or a halogen atom; X represents a halogen atom, cyano group, or an alkyl group having 1 to 10 carbon atoms in which alkyl group not-adjacent any methylene group may be replaced by oxygen atom, sulfur atom, -CH=CH-, -C≡C-, -CO-, -COO-, -OCO-, or -SiH₂- and any hydrogen atom in this group may be replaced by a halogen atom; and each atom which constitutes those compounds may be replaced by its isotope.
(13) The thirteenth aspect of the present invention is concerned with the liquid crystalline compound recited in the aspect (12) above wherein X is -CF₃, -CHF₂, -OCF₃, -OCHF₂, or -OCF₂H in the general formula (1-1), (1-2), (1-3), or (1-4).
(14) The fourteen aspect of the present invention is concerned with a liquid crystal composition comprising at least two components and comprising at least one liquid crystalline compound expressed by the general formula (1) recited in any one of the aspects (1) to (13) above.
(15) The fifteenth aspect of the present invention is concerned with a liquid crystal composition comprising, as a first component, at least one compound recited in any one of the aspects (1) to (13) above, and comprising, as a second component, at least one compound selected from the group consisting of the compounds expressed by any one of the general formulas (2), (3), and (4) 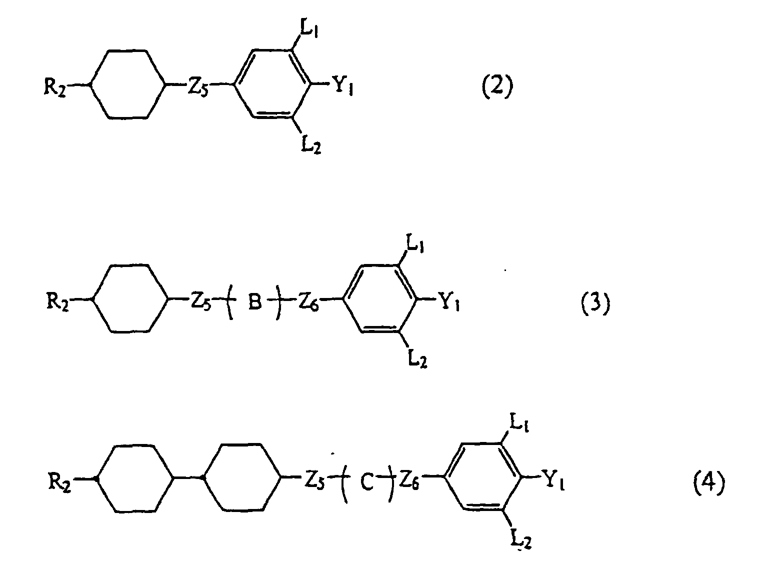 wherein R₂ represents an alkyl group having 1 to 10 carbon atoms in which group not-adjacent any methylene group may be replaced by oxygen atom or -CH=CH-, and any hydrogen atom in the group may be replaced by fluorine atom; Y₁ represents fluorine atom, chlorine atom, -CF₃, -CF₂H, -OCF₃, -OCF₂H, -OCF₂CF₂H, or -OCF₂CFHCF₃; L₁ and L₂ each independently represent hydrogen atom or fluorine atom; Z₅ and Z₆ each independently represent -(CH₂)₂-, -(CH₂)₄-, -CH=CH-, -COO-, -CF₂O-, -OCF₂-, or single bond; ring B represents trans-1,4-cyclohexylene group or 1,3-dioxane-2,5-diyl group, or 1,4-phenylene group in which any hydrogen atom on the ring may be replaced by fluorine atom; ring C represents trans-1,4-cyclohexylene group, or 1,4-phenylene group in which any hydrogen atom on the ring may be replaced by fluorine atom; and any atom which constitutes those compounds may be replaced by its isotope.
(16) The sixteenth aspect of the present invention is concerned with a liquid crystal composition comprising, as a first component, at least one compound recited in any one of the aspects (1) to (13) above, and comprising, as a second component, at least one compound selected from the group consisting of the compounds expressed by the general formula (5) or (6) 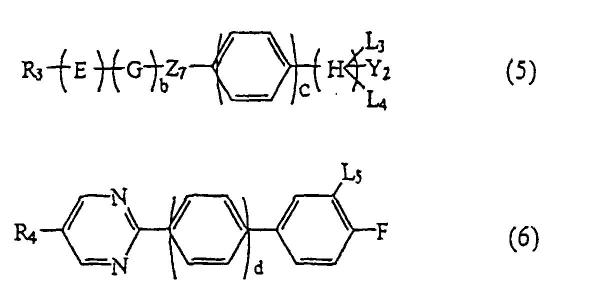 wherein R₃ and R₄ each independently represent an alkyl group having 1 to 10 carbon atoms in which group not-adjacent any methylene group may be replaced by oxygen atom or -CH=CH-, and any hydrogen atom in the group may be replaced by fluorine atom; Y₂ represents cyano group or -C≡C-CN; ring E represents trans-1,4-cyclohexylene group, 1,4-phenylene group, 1,3-dioxane-2,5-diyl group, or pyrimidine-2,5-diyl group; ring G represents trans-1,4-cyclohexylene group, or pyrimidine-2,5-diyl group, or 1,4-phenylene group in which hydrogen atom on the ring may be replaced by fluorine atom; ring H represents trans-1,4-cyclohexylene group or 1,4-phenylene group; Z₇ represents -(CH₂)₂-, -COO-, or single bond; L₃, L₄, and L₅ each independently represent hydrogen atom or fluorine atom; b, c, and d are each independently 0 or 1; and each atom which constitutes those compounds may be replaced by its isotope.
(17) The seventeenth aspect of the present invention is concerned with a liquid crystal composition comprising, as a first component, at least one compound recited in any one of the aspects (1) to (13) above, comprising, as a second component, at least one compound selected from the group consisting of the compounds expressed by any one of the general formulas (2), (3), and (4) described above, and comprising, as a third component, at least one compound selected from the group consisting of the compounds expressed by any one of the general formulas (7), (8), and (9) 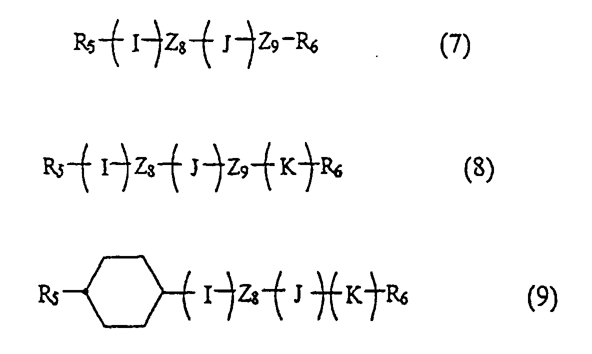 wherein R₅ and R₆ each independently represent an alkyl group having 1 to 10 carbon atoms in which group not-adjacent any methylene group may be replaced by oxygen atom or -CH=CH-, and any hydrogen atom in the group may be replaced by fluorine atom; ring I, ring J, and ring K each independently represent trans-1,4-cyclohexylene group or pyrimidine-2,5-diyl group, or 1,4-phenylene group in which hydrogen atom may be replaced by fluorine atom; Z₈ and Z₉ each independently represent -(CH₂)₂-, -CH=CH-, -C≡C-, -COO-, or single bond; and each atom which constitutes those compounds may be replaced by its isotope.
(18) The eighteenth aspect of the present invention is concerned with a liquid crystal composition comprising, as a first component, at least one compound recited in any one of the aspects (1) to (13), and comprising, as a second component, at least one compound selected from the group consisting of the compounds expressed by any one of the general formulas (10), (11), and (12) 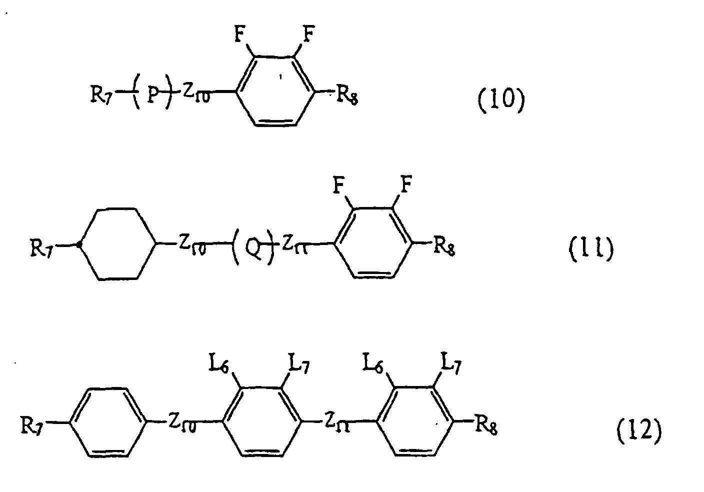 wherein R₇ and R₈ each independently represent an alkyl group having 1 to 10 carbon atoms in which group not-adjacent any methylene group may be replaced by oxygen atom or -CH=CH, and any hydrogen atom in the group may be replaced by fluorine atom; ring P and ring Q each independently represent trans-1,4-cyclohexylene group or 1,4-phenylene group; L₆ and L₇ each independently represent hydrogen atom or fluorine atom but in no case represent L₆ and L₇ simultaneously hydrogen atom; Z₁₀ and Z₁₁ each independently represent -(CH₂)₂-, -COO-, or single bond; and any atom which constitutes those compounds may be replaced by its isotope.
(19) The nineteenth aspect of the present invention is concerned with a liquid crystal composition comprising, as a first component, at least one compound recited in any one of the aspects (1) to (13) above, comprising, as a second component, at least one compound selected from the group consisting of the compounds expressed by any one of the general formulas (7), (8), and (9) described above, and comprising, as a third component, at least one compound selected from the group consisting of the compounds expressed by any one of the general formulas (10), (11), and (12) described above.
(20) The twentieth aspect of the present invention is concerned with a liquid crystal composition comprising, as a first component, at least one compound recited in any one of the aspects (1) to (13) above, comprising, as a second component, at least one compound selected from the group consisting of the compounds expressed by any one of the general formulas (2), (3), and (4) described above, and comprising, as a third component, at least one component selected from the group consisting of the compounds expressed by any one of the general formulas (7), (8), and (9) described above.
(21) The twenty-first aspect of the present invention is concerned with a liquid crystal composition comprising, as a first component, at least one compound recited in any one of the aspects (1) to (13) above, comprising, as a second component, at least one compound selected from the group consisting of the compounds expressed by the general formula (5) or (6) described above, and comprising, as a third component, at least one compound selected from the group consisting of the compounds expressed by any one of the general formulas (7), (8), and (9) described above.
(22) The twenty-second aspect of the present invention is concerned with a liquid crystal composition comprising, as a first component, at least one compound recited in any one of the aspects (1) to (13) above, comprising, as a second component, at least one compound selected from the group consisting of the compounds expressed by any one of the general formulas (2), (3), and (4) described above, comprising, as a third component, at least one compound selected from the group consisting of the compounds expressed by the general formula (5) or (6) described above, and comprising, as a fourth component, at least one compound selected from the group consisting of the compounds expressed by any one of the general formulas (7), (8), and (9) described above.
(23) The twenty-third aspect of the present invention is concerned with a liquid crystal composition further comprising an optically active compound in addition to the liquid crystal composition recited in any one of the aspects (14) to (22) above.
(24) The twenty-fourth aspect of the present invention is concerned with a liquid crystal display device fabricated by using the liquid crystal composition recited in any one of the aspects (14) to (23) above.

Compounds of the present invention expressed by the general formula (1) are high in voltage holding ratio and small in elastic constant ratio. The compounds are excellent in the miscibility with other liquid crystalline compounds, particularly in the miscibility at low temperatures when they are added to liquid crystal compositions.

Further, as an embodiment of the compounds of the present invention, the compounds in which an alkyl group is selected as substituent R₁, a group other than CN group is selected as X, and a group other than ester group is selected as bonding group (Z), respectively, exhibit a remarkably high voltage holding ratio and show preferable characteristics for liquid crystal materials used for TFT mode display, particularly for driving at a low voltage. Also, the compounds in which an alkenyl group is selected as substituent R₁, or CN group is selected as X exhibit a high dielectric anisotropy value and are very useful as liquid crystal materials driven at a low voltage for STN display devices.

Still further, while the compounds of the present invention expressed by the general formula (1) exhibit about the same or higher voltage holding ratio compared with that of the known compounds of the formula (14), (15-1), (15-2), (16), (17), or (18) shown in the section of BACKGROUND ART, the compounds of the present invention have such an excellent chemical stability that they are not deteriorated even under such severe conditions as exposure to heat and ultraviolet radiation, and possess extremely superior characteristics as liquid crystalline compound for producing liquid crystal compositions of which primary importance is placed on reliability.

By the use of the compounds of the present invention, it is possible to provide novel liquid crystalline compositions which are excellent in miscibility, particularly in miscibility at low temperatures, with which driving at a low voltage and a high speed response are possible in various display modes by proper selection of substituents, and which have a high reliability; and to provide liquid crystal display devices.

As will be clear from Examples and Comparative Examples, a remarkably excellent miscibility at low temperatures and a high voltage holding ratio of the compounds of the present invention are caused by the effect of bicyclo[1.1.1]pentane-1,3-diyl grop.

That is, when two to four ring compound (A) having 1,4-cyclohexylene group or 1,4-phenylene group as mother skeleton and compound (B) which is prepared by replacing oen of the 1,4-cyclohexylene group or 1,4-phenylene group in the compound (A) by bicyclo[1.1.1]pentane-1,3-diyl group were imagined, and when the compound (B) was assumed to have been rotated with the position 1 and position 3 of the bicyclo[1.1.1]pentane-1,3-diyl group being its axis, it can readily be presumed that the compound (B) becomes more uneven in molecular shape compared with the case in which the compound (A) was rotated with the position 1 and position 4 of 1,4-cyclohexylene group or 1,4-phenylene group being its axis since the compound (B) has a bulky structure and thus is large in rotational radius of the molecule. It is considered that such a change in molecular shape subtly affect to the interaction between or orientation of the molecules in liquid crystal compositions, and that its effect contributes to the improvement in miscibility, particularly in miscibility at low temperatures.

Further, by the report from B. W. Van der Meer et al. (Mol. Phys., 45, 1227 (1982)) or F. Leenhouts et al. (Phys. Lett., 72A, 155 (1979)), it is well known that elastic constant ratio (K33/K11) is proportional to the ratio of molecular length ℓ, to molecular width w, that is ℓ/w, and it is considered to be based on this ℓ/w that the compounds of the present invention characterized by having bicyclo[1.1.1]pentane-1,3-diyl group in their skeleton exhibit a small elastic constant ratio.

As described in the literatures such as J. Nehring "Advance in Liquid Crystal Research and Application", L. Bata(ed.), Pergamon Press, Budapest, Oxford and New York (1980), p. 1155, G. Baur "The Physics and Chemistry of Liquid Crystal Devices", G. J. Sprokel(ed.), Plenum, New York and London (1980), p. 61, and Y. Takahashi, T. Uchida, and M. Wada, Mol. Crystal. Liq. Cryst., 66, 171 (1981), it is known that generally in display devices of TN (simple matrix, active matrix) mode, compounds having a small K33/K11 are excellent in the steepness of V-T curve, and it is understood that the compounds of the present invention have an excellent characteristic even on this point.

While any of the compounds of the present invention exhibits preferable physical properties, liquid crystal compositions suitable for the purpose can be produced by using the compounds expressed by the general formula (1) in which R₁, A₀, A₁, A₂, A₃, Z₀, Z₁, Z₂, Z₃, X, ℓ, m, n, and o are properly selected.

That is, when the compounds are used for the production of liquid crystal compositions of which temperature range of liquid crystal phase must be at high temperature side, it is sufficient that five ring compounds in which $\text{ℓ + m + n + o = 4}$ are selected. On the other hand, when the temperature range of liquid crystal phase is not necessary to be at high temperature region, it is sufficient to use two or three ring compounds.

When a high voltage holding ratio for liquid crystal compositions used, for example, for active matrix is necessary, the purpose can be achieved by using compounds in which an alkyl group or fluoroalkyl group is selected as side chain R₁, a group other than ester group is selected for Z₀, Z₁, Z₂, and Z₃, and a group other than CN group is selected for substituent X.

Further, in the case of liquid crystal materials of which a negative dielectric anisotropy is required and represented by the IPS mode or VA mode described in the section of BACKGROUND ART, compounds having a negative and large dielectric anisotropy are obtained by selecting one or more 2,3-difluoro-1,4-phenylene groups for skeletal portions other than bicyclopentane ring.

In the bicyclo[1.1.1]pentane derivatives of the present invention expressed by the general formula (1), R₁ represents an alkyl group having 1 to 10 carbon atoms in which group not-adjacent any methylene group may be replaced by oxygen atom, sulfur atom, -CH=CH-, -C≡C-, -CO-, -COO-, -OCO-, or -SiH₂-, and any hydrogen atom in this group may be replaced by a halogen atom.

In the above, R₁ specifically represents an alkyl group, alkoxy group, alkoxyalkyl group, alkenyl group, alkynyl group, alkenyloxy group, alkynyloxy group, halogen substituted alkyl group, and halogen substituted alkenyl group.

More specifically, methyl group, ethyl group, propyl group, butyl group, pentyl group, hexyl group, heptyl group, octyl group, nonyl group, decyl group, methoxy group, ethoxy group, propoxy group, butoxy group, pentoxy group, hexyloxy group, nonyloxy group, decyloxy group, methoxymethyl group, ethoxymethyl group, propoxymethyl group, butoxymethyl group, methoxyethyl group, methoxypropyl group, methoxybutyl group, ethoxyethyl group, ethoxypropyl group, propoxyethyl group, propoxypropyl group, vinyl group, 1-propenyl group, 1-butenyl group, 1-pentenyl group, 3-butenyl group, 3-pentenyl group, allyloxy group, ethynyl group, 1-propynyl group, 1-butynyl group, 1-pentynyl group, 3-butynyl group, 3-pentynyl group, trifluoromethyl group, difluoromethyl group, difluorochloromethyl group, 2,2,2-trifluoroethyl group, 2,2-difluoroethyl group, 2-fluoroethyl group, 3-fluoropropyl group, 4-fluorobutyl group, 5-fluoropentyl group, 3-chloropropyl group, trifluoromethoxy group, difluoromethoxy group, 2,2,2-trifluoroethoxy group, 2,2-difluoroethoxy group, difluorochloromethoxy group, trifluoromethoxymethyl group, 2-fluoroethenyl group, 2,2-difluoroethenyl group, 1,2,2-trifluoroethenyl group, 3-fluoro-1-butenyl group, 4-fluoro-1-butenyl group, and 3,3,3-trifluoro-1-propynyl group are preferable.

In the general formula (1), X represents a halogen atom, cyano group, or an alkyl group having 1 to 10 carbon atoms in which alkyl group not-adjacent any methylene group may be replaced by oxygen atom, sulfur atom, -CH=CH-, -C≡C-, -CO-, -COO-, -OCO-, or -SiH₂-, and any hydrogen atom in this group may be replaced by a halogen atom.

Specifically, X represents fluorine atom, chlorine atom, bromine atom, cyano group, an alkyl group, alkoxy group, alkoxyalkyl group, alkenyl group, alkynyl group, alkenyloxy group, alkynyloxy group, halogen substituted alkyl group, halogen substituted alkoxy group, halogen substituted alkoxyalkyl group, halogen substituted alkenyl group, or halogen substituted alkynyl group.

As the substituting group described above after the alkyl group, specifically the same substituting groups as those in the R₁ described above are preferable.

In the first aspect of the present invention, preferable embodiments of bicyclo[1.1.1]pentane derivatives expressed by the general formula (1) are compounds expressed by one of the following general formulas (20-1) to (20-17). 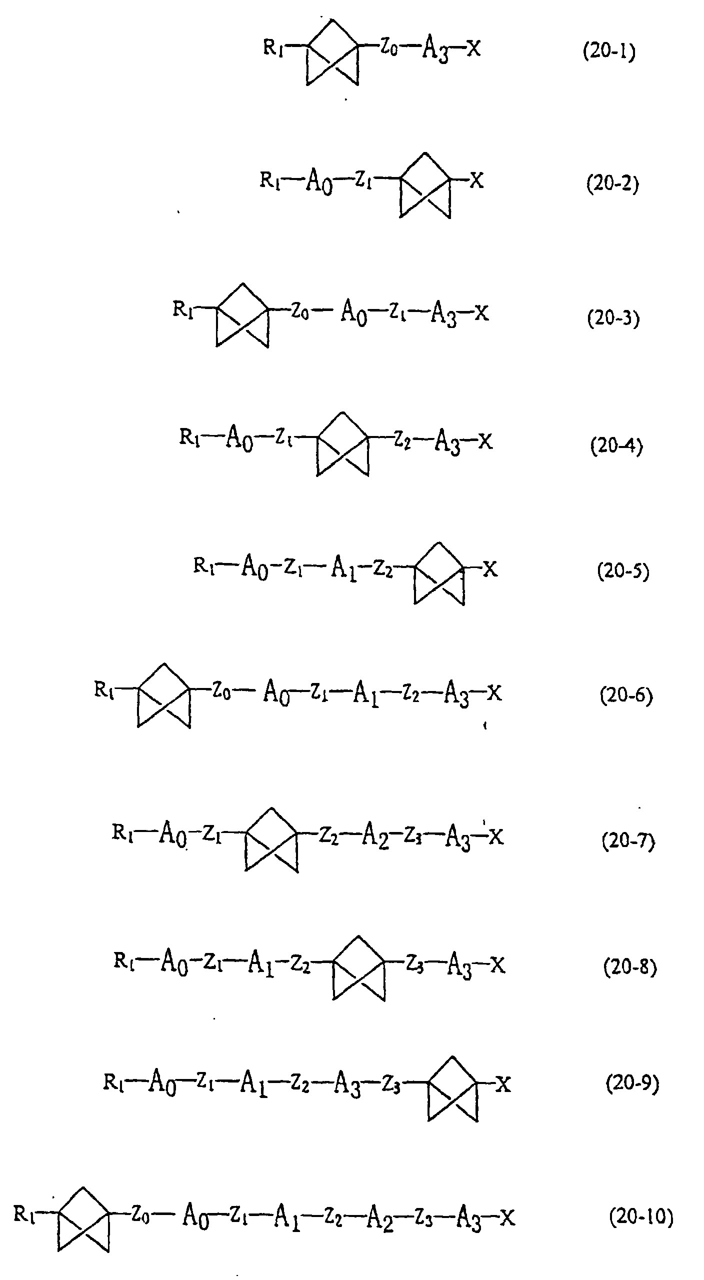 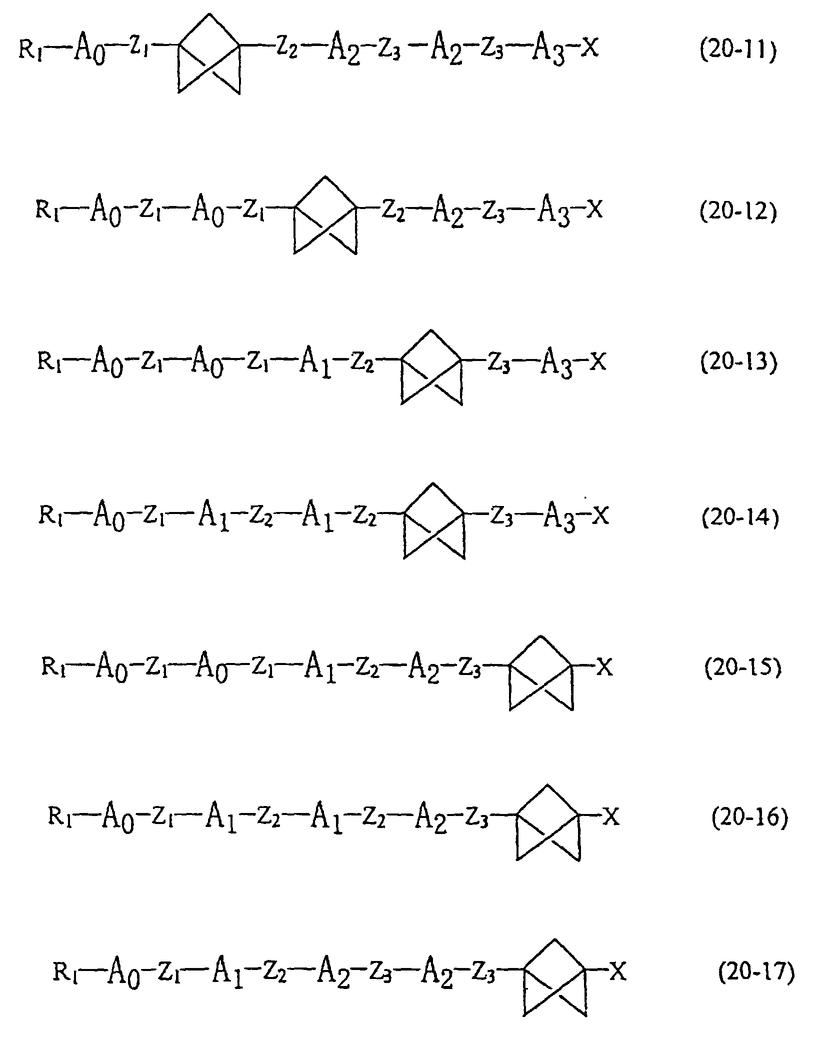 wherein R₁, A₀, A₁, A₂, A₃, Z₀, Z₁, Z₂, Z₃, and X have the same meaning as described above.

When added as component of liquid crystal compositions, two ring compounds expressed by the general formula (20-1) or (20-2) can stabilize miscibility at low temperatures, that is, nematic phase of compositions at low temperatures and can efficiently lower elastic constant ratio (K33/K11) while maintaining viscosity, and are able to improve the steepness of "voltage-transmittance characteristic" of liquid crystal compositions principally for TN or TFT.

When added as component of liquid crystal compositions, three ring, four ring, or five ring compounds expressed by one of the general formulas (20-3) to (20-17) can widen the range of a liquid crystal phase while maintaining miscibility at low temperatures, and are able to increase the voltage holding ratio particularly at a high temperature (100°C).

In order to develop excellent characteristics, it is preferable that the liquid crystal compositions of the present invention comprise at least one compound expressed by the general formula (1) in an amount of 0.1 to 99.9 % by weight, desirably 1 to 50 % by weight, and more desirably 3 to 20 % by weight.

Liquid crystal compositions of the present invention are completed by mixing compounds selected from the group consisting of the compounds expressed by one of the general formulas (2) to (9) depending on the purpose of liquid crystal compositions with the first component comprising at least one compound expressed by the general formula (1).

As preferable examples of the compounds expressed by one of the general formulas (2) to (4), the compounds of the following formulas can be mentioned. 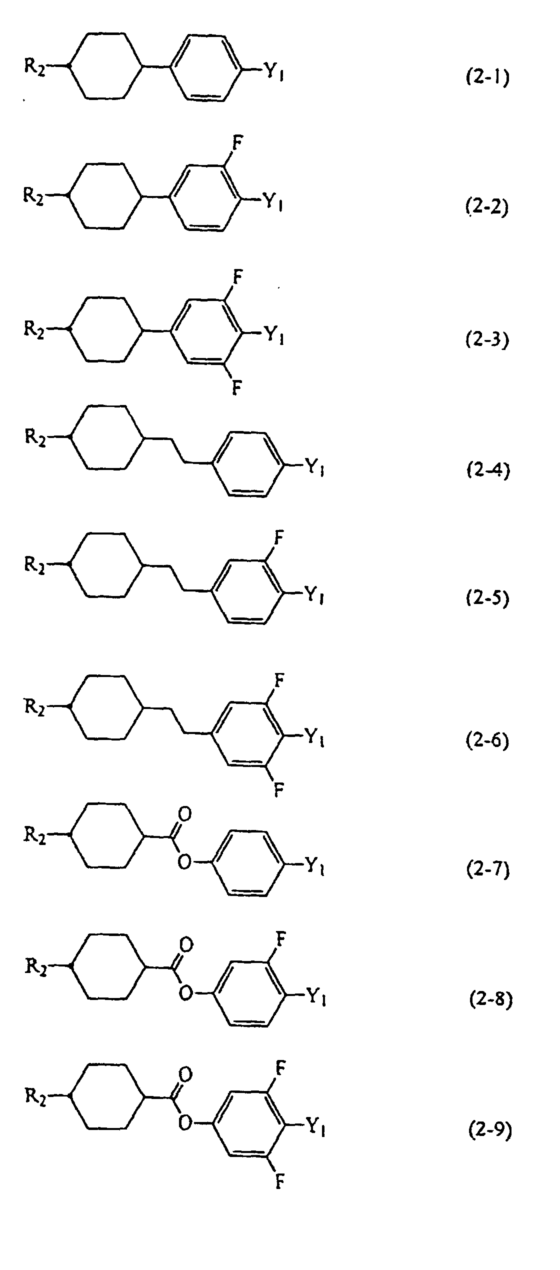 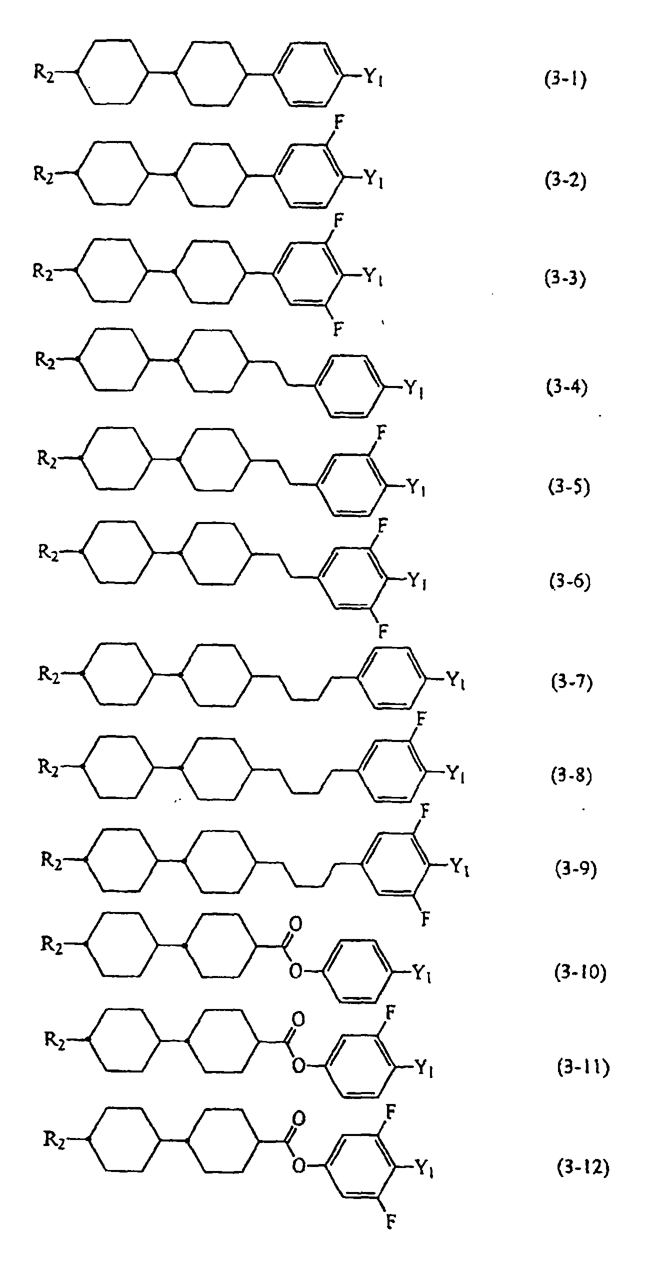 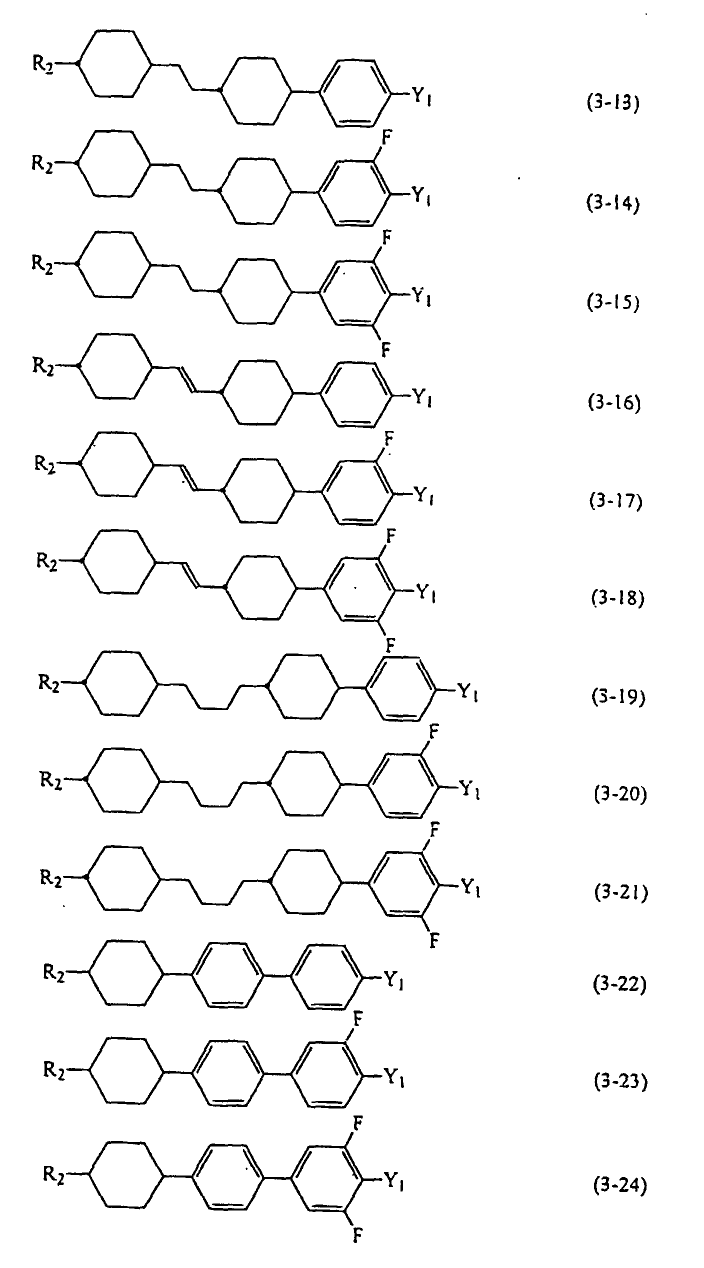 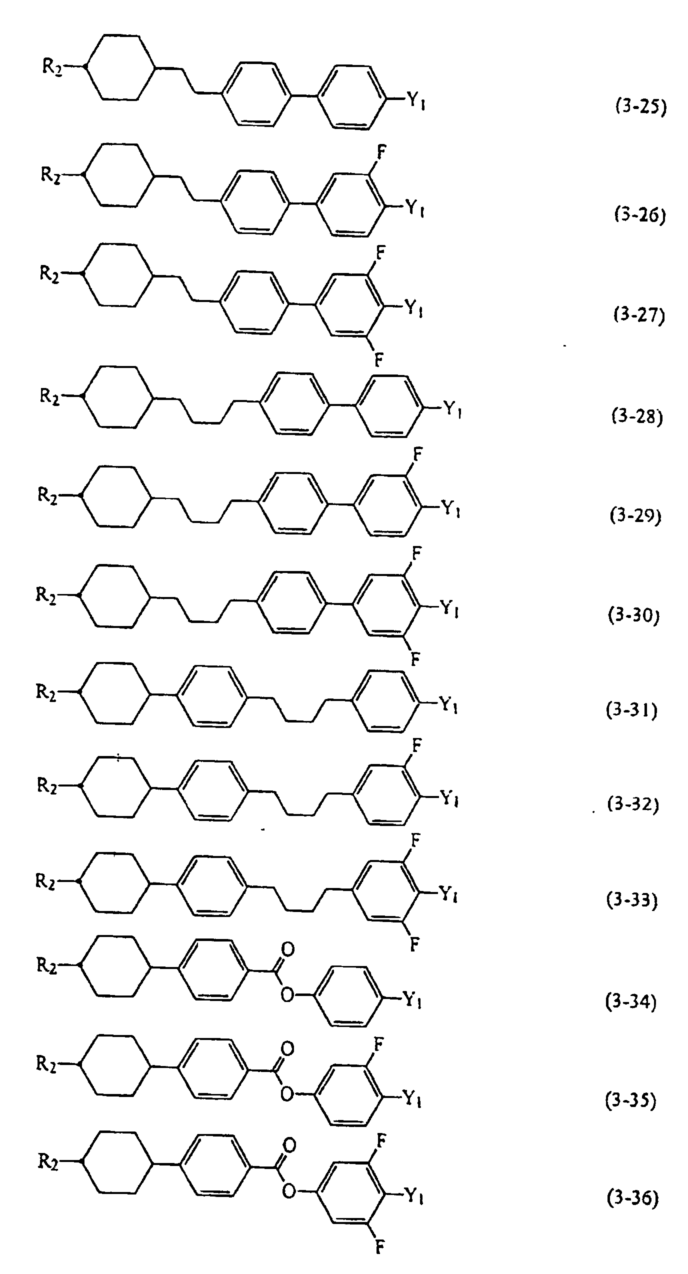 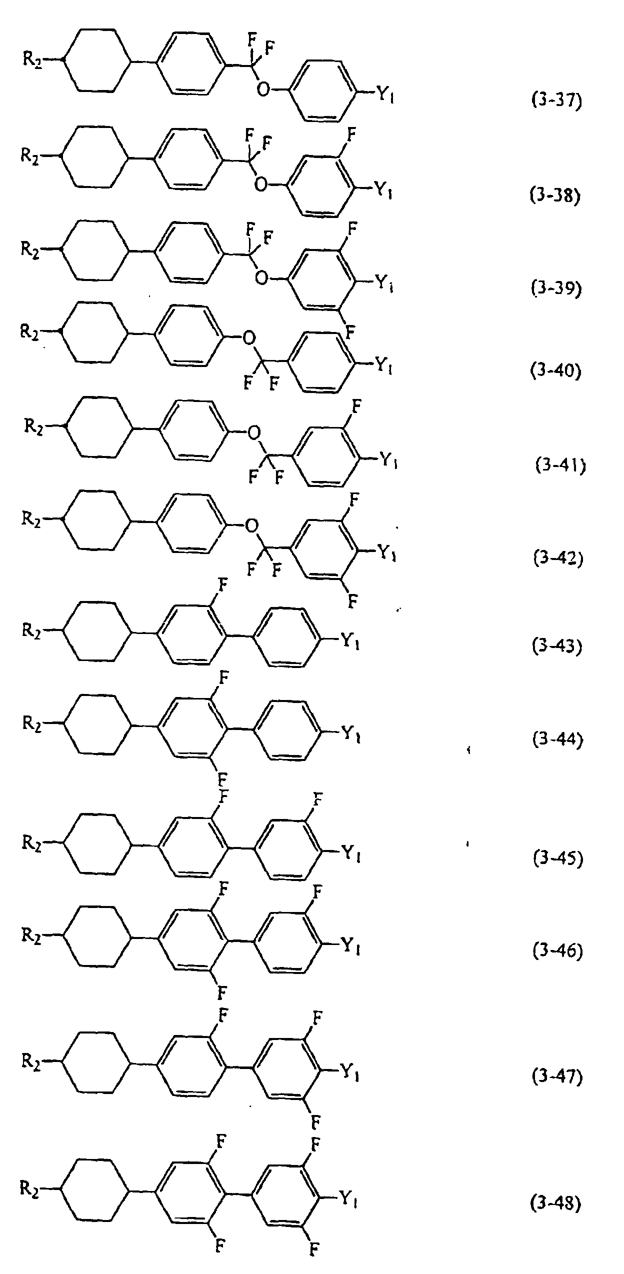 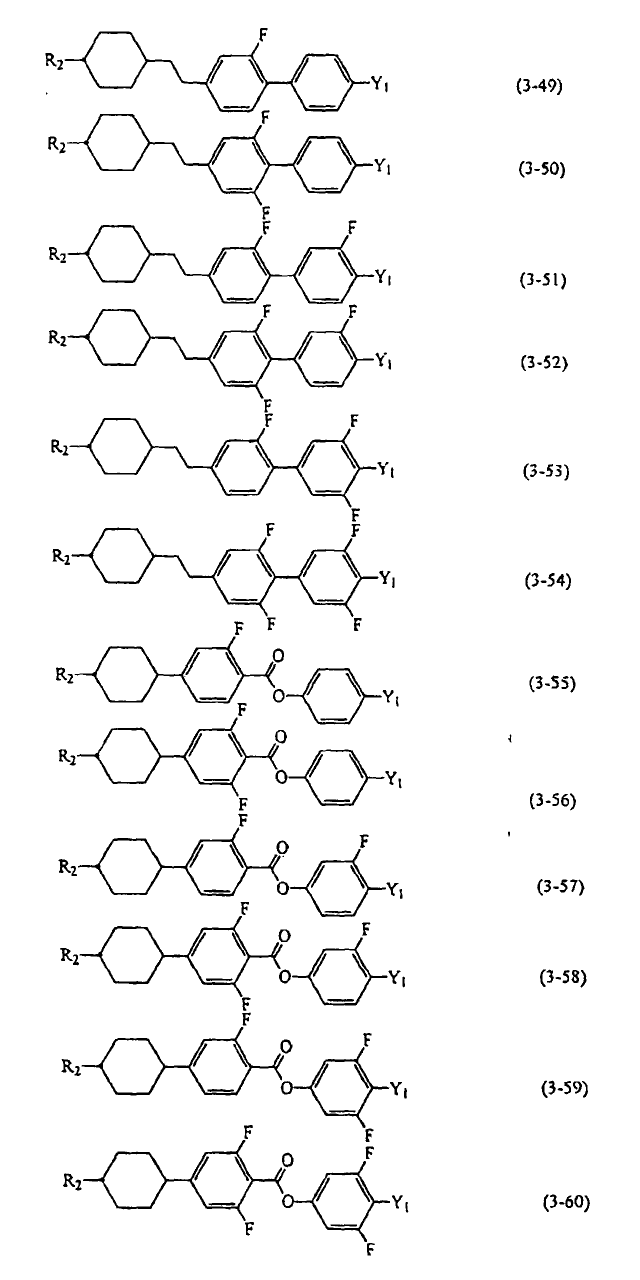 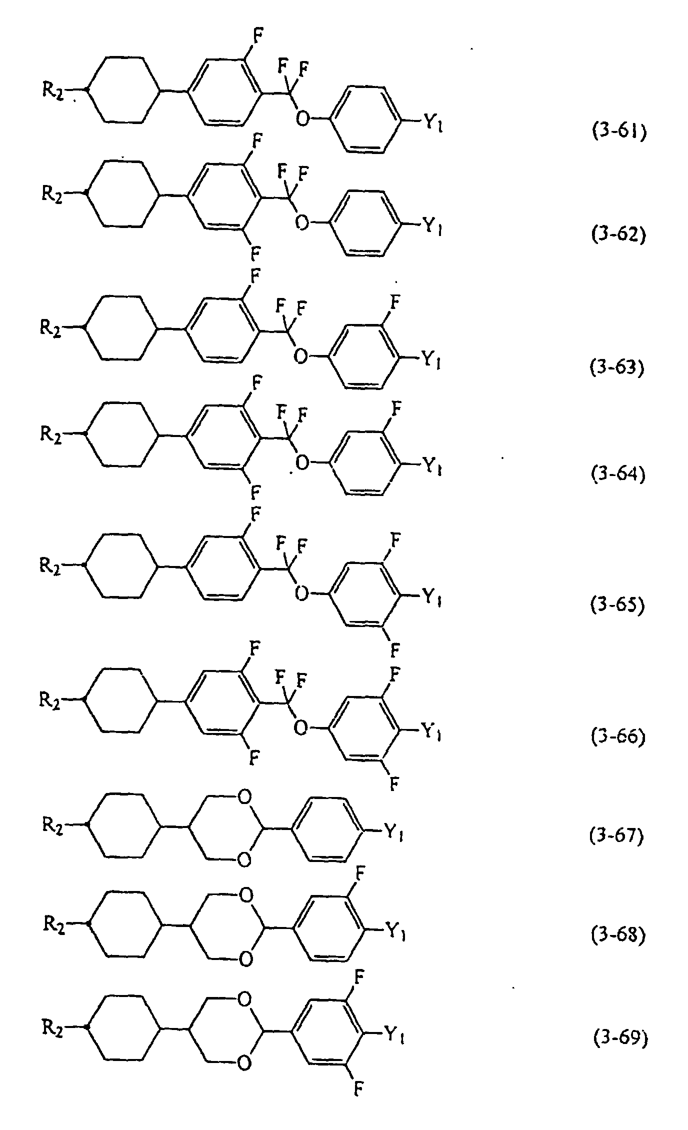 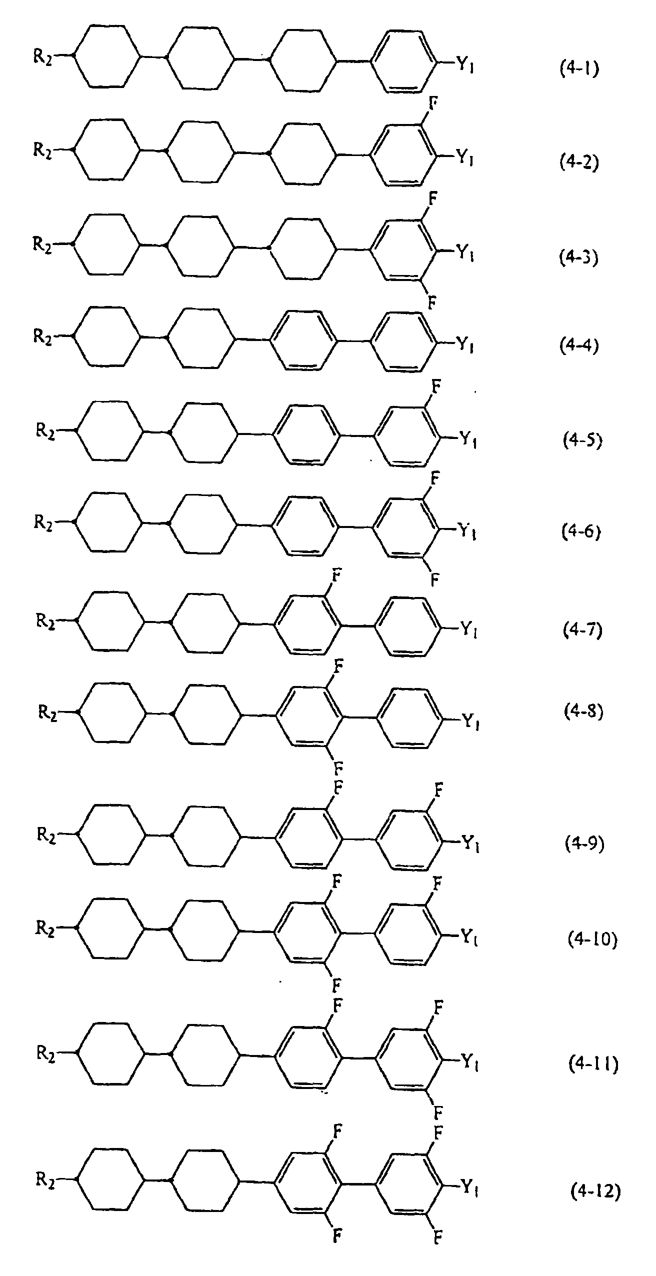 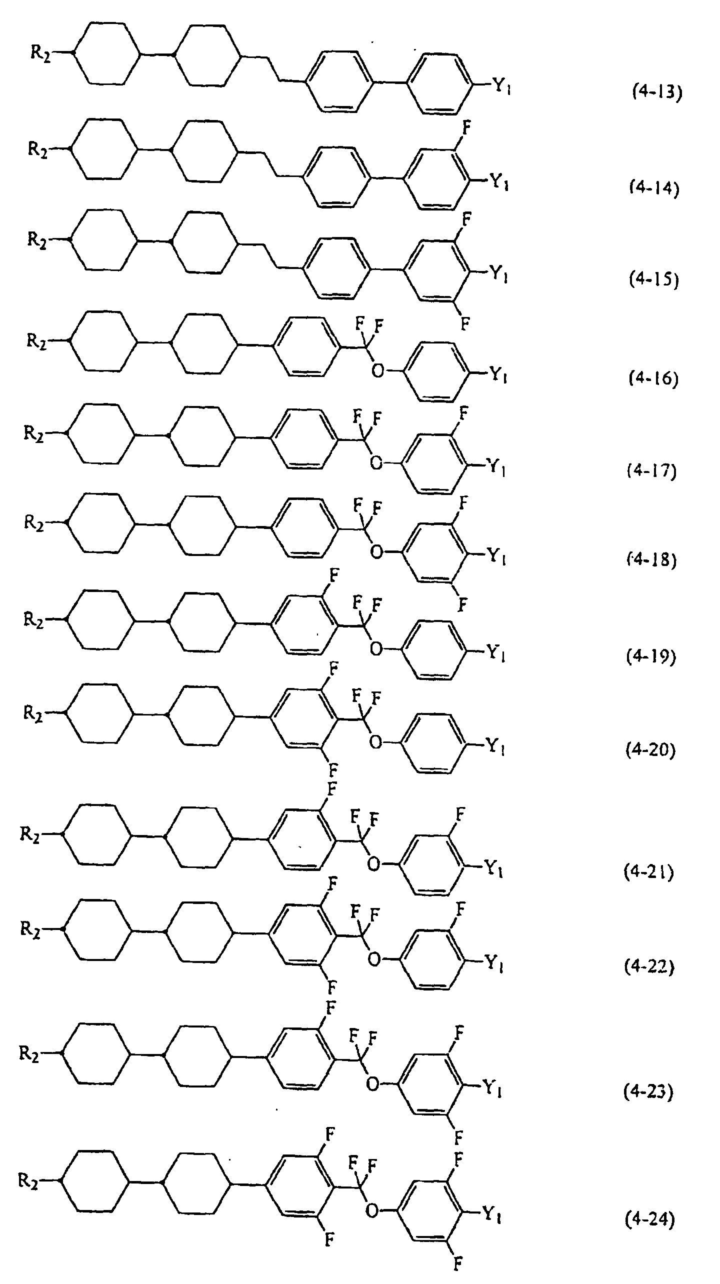 wherein R₂ and Y₁ have the same meaning as described above.

Compounds expressed by one of the general formulas (2) to (4) have a positive dielectric anisotropy value, are excellent in thermal stability and chemical stability, are high in voltage holding ratio (large in specific resistance), and are useful when liquid crystal compositions for TFT (AM-LCD) of which a high reliability is required are produced.

When liquid crystal compositions for TFT are produced, while the compounds expressed by one of the general formulas (2) to (4) can be used in the range of 1 to 99 % by weight based on the total amount of liquid crystal composition, the amount is preferably 10 to 97 % by weight and more desirably 40 to 95 % by weight. Besides, compounds expressed by one of the general formulas (7) to (9) may be blended at that time.

Also, when liquid crystal compositions for STN display mode or TN display mode are produced, compounds expressed by one of the general formulas (2) to (4) can be used. In that case, the amount of the compounds used is preferably 50 % by weight or less since the compounds are small in the effect of lowering the threshold voltage of liquid crystal compositions compared with the compounds of the general formula (5) or (6).

As preferable examples of the compounds expressed by the general formula (5) or (6), the compounds of the following formulas can be mentioned. 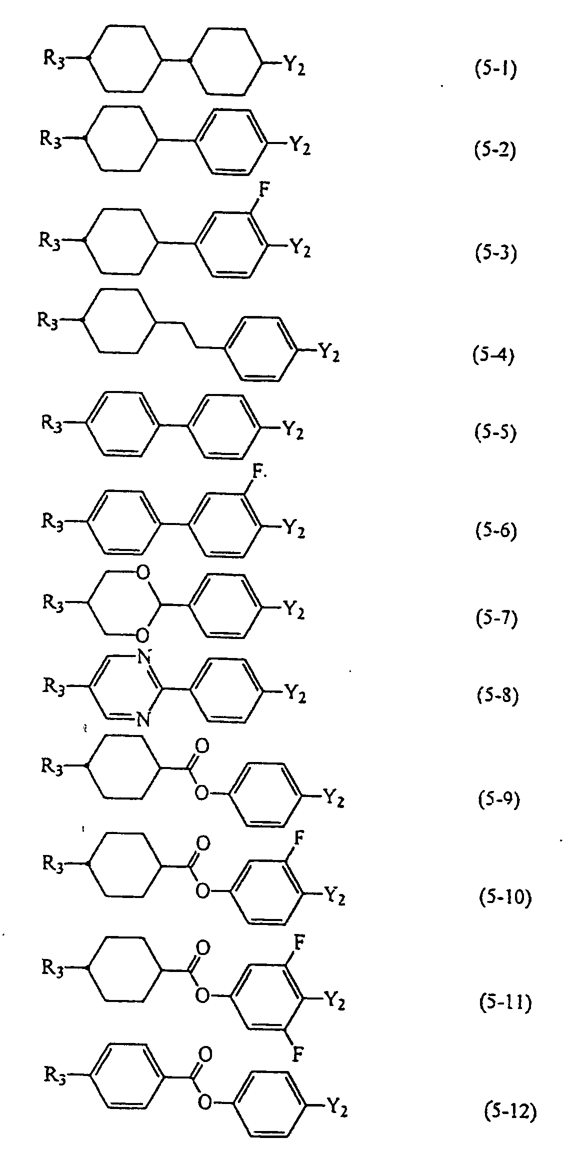 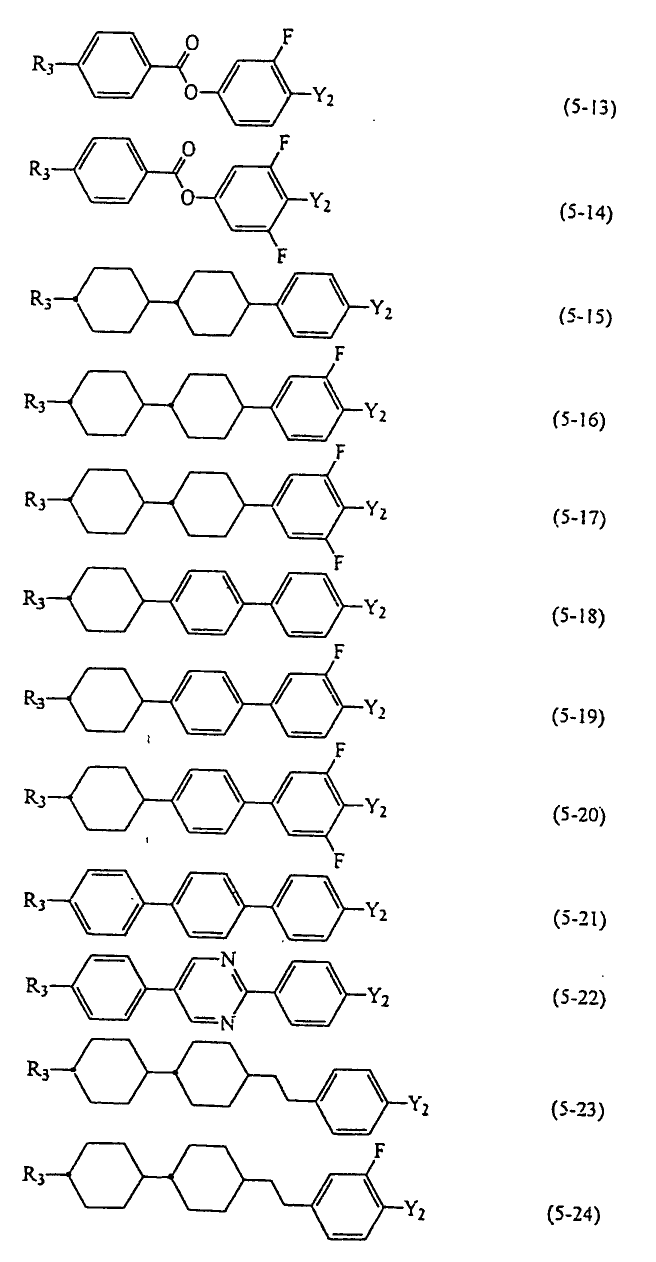 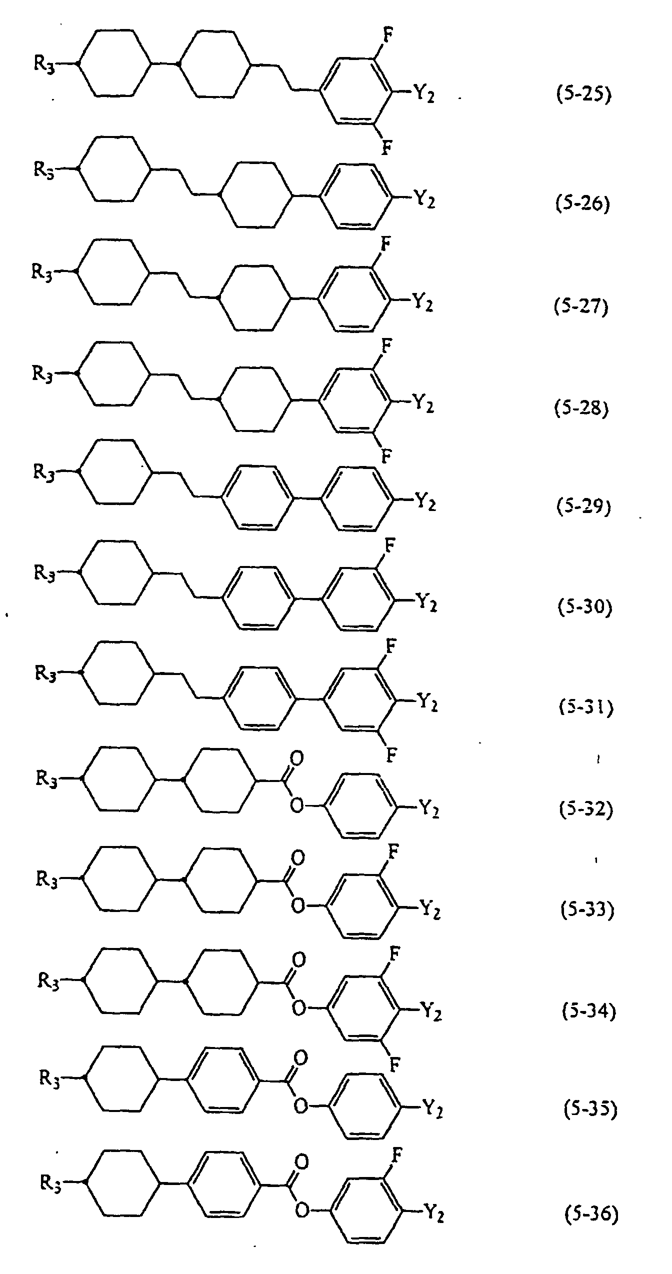 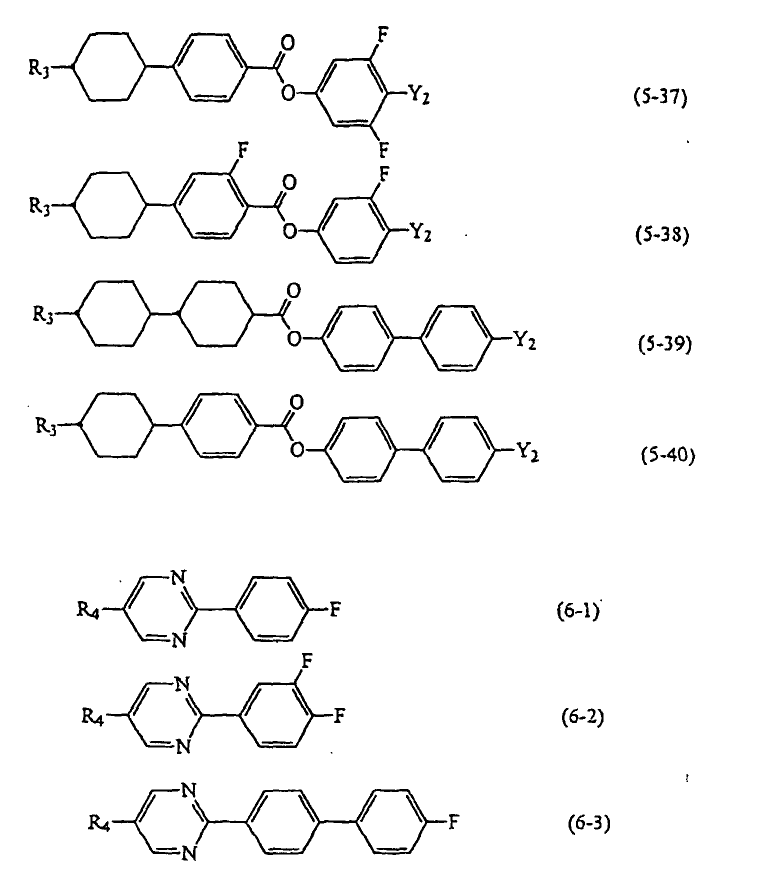 wherein R₃, R₄, and Y₂ have the same meaning as described above.

Compounds expressed by the general formula (5) or (6) have a positive large dielectric anisotropy value, are used particularly for the purpose of lowering the threshold voltage of liquid crystal compositions, and are also used for the purpose of adjusting optical anisotropy value and widening nematic range such as raising clearing point. Further, they are used even for the purpose of improving the steepness of "voltage-transmittance characteristic" of liquid crystal compositions for STN display mode or TN display mode.

Compounds expressed by the general formula (5) or (6) are especially useful when liquid crystal compositions for STN display mode or TN display mode are produced.

When the amount of the compounds expressed by the general formula (5) or (6) used in liquid crystal compositions is increased, the threshold voltage of liquid crystal compositions lowers and viscosity rises. Accordingly, it is advantageous to use them in a large quantity since driving at a low voltage is possible, so far as the viscosity of liquid crystal compositions to be obtained satisfies required characteristics.

While the compounds expressed by the general formula (5) or (6) can be used in any amount in the range of 0.1 to 99.9 % by weight, the amount is preferably 10 to 97 % by weight and more desirably 40 to 95 % by weight when liquid crystal compositions for STN display mode or TN display mode are produced.

As preferable examples of the compounds expressed by one of the general formulas (7) to (9), the compounds of the following formulas can be mentioned. 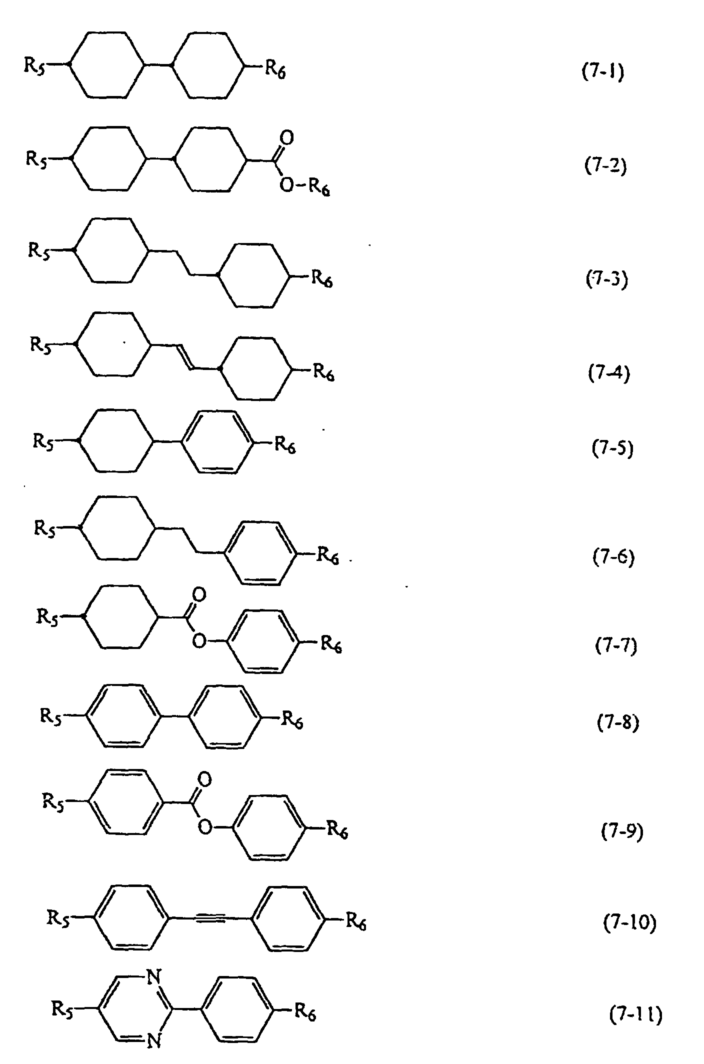 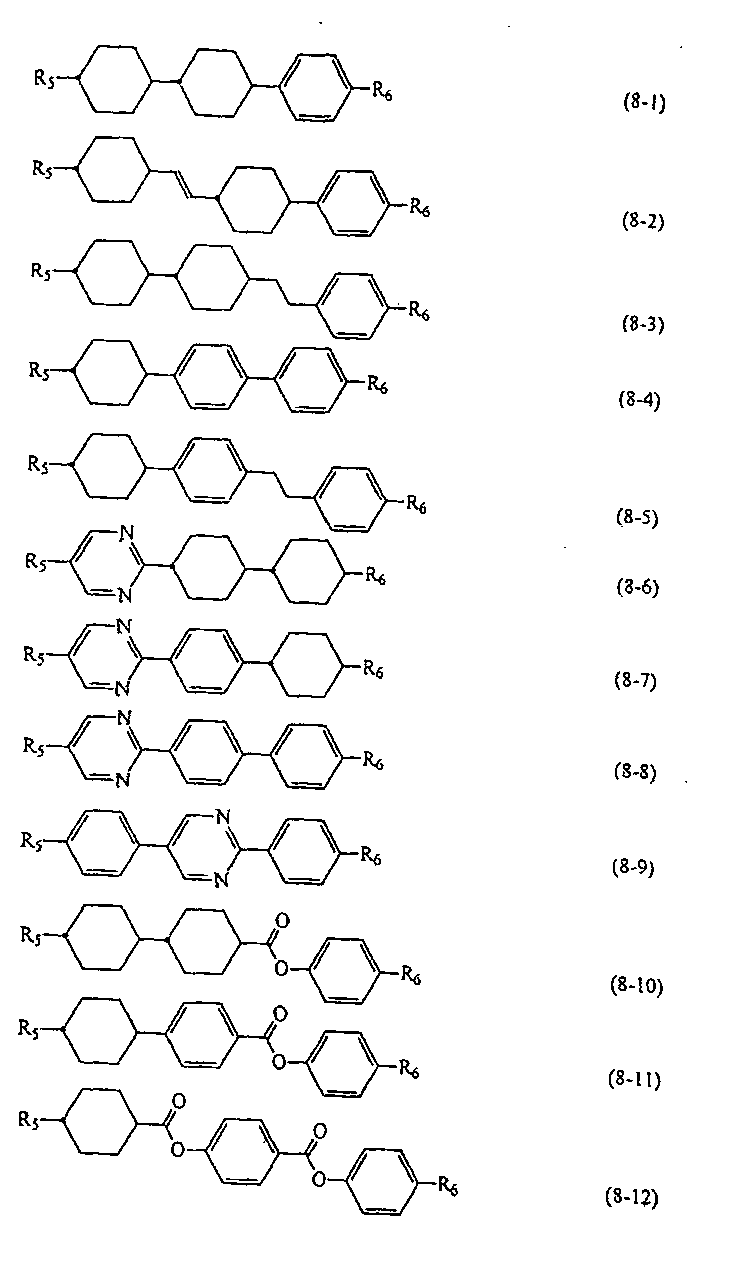 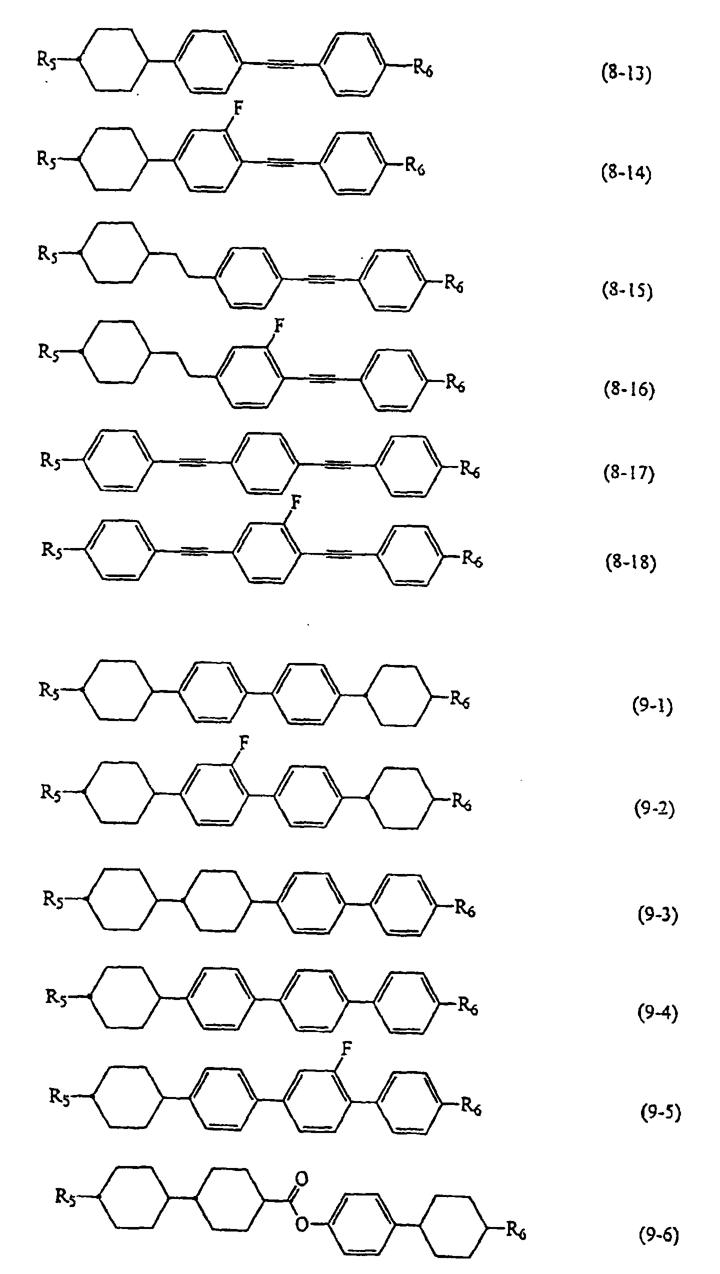 wherein R₅ and R₆ have the same meaning as described above.

Compounds expressed by one of the general formulas (7) to (9) have a small absolute value of dielectric anisotropy and the value is close to 0. Compounds expressed by the general formula (7) are used principally for the purpose of adjusting viscosity or adjusting optical anisotropy value. Compounds expressed by the general formula (8) or (9) are used for the purpose of widening nematic range such as raising clearing point or for the purpose of adjusting optical anisotropy value.

When the amount of the compounds expressed by one of the general formulas (7) to (9) used in liquid crystal compositions is increased, the threshold voltage of liquid crystal compositions rises and their viscosity lowers. Accordingly, it is desirable to use the compounds in a large quantity so far as the threshold voltage of liquid crystal compositions satisfies required values. The amount of the compounds expressed by one of the general formulas (7) to (9) to be used is preferably 40 % by weight or less and more desirably 35 % by weight or less when liquid crystal compositions for TFT are produced. When the liquid crystal compositions for STN display mode or TN display mode are produced, the amount is preferably 70 % by weight or less and more desirably 60 % by weight or less.

As preferable examples of the compounds expressed by one of the general formulas (10) to (12), the compounds of the following formulas can be mentioned. 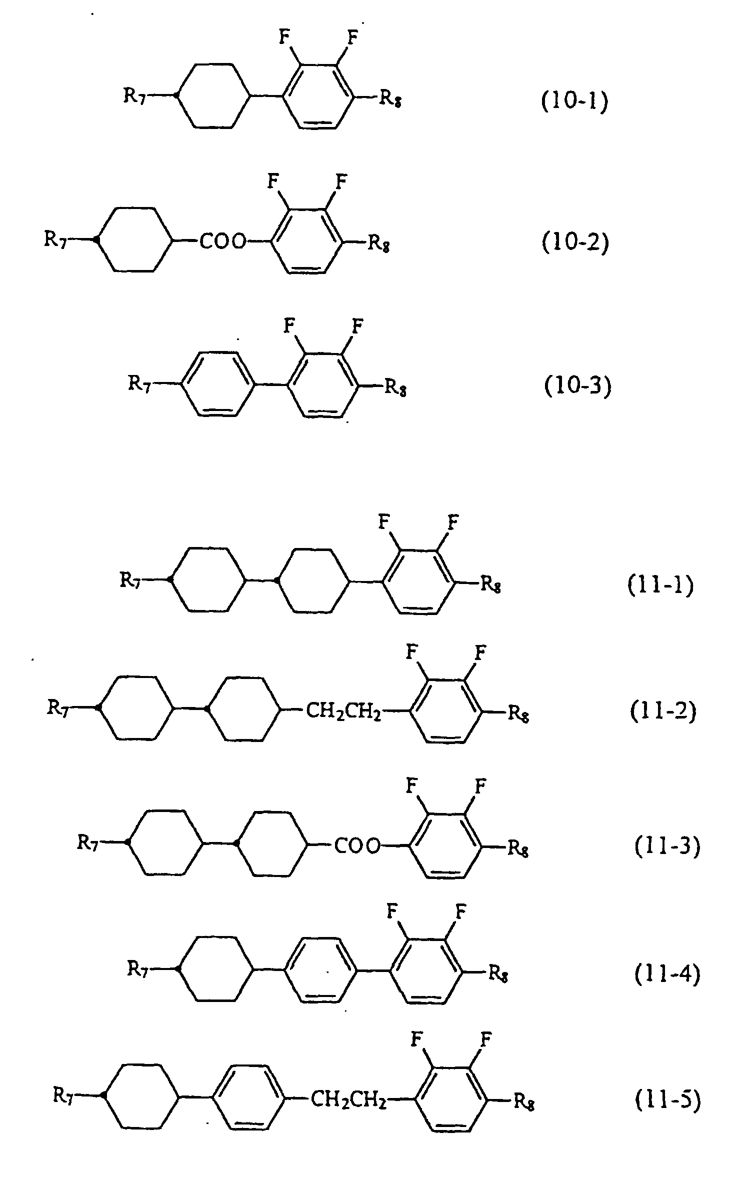 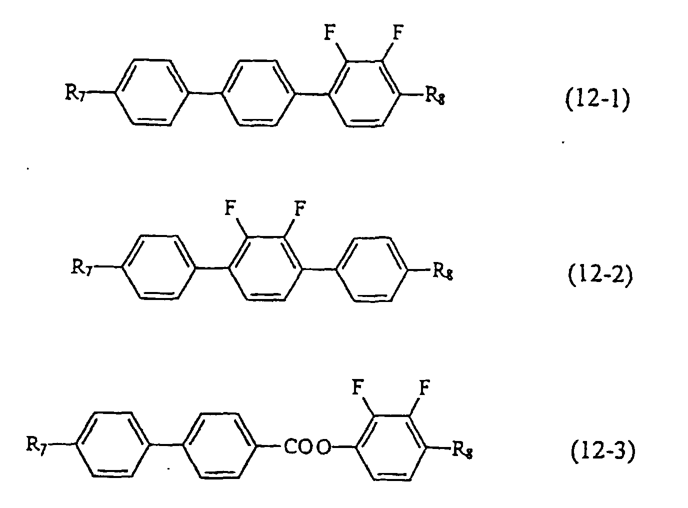 wherein R₇ and R₈ have the same meaning as described above.

Compounds expressed by one of the general formulas (10) to (12) have a negative dielectric anisotropy value. Since the compounds expressed by the general formula (7) are two ring compounds, they are used principally for the purpose of adjusting threshold voltage, adjusting viscosity, or adjusting optical anisotropy value. Compounds expressed by the general formula (11) are used for the purpose of widening nematic range such as raising clearing point or for the purpose of adjusting optical anisotropy value. Compounds expressed by the general formula (12) are used for the purpose of lowering threshold voltage and for the purpose of increasing optical anisotropy value in addition to the purpose of widening nematic range.

Compounds expressed by one of the general formulas (10) to (12) are used principally in N type (dielectric anisotropy Δε is negative) compositions, and when the amount used is increased, the threshold voltage of liquid crystal compositions lowers and their viscosity rises. Accordingly, it is desirable to use the compounds in a small quantity so far as the threshold voltage of liquid crystal compositions satisfies required value. However, since the absolute value of dielectric anisotropy of these compounds is 5 or less, when the amount is 40 % by weight or less, it sometimes becomes impossible to drive by a voltage. While the amount of the compounds expressed by one of the general formulas (10) to (12) to be used is preferably 40 % by weight or more, the amount is more desirably 50 to 95 % by weight when liquid crystal compositions for N type TFT are produced.

Compounds of one of the general formulas (10) to (12) are sometimes mixed with P type (dielectric anisotropy Δε is positive) compounds for the purpose of controlling elastic constants and adjusting the voltage-transmittance curve (V-T curve) of liquid crystal compositions. In this case, the amount of the compounds expressed by one of the general formulas (10) to (12) used is preferably 30 % by weight or less.

Further, with the exception of such specific cases as liquid crystal compositions for OCB (Optically Compensated Birefringence) mode, an optically active compound is added to the liquid crystal compositions of the present invention generally for the purpose of inducing helical structure of liquid crystal composition to adjust required twist angle and to prevent reverse twist. As optically active compounds used for such purposes, while any known optically active compounds can be employed, the optically active compounds of the following formulas can be mentioned as preferable examples.

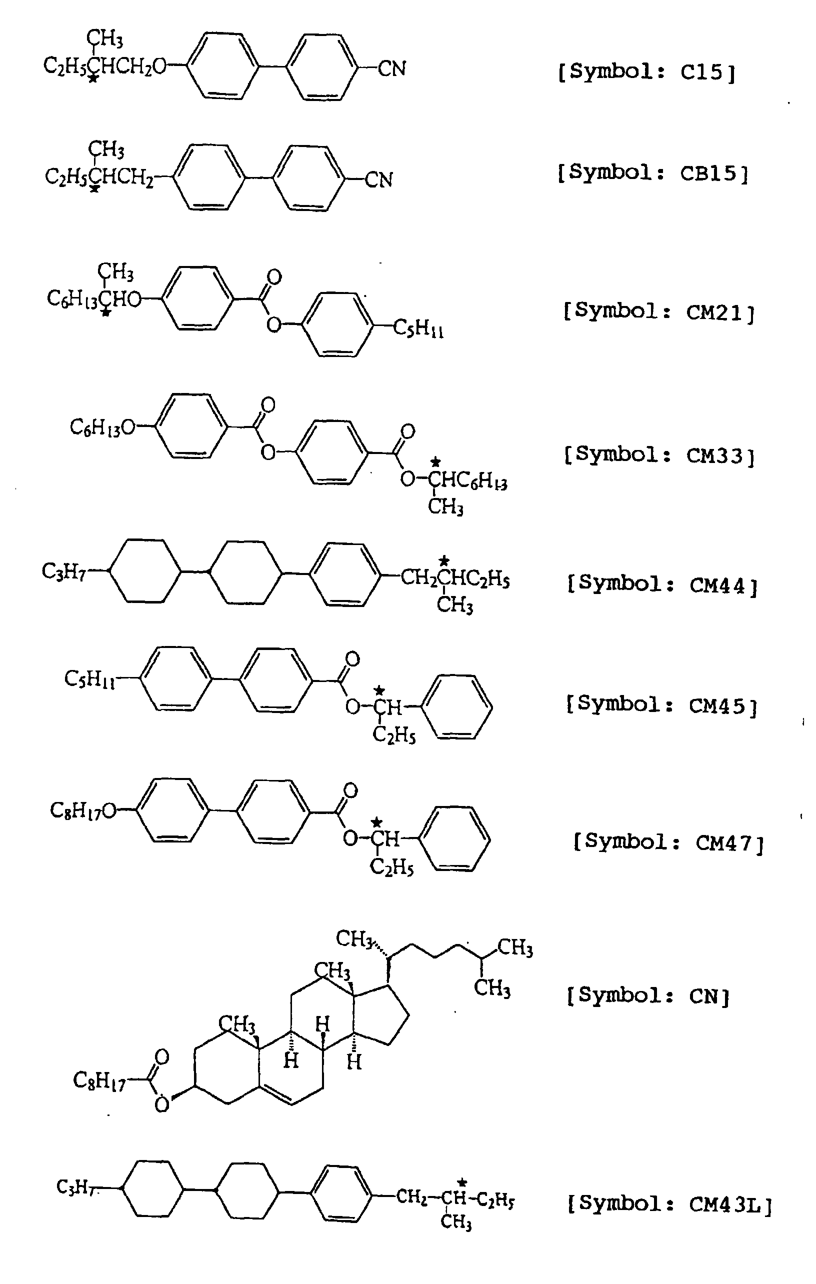

Usually, these optically active compounds are added to the liquid crystal compositions of the present invention to adjust the pitch of the twist. Pitch of the twist is preferably adjusted in the range of 40 to 200 µm in the case of liquid crystal compositions for TFT or TN, and preferably adjusted in the range of 6 to 20 µm in the case of liquid crystal compositions for STN. In the case of liquid crystal compositions for bistable TN mode, it is preferably adjusted in the range of 1.5 to 4 µm. Besides, two or more kind of optically active compounds may be added for the purpose of adjusting the dependency of the pitch on temperature.

Liquid crystal compositions of the present invention can be produced by conventional methods. Generally, a method in which various components are dissolved in one another at a high temperature is adopted.

Further, the liquid crystal compositions of the present invention can be used as ones for guest-host (GH) mode by adding a dichroic dye such as merocyanine type, styryl type, azo type, azomethine type, azoxy type, quinophthalone type, anthraquinone type, and tetrazine type thereto. Alternatively, the liquid crystal compositions can be used as NCAP which is prepared by the microencapsulation of a nematic liquid crystal, or as liquid crystal compositions for polymer dispersed liquid crystal display devices (PDLCD) represented by polymer net work liquid crystal display devices (PNLCD) prepared by forming a polymer of three-dimensional reticulated structure in a liquid crystal. Still further, the liquid crystal compositions of the present invention can be used as ones for electrically controlled birefringence (ECB) mode or dynamic scattering (DS) mode.

As nematic liquid crystal compositions comprising the compound of the present invention, the following Use Examples (composition examples 1 to 27) can be mentioned. Compounds in the following Use Examples are designated by abbreviations according to the definitions shown in Table 1.

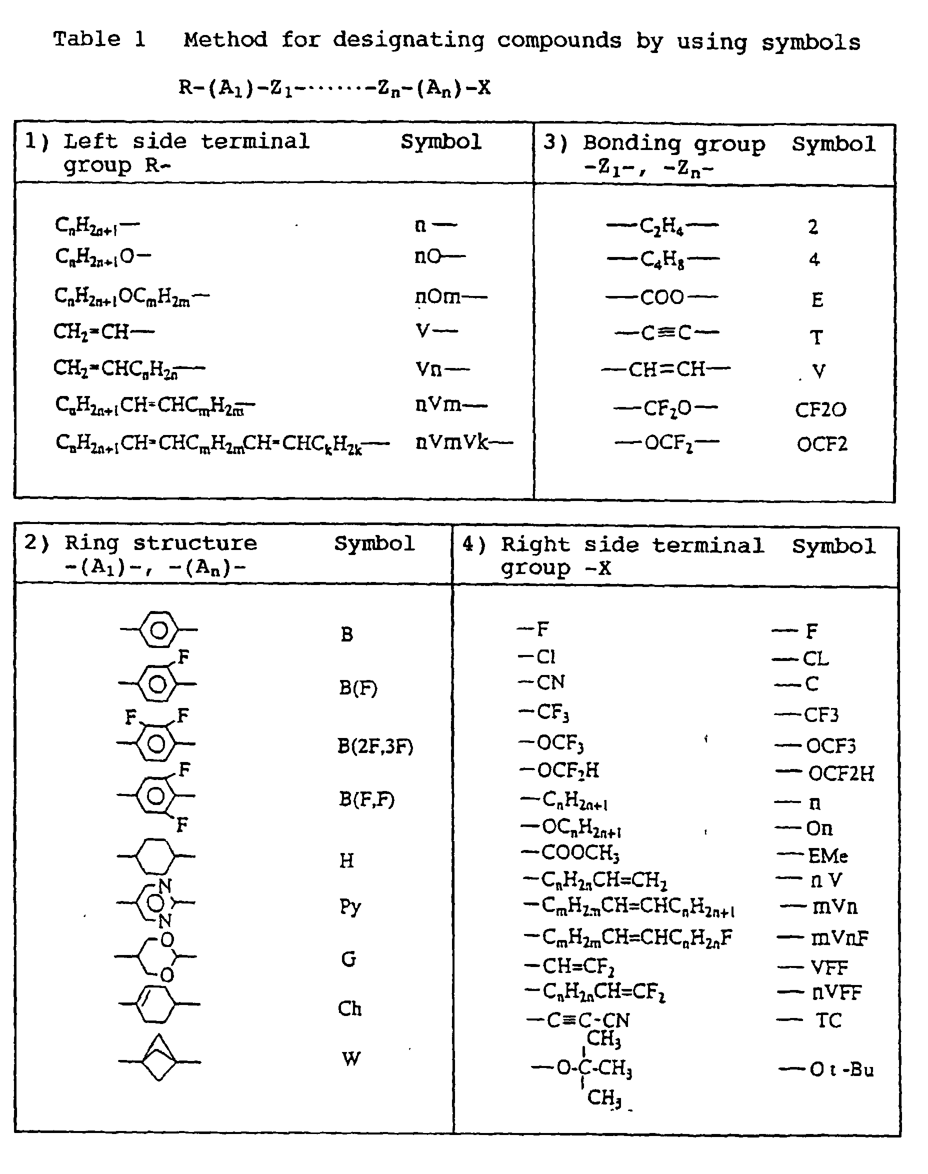

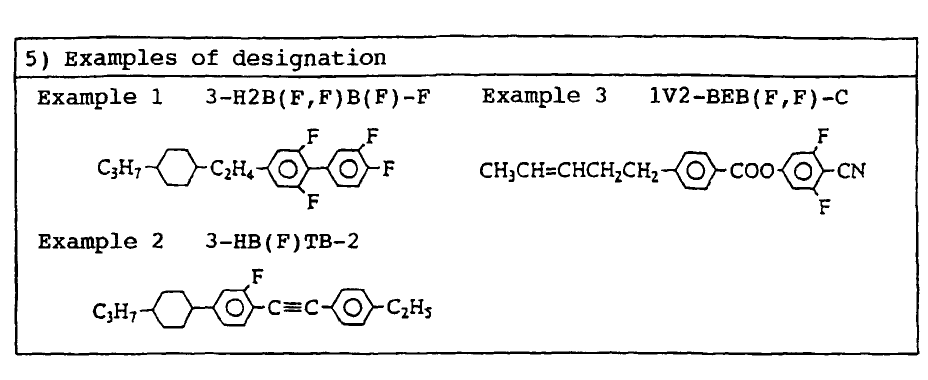

In each Use Example, TNI indicates phase transition temperature of nematic phase-isotropic liquid (°C), Δε: dielectric anisotropy value, Δn: optical anisotropy value, η: viscosity (mPa·s), and Vth: threshold voltage (V). In this connection, η was determined at 20°C, and Δε, Δn, Vth, and the twist pitch (P) (µm) indicate the values determined each at 25°C.

### Use Example 1

| | |
|---|---|
| V-WBB-2 | 4.0% |
| V1-WBB-2 | 4.0% |
| V2-HB-C | 12.0% |
| 1V2-HB-C | 12.0% |
| 3-HB-C | 15.0% |
| 3-H[1D,2D,3D]-C | 9.0% |
| 3-HB(F)-C | 5.0% |
| 2-BTB-1 | 2.0% |
| 3-HH-4 | 3.0% |
| 3-HH-VFF | 3.0% |
| 2-H[1D,2D,3D]HB-C | 3.0% |
| 3-HHB-C | 6.0% |
| 3-HB(F)TB-2 | 8.0% |
| 3-H2BTB-2 | 5.0% |
| 3-H2BTB-3 | 5.0% |
| 3-H2BTB-4 | 4.0% |

TNI= 88.1 (°C)
η = 20.1 (mPa·s)
Δn = 0.167
Δε = 8.7
Vth = 2.00 (V)

### Use Example 2

| | |
|---|---|
| 4-WBBW-4 | 9.0% |
| 1V2-BEB(F,F)-C | 5.0% |
| 3-HB-C | 25.0% |
| 3-HB-O2 | 3.0% |
| 1-BTB-3 | 5.0% |
| 2-BTB-1 | 7.0% |
| 3-HH-4 | 11.0% |
| 3-HHB-1 | 11.0% |
| 3-H2BTB-2 | 4.0% |
| 3-H2BTB-3 | 4.0% |
| 3-H2BTB-4 | 4.0% |
| 3-HB(F)TB-2 | 6.0% |
| 3-HB(F)TB-3 | 6.0% |

TNI= 90.1 (°C)
η = 17.8 (mPa·s)
Δn = 0.161
Δε = 7.0
Vth = 2.08 (V)

Pitch of the composition prepared by adding 0.8 part by weight of CM-33 to 100 parts by weight of the composition described above is shown below.$\text{P = 11.3 µm}$

### Use Example 3

| | |
|---|---|
| 4-WBEB(F,F)-C | 10.0% |
| 2O1-BEB(F)-C | 5.0% |
| 3O1-BEB(F)-C | 15.0% |
| 4O1-BEB(F)-C | 13.0% |
| 5O1-BEB(F)-C | 3.0% |
| 2-HHB(F)-C | 15.0% |
| 3-HHB(F)-C | 15.0% |
| 3-HB(F)TB-2 | 4.0% |
| 3-HB(F)TB-3 | 4.0% |
| 3-HB(F)TB-4 | 4.0% |
| 3-HHB-1 | 8.0% |
| 3-HHB-O1 | 4.0% |

### Use Example 4

| | |
|---|---|
| 4-WBBW-4 | 12.0% |
| V-WBB-2 | 4.0% |
| V1-WBB-2 | 7.0% |
| 3-HB-C | 18.0% |
| 7-HB-C | 3.0% |
| 1O1-HB-C | 10.0% |
| 3-HB(F)-C | 10.0% |
| 2-PyB-2 | 2.0% |
| 1O1-HH-3 | 5.0% |
| 2-BTB-O1 | 2.0% |
| 3-HHB-1 | 7.0% |
| 3-HHB-O1 | 4.0% |
| 3-H2BTB-2 | 3.0% |
| 3-H2BTB-3 | 3.0% |
| 2-PyBH-3 | 4.0% |
| 3-PyBH-3 | 3.0% |
| 3-PyBB-2 | 3.0% |

TNI= 78.1 (°C)
η = 23.4 (mPa·s)
Δn = 0.146
Δε = 8.3
Vth = 1.77 (V)

### Use Example 5

| | |
|---|---|
| V1-WBB-2 | 4.0% |
| 2-BBW-3Ot-Bu | 3.0% |
| 3-GB-C | 10.0% |
| 4-GB-C | 10.0% |
| 2-BEB-C | 12.0% |
| 3-BEB-C | 4.0% |
| 3-PyB(F)-F | 4.0% |
| 3-HEB-O4 | 8.0% |
| 4-HEB-O2 | 6.0% |
| 5-HEB-O1 | 6.0% |
| 3-HEB-O2 | 5.0% |
| 5-HEB-O2 | 4.0% |
| 5-HEB-5 | 5.0% |
| 4-HEB-5 | 5.0% |
| 1O-BEB-2 | 2.0% |
| 3-HHB-1 | 3.0% |
| 3-HHEBB-C | 3.0% |
| 3-HBEBB-C | 3.0% |
| 5-HBEBB-C | 3.0% |

TNI= 66.7 (°C)
η = 41.7 (mPa·s)
Δn = 0.120
Δε = 11.2
Vth = 1.34 (V)

### Use Example 6

| | |
|---|---|
| 4-WBBW-4 | 8.0% |
| V-WBB-2 | 6.0% |
| 5-PyB-F | 4.0% |
| 3-PyB(F)-F | 4.0% |
| 2-BB-C | 5.0% |
| 4-BB-C | 4.0% |
| 5-BB-C | 5.0% |
| 2-PyB-2 | 2.0% |
| 3-PyB-2 | 2.0% |
| 4-PyB-2 | 2.0% |
| 6-PyB-O5 | 3.0% |
| 6-PyB-O6 | 3.0% |
| 3-PyBB-F | 6.0% |
| 4-PyBB-F | 6.0% |
| 5-PyBB-F | 6.0% |
| 3-HHB-1 | 6.0% |
| 2-H2BTB-2 | 4.0% |
| 2-H2BTB-3 | 4.0% |
| 2-H2BTB-4 | 5.0% |
| 3-H2BTB-2 | 5.0% |
| 3-H2BTB-3 | 5.0% |
| 3-H2BTB-4 | 5.0% |

TNI= 91.9 (°C)
η = 38.8 (mPa·s)
Δn = 0.201
Δε = 6.3
Vth = 2.30 (V)

### Use Example 7

| | |
|---|---|
| 4-WBBW-4 | 5.0% |
| V-WBB-2 | 7.0% |
| 5-BEB(F)-C | 5.0% |
| V-HB-C | 15.0% |
| 5-PyB-C | 6.0% |
| 4-BB-3 | 4.0% |
| 3-HH-2V | 10.0% |
| 5-HH-V | 9.0% |
| V-HHB-1 | 7.0% |
| V2-HHB-1 | 13.0% |
| 3-HHB-1 | 4.0% |
| 1V2-HBB-2 | 10.0% |
| 3-HHEBH-3 | 5.0% |

TNI= 90.8 (°C)
η = 19.9 (mPa·s)
Δn = 0.117
Δε = 5.3
Vth = 2.28 (V)

### Use Example 8

| | |
|---|---|
| 4-W2HB(F,F)B(F)-OCF3 | 8.0% |
| 4-WBEB(F,F)-C | 12.0% |
| 2O1-BEB(F)-C | 5.0% |
| 3O1-BEB(F)-C | 4.0% |
| 1V2-BEB(F,F)-C | 16.0% |
| 3-HB-O2 | 10.0% |
| 3-HH-4 | 3.0% |
| 3-HHB-F | 3.0% |
| 3-HHB-O1 | 4.0% |
| 3-HBEB-F | 4.0% |
| 3-HHEB-F | 7.0% |
| 5-HHEB-F | 7.0% |
| 3-H2BTB-2 | 4.0% |
| 3-H2BTB-3 | 4.0% |
| 3-H2BTB-4 | 4.0% |
| 3-HB(F)TB-2 | 5.0% |

### Use Example 9

| | |
|---|---|
| 4-WBBW-4 | 10.0% |
| V-WBB-2 | 3.0% |
| 2-BEB-C | 10.0% |
| 5-BB-C | 12.0% |
| 7-BB-C | 7.0% |
| 1-BTB-3 | 4.0% |
| 2-BTB-1 | 10.0% |
| 1O-BEB-2 | 10.0% |
| 1O-BEB-5 | 12.0% |
| 2-HHB-1 | 4.0% |
| 3-HHB-F | 4.0% |
| 3-HHB-1 | 7.0% |
| 3-HHB-O1 | 4.0% |
| 3-HHB-3 | 3.0% |

TNI= 64.7 (°C)
η = 24.5 (mPa·s)
Δn = 0.163
Δε = 6.4
Vth = 1.76 (V)

### Use Example 10

| | |
|---|---|
| 4-WBBW-4 | 7.0% |
| V1-WBB-2 | 5.0% |
| 1V2-BEB(F,F)-C | 8.0% |
| 3-HB-C | 10.0% |
| V2V-HB-C | 14.0% |
| V2V-HH-3 | 14.0% |
| 3-HB-O2 | 4.0% |
| 3-HHB-1 | 10.0% |
| 3-HHB-3 | 8.0% |
| 3-HB(F)TB-2 | 4.0% |
| 3-HB(F)TB-3 | 4.0% |
| 3-H2BTB-2 | 4.0% |
| 3-H2BTB-3 | 4.0% |
| 3-H2BTB-4 | 4.0% |

TNI= 99.8 (°C)
η = 20.3 (mPa·s)
Δn = 0.137
Δε = 7.8
Vth = 2.08 (V)

### Use Example 11

| | |
|---|---|
| 4-WBBW-4 | 7.0% |
| V-WBB-2 | 5.0% |
| 5-BTB(F)TB-3 | 10.0% |
| V2-HB-TC | 10.0% |
| 3-HB-TC | 10.0% |
| 3-HB-C | 10.0% |
| 5-HB-C | 7.0% |
| 5-BB-C | 3.0% |
| 2-BTB-1 | 5.0% |
| 2-BTB-O1 | 5.0% |
| 3-HH-4 | 5.0% |
| 3-HHB-1 | 10.0% |
| 3-HHB-3 | 4.0% |
| 3-H2BTB-2 | 3.0% |
| 3-H2BTB-3 | 3.0% |
| 3-HB(F)TB-2 | 3.0% |

TNI= 100.6 (°C)
η = 17.8 (mPa·s)
Δn = 0.204
Δε = 6.7
Vth = 2.08 (V)

### Use Example 12

| | |
|---|---|
| 4-WBBW-4 | 7.0% |
| V1-WBB-2 | 5.0% |
| 1V2-BEB(F,F)-C | 6.0% |
| 3-HB-C | 18.0% |
| 2-BTB-1 | 5.0% |
| 5-HH-VFF | 30.0% |
| 1-BHH-VFF | 8.0% |
| 1-BHH-2VFF | 4.0% |
| 3-H2BTB-2 | 5.0% |
| 3-H2BTB-3 | 4.0% |
| 3-H2BTB-4 | 4.0% |
| 3-HHB-1 | 4.0% |

TNI= 80.9 (°C)
η = 15.9 (mPa·s)
Δn = 0.131
Δε = 6.2
Vth = 2.11 (V)

### Use Example 13

| | |
|---|---|
| 4-WBCF2OB(F,F)B(F)-F | 5.0% |
| 4-W2HB(F,F)B(F)-OCF3 | 5.0% |
| 4-WBB(2F,3F)B(2F,3F)-O2 | 3.0% |
| 2-HHB(F)-F | 17.0% |
| 3-HHB(F)-F | 17.0% |
| 5-HHB(F)-F | 16.0% |
| 2-H2HB(F)-F | 10.0% |
| 3-H2HB(F)-F | 5.0% |
| 5-H2HB(F)-F | 10.0% |
| 2-HBB(F)-F | 6.0% |
| 3-HBB(F)-F | 6.0% |

### Use Example 14

| | |
|---|---|
| 4-WBBW-4 | 8.0% |
| V1-WBB-2 | 4.0% |
| 7-HB(F)-F | 4.0% |
| 5-H2B(F)-F | 4.0% |
| 3-HB-O2 | 10.0% |
| 3-HH-4 | 2.0% |
| 3-HH[5D,6D,7D]-4 | 3.0% |
| 2-HHB(F)-F | 10.0% |
| 3-HHB(F)-F | 10.0% |
| 5-HH[5D,6D,7D]B(F)-F | 10.0% |
| 3-H2HB(F)-F | 3.0% |
| 2-HBB(F)-F | 3.0% |
| 3-HBB(F)-F | 3.0% |
| 5-HBB(F)-F | 6.0% |
| 2-H2BB(F)-F | 5.0% |
| 3-H2BB(F)-F | 6.0% |
| 3-HHB-O1 | 5.0% |
| 3-HHB-3 | 4.0% |

TNI= 87.3 (°C)
η = 21.7 (mPa·s)
Δn = 0.099
Δε = 3.0
Vth = 2.71 (V)

### Use Example 15

| | |
|---|---|
| 4-WBBW-4 | 7.0% |
| 2-BBW-3Ot-Bu | 2.0% |
| 7-HB(F,F)-F | 3.0% |
| 3-HB-O2 | 7.0% |
| 2-HHB(F)-F | 10.0% |
| 3-HHB(F)-F | 10.0% |
| 5-HHB(F)-F | 10.0% |
| 2-HBB(F)-F | 9.0% |
| 3-HBB(F)-F | 9.0% |
| 5-HBB(F)-F | 16.0% |
| 2-HBB-F | 4.0% |
| 3-HBB(F,F)-F | 5.0% |
| 5-HBB(F,F)-F | 8.0% |

TNI= 81.6 (°C)
η = 27.9 (mPa·s)
Δn = 0.113
Δε = 5.4
Vth = 2.09 (V)

### Use Example 16

| | |
|---|---|
| 4-WBCF2OB(F,F)B(F)-F | 8.0% |
| 4-W2HB(F,F)B(F)-OCF3 | 8.0% |
| 4-WBB(F,F)B(F,F)-F | 4.0% |
| 7-HB(F,F)-F | 3.0% |
| 3-H2HB(F,F)-F | 12.0% |
| 4-H2HB(F,F)-F | 10.0% |
| 3-HHB(F,F)-F | 5.0% |
| 4-HHB(F,F)-F | 5.0% |
| 3-HH2B(F,F)-F | 15.0% |
| 3-HBB(F,F)-F | 12.0% |
| 5-HBB(F,F)-F | 12.0% |
| 3-HBCF2OB(F,F)-F | 6.0% |

### Use Example 17

| | |
|---|---|
| 4-WBCF2OB(F,F)B(F)-F | 6.0% |
| 4-W2HB(F,F)B(F)-OCF3 | 6.0% |
| 4-WBB(F,F)B(F,F)-F | 3.0% |
| 7-HB(F,F)-F | 5.0% |
| 3-H2HB(F,F)-F | 7.0% |
| 3-HHB(F,F)-F | 10.0% |
| 4-HHB(F,F)-F | 5.0% |
| 3-HBB(F,F)-F | 10.0% |
| 3-HHEB(F,F)-F | 10.0% |
| 4-HHEB(F,F)-F | 3.0% |
| 5-HHEB(F,F)-F | 3.0% |
| 2-HBEB(F,F)-F | 3.0% |
| 3-HBEB(F,F)-F | 5.0% |
| 5-HBEB(F,F)-F | 3.0% |
| 3-HGB(F,F)-F | 15.0% |
| 3-HHBB(F,F)-F | 6.0% |

### Use Example 18

| | |
|---|---|
| 4-WBBW-4 | 8.0% |
| 3-HB-CL | 10.0% |
| 5-HB-CL | 4.0% |
| 7-HB-CL | 4.0% |
| 1O1-HH-5 | 5.0% |
| 2-HBB(F)-F | 8.0% |
| 3-HBB(F)-F | 8.0% |
| 5-HBB(F)-F | 14.0% |
| 4-HHB-CL | 8.0% |
| 5-HHB-CL | 4.0% |
| 3-H2HB(F)-CL | 4.0% |
| 3-HBB(F,F)-F | 10.0% |
| 5-H2BB(F,F)-F | 9.0% |
| 3-HB(F)VB-2 | 4.0% |

TNI= 90.0 (°C)
η = 23.1 (mPa·s)
Δn = 0.125
Δε = 4.7
Vth = 2.36(V)

### Use Example 19

| | |
|---|---|
| 4-WBBW-4 | 7.0% |
| 5-HB-F | 12.0% |
| 6-HB-F | 9.0% |
| 7-HB-F | 7.0% |
| 2-HHB-OCF3 | 7.0% |
| 3-HHB-OCF3 | 7.0% |
| 5-HHB-OCF3 | 5.0% |
| 3-HH2B-OCF3 | 4.0% |
| 5-HH2B-OCF3 | 4.0% |
| 3-HHB(F,F)-OCF3 | 5.0% |
| 3-HBB(F)-F | 10.0% |
| 5-HBB(F)-F | 10.0% |
| 3-HH2B(F)-F | 3.0% |
| 3-HB(F)BH-3 | 3.0% |
| 5-HBBH-3 | 3.0% |
| 3-HHB(F,F)-OCF2H | 4.0% |

TNI= 84.5 (°C)
η = 17.4 (mPa·s)
Δn = 0.097
Δε = 4.2
Vth = 2.52 (V)

### Use Example 20

| | |
|---|---|
| 4-WBBW-4 | 5.0% |
| V1-WBB-2 | 5.0% |
| 5-H4HB(F,F)-F | 7.0% |
| 5-H4HB-OCF3 | 15.0% |
| 3-H4HB(F,F)-CF3 | 8.0% |
| 3-HB-CL | 6.0% |
| 5-HB-CL | 4.0% |
| 2-H2BB(F)-F | 5.0% |
| 3-H2BB(F)-F | 10.0% |
| 5-HVHB(F,F)-F | 5.0% |
| 3-HHB-OCF3 | 5.0% |
| 3-H2HB-OCF3 | 5.0% |
| V-HHB(F)-F | 5.0% |
| 3-HHB(F)-F | 5.0% |
| 5-HHEB-OCF3 | 2.0% |
| 3-HBEB(F,F)-F | 5.0% |
| 5-HH-V2F | 3.0% |

TNI= 74.2(°C)
η = 25.7 (mPa·s)
Δn = 0.104
Δε = 6.9
Vth = 1.90 (V)

### Use Example 21

| | |
|---|---|
| 4-WBBW-4 | 10.0% |
| 2-HHB(F)-F | 2.0% |
| 3-HHB(F)-F | 2.0% |
| 5-HHB(F)-F | 2.0% |
| 2-HBB(F)-F | 6.0% |
| 3-HBB(F)-F | 6.0% |
| 5-HBB(F)-F | 10.0% |
| 2-H2BB(F)-F | 9.0% |
| 3-H2BB(F)-F | 9.0% |
| 3-HBB(F,F)-F | 20.0% |
| 5-HBB(F,F)-F | 19.0% |
| 1O1-HBBH-4 | 5.0% |

TNI= 95.8 (°C)
η = 36.4 (mPa·s)
Δn = 0.134
Δε = 6.8
Vth = 2.02 (V)

Pitch when 0.25 part of CM-43L was mixed to 100 parts of the composition described above as shown below.$\text{P = 61 µm}$

### Use Example 22

| | |
|---|---|
| 4-WBBW-4 | 5.0% |
| V1-WBB-2 | 3.0% |
| 5-HB-CL | 12.0% |
| 3-HH-4 | 4.0% |
| 3-HB-O2 | 20.0% |
| 3-H2HB(F,F)-F | 8.0% |
| 3-HHB(F,F)-F | 8.0% |
| 3-HBB(F,F)-F | 6.0% |
| 2-HHB(F)-F | 5.0% |
| 3-HHB(F)-F | 5.0% |
| 5-HHB(F)-F | 5.0% |
| 2-H2HB(F)-F | 2.0% |
| 3-H2HB(F)-F | 1.0% |
| 5-H2HB(F)-F | 2.0% |
| 3-HHBB(F,F)-F | 4.0% |
| 3-HBCF2OB-OCF3 | 4.0% |
| 5-HBCF2OB(F,F)-CF3 | 4.0% |
| 3-HHB-O1 | 2.0% |

TNI= 70.8 (°C)
η = 17.6 (mPa·s)
Δn = 0.091
Δε = 4.1
Vth = 2.16 (V)

### Use Example 23

| | |
|---|---|
| 4-WBB(2F)B(2F,3F)-O2 | 15.0% |
| 3-HEB-O4 | 24.0% |
| 4-HEB-O2 | 17.0% |
| 5-HEB-O1 | 17.0% |
| 3-HEB-O2 | 15.0% |
| 5-HEB-O2 | 12.0% |

### Use Example 24

| | |
|---|---|
| 4-WBB(2F)B(2F,3F)-O2 | 10.0% |
| 3-HH-4 | 6.0% |
| 3-HH-O1 | 6.0% |
| 3-HH-O3 | 6.0% |
| 5-HH-O1 | 6.0% |
| 3-HB(2F,3F)-O2 | 12.0% |
| 5-HB(2F,3F)-O2 | 11.0% |
| 3-HHB(2F,3F)-O2 | 14.0% |
| 5-HHB(2F,3F)-O2 | 15.0% |
| 3-HHB(2F,3F)-2 | 14.0% |

### Use Example 25

| | |
|---|---|
| 4-WBBW-4 | 8.0% |
| 4-W2HB(F,F)B(F)-OCF3 | 9.0% |
| 4-WBEB(F,F)-C | 12.0% |
| 3-WHVH-5 | 3.0% |
| 4-WBTW-5 | 3.0% |
| 2O1-BEB(F)-C | 5.0% |
| 5O1-BEB(F)-C | 4.0% |
| 1V2-BEB(F,F)-C | 10.0% |
| 3-HH-EMe | 4.0% |
| 3-HB-O2 | 18.0% |
| 7-HEB-F | 2.0% |
| 3-HHEB-F | 2.0% |
| 5-HHEB-F | 2.0% |
| 3-HBEB-F | 4.0% |
| 2O1-HBEB(F)-C | 2.0% |
| 3-HB(F)EB(F)-C | 2.0% |
| 3-HBEB(F,F)-C | 2.0% |
| 3-HHB-O1 | 4.0% |
| 3-HEBEB-F | 2.0% |
| 3-HEBEB-1 | 2.0% |

### Use Example 26

| | |
|---|---|
| 4-WBBW-4 | 7.0% |
| 4-WBCF2OB(F,F)B(F)-F | 8.0% |
| 4-W2HB(F,F)B(F)-OCF3 | 8.0% |
| 4-WBB(F,F)B(F,F)-F | 5.0% |
| 4-WHHBB(F,F)-F | 2.0% |
| 2-HB-C | 5.0% |
| 3-HB-C | 12.0% |
| 3-HB-O2 | 15.0% |
| 2-BTB-1 | 3.0% |
| 3-HHB-1 | 4.0% |
| 3-HHB-F | 4.0% |
| 3-HHEB-F | 4.0% |
| 5-HHEB-F | 4.0% |
| 2-HHB(F)-F | 7.0% |
| 3-HHB(F)-F | 7.0% |
| 3-HHB(F,F)-F | 5.0% |

### Use Example 27

| | |
|---|---|
| V-WBB-2 | 8.0% |
| 4-WBCF2OB(F,F)B(F)-F | 5.0% |
| 4-W2HB(F,F)B(F)-OCF3 | 5.0% |
| 4-WBB(2F)B(2F,3F)-O2 | 5.0% |
| 3-WHHBB(F,F)-F | 2.0% |
| 4-WBTW-5 | 5.0% |
| 3-HHB(F,F)-F | 9.0% |
| 3-H2HB(F,F)-F | 8.0% |
| 3-HBB(F,F)-F | 21.0% |
| 5-HBB(F,F)-F | 10.0% |
| 3-H2BB(F,F)-F | 10.0% |
| 5-HHBB(F,F)-F | 3.0% |
| 5-HHEBB-F | 2.0% |
| 3-HH2BB(F,F)-F | 3.0% |
| 1O1-HBBH-4 | 4.0% |

### Methods for producing the compounds:

Compounds of the present invention expressed by the general formula (1) can readily be synthesized by selecting proper procedures in ordinary organic synthetic chemistry, for examples, the procedures described in Organic Synthesis, Organic Reactions, and Jikken Kagaku Kouza (Course of Chemical Experiment) (Maruzen Co., Ltd.) and using them in combination while using the methods described in the thesis of a degree of Dr. Mattias Messner as reference.

In the case of compound (1-A) expressed by the general formula (1) in which ℓ = 1, A₀ is trans-1,4-cyclohexylene group, the compound can readily be produced by the following method.

That is, methyl lithium and then an alkyl iodide are reacted with [1.1.1]propellane (20) which can be synthesized by a method described in G. Szeimies, J. Bellzer et al., J. Am. Chem. Soc., 107, 6410 (1985), Chem. Ber., 122, 3978 (1989), in diethyl ether to synthesize compound (21). Subsequently, t-butyl lithium is reacted with the compound (21) in diethyl ether or tetrahydrofuran (hereinafter abbreviated to THF) to lithiate, cyclohexanone derivative (22) is reacted thereto to form compound (23), phosphorus oxychloride is reacted thereto in the presence of pyridine to dehydrate, subjected to a catalytic hydrogen reduction using a Raney-nickel or palladium carbon as catalyst, and then the reduced product thus obtained is recrysallized to produce the objective compound (1-A).

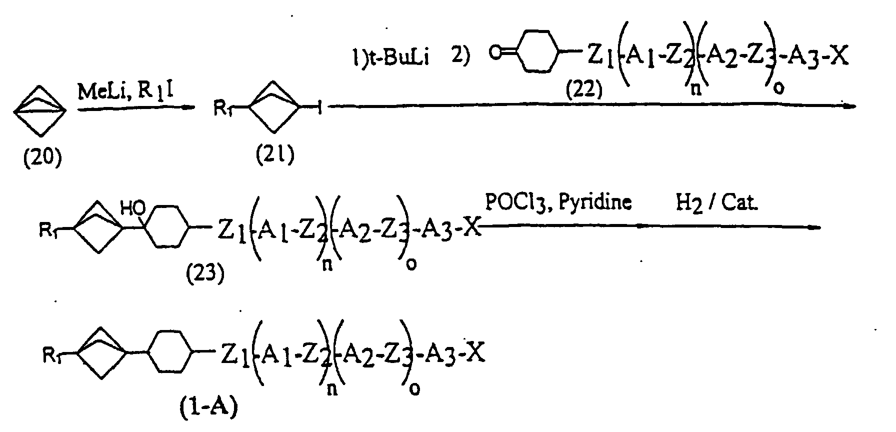

Compound (1-B) expressed by the general formula (1) in which ℓ = 1, A₀ is 1,4-phenylene group can readily be produced by the following method.

That is, the objective compound (1-B) can be produced by reacting t-butyl lithium and then zinc chloride with the compound (21) which can be prepared by the same manner as described above to convert it into organic metal compound (24), and then subjecting it to a coupling reaction with aryl halide (25) in the presence of dichloro[1,2-bis(diphenylphosphino)ethane]nickel (II) (hereinafter abbreviated to NiCl₂(dppe)₂) as catalyst.

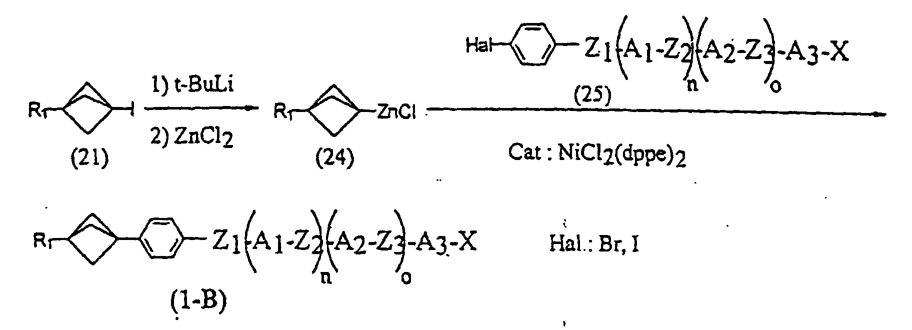

Also, compounds in which bicyclo[1.1.1]pentane-1,3-diyl group is inserted between two benzene rings, for example, compound (1-C) expressed by the general formula (1) in which ℓ = 0, $\text{m = n = o = 1}$, both A₀ and A₂ are 1,4-phenylene group, A₁ is bicyclo[1.1.1]pentane-1,3-diyl group, and both Z₁ and Z₂ are single bonds can readily be produced by the following method.

That is, the objective compound (1-C) can be produced by reacting a Grignard reagent (26) with [1.1.1]propellane (20) and then subjecting it to a coupling reaction with aryl bromide (27) employing NiCl₂(dppe)₂ as catalyst while using the coupling method described in M. Kumada et al., Bull. Chem. Soc. Jpn., 49 (7), 1959 (1976) as reference.

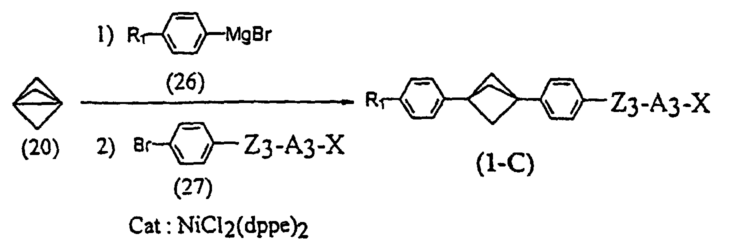

Compound (1-D) expressed by the general formula (1) in which ℓ = 1 and Z₀ is 1,2-ethylene group can readily be produced by the following method.

That is, compound (1-D) can be produced by reacting halide (28) with the organic metal compound (24) shown with reference to the production of the compound (1-B) described above by using NiCl₂(dppe)₂ or dichloro[1.1'-bis(diphenylphosphino)ferrocene]palladium (II) (hereinafter abbreviated to PdCl₂(dppf)₂) as catalyst.

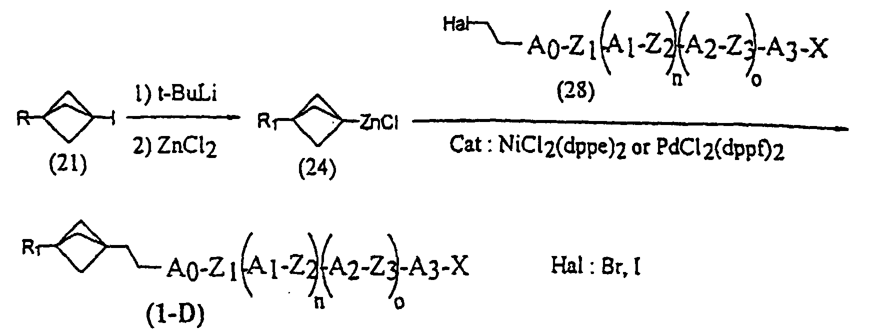

The halide (28) described above can be produced by the following method.

That is, compound (28-1) expressed by the formula (28) in which A₀ is trans-1,4-cyclohexylene group can be produced by the following method. Cyclohexane derivative (29) is first reacted with an ylide which is prepared by reacting a base such as sodium hydride, an alkyl lithium, and a sodium alkoxide with ethyl diethylphosphinoacetate according to the method of W. D. Emmons et al. (J. Am. Chem. Soc. 83, 1733 (1961), Org. Synth., 45, 44 (1965)) to produce cyclohexylidene derivative (30). Subsequently, the derivative (30) is subjected to a catalytic hydrogen reduction in the presence of a Raney-nickel or palladium carbon catalyst, the ester derivative thus obtained is further reduced with lithium aluminum hydride (hereinafter abbreviated to LAH) to produce alcohol derivative (31). Then, hydrobromic acid is reacted, or carbon tetrabromide is reacted in the presence of triphenylphosphine according to the method of J. G. Calzada et al. (Org. Synth., 54, 63 (1974)), with the alcohol derivative (31) to obtain compound (28-1).

Also, compound (28-2) expressed by the formula (28) in which A₀ is 1,4-phenylene group is produced by the following method.

That is, first, aldehyde derivative (33) in which carbon chain is extended by one is produced by reacting an ylide which is obtained by reacting a base such as sodium hydride, an alkyl lithium, and a sodium alkoxide with methoxymethyltriphenylphosphonium chloride according to the method described in Organic Reactions vol. 14, Chapter 3 (Wittigh Reaction) with benzaldehyde derivative (32), and then deprotecting the reaction product in the presence of an inorganic acid such as hydrochloric acid and sulfuric acid, an organic acid such as p-toluenesulfonic acid and formic acid, or an acidic ion exchanging resin. Compound (28-2) can be produced by reducing the compound (33) with sodium borohydride to an alcohol, and then brominating it in the same manner as in the case of the compound (31) described above.

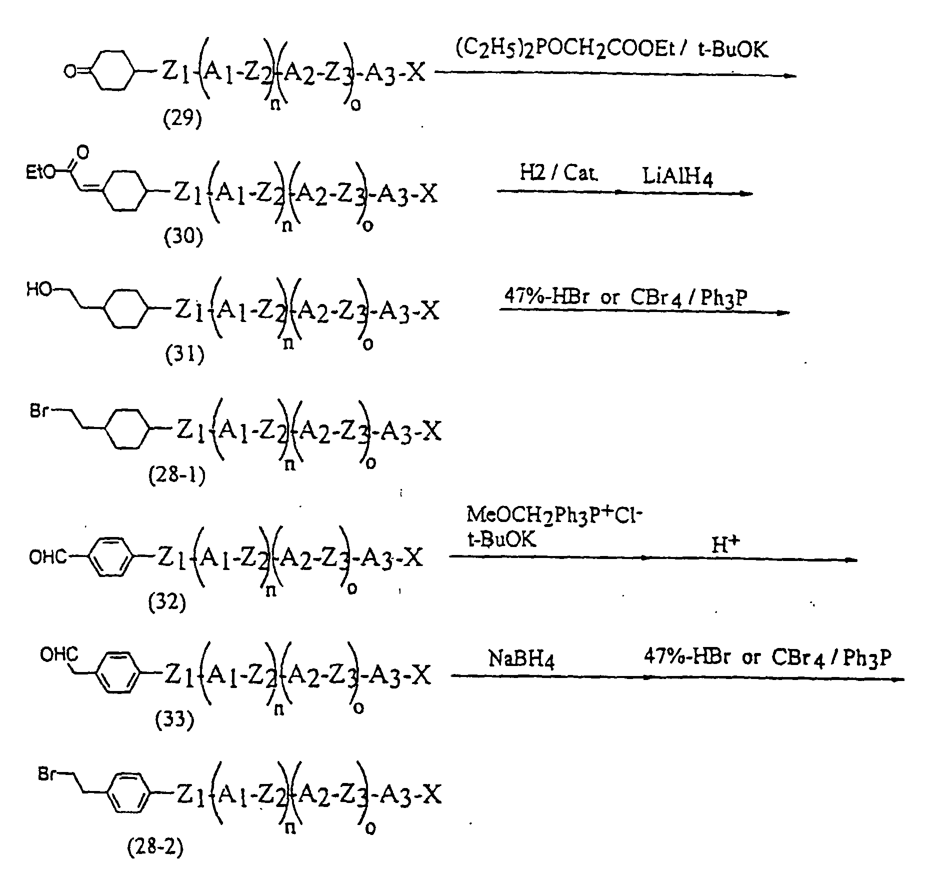

Compound (1-E) expressed by the general formula (1) in which ℓ = 1 and Z₀ is -COO-, and compound (1-F) expressed by the general formula (1) in which ℓ = 1 and Z₀ is -CF₂O- can be produced by the following methods.

That is, the objective ester derivative (1-E) can be produced by reacting alcohol derivative (35) with carboxylic acid derivative (34) which is obtained by reacting t-butyl lithium and then carbon dioxide with compound (21), in the presence of dicyclohexylcarbodimide (hereinafter abbreviated to DCC) and 4,4-dimethylaminopyridine (hereinafter abbreviated to DMAP).

Objective compound (1-F) can be produced by further reacting Lawesson's Reagent (S. -O. Lawesson et al., Bull. Soc. Chim. Belg., 87, 223 (1978)) with compound (1-E) according to the method of S. -O. Lawesson et al. (S. -O. Lawesson et al., Bull. Soc. Chim. Belg., 87, 293 (1978)) to derive to thion-O-ester derivative (36), and then reacting diethylaminosulfur trifluoride (hereinafter abbreviated to DAST) with the derivative according to the method of W. J. Middleton, J. Org. Chem., 40, 574 (1975), or reacting tetrabutylammonium dihydrogentrifluoride (hereinafter abbreviated to TBAH2F3) with the derivative in the presence of an oxidizing agent such as N-bromosuccinimide (hereinafter abbreviated to NBS) and 1,3-dibromo-5,5-dimethylhydantoin (hereinafter abbreviated to DBH) according to the method described in Laid-open Japanese Patent Publication No. Hei 5-255165, to fluorinating thiocarbonyl group.

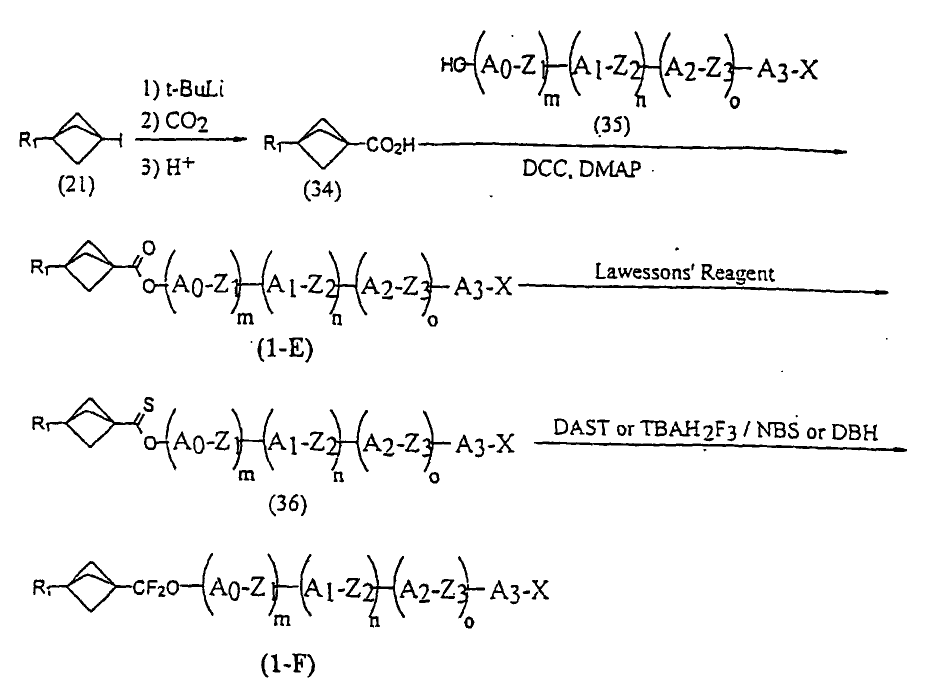

Compound (1-G) expressed by the general formula (1) in which ℓ = 1 and Z₀ is 1,2-ethenylene group can preferably be produced by the following method.

That is, the objective compound (1-G) can be produced by reacting a base such as sodium hydride, an alkyl lithium, and a sodium alkoxide with the Wittig Reagent (37) which is prepared from compound (21) and triphenylphosphine, to prepare an ylide, and then reacting aldehyde derivative (38) with the ylide.

When producing compound (1-G) by the method described above, compound in which 1,2-ethenylene group is cis-isomer (Z isomer) is mainly obtained. When the compound in which 1,2-ethenylene group is trans-isomer (E isomer) is necessary, isomerization is possible by the method shown below. That is, first, m-chloroperbenzoic acid is reacted with a mixture of E isomer and Z isomer (1-G) in the presence of potassium carbonate according to the method described in Japanese Patent Publication No. Hei 6-62462 to produce oxirane derivative (39). Subsequently, the derivative (39) is brominated with dibromotriphenylphosphorane, subjected to a recrystallization to purify and obtain only erythro-isomer, and then reduced with hydrochloric acid, acetic acid, or the like by using metal zinc as catalyst to produce compound (1-G-e) in which 1,2-ethenylene group is trans-isomer (E isomer).

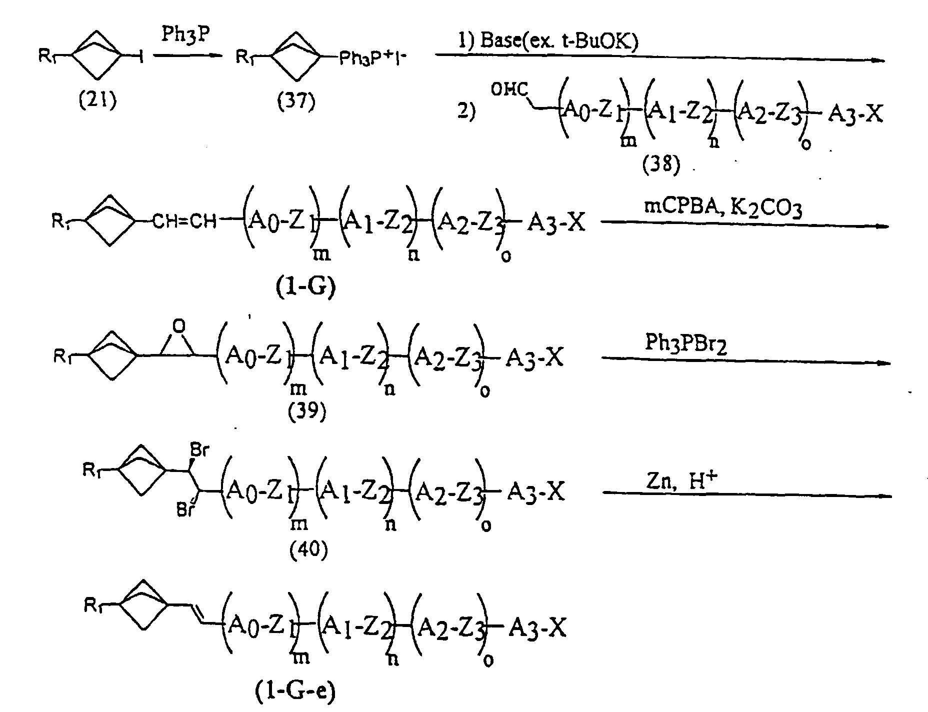

Compound (1-H)expressed by the general formula (1) in which $\text{ℓ = m = n = 1}$, A₀ and A₁ are trans-1,4-cyclohexylene group, Z₀ is single bond, Z₁ is trans-1,2-ethenylene group can preferably be produced by the following method. That is, first, an alcohol derivative which is obtained by reacting t-butyl lithium and cyclohexanedionemonopropylene ketal (41) with compound (21) is subjected to a dehydration and catalytic hydrogen reduction in the same manner as in the case where compound (1-A) is produced from compound (23), and then deprotected in the presence of an acid or acidic ion exchange resin to produce cyclohexanone derivative (42). Subsequently, an ylide which is obtained by reacting a base such as sodium hydride, an alkyl lithium, and a sodium alkoxide with methoxymethyltriphenyl-phosphonium chloride is reacted with compound (42) to obtain compound (43), and the compound (43) is deprotected in the presence of an acid catalyst in the same manner as described above and recrystallized to obtain aldehyde derivative (44). Then, the objective compound (1-H) can be produced by reacting an ylide which is obtained by reacting a base such as sodium hydride, an alkyl lithium, and a sodium alkoxide with the Wittig Reagent (45) which can be produced according to the method described in Organic Reactions vol. 14, Chapter 3 (Wittig Reaction) with compound (44), and then conducting the isomerization described with reference to the case wherein the compound (1-G-e) described above is produced.

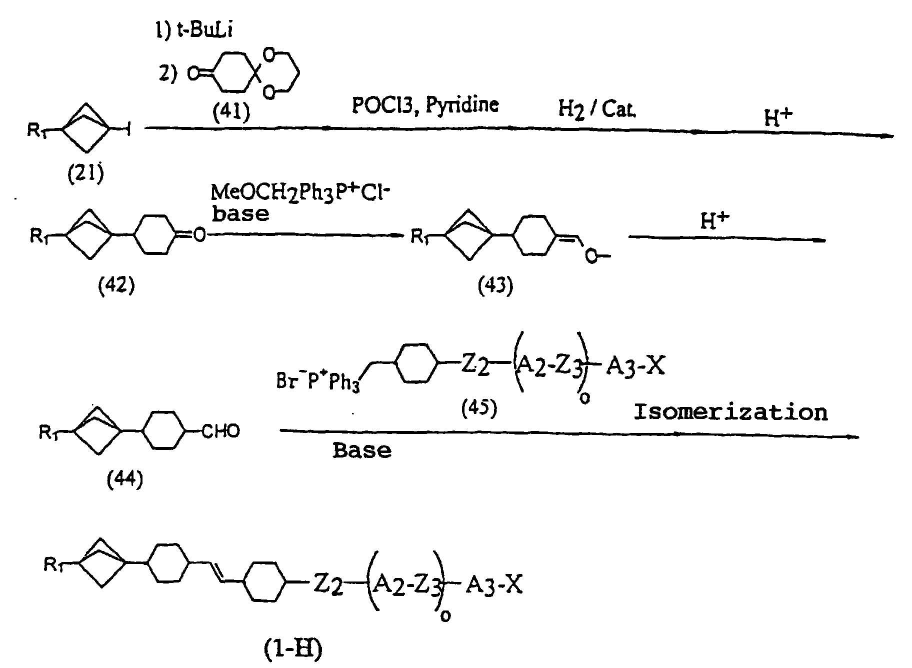

Derivative expressed by the general formula (1) in which R₁ is an alkenyl group can preferably produced by the following method.

That is, the derivative in which R₁ is an alkenyl group can be produced by using 1-alkenyl-3-iodobicyclo[1.1.1]pentane (47) which is obtained by reacting methyl lithium and alkenyl iodide (46) with [1.1.1]propellane (20), in place of the compound (21) used when the compounds (1-B), (1-C), (1-D), (1-E), (1-F), and (1-G) are produced.

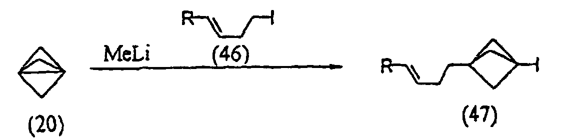

Also, terminal substituent X of compounds can preferably introduced by the methods described below. That is, derivative (1-I) in which A₃ is 1,4-phenylene group hydrogen atom of which may be replaced by fluorine atom, and terminal substituent X is -OCF₃ is produced by the method as follows. First, butyl lithium is reacted with fluorobenzene derivative (48) according to the method described in R. L. Kidwell et al., Org. Synth., V, 918 (1973) to lithiate, reacted with a trialkyl borate to convert into boric acid ester (49), and then reacted with an organic carboxylic peroxide such as peracetic acid to produce phenol derivative (50). Subsequently, the objective compound (1-I) can be produced by reacting sodium hydride, carbon disulfide, and methyl iodide with the phenol derivative (50) to convert it into xanthate derivative (51) according to the method reported by Manabu KUROHOSHI et al. in the general remarks (Journal of Organic Synthetic Chemical Society, vol. 51, No. 12, p 22 (1993)), and then reacting (HF) pyridine with the derivative in the presence of DBH.

Derivative (1-J) in which terminal substituent X is -OCF₂H can also be produced by reacting sodium hydride with phenol derivative (50) in a dipolar aprotic solvent such as DMF, and then reacting chlorodifluoromethane therewith.

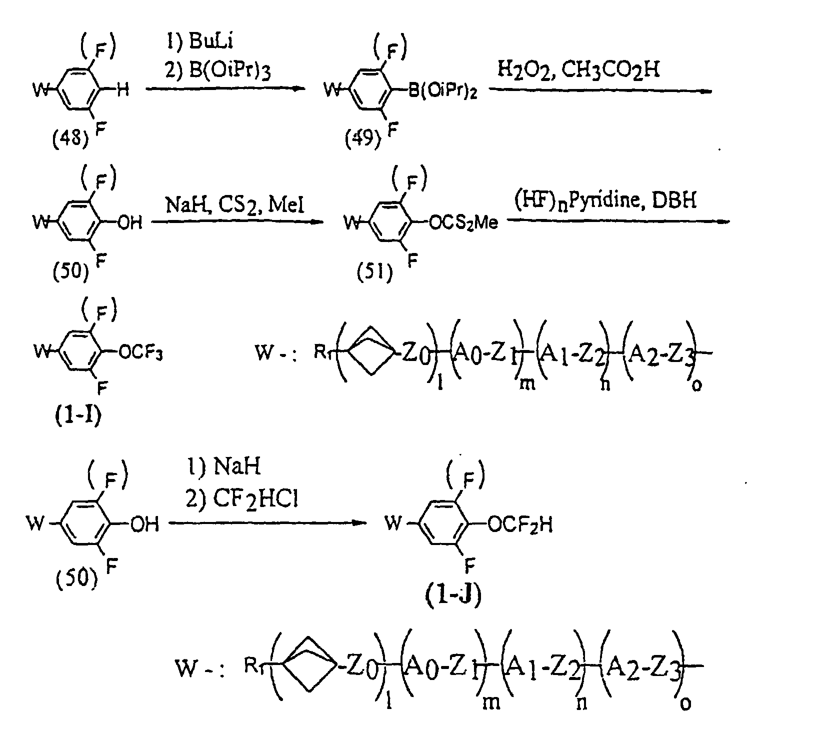

Derivative (1-K) in which A₃ is 1,4-phenylene group hydrogen atom of which may be replaced by fluorine atom, and substituent X is -CF₃ group can preferably be produced by the following method. That is, the objective trifluoromethylated isomer (1-K) can be produced by reacting butyl lithium with fluorobenzene derivative (48) to lithiate, reacting iodide to convert it into iodo derivative (52), and then reacting methyl fluorosulfonyldifluoroacetate with the iodo derivative (52) by using cuprous iodide as catalyst. Derivative (1-L) in which substituent X is difluoromethyl group can be produced by reacting DAST with benzaldehyde derivative (53) which is obtained by reacting N-formylpiperidine with lithiated product of fluorobenzene derivative (48).

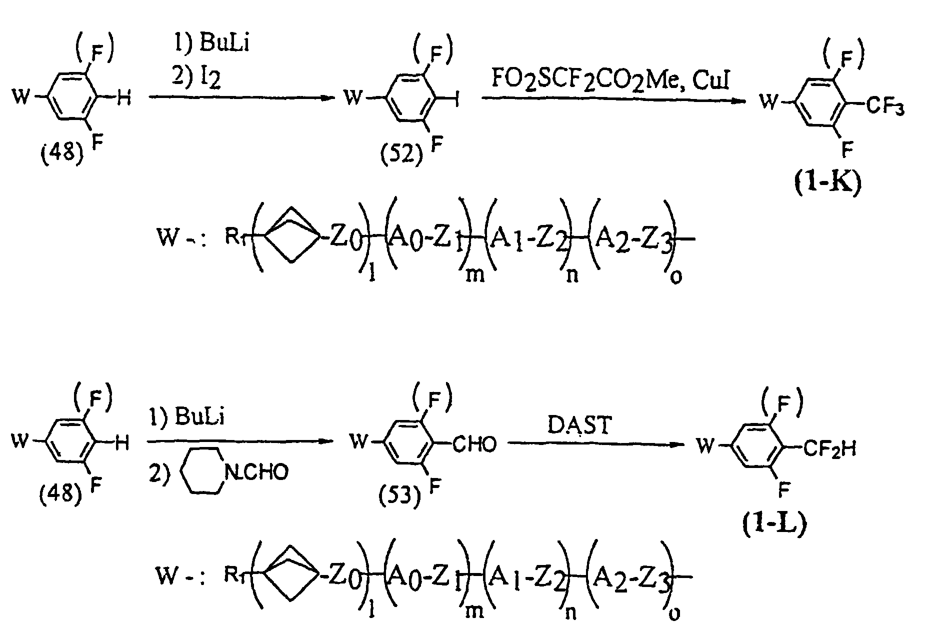

Compound (1-M) in which A₃ is 1,4-phenylene group hydrogen atom of which may be replaced by fluorine atom, and terminal substituent X is a fluoroalkoxy group (for example, substituent X is 2,2,2-trifluoroethoxy group, 2,2-difluoroethoxy group, 2,2,3,3,3-pentafluoropropoxy group, or 2,2,3,3-tetrafluoropropoxy group) can preferably be produced by the following method.

That is, the objective compound (1-M) can be produced by reacting sodium hydride with phenol derivative (50) in 1,3-dimethyl-2-imidazolidinone according to a method described in the literature of F. Camps et al. (Synthesis, 1980, 727), and then reacting a fluoroalkyl mesylate (54).

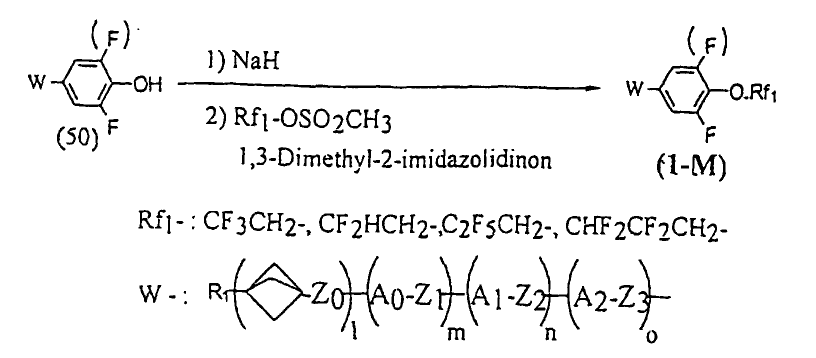

Compound (1-N) in which terminal substituent X is a fluoroalkyl group or fluoroalkoxy group can preferably be produced by using a commercially available p-bromo-fluoroalkyl substituted benzene or p-bromo-fluoroalkoxybenzene derivative (for example, a derivative in which terminal X is 1,1,2,2-tetrafluoroethyl group, 1,1,2,3,3,3-hexafluoropropyl group, pentafluoroethyl group, heptafluoropropyl group, 1,1,2,3,3,3-hexafluoropropoxy group). That is, the objective compound (1-N) can be produced by reacting bromobenzene derivative (56) with boric acid derivative (55) which is obtained by acid hydrolyzing boric acid ester (54) which is obtained in the same manner as in the case of production of boric acid ester (49) which is used for the production of the compound (1-I) described above, by using tetrakistriphenylphosphine palladium (0) as catalyst according to a coupling method of Akira SUZUKI et al. (described in the general remarks, Journal of Organic Synthetic Chemical Society, vol. 46, No. 9, p 848 (1988)) to coupling.

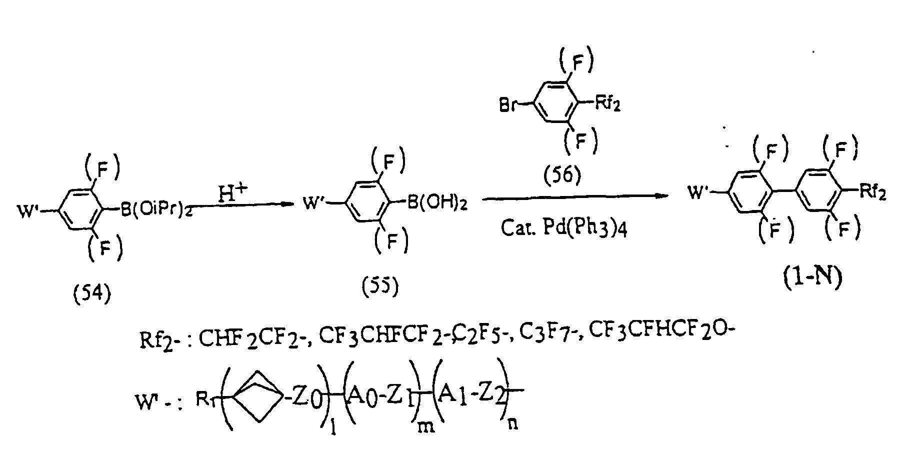

Compounds other than those described above can be produced by using known literatures such as patent publications as reference. For example, the compounds having 1,2-ethylene group as bonding group can be produced according to the production method described in Japanese Patent Publication No. Hei 3-03643 or Laid-open Japanese Patent Publication No. Sho 59-25338. Compounds having 1,4-butylene group as bonding group can be produced according to the production method described in Laid-open Japanese Patent Publication No. Hei 3-66632, Laid-open Japanese Patent Publication No. Hei 4-501575, or Laid-open Japanese Patent Publication No. Hei 5-310605. Compounds having 1,2-ethenyl group as bonding group can be produced according to the production method described in Laid-open Japanese Patent Publication No. Sho 61-215336 or Laid-open Japanese Patent Publication No. Hei 3-127748. Compounds having 1,2-ethynyl group as bonding group can be produced according to the production method described in Laid-open Japanese Patent Publication No. Sho 61-280441 or Laid-open Japanese Patent Publication No. Hei 1-502908. Also, the compounds having difluoromethylenoxy group (-CF₂O-) as bonding group can be produced according to the production method described in Laid-open Japanese Patent Publication No. Hei 5-112778 or Laid-open Japanese Patent Publication No. Hei 5-255165.

### BEST MODE FOR CARRYING OUT THE INVENTION

Now, the production methods and use examples of the compounds of the present invention are described in more detail with reference to Examples. However, it should be understood that the scope of the present invention is by no means restricted by such specific Examples. In each of the Examples, Cr indicates crystal, N: nematic phase, Sm: smectic phase, and Iso: isotropic liquid, and the unit of all phase transition temperatures is °C. Structure of the compounds was confirmed by the spectrums of nuclear magnetic resonance (hereinafter abbreviated to 1H-NMR) and mass spectrums (hereinafter abbreviated to MS). In the Examples, d indicate a doublet, t: a triplet, and m: a multiplet in 1H-NMR, and J shows a coupling constant (Hz). In the MS, M+ indicates molecular ion peak.

NI point, Δε (dielectric anisotropy value), Δn (optical anisotropy value), and viscosity η indicate the values obtained by extrapolation from physical properties of liquid crystal compositions obtained by adding a particular compound in Mother liquid crystal A or Mother liquid crystal B, physical properties of the mother liquid crystal itself, and the concentration of the particular compound in the liquid crystal composition (Δε and Δn were determined at 25°C, and η was determined at 20°C).

### Chemical composition of Mother liquid crystal A

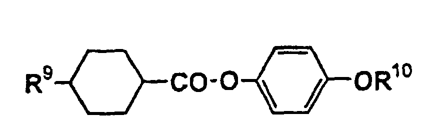

Five kind of ester compounds which are expressed by the general formula described above, but the alkyl groups at both terminals (R⁹ and R¹⁰) are different from one another were mixed in the ratio shown below, and used as Mother liquid crystal A.

| | | % by weight |
|---|---|---|
| R⁹ = C₃H₇, | R¹⁰ = C₄H₉ | 27.6 |
| R⁹ = C₄H₉, | R¹⁰ = C₂H₅ | 20.7 |
| R⁹ = C₅H₁₁, | R¹⁰ = CH₃ | 20.7 |
| R⁹ = C₃H₇, | R¹⁰ = C₂H₅ | 17.2 |
| R⁹ = C₅H₁₁, | R¹⁰ = C₂H₅ | 13.8 |
| | | 100.0 |

NI = 74.0°C, Δε = -1.5, and Δn = 0.087, η = 17.9 mPa.s

### Chemical composition of Mother liquid crystal B

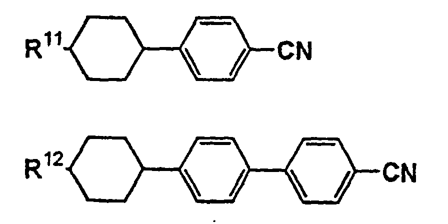

Four kind of compounds which are expressed by either general formulas described above, but the alkyl groups at terminals (R¹¹ and R¹²) are different from one another were mixed in the ratio shown below, and used as Mother liquid crystal B.

| | % by weight |
|---|---|
| R¹¹ = C₃H₇, | 24.0 |
| R¹¹ = C₅H₁₁, | 36.0 |
| R¹¹ = C₇H₁₅, | 25.0 |
| R¹² = C₅H₁₁, | 15.0 |
| | 100.0 |

NI = 71.7°C, Δε = 11.0, and Δn = 0.137, η = 26.7 mPa.s

### Example 1

### Preparation of 4''-(3-n-butylbicyclo[1.1.1]pent-1-yl)-3,4-5,2',6'-pentafluoroterphenyl (Compound No. 599)

### First step

Under nitrogen gas atmosphere, 100 g (1.51 mol) of [1.1.1]propellane was dissolved in 300 ml of diethyl ether and cooled down to -60°C with a refrigerant. Methyl lithium in an amount of 63.3 g (2.89 mol) was added dropwise thereto and stirred at the same temperature for 2 hours. n-Butyl iodide in an amount of 415.6 g (2.26 mol) was added dropwise at the same temperature thereto. After the finishing of the dropping, it was gradually warned and stirred at room temperature for 6 hours. After the termination of the reaction, the reaction product was put in 1 ℓ of an ice-cold water, extracted with 200 ml of diethyl ether twice, washed with 200 ml of water thrice, and dried over anhydrous magnesium sulfate. The solvent was distilled off under a reduced pressure, the residue was subjected to silica gel column chromatography (eluent: n-heptane), and concentrated under a reduced pressure to obtain 260.6 g of 1-iodo-3-n-butyl bicyclo[1.1.1]pentane.

### Second step

Under nitrogen gas stream, 50 g (0.20 mol) of the 1-iodo-3-n-butylbicyclo[1.1.1]pentane obtained in the first step was dissolved in 250 ml of THF and cooled down to -30°C with a refrigerant. n-Butyl lithium (1.6M, n-hexane solution) in an amount of 150 ml (0.24 mol) was added dropwise thereto, stirred at the same temperature for 20 min, further cooled down to -50°C, and stirred for 1 hour.

Zinc chloride (0.5M, THF solution) in an amount of 525 ml was added dropwise thereto, stirred at the same temperature for 1 hour, and then stirred at room temperature for further 1 hour.

Tetrakis(triphenylphosphine)palladium (0) in an amount of 1.2 g was added thereto, a solution of 56.6 g (0.19 mol) of 4-iodobromobenzene in 200 ml of THF was added dropwise, and heated while being stirred for 3 hours. The reaction mixture was added to 1 ℓ of water and extracted with 500 ml of toluene twice. The organic layer was washed with 1 ℓ of water thrice and then dried over anhydrous magnesium sulfate. The solvent was distilled off under a reduced pressure, and the residue was subjected to silica gel column chromatography (eluent: n-heptane) and recrystallized from n-heptane to obtain 36.2 g of 4-(3-n-butylbicyclo[1.1.1]pent-1-yl)bromobenzene.

### Third step

Under nitrogen gas atmosphere, 36.2 g (0.13 mol) of the 4-(3-n-butylbicyclo[1.1.1]pent-1-yl)bromobenzene obtained in the second step, 26.6 g (0.17 mol) of 3,5-difluorophenyl boric acid, and 1.81 g of 5 weight % palladium/carbon catalyst were suspended in 300 ml of mixed solvent of toluene/Solmix/water = 1/1/1 and heated under reflux for 10 hours. After the termination of the reaction, the palladium/carbon catalyst was removed by filtration, and extracted with 150 ml of toluene twice. The organic layer was washed with 300 ml of water thrice and dried over anhydrous magnesium sulfate. The solvent was distilled off under a reduced pressure, and the residue was subjected to silica gel column chromatography (eluent: n-heptane) and recrystallized from n-heptane to obtain 17.5 g of 4'-(3-n-butylbicyclo[1.1.1]pent-1-yl)-3,5-difluorobiphenyl.

### Fourth step

Under nitrogen gas stream, 17.5 g (56.0 mmol) of the 4'-(3-n-butylbicyclo[1.1.1]pent-1-yl)-3,5-difluorobiphenyl obtained in the third step was dissolved in 100 ml of THF and cooled down to -30°C under a refrigerant. n-Butyl lithium (1.6M, n-hexane solution) in an amount of 42 ml (67.2 mmol) was added dropwise, stirred at the same temperature for 20 min, further cooled down to -50°C, and stirred four 1 hour. Subsequently, 134.4 ml (67.2 mmol) of zinc chloride (0.5M, THF solution) was added thereto, stirred at the same temperature for 1 hour, and then stirred at room temperature for further 1 hour. Tetrakis-(triphenylphosphine)palladium (0) in an amount of 3.23 g was added to the reaction liquid, a solution of 14.2 g (67.2 mmol) of 3,4,5-trifluorobromobenzene in 80 ml of THF was added thereto, and heated under reflux for 3 hours. The reaction mixture was added to 200 ml of water and extracted with 100 ml of toluene twice. The organic layer was washed with 200 ml of water thrice and dried over anhydrous sodium sulfate. The solvent was distilled off under a reduced pressure, and the residue was subjected to silica gel column chromatography (eluent: n-heptane) and recrystallized from n-heptane twice to obtain 9.9 g of the subject compound, 4''-(3-n-butylbicyclo[1.1.1]pent-1-yl)-3,4,5,2',6'-pentafluoroterphenyl. Results of determination of various kind of spectrums strongly supported the structure of the objective product.
GC-MS m/z 442 (M+)

### Example 2

### Preparation of 4'-(trans-4-(trans-4-(3-n-propylbicyclo[1.1.1]pent-1-yl)cyclohexyl)cyclohexyl)-3,4,5-trifluorobiphenyl (Compound No. 733)

### First step

Under nitrogen gas atmosphere, 50 g (0.21 mol) of 1-iodo-3-n-propylbicyclo[1.1.1]pentane was dissolved in 250 ml of THF and cooled down to -60°C with a refrigerant. t-Butyl lithium (1.5M, n-pentane solution) in an amount of 300 ml was added dropwise thereto and stirred at the same temperature for 1 hour. To this mixture was added dropwise a solution of 53.8 g (0.21 mol) of 4-(trans-4-phenylcyclohexyl)cyclohexanone in 250 ml of THF while being maintained at a temperature lower than -60°C. After the finishing of the dropping, the reaction temperature was gradually raised up to room temperature and further stirred for 5 hours. The reaction solution was subjected to Celite filtration, the solvent was distilled off under a reduced pressure, and the residue was subjected to silica gel column chromatography (eluent: n-heptane/toluene = 9/1) and the solvent was distilled off under a reduced pressure. Further, this product was dissolved in 150 ml of pyridine under nitrogen gas atmosphere, and 64.5 g (0.42 mol) of phosphorus oxychloride was added dropwise thereto under cooling with ice. After the finishing of the dropping, the solution was gradually warmed up to room temperature and stirred for 48 hours. After the termination of the reaction, the reaction mixture was put in 300 ml of water and extracted with 100 ml of diethyl ether twice. The organic layer was washed 200 ml of 2N aqueous hydrochloric acid solution twice,then washed with 200 ml of water thrice, and dried over anhydrous magnesium sulfate. The solvent was distilled off under a reduced pressure, and the residue was subjected to silica gel chromatography (eluent: n-heptane) to obtain 30.6 g of 1-(3-n-propylbicyclo[1.1.1]pent-1-yl)-4-(trans-4-phenylcyclohexyl)cyclohexene.

### Second step

To 200 ml of Solmix was dissolved 30.6 g (90.1 mmol) of the crude 1-(3-n-propylbicyclo[1.1.1]pent-1-yl)-4-(trans-4-phenylcyclohexyl)cyclohexene obtained in the first step, 3 g of 10 weight % Raney-Ni catalyst was added thereto, and stirred at room temperature under hydrogen pressure of 1 to 2 kg/cm² for 12 hours. After the catalyst was filtered off, the solvent was distilled off under a reduced pressure, and the residue was subjected to silica gel column chromatography (eluent: n-heptane) and recrystallized from n-heptane to obtain 19.7 g of trans-4-(trans-4-(3-n-propylbicyclo[1.1.1]pent-1-yl)-cyclohexyl)cyclohexylbenzene.

### Third step

Under nitrogen gas atmosphere, 19.7 g (57.7 mmol) of trans-4-(trans-4-(3-n-propylbicyclo[1.1.1]pent-1-yl)cyclohexyl)cyclohexylbenzene was dissolved in a mixed solvent (acetic acid 200 ml/carbon tetrachloride 40 ml/ water 40 ml), 12.1 g (69.2 mmol) of iodic acid, 17.6 g (69.2 mmol) of iodine, and 5 ml of sulfuric acid were added thereto and heated under reflux for 8 hours. After the termination of the reaction, the reaction mixture was put in 1 ℓ of water and extracted with 200 ml of toluene twice. The organic layer was washed with saturated aqueous sodium sulfite solution, saturated aqueous sodium carbonate solution, and water in turn, and dried over anhydrous sodium sulfate. The solvent was distilled off under a reduced pressure, and the residue was subjected to silica gel column chromatography (eluent: n-heptane) and concentrated under a reduced pressure to obtain 14.8 g of 4-(trans-4-(trans-4-(3-n-propylbicyclo[1.1.1]pent-1-yl)cyclohexyl)cyclohexyl)iodo benzene.

### Fourth step

In 200 ml of THF were dissolved 14.8 g (31.7 mmol) of the crude 4-(trans-4-(trans-4-(3-n-propylbicyclo[1.1.1]pent-1-yl)cyclohexyl)cyclohexyl)iodo benzene obtained in the third step and 5.6 g (31.7 mmol) of 3,4,5-trifluorophenyl boric acid, 1.83 g of tetrakis(triphenylphosphine)palladium (0) was added thereto, and heated under reflux for 6 hours. The reaction mixture was put in 500 ml of water and extracted with 150 ml of toluene twice. The organic layer was washed 200 ml of water thrice and dried over anhydrous magnesium sulfate. The solvent was distilled off under a reduced pressure, and the residue was subjected to silica gel column chromatography (eluent: n-heptane) and recrystallized from n-heptane twice to obtain 5.8 g of the subject compound, 4'-(trans-4-(trans-4-(3-n-propylbicyclo[1.1.1]pent-1-yl)cyclohexyl)cyclohexyl)-3,4,5-trifluorobiphenyl. Results of determination of various kind of spectrums strongly supported the structure of the objective product.
GC-MS m/z 480 (M+)

### Example 3

### Preparation of 1-(3-n-butylcyclo[1.1.1]pent-1-yl)-2-(trans-4-(3,2',6'-trifluoro-4-trifluoromethoxybiphenyl-4'-yl)cyclohexyl)ethane (Compound No. 659)

### First step

In 150 ml of THF was dissolved 30 g (118 mmol) of 4-(3,5-difluorophenyl)cyclohexanone ethylene ketal and cooled down to -30°C under refrigerant. n-Butyl lithium (1.6M, n-hexane solution) in an amount of 110 ml was added dropwise thereto, stirred at the same temperature for 20 min, further cooled down to -50°C, and stirred for 1 hour. After 284 ml of zinc chloride (0.5M, THF solution) was added thereto and stirred at the same temperature for 1 hour, it was further stirred at room temperature for 1 hour. Tetrakis(triphernylphosphine)palladium (0) in an amount of 6.81 g was added thereto, a solution of 30.6 g of 3-fluoro-4-trifluoromethoxybromobenzene in 100 ml of THF was added dropwise, and heated while being stirred for 3 hours. The reaction mixture was put in 500 ml of water, and extracted with 200 ml of toluene twice. The organic layer was washed with 500 ml of water thrice, and dried over anhydrous magnesium sulfate. The solvent was distilled off under a reduced pressure, and the residue was subjected to silica gel column chromatography (eluent: n-heptane) and the solvent was distilled off under a reduced pressure to obtain a ketal derivative. This derivative was dissolved in 200 ml of toluene, 5.4 g of formic acid was added thereto, and heated under reflux for 2 hours. After the termination of the reaction, the product was put in 300 ml of water and extracted with 100 ml of toluene twice. The organic layer was washed with 19 % by weight of aqueous sodium carbonate solution twice and 200 ml of water thrice, and dried over anhydrous magnesium sulfate. The solvent was distilled off under a reduced pressure, and the residue was subjected to silica gel column chromatography (eluent: n-heptane), and the solvent was distilled off under a reduced pressure to obtain 11.0 g 4-(3,2',6'-trifluoro-4-trifluoromethoxybiphenyl-4'-yl)cyclohexanone.

### Second step

Under nitrogen gas stream, 3.8 g of potassium t-butoxide was added to 7.6 g of ethyl diethylphosphonate, stirred for 2 hours, 11.0 g (28.3 mmol) of 4-(3,2',6'-trifluoro-4-trifluoromethoxybiphenyl-4'-yl)cyclohexanone was added, and then further stirred for 2 hours. After the termination of the reaction, water was added and extracted with toluene twice. The organic layer was washed with water thrice and dried over anhydrous magnesium sulfate. The solvent was distilled off under a reduced pressure, and the residue was subjected to silica gel column chromatography (eluent: n-heptane/toluene = 1/1), and the solvent was distilled off under a reduced pressure to obtain 7.8 g of the objective compound. This crude compound was dissolved in 80 ml of ethyl acetate, 1.0 g of 5 weight % palladium/carbon catalyst was added thereto, and stirred at room temperature under a hydrogen pressure of 1 to 2 kg/cm² for 5 hours. After the catalyst was filtered off, the solvent was distilled off under a reduced pressure, and the residue was subjected to silica gel column chromatography (eluent: n-heptane/toluene = 4/1) and recrystallized from n-heptane to obtain 5.1 g of an ethyl ester derivative.

### Third step

Under nitrogen gas atmosphere, a solution of 5.1 g (11.1 mmol) of the ethyl ester derivative obtained in the second step in 50 ml of THF was added dropwise in a suspension of 0.42 g of LAH in 30 ml of THF. After the finishing of the dropping, it was stirred under cooling with ice, and further stirred at room temperature for 1 hour. After each 100 ml of ethyl acetate, water, and 6N-hydrochloric acid was added to the reaction liquid in turn, it was extracted with toluene twice. The organic layer was washed with water thrice and dried over anhydrous magnesium sulfate. The solvent was distilled off under a reduced pressure, and the residue was subjected to silica gel column chromatography (eluent: n-heptane/toluene = 4/1), and then the solvent was distilled off under a reduced pressure to obtain 3.0 g of 4'-(trans-4-(2-hydroxyethyl)-cyclohexyl)-3,2',6'-trifluoro-4-trifluoromethoxybiphenyl.

### Fourth step

Under nitrogen gas stream, 3.0 g (7.18 mmol) of 4'-(trans-4-(2-hydroxyethyl)cyclohexyl)-3,2'-6'-trifluoro-4-trifluoromethoxybiphenyl and 0.78 g of triphenylphosphine were dissolved in 60 ml of dichloromethane and cooled down to 0°C. A solution of 2.38 g of carbon tetrabromide in 25 ml of dichloromethane was added dropwise thereto. After the finishing of the dropping, the solution was stirred at the same temperature for 1 hour and further stirred at room temperature for 4 hours. After the termination of the reaction, the reaction mixture was put in 150 ml of water and extracted with 50 ml of dichloromethane twice. The organic layer was washed with 100 ml of water twice and dried over anhydrous magnesium sulfate. The solvent was distilled off under a reduced pressure, and the residue was subjected to silica gel column chromatography (eluent: n-heptane/ethyl acetate = 4/1) and the concentrated under a reduced pressure to obtain 1.93 g of the objective bromide.

### Fifth step

Under nitrogen gas stream, 2.0 g (8.04 mmol) of 1-iodo-3-n-butylbicyclo[1.1.1]pentane was dissolved in 20 ml of THF and cooled down to -30°C with a refrigerant. t-Butyl lithium (1.5M, n-pentane solution) in an amount of 8 ml was added thereto, stirred at the same temperature for 20 min, further cooled down to -50°C, and stirred for 1 hour.

Zinc chloride (0.5M, THF solution) in an amount of 25 ml was added thereto, stirred at the same temperature for 1 hour, and then stirred at room temperature for further 1 hour. Tetrakis(triphenylphosphine)palladium (0) in an amount of 0.46 g was added to the reaction liquid, a solution of 1.93 g (4.02 mmol) of the bromide obtained in the fourth step in 20 ml of THF was added dropwise to the solution, and heated under reflux for 6 hours. After the termination of the reaction, the product was put in 100 ml of water and extracted with 50 ml of diethyl ether twice. The organic layer was washed with 100 ml of water thrice and dried over anhydrous magnesium sulfate. The solvent was distilled off under a reduced pressure, and the residue was subjected to silica gel column chromatography (eluent: n-heptane) and recrystallized from n-heptane to obtain 0.88 g of the subject compound, 1-(3-n-butylbicyclo[1.1.1]pent-1-yl)-2-(trans-4-(3,2',6'-trifluoro-4-trifluoromethoxybiphenyl-4'-yl)cyclohexyl)ethane. Results of determination of various kind of spectrums strongly supported the structure of the objective product.
GC-MS m/z 524 (M+)

### Example 4

### Preparation of 4-(3-n-butylbicyclo[1.1.1]pent-1-yl)-α,α-difluorobenzyl-(2,6,3',4'-tetrafluoro-biphenyl-4-yl)ether (Compound No. 663)

### First step

Under nitrogen gas stream, 2.74 g (112.8 mmol) of magnesium was added in 50 ml of THF, and a solution of 30 g (107.7 mmol) of the 4-(3-n-butylbicyclo[1.1.1]pent-1-yl)bromobenzene obtained in the second step in Example 1 in 200 ml of THF was added dropwise while maintaining the reaction temperature at about 50°C. After stirred at room temperature for 1 hour, it was cooled to about 10°C and 81.9 g (1.07 mol) of carbon disulfide was gradually added dropwise. After the finishing of the dropping, it was stirred at room temperature for 24 hours. After the termination of the reaction, it was cooled again with ice, 100 ml of 6N hydrochloric acid was gradually added dropwise, and then extracted with 100 ml of diethyl ether twice. The organic layer was washed with 100 ml of water thrice and dried over anhydrous magnesium sulfate. The solvent was distilled off under a reduced pressure and the residue was recrystallized from n-heptane to obtain 13.6 g of 4-(3-n-butylbicyclo[1.1.1]pent-1-yl)dithiobenzoic acid.

### Second step

Under nitrogen gas stream, 13.6 g (49.2 mmol) of the dithiobenzoic acid derivative obtained in the first step was dissolved in 100 ml of diethyl ether and 0.3 ml of pyridine was added. At room temperature, 11.7 g (98.4 mmol) of thionyl chloride was added dropwise thereto. After the finishing of the dropping, it was heated under reflux for 10 hours. After the termination of the reaction, unreacted thionyl chloride and the solvent were distilled off under a reduced pressure to obtain 12.2 g of a dark purplish red oily product.

### Third step

In 50 ml of toluene were dissolved 13.1 g (54.1 mmol) of 3,5-difluoro-4-(3,4-difluorophenyl)phenol and 8.6 g (108.2 mmol) of pyridine, and a solution of 12.2 g (43.7 mmol) of the 4-(3-n-butylbicyclo[1.1.1]pent-1-yl)dithiobenzoic acid chloride obtained in the second step described above in 25 ml of toluene was added dropwise at room temperature while being stirred. After the finishing of the dropping, it was stirred while maintaining at 65°C for 8 hours. Water in an amount of 100 ml was added to the reaction solution and extracted with 100 ml of toluene twice. The organic layer was washed with 100 ml 2N hydrochloric acid once, 150 ml of water twice, 100 ml of saturated aqueous sodium carbonate solution once, and then 150 ml of water twice in turn, and then dried over anhydrous magnesium sulfate. The solvent was distilled off under a reduced pressure, and the residue was subjected to silica gel column chromatography (eluent: n-heptane) and recrystallized from n-heptane to obtain 8 g of thione-O-ester derivative.

### Fourth step

Under nitrogen gas atmosphere, DBH was suspended in 50 ml of dichloromethane, cooled down to -60°C, 39.5 g of TBAH2F was added, and stirred for 5 min. A solution of 8 g (16.5 mmol) of the thione-O-ester derivative in 30 ml of dichloromethane was gradually added dropwise thereto, stirred at the same temperature for 2 hours, and then further stirred at room temperature for 24 hours.

The reaction liquid was put in 300 ml of saturated aqueous sodium carbonate solution to terminate the reaction and extracted with 100 ml of diethyl ether twice. The organic layer was washed with 150 ml of 10 weight % sodium sulfite and then 150 ml of water thrice, and dried over anhydrous magnesium sulfate. The solvent was distilled off under a reduced pressure, and the residue was subjected to silica gel column chromatography (eluent: n-heptane) and recrystallized from n-heptane to obtain 2.59 g of the subjective compound, 4-(3-n-butylbicyclo[1.1.1]pent-1-yl)-α,α-difluorobenzyl-(2,6,3',4'-tetrafluorobiphenyl-4-yl)ether. Results of determination of various kind of spectrums strongly supported the structure of the objective product.
GC-MS m/z 490 (M+)

### Example 5

### Preparation of 4''-(3-n-butylbicyclo[1.1.1]pent-1-yl)-2,3,3'-trifluoro-4-ethoxyterphenyl (Compound No. 600)

### First step

Under nitrogen gas stream, 24 g (0.50 mol) of sodium hydride (50 % oily state) was suspended in 50 ml of DMF, and a solution of 50 g (0.38 mol) of 2,3-difluorophenol in 200 ml of DMF was gradually added dropwise thereto. After the finishing of the dropping, the suspension was gradually heated and 105.7 g (0.76 mol) of ethyl iodide was added dropwise at about 50°C. After the finishing of the dropping, it was heated under reflux for 20 hours. After the termination of the reaction, 500 ml of water was added dropwise under cooling with ice, extracted with 150 ml of n-heptane twice, washed with 200 ml of water thrice, and dried over anhydrous magnesium sulfate.

The solvent was distilled off under a reduced pressure, and recrystallized from n-heptane to obtain 40.8 g (0.25 mol) of 2,3-difluoroethoxybenzene. This compound was dissolved in 300 ml of THF and cooled down to -60°C under a refrigerant. sec-Butyl lithium (1.05M, cyclohexane solution) in an amount of 370 ml was gradually added dropwise thereto. After the finishing of the dropping, it was stirred at the same temperature for 2 hours, and further a solution of 52 g of trimethyl borate in 150 ml of THF was added dropwise. After the finishing of the dropping, the temperature of the solution was allowed to rise back to room temperature and stirred for 24 hours. After the termination of the reaction, it was again cooled with ice, 500 ml of 3N hydrochloric acid was added dropwise, extracted with 300 ml of toluene twice, washed with 500 ml of water thrice, and dried over anhydrous magnesium sulfate. The solvent was distilled off under a reduced pressure, and the residue was thoroughly washed with n-heptane to obtain 23,.4 g of 2,3-difluoro-4-ethoxyphenyl boric acid.

### Second step

Under nitrogen gas stream, 19.8 g (89.1 mmol) of 3-fluoroiodobenzene, 23.4 g (115.8 mmol) of 2,3-difluoro-4-ethoxyphenyl boric acid, and 1.3 g of palladium/carbon catalyst were suspended in 300 ml of THF, and heated under reflux for 8 hours. After it was put in 600 ml of water and extracted with 300 ml of toluene twice. The organic layer was washed with 300 ml of water thrice and dried over anhydrous magnesium sulfate. The solvent was distilled off under a reduced pressure, and the residue was subjected to silica gel column chromatography (eluent: n-heptane) and recrystallized from n-heptane to obtain 14.5 g of 2,3,3'-trifluoro-4-ethoxybiphenyl.

### Third step

Under nitrogen gas stream, 14.5 g (57.9 mmol) of 2,3,3'-trifluoro-4-ethoxybiphenyl was dissolved in 250 ml of THF and cooled down to -30°C under a refrigerant. sec-Butyl lithium (1.05M, cyclohexane solution) in an amount of 66 ml (69.5 mmol) was gradually added dropwise thereto. After the finishing of the dropping, it was stirred at the same temperature for 2 hours, and a solution of 21.8 g of triisopropyl borate in 80 ml of THF was further added dropwise. After the finishing of the dropping, the temperature of the solution was allowed to rise back to room temperature and stirred for 24 hours. After the termination of the reaction, it was again cooled with ice, 200 ml of 3N hydrochloric acid was added dropwise, extracted with 200 ml of toluene, washed with 300 ml of water thrice, and dried over anhydrous magnesium sulfate. The solvent was distilled off under a reduced pressure, and the residue was thoroughly washed with n-heptane to obtain 11 g of 2,3,3'-trifluoro-4-ethoxy-4'-boric acid biphenyl.

### Fourth step

Under nitrogen gas atmosphere, 11 g (37.3 mmol) of 2,3,3'-trifluoro-4-ethoxy-4'-boric acid biphenyl, 8.7 g (31.1 mmol) of the 4-(3-n-butylbicyclo[1.1.1]pent-1-yl)bromobenzene obtained in the procedures in the second step of Example 1, and 0.6 g of 5 weight % palladium/carbon catalyst were suspended in 200 ml of mixed solvent of toluene/Solmix/water = 1/1/1, and heated under reflux for 10 hours. After the termination of the reaction, the palladium/carbon catalyst was filtered off and the reaction product was extracted with 150 ml of toluene twice. The organic layer was washed with 300 ml of water thrice and dried over anhydrous magnesium sulfate. The solvent was distilled off under a reduced pressure, and the residue was subjected to silica gel column chromatography (eluent: n-heptane) and recrystallized from n-heptane to obtain 3.3 g of the subject compound, 4''-(3-n-butylbicyclo[1.1.1]pent-1-yl)-2,3,3'-trifluoro-4-ethoxyterphenyl. Results of determination of various kind of spectrums strongly supported the structure of the objective product.
GC-MS m/z 433 (M+)

### Example 6

### Preparation of 4-(4-(3-(3-pentenylbicyclo[1.1.1]pent-1-yl)benzoyloxy)-2,6-difluorobenzonitrile (Compound No. 301)

### First step

Under nitrogen gas atmosphere, 10 g (49.7 mmol) of 4-bromobenzoic acid was suspended in 80 ml of dichloromethane under cooling with ice, and 18.5 g (89.5 mmol) of DCC was added at a time. After the suspension was stirred at the same temperature for 30 min, 7.7 g (49.7 mmol) of 4-hydroxy-2,6-difluorobenzonitrile and 0.6 g (4.97 mmol) of DMAP were added in turn, stirred at the same temperature for 1 hour, and further stirred at room temperature for 24 hours. After the termination of the reaction, the precipitates were filtered off, the solvent was distilled off under a reduced pressure and the product was extracted with 100 ml of diethyl ether. The organic layer was washed with 150 ml of water thrice and dried over anhydrous magnesium sulfate. The solvent was distilled off under a reduced pressure, and the residue was subjected to silica gel column chromatography (eluent: toluene) to obtain 11.6 g of the objective compound, 4-(4-bromobonzoyloxy)-2,6-difluorobenzonitrile.

### Second step

Under nitrogen atmosphere, 20 g (0.29 mol) of [1.1.1]-propellane was dissolved in 100 ml of diethyl ether and cooled down to -60°C under a refrigerant. Methyl lithium (1.0M, diethyl ether solution) in an amount of 523 ml (0.53 mol) was added dropwise thereto and stirred at the same temperature for 2 hours. Then, 80 g (0.44 mol) of 3-pentenyliodide was added dropwise at the same temperature. After the finishing of the dropping, it was gradually warmed, and stirred at room temperature for 6 hours. After the termination of the reaction, the product was put in 300 ml of water, extracted with 50 ml of diethyl ether twice, washed with 100 ml of water thrice, and dried over anhydrous magnesium sulfate. The solvent was distilled off under a reduced pressure, and the residue was subjected to silica gel column chromatography (eluent: n-heptane) and concentrated under a reduced pressure to obtain 49.9 g of an oily product.

### Third step

Under nitrogen gas atmosphere, 17.2 g (68.7 mmol) of the iodide derivative obtained in the second step was dissolved in 100 ml of THF and cooled down to -30°C under cooling with ice. Then, 69 ml (103 mmol) of t-butyl lithium (1.5M, n-pentane solution) was added thereto, stirred at the same temperature for 20 min, further cooled down to -50°C, and stirred for 1 hour. Zinc chloride (0.5M, THF solution) in an amount of 206 ml was added dropwise, stirred at the same temperature for 1 hour, and further stirred at room temperature for 1 hour. Then, 21.2 g of NiCl₂ (dppe) was added thereto, a solution of 11.6 g (34.3 mmol) of 4-(4-bromobenzoyloxy)-2,6-difluorobenzonitrile in 50 ml of THF was added dropwise, and heated under reflux for 3 hours. The product was put in 500 ml of water and extracted with 100 ml of toluene twice. The organic layer was washed with 200 ml of water thrice and dried over anhydrous magnesium sulfate. The solvent was distilled off under a reduced pressure, and the residue was subjected to silica gel column chromatography (eluent: n-heptane/ethyl acetate = 4/1) and recrystallized from ethanol twice to obtain 5.2 g of the subject compound, 4-(4-(3-(3-pentenylbicyclo[1.1.1]pent-1-yl)benzoyloxy)-2,6-difluorobenzonitrile. Results of determination of various kind of spectrums strongly supported the structure of the objective product.
GC-MS m/z 379 (M+)

### Example 7

### Preparation of (E)-1-(trans-4-(3-n-propylbicyclo[1.1.1]-pent-1-yl)cyclohexyl)-2-(trans-4-pentylcyclohexyl)ethene (Compound No. 349)

### First step

Under nitrogen gas stream, 30 g (127 mmol) of 1-iodo-3-n-propylbicyclo[1.1.1]pentane was dissolved in 150 ml of THF and cooled down to -60°C. Then, 112 ml of t-butyl lithium (1.7M, pentane solution) was added dropwise thereto and stirred at the same temperature for 1 hour. A solution of 19.8 g (127 mmol) of 1,4-cyclohexanedione monoethylene ketal in 80 ml of THF was added dropwise while being maintained at a temperature lower than -60°C. After the finishing of the dropping, the reaction temperature was gradually raised up to room temperature, and further stirred for 5 hours. The reaction solution was subjected to Celite filtration, the solvent was distilled off under a reduced pressure, and the residue was subjected to silica gel column chromatography (eluent: toluene), and concentrated under a reduced pressure. Further, this product was dissolved in 100 ml of pyridine while being cooled with ice under nitrogen gas atmosphere, 25.4 g (165.2 mmol) of phosphorus oxychloride was added dropwise thereto. After the finishing of the dropping, it was gradually warmed up to room temperature and stirred for 48 hours. After the termination of the reaction, the solution was put in 300 ml of water and extracted with 100 ml of diethyl ether twice. The organic layer was washed with 200 ml of 2N aqueous ammonium chloride solution twice, further washed with 200 ml of water thrice, and dried over anhydrous magnesium sulfate. The solvent was distilled off under a reduced pressure, and the residue was subjected silica gel column chromatography (eluent: n-heptane) and concentrated under a reduced pressure to obtain 13.9 g of an oily product. This product was dissolved in 150 ml of Solmix, 1.4 g of 5 weight % palladium/carbon catalyst was added thereto, and stirred at room temperature under a condition of hydrogen pressure of 1 to 2 kg/cm² for 6 hours. After the catalyst was filtered off, the solvent was distilled off under a reduced pressure, and the residue was subjected to silica gel column chromatography (eluent: n-heptane) and concentrated under a reduced pressure to obtain 9.1 g of 4-(3-n-propylbicyclo[1.1.1]pent-1-yl)cyclohexanone ethylene ketal.

### Second step

Under nitrogen gas stream, 9.1 g (36.4 mmol) of 4-(3-n-propylbicyclo[1.1.1]pent-1-yl)cyclohexanone ethylene ketal was dissolved in 60 ml of toluene, 3.3 g of formic acid was added thereto, and heated under reflux for 2 hours. After the termination of the reaction, the solution was put in 150 ml of water, extracted with 60 ml of toluene twice, washed with 100 ml of 10 % by weight of aqueous sodium carbonate solution twice, further washed with 150 ml of water thrice, and dried over anhydrous magnesium sulfate. The solvent was distilled off under a reduced pressure, and the residue was subjected to silica gel column chromatography (eluent: toluene) and concentrated under a reduced pressure to obtain 5.25 g of 4-(3-n-propylbicyclo[1.1.1]pent-1-yl)cyclohexanone.

### Third step

Under nitrogen gas stream, 10.5 g (30.6 mmol) of methoxymethyltriphenylphosphonium chloride was dissolved in 50 ml of THF and cooled down to -10°C. Then, 5.25 g (25.5 mmol) of 4-(3-n-propylbicyclo[1.1.1]pent-1-yl)cyclohexanone was added thereto and stirred at the same temperature for 2 hours. Further, 3.4 g of potassium t-butoxide was added at a time and stirred at -10°C for 2 hours. After the termination of the reaction, 100 ml of water was added thereto and the product was extracted with 50 ml of toluene twice. The organic layer was washed with 100 ml of water thrice and dried over anhydrous magnesium sulfate. The solvent was distilled off under a reduced pressure, and the residue was subjected to silica gel column chromatography (eluent: toluene) and further concentrated under a reduced pressure to obtain 3.65 g of an oily product. This roughly purified compound in an amount of 3.65 g (15.3 mmol) was dissolved in 40 ml of acetone under cooling with ice, 10 ml of 6N aqueous hydrochloric acid solution was added dropwise. After the finishing of the dropping, the solution was stirred at room temperature for 16 hours. The solvent was distilled off under a reduced pressure and the product was extracted with diethyl ether. The organic layer was washed with saturated aqueous sodium carbonate solution and water each twice in turn, and dried over anhydrous magnesium sulfate. The solvent was distill off under a reduced pressure, and the residue was subjected to silica gel column chromatography (eluent: toluene) and concentrated under a reduced pressure to obtain 2.29 g of 4-(3-n-propylbicyclo[1.1.1]pent-1-yl)cyclohexylcarbaldehyde.

### Fourth step

Under nitrogen gas stream, 5.3 g (10.4 mmol) of the 4-pentylcyclohexylmethyltriphenyiphosphonium bromide obtained by the method described in patent publication (Japanese patent Publication No. Hei 6-62462) was dissolved in 30 ml of toluene and cooled down to -10°C under a refrigerant. Then, 2.29 g (10.4 mmol) of 4-(3-n-propylbicyclo[1.1.1]pent-1-yl)cyclohexylcarbaldehyde was added dropwise thereto and stirred at the same temperature for 2 hours. Further, 1.4 g of potassium t-butoxide was added at a time and stirred at -10°C for 2 hours. After the termination of the reaction, the product was put in 50 ml of water and extracted with 50 ml of toluene twice. The organic layer was washed with 100 ml of water thrice and dried over anhydrous magnesium sulfate. The solvent was distilled off under a reduced pressure, and the residue was subjected to silica gel column chromatography (eluent: n-heptane) to obtain 2.16 g of a crude (Z)-1-(trans-4-(3-n-propylbicyclo[1.1.1]pent-1-yl)cyclohexyl)-2-(trans-4-pentylcyclohexyl)ethene. Since ethenyl group in this compound was in cis-configuration, the compound was isomerized by the following method.

### Fifth step

Under nitrogen gas atmosphere, 2.6 g of metachloroperbenzoic acid and 1.5 g of potassium carbonate were suspended in 10 ml of dichloromethane and cooled down to 10°C. Then, a solution of 2.16 g (5.83 mmol) of the (Z)-1-(trans-4-(3-n-propylbicyclo[1.1.1]pent-1-yl)cyclohexyl)-2-(trans-4- pentylcyclohexyl)ethene obtained in the fourth step described above in 10 ml of dichloromethane was added dropwise thereto and stirred at room temperature for 10 hours. Subsequently, 30 ml of 10 % by weight of aqueous sodium thiosulfate solution was added to the reaction solution and stirred for 10 min. The product was extracted with 30 ml of diethyl ether twice. The organic layer was washed with saturated aqueous sodium carbonate solution and then saturated aqueous sodium chloride solution, and dried over anhydrous magnesium sulfate. The solvent was distilled off under a reduced pressure, and the residue was subjected to silica gel column chromatography (eluent: toluene) and concentrated under a reduced pressure to obtain 1.41 g of an oily product. This oily product in an amount of 1.41 g was dissolved in 10 ml of toluene, a solution of 2.2 g of dibromotriphenylphosphorane in 10 ml of toluene was added thereto and heated under reflux for 6 hours. After the termination of the reaction, the product was subjected to silica gel column chromatography (eluent: n-heptane), concentrated under a reduced pressure, and recrystallized from ethanol to obtain 1.32 g of erythro-1,2-dibromo-1-(trans-4-(3-propylbicyclo[1.1.1]pent-1-yl)cyclohexyl)-2-(trans-4-pentylcyclohexyl)ethane.

### Sixth step

Under nitrogen gas stream, 1.32 g (2.50 mmol) of erythro-1,2-dibromo-1-(trans-4-(3-propylbicyclo[1.1.1]pent-1-yl)-cyclohexyl)-2-(trans-4-pentylcyclohexyl)ethane was dissolved in 15 ml of acetic acid, 0.33 g of zinc was added thereto, and stirred at room temperature for 5 hours. The reaction liquid was put in 60 ml of water and extracted with 30 ml of ethyl acetate twice. The organic layer was washed with saturated aqueous sodium carbonate solution and then saturated aqueous sodium chloride solution in turn, and dried over anhydrous magnesium sulfate. The solvent was distilled off under a reduced pressure, and the residue was subjected to silica gel column chromatography (eluent: n-heptane) and recrystallized from n-heptane to obtain 0.39 g of the subject compound, (E)-1-(trans-4-(3-n-propylbicyclo[1.1.1]pent-1-yl)cyclohexyl)-2-(trans-4-pentylcyclohexyl)ethene. Results of determination of various kind of spectrums strongly supported the structure of the objective product.
GC-MS m/z 370 (M+)

The following compounds can be prepared by selecting known procedures in organic synthesis and using them in combination while using the methods described in the Examples described above as reference.

Value of NI point, Δε, Δn, and viscosity η (extrapolated values) of several examples of the liquid crystal compounds of the present invention are shown in Table 2.

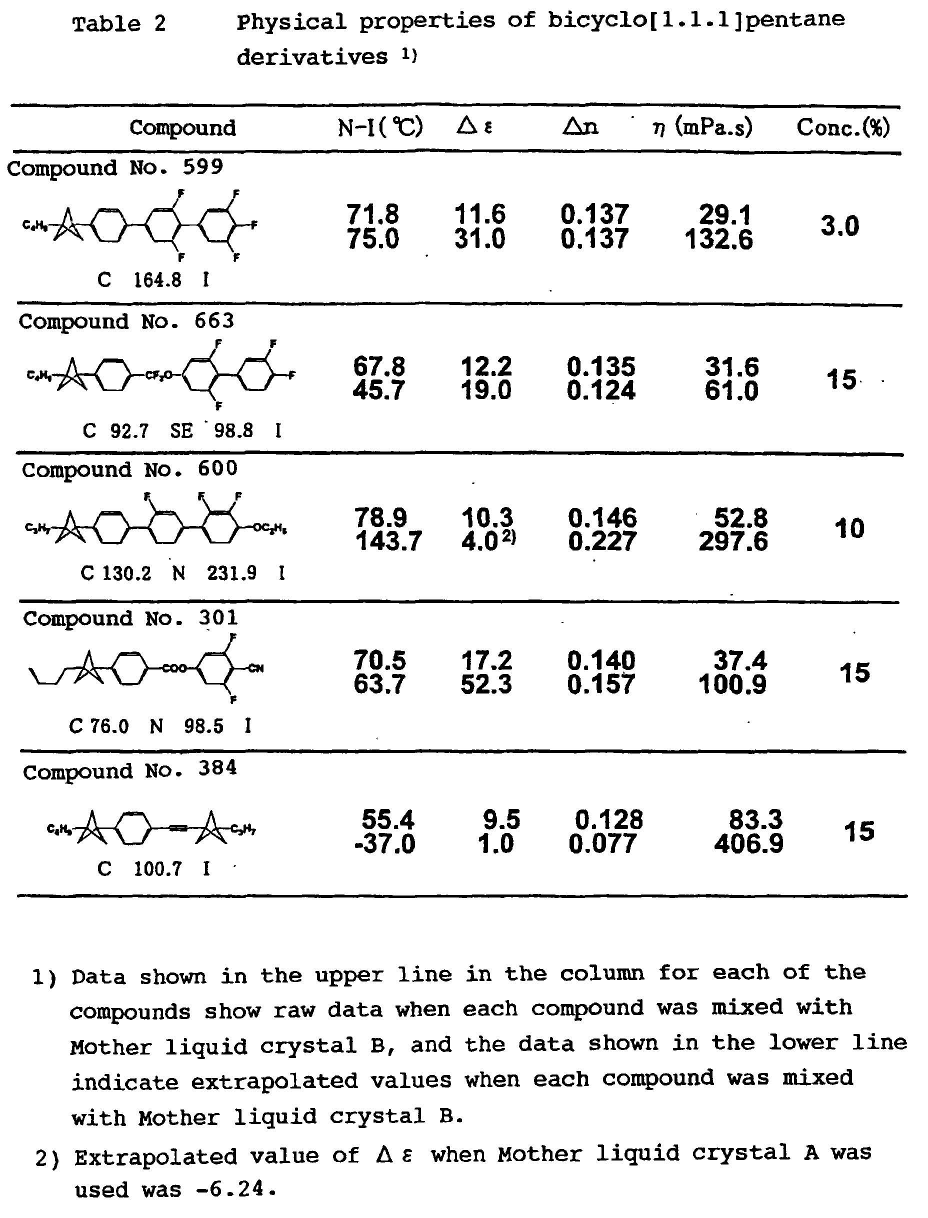

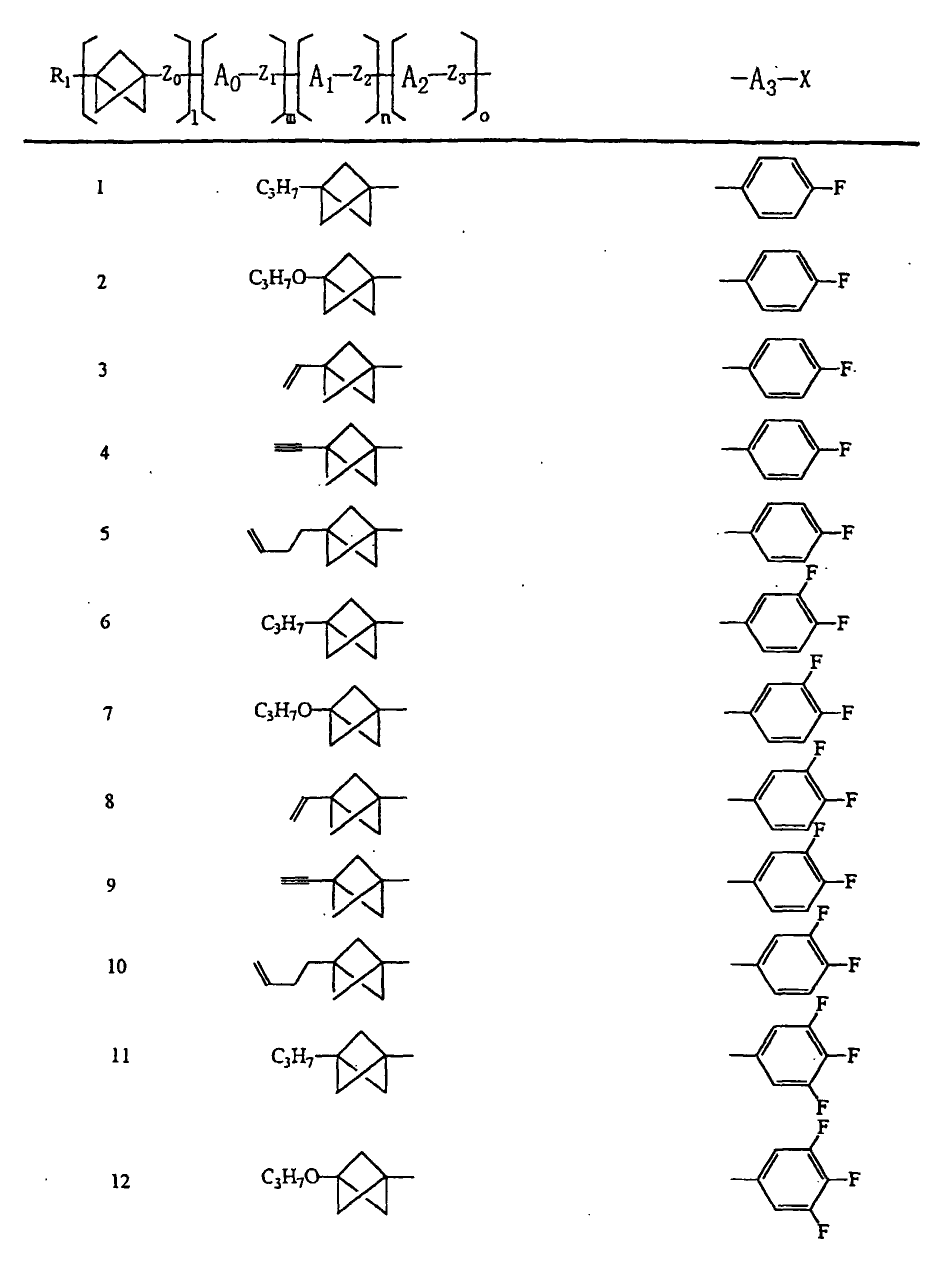

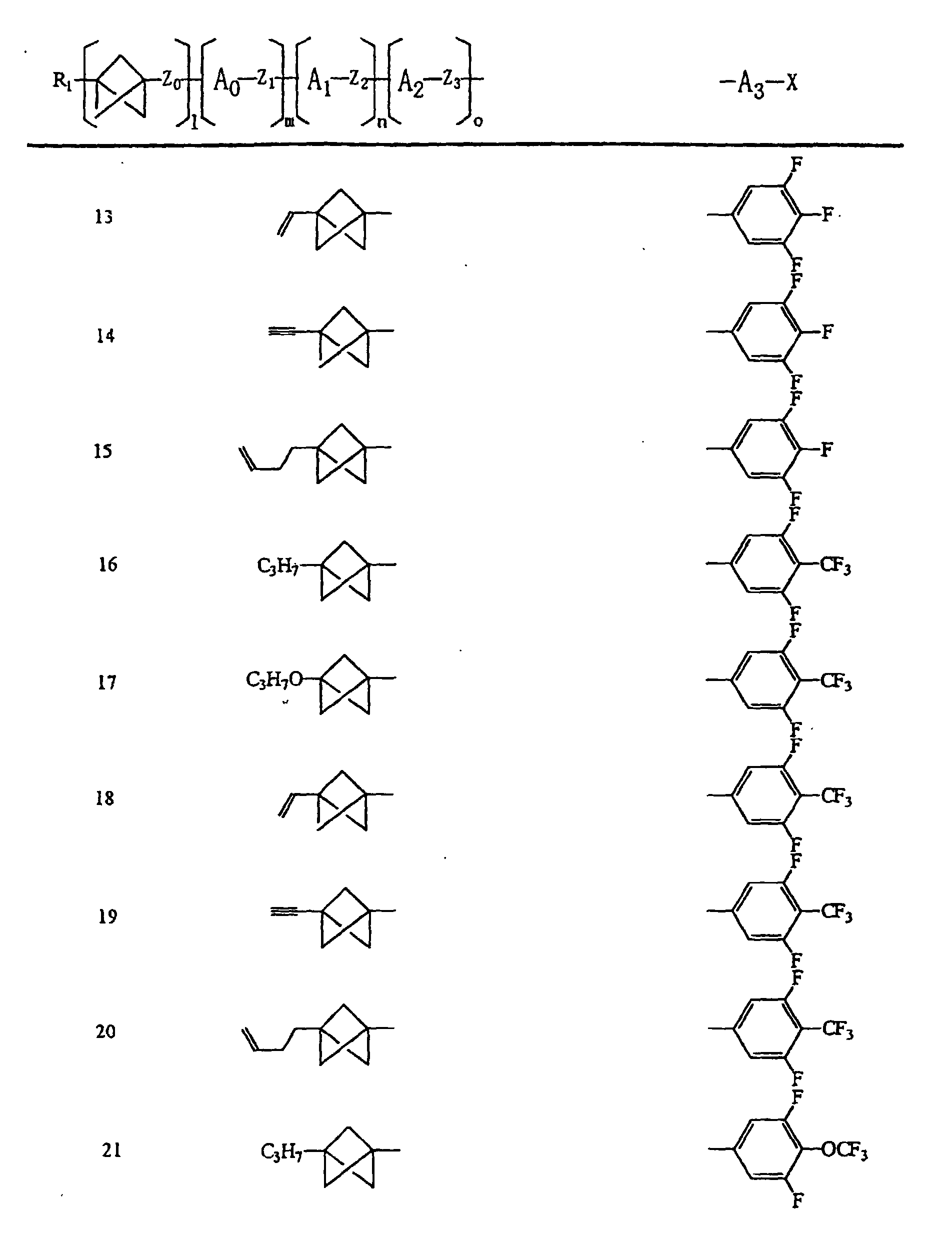

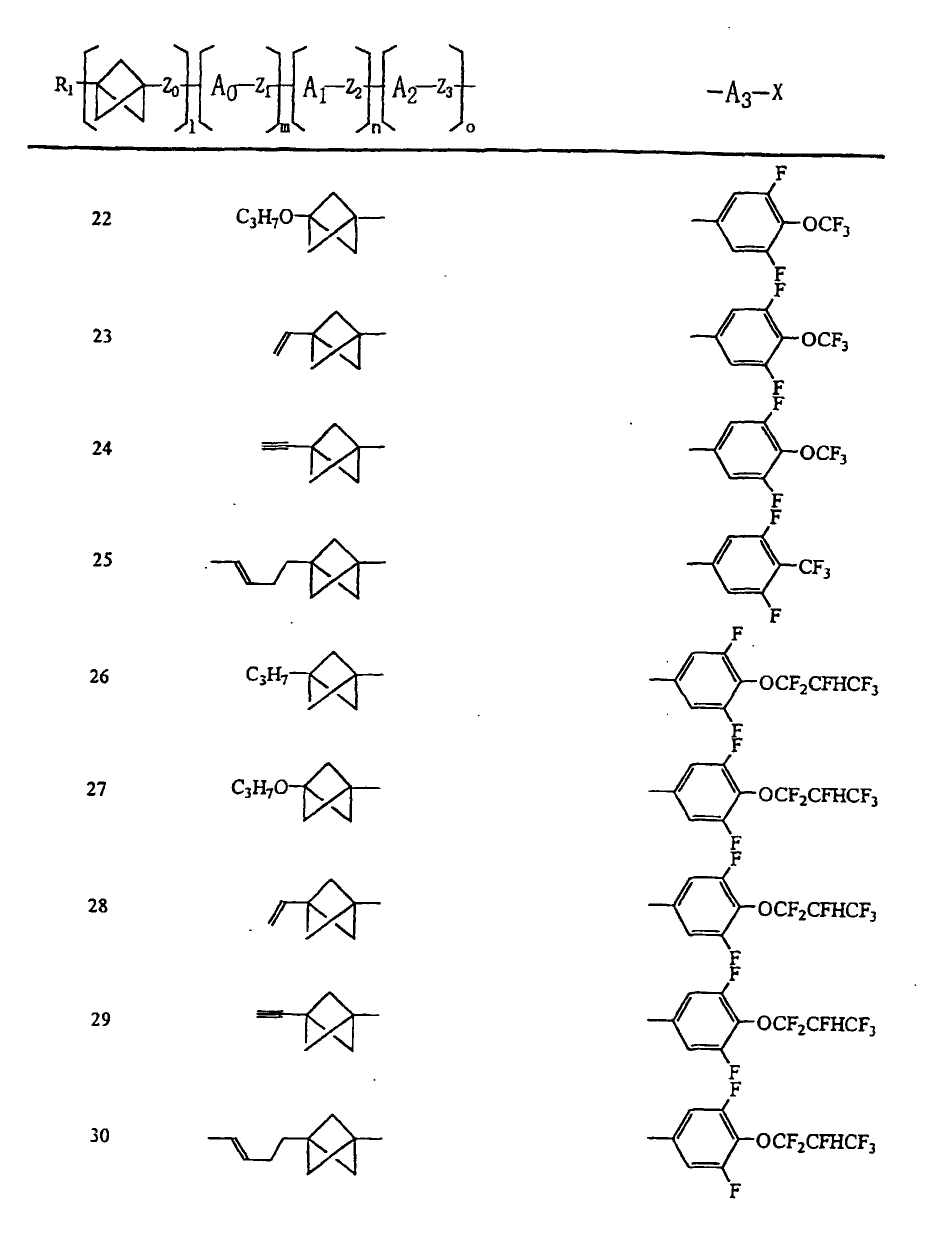

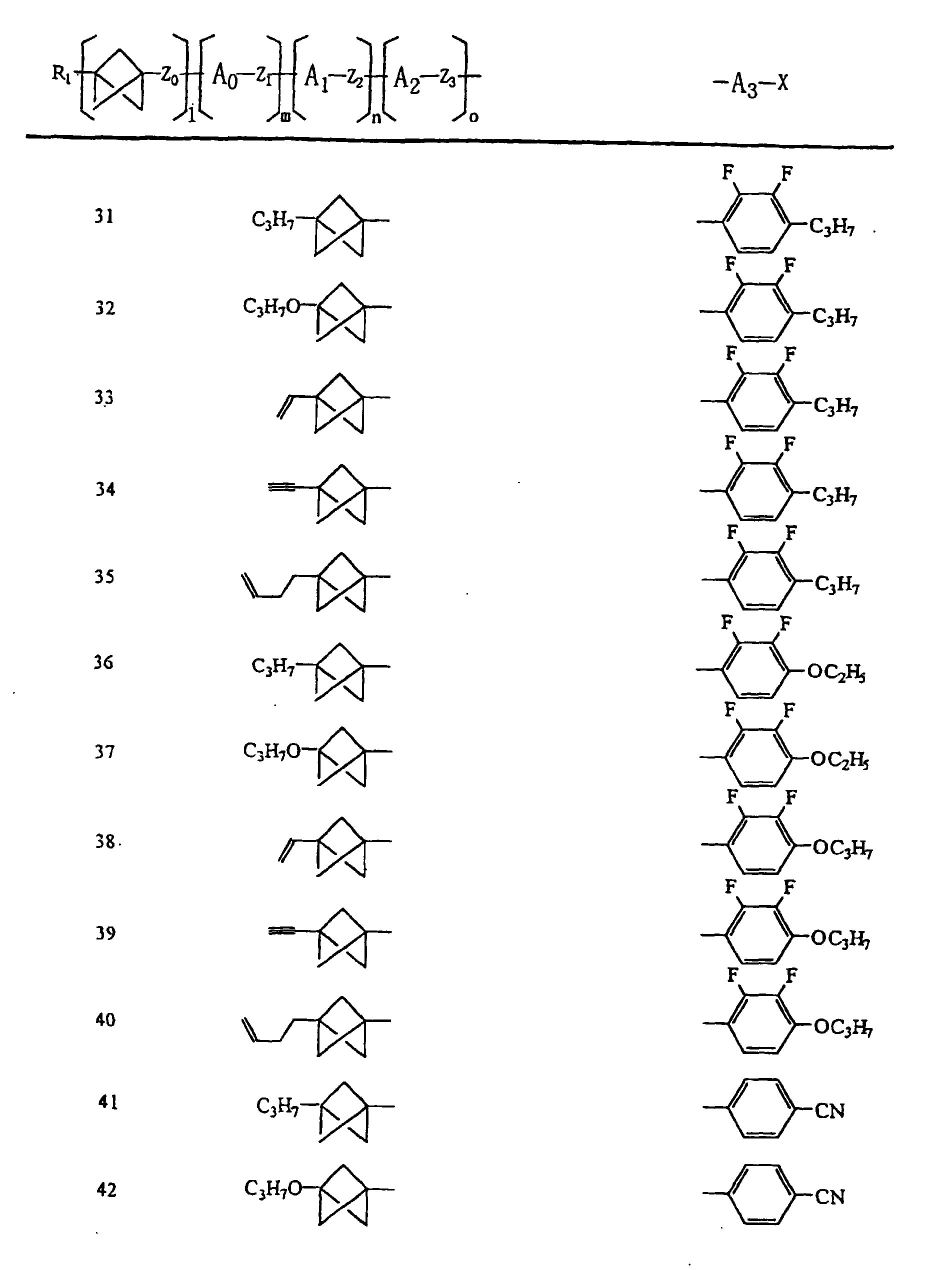

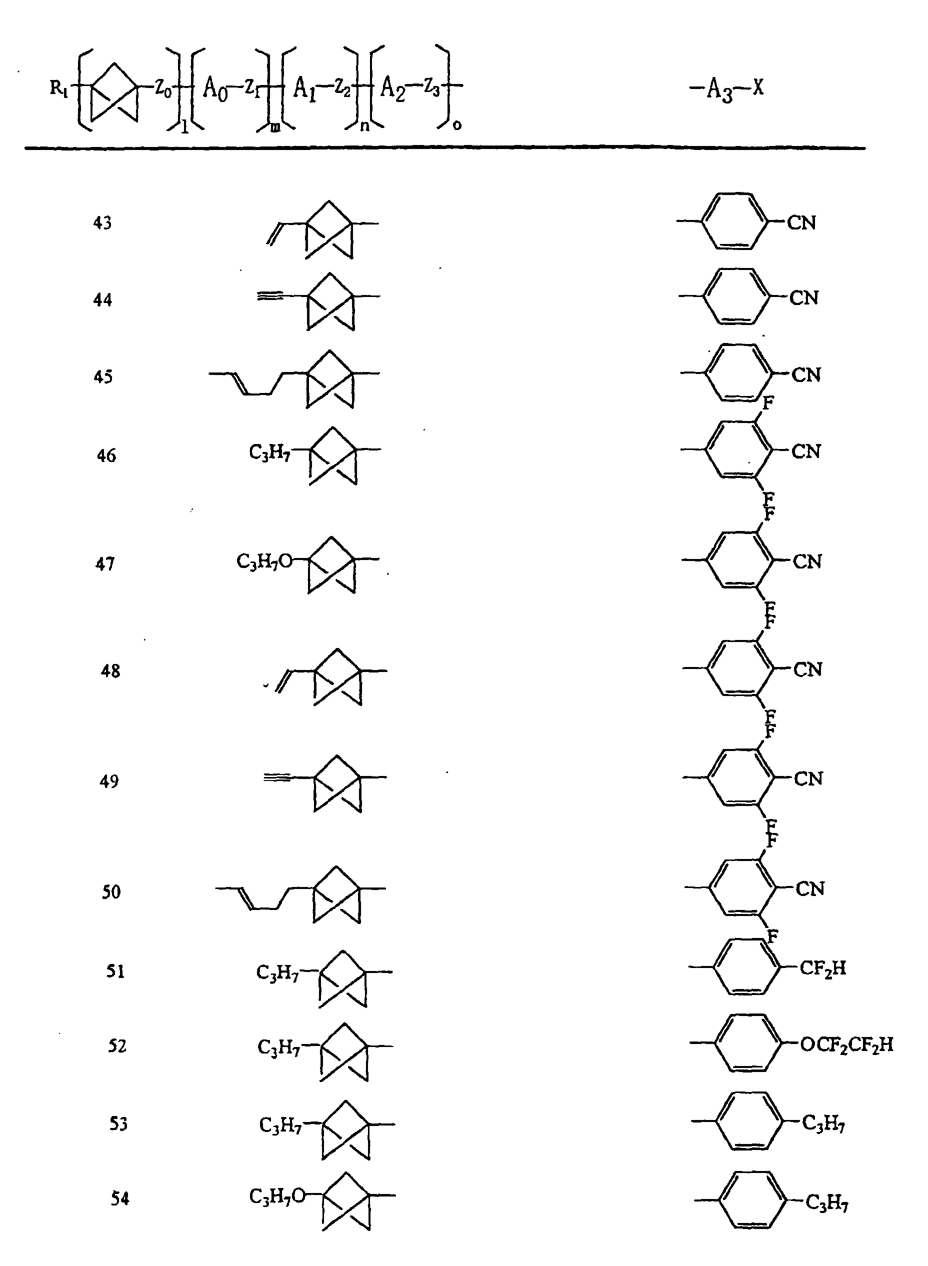

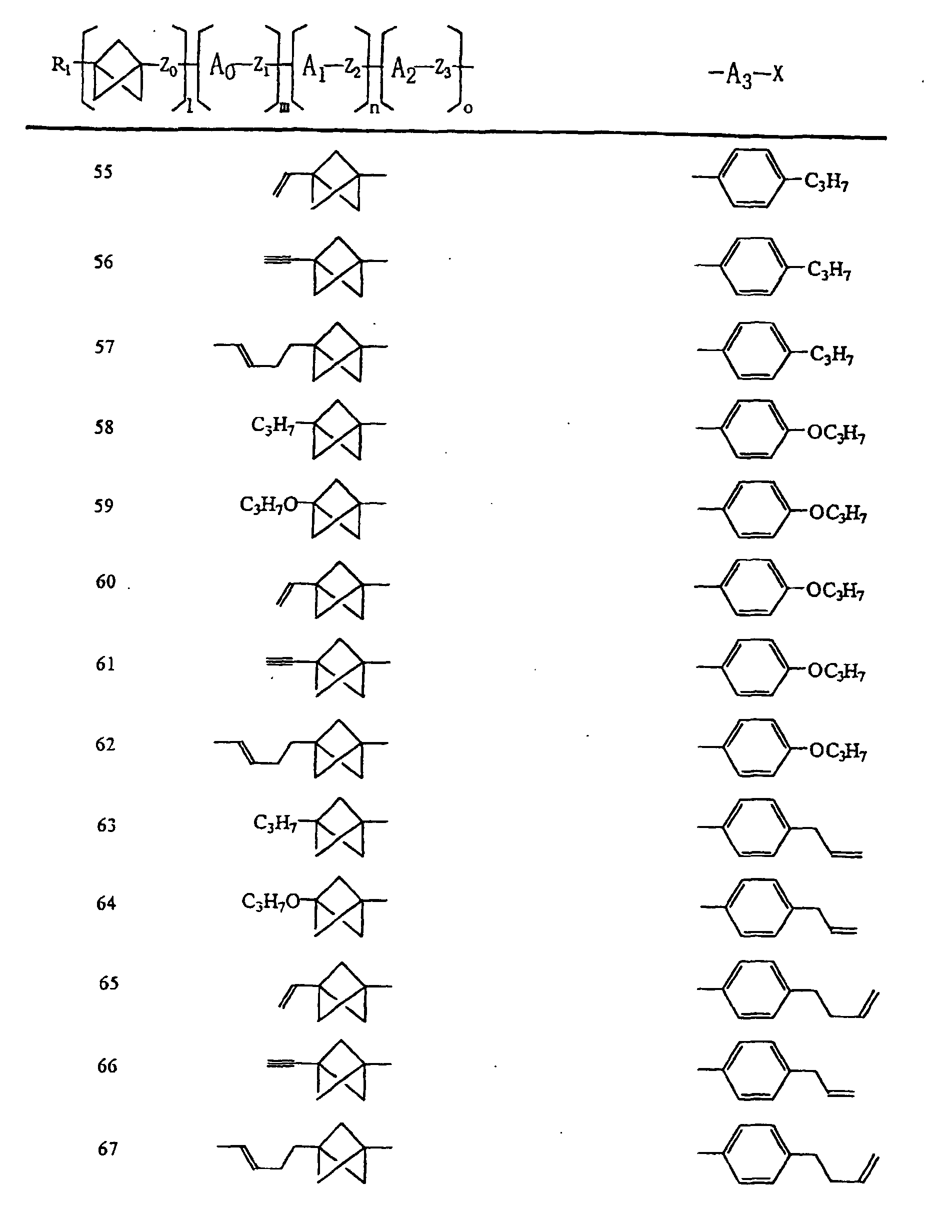

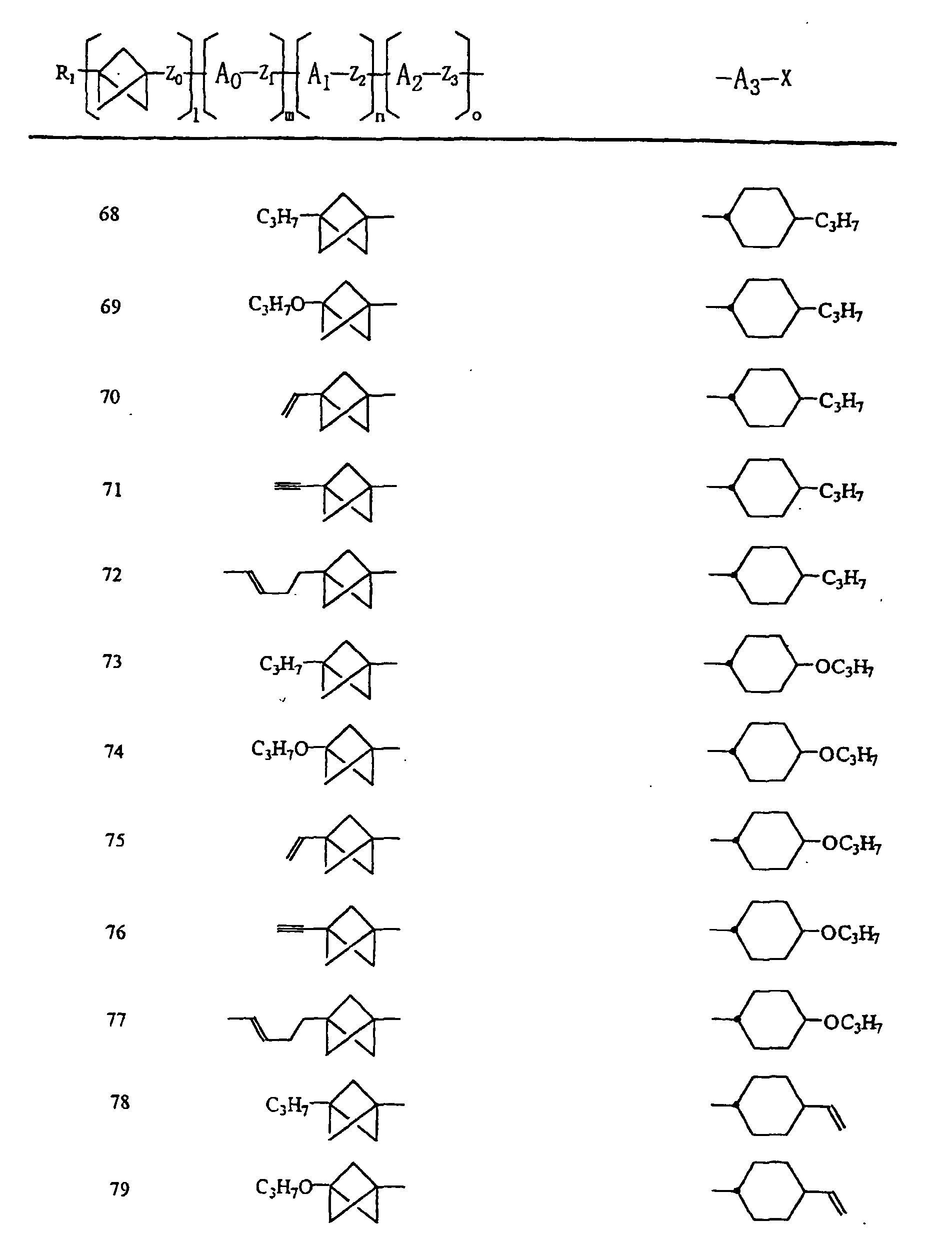

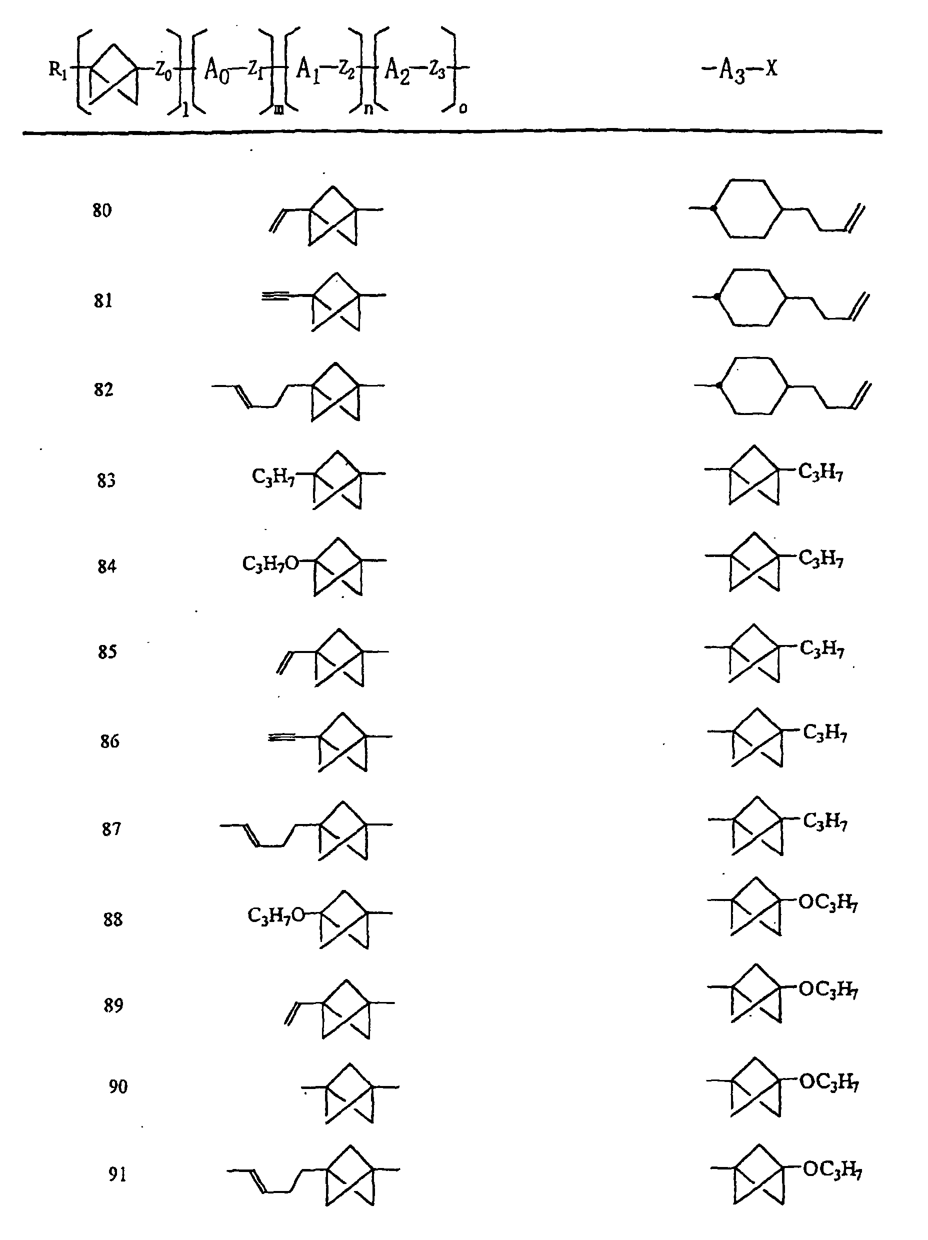

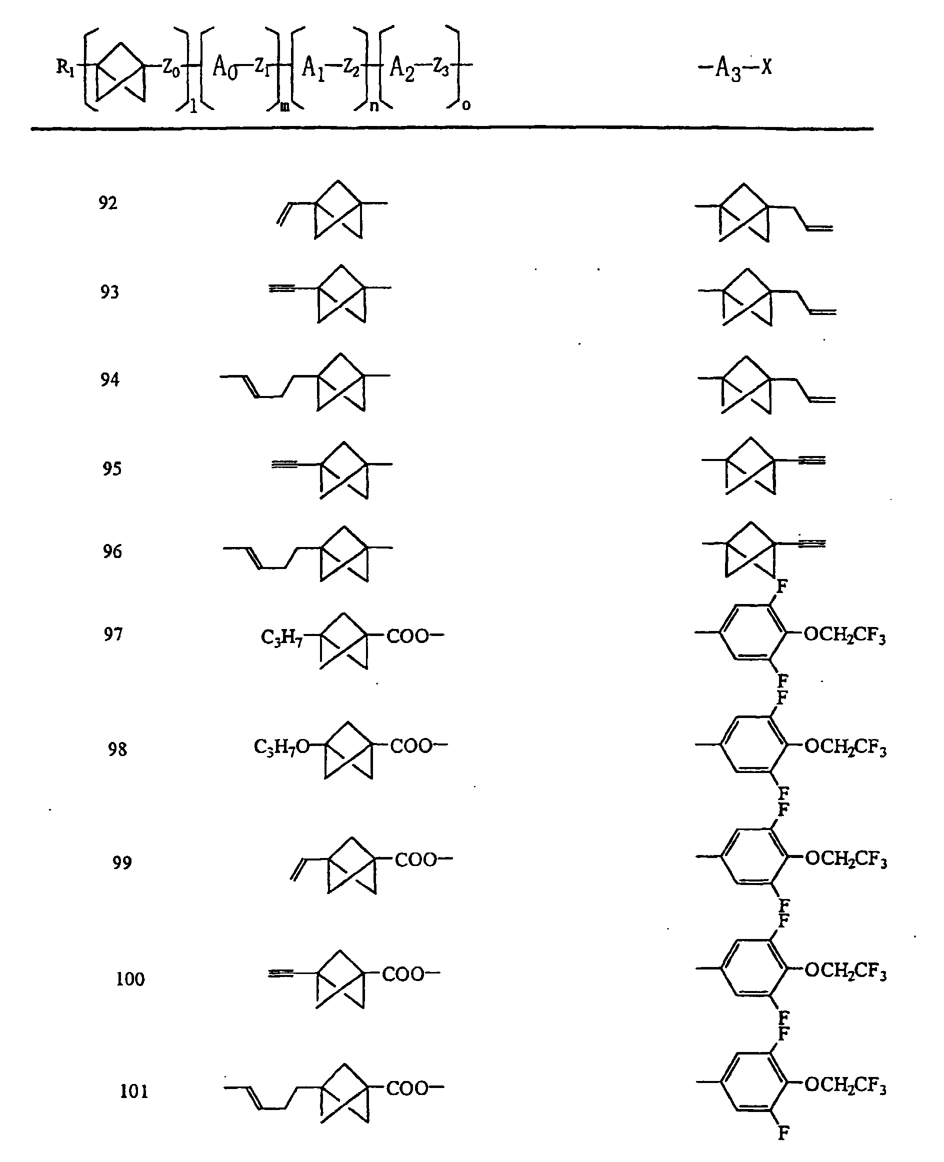

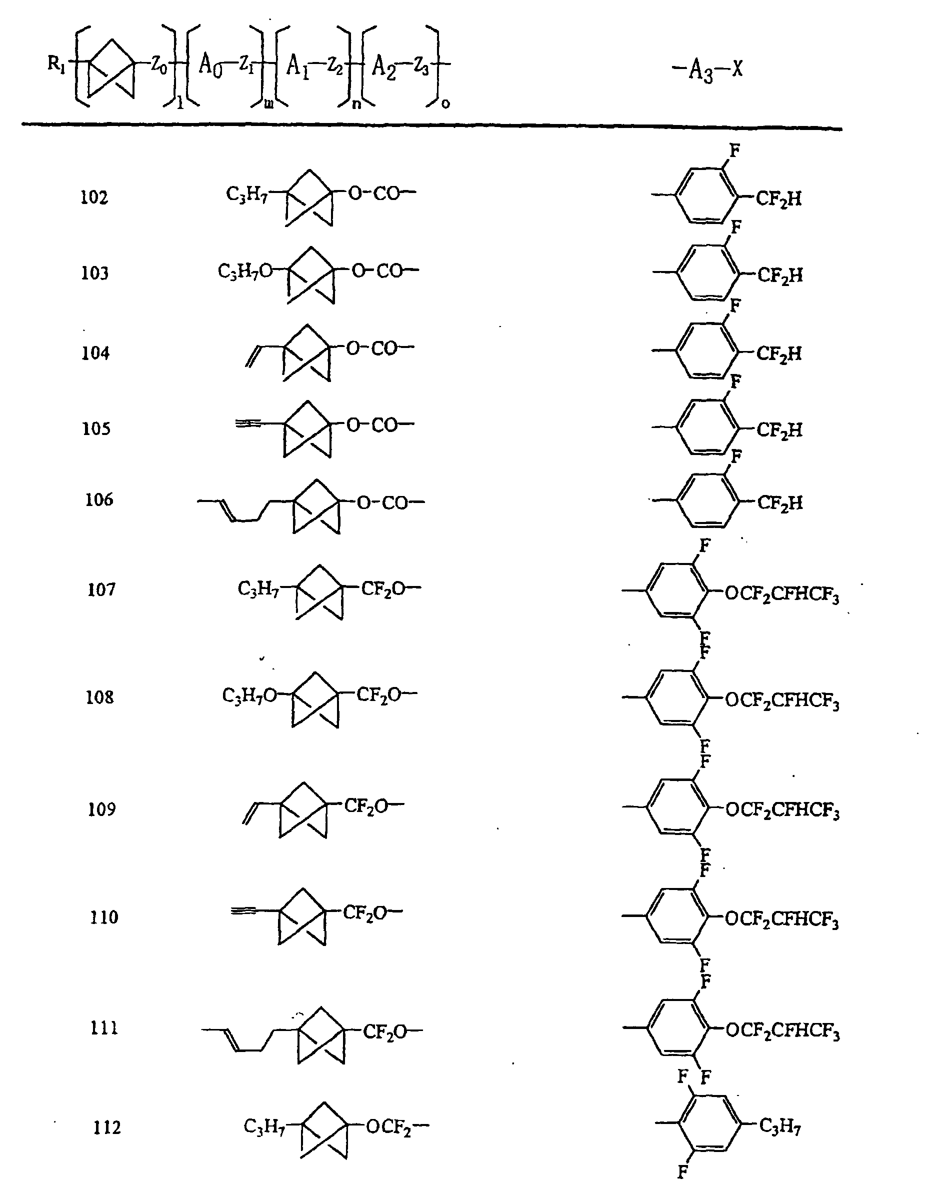

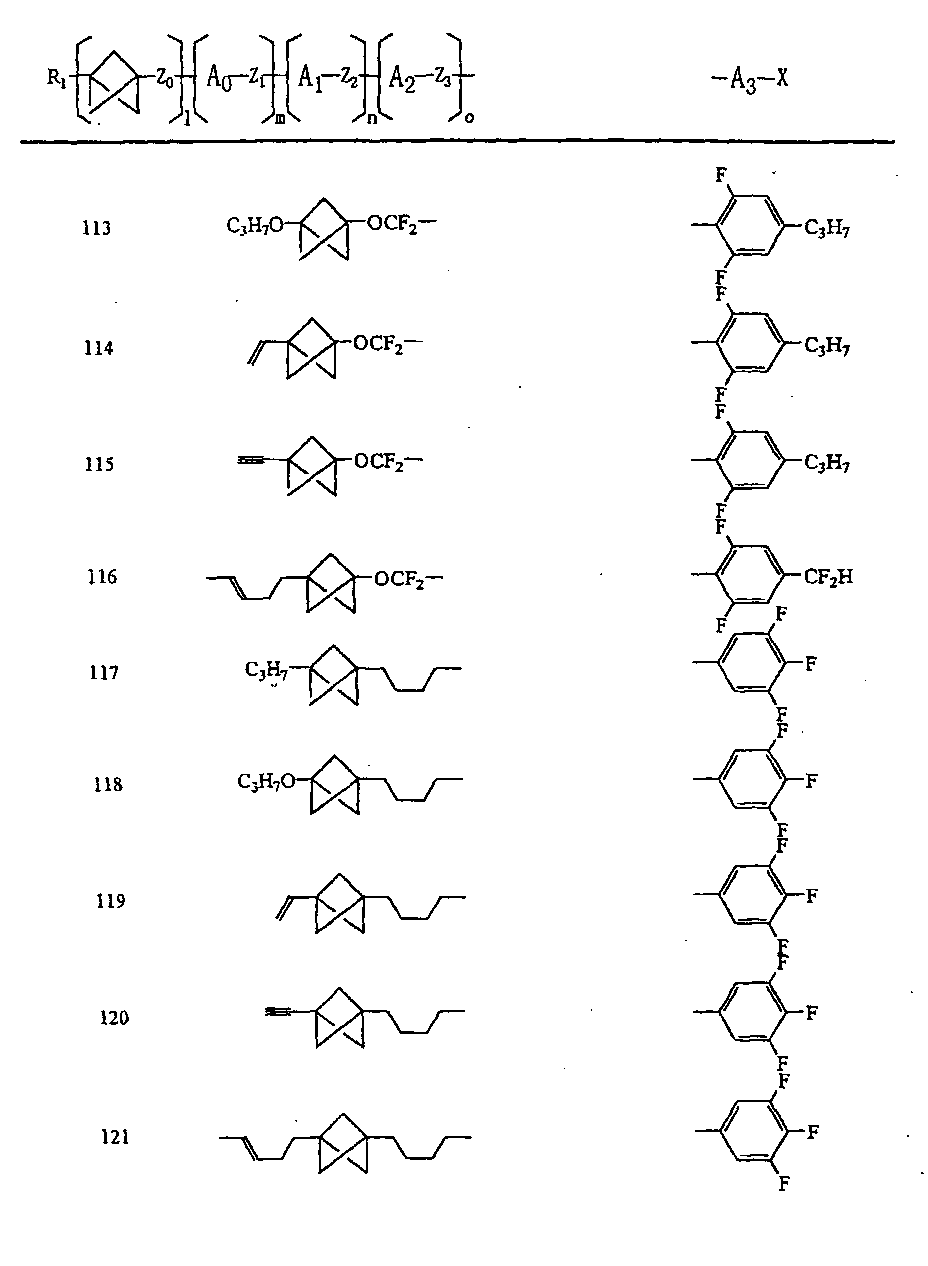

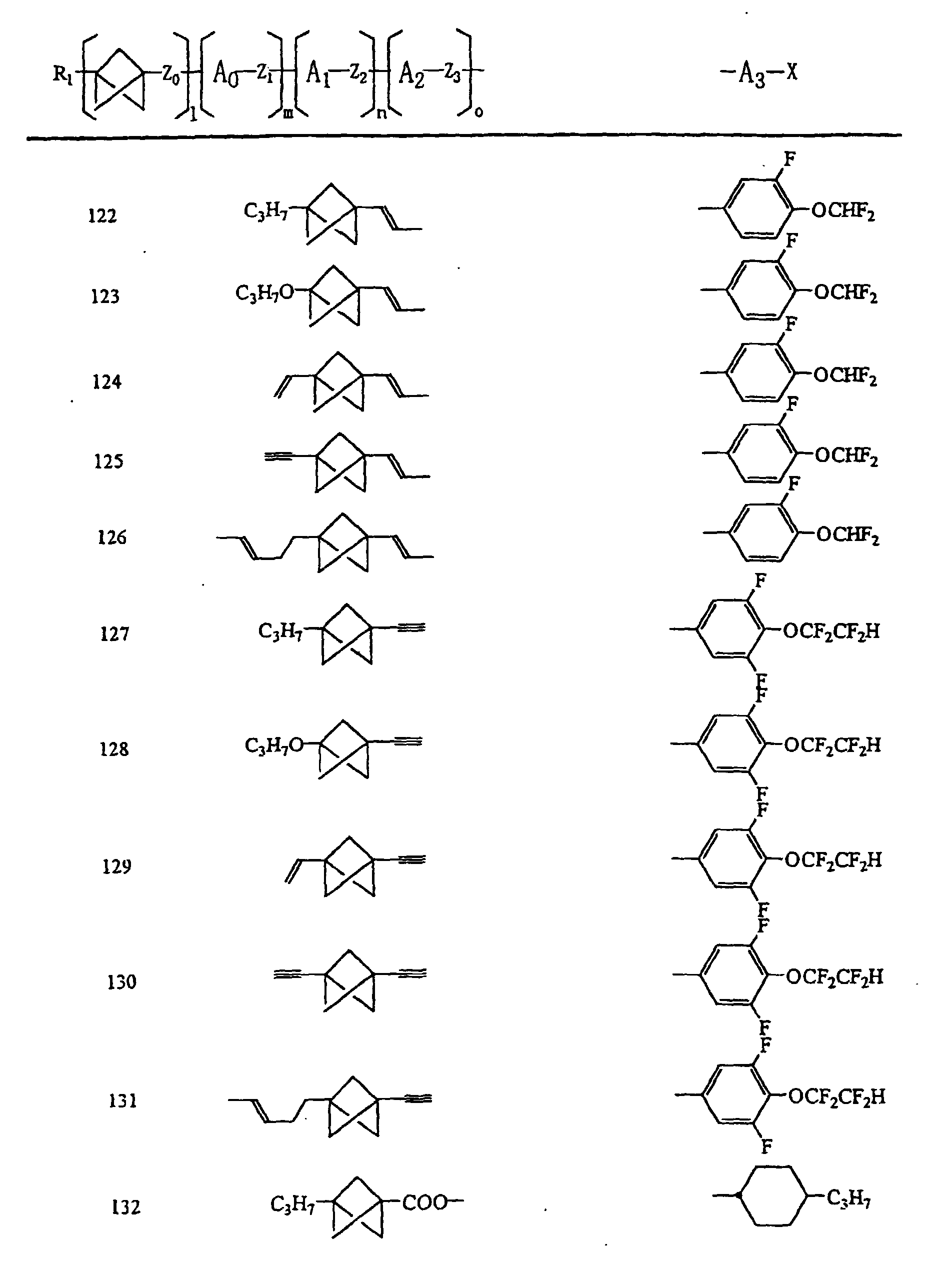

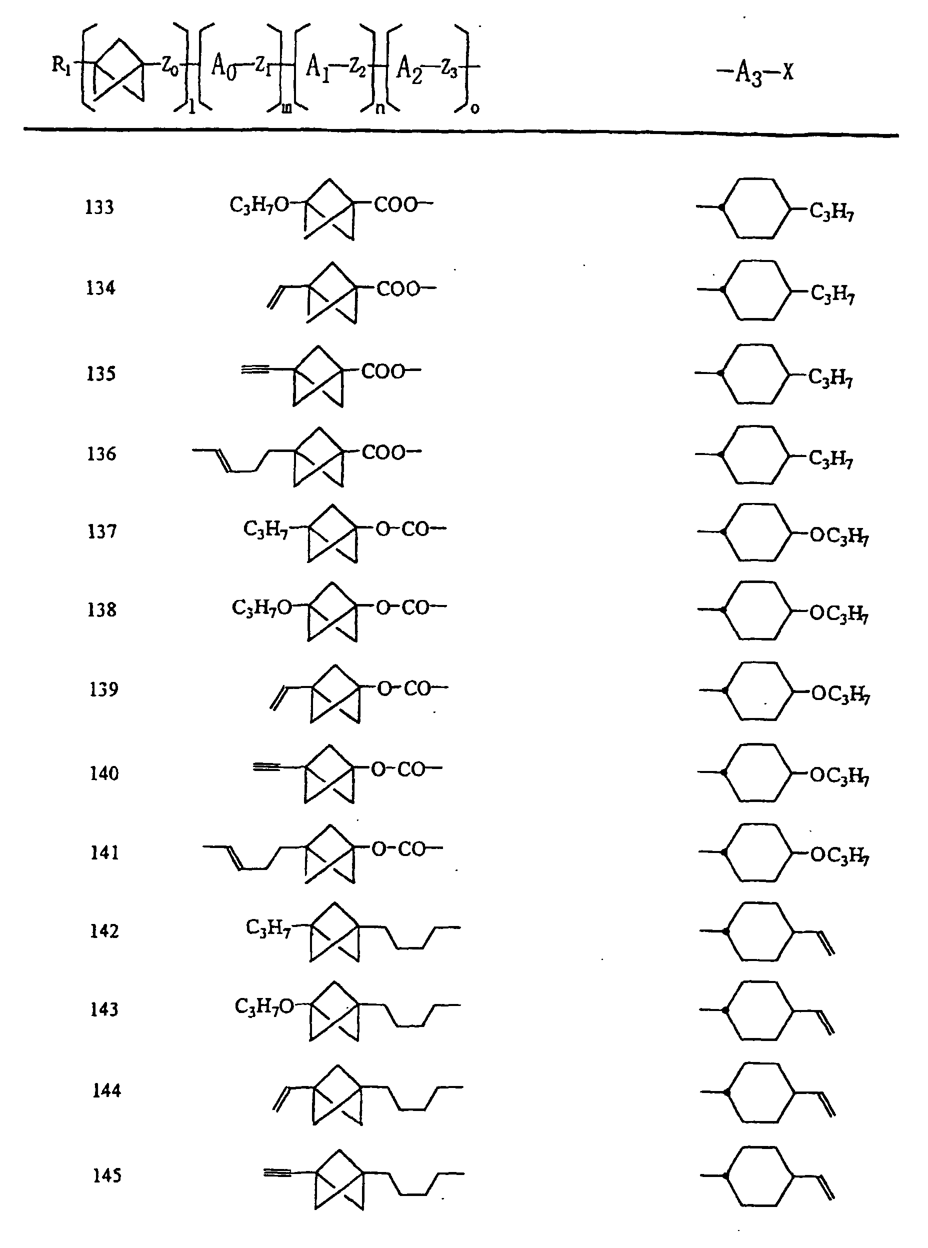

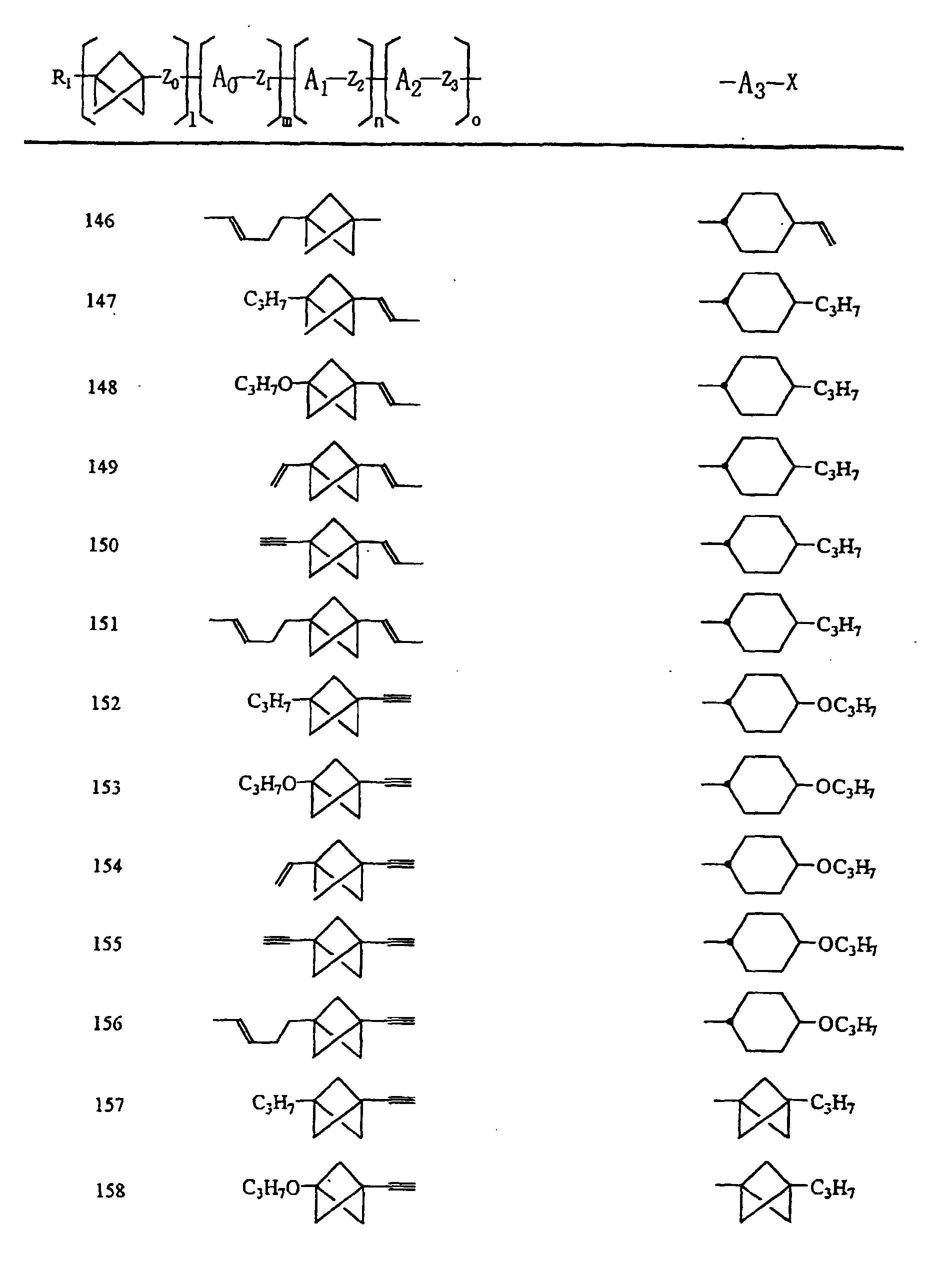

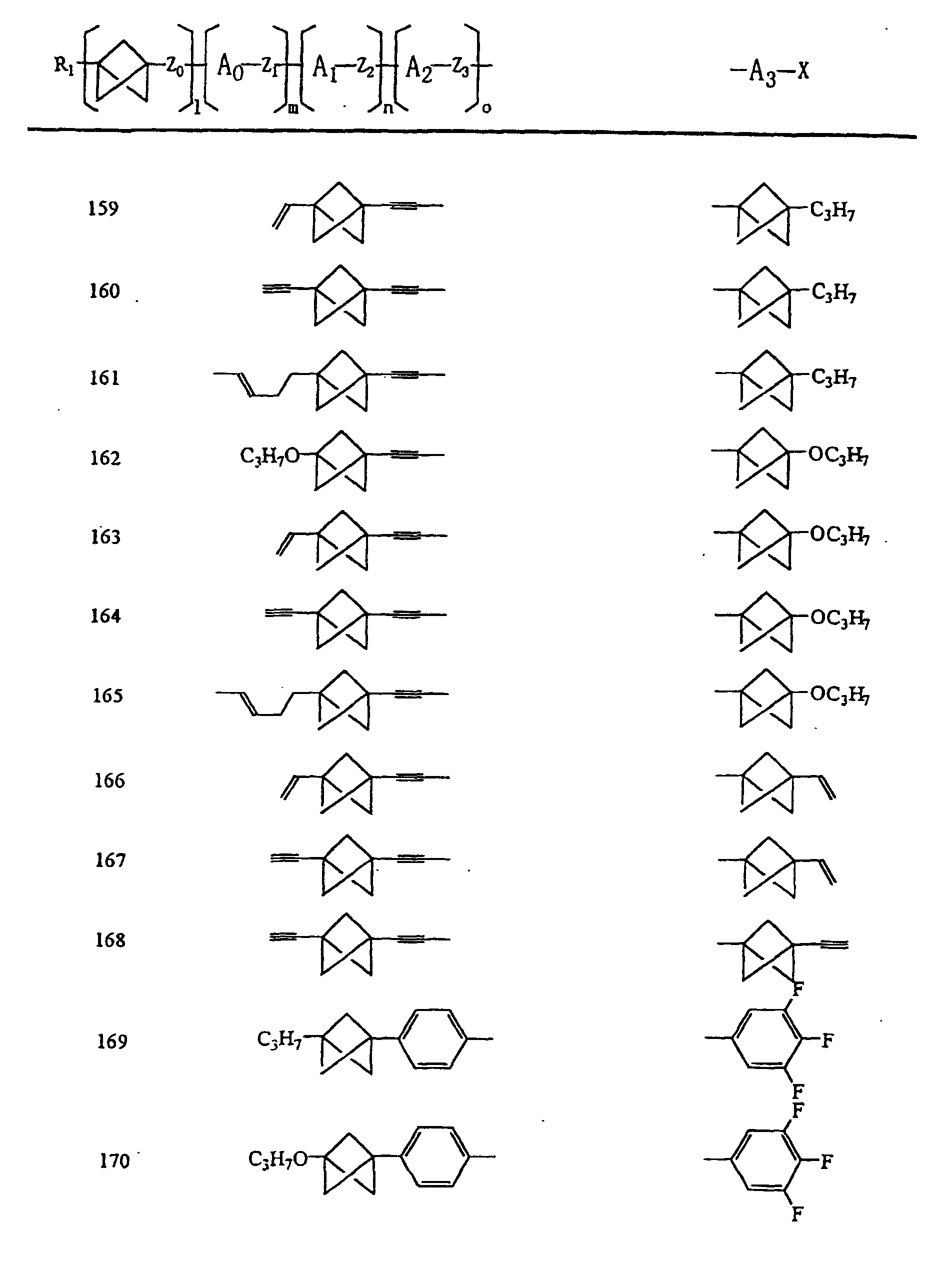

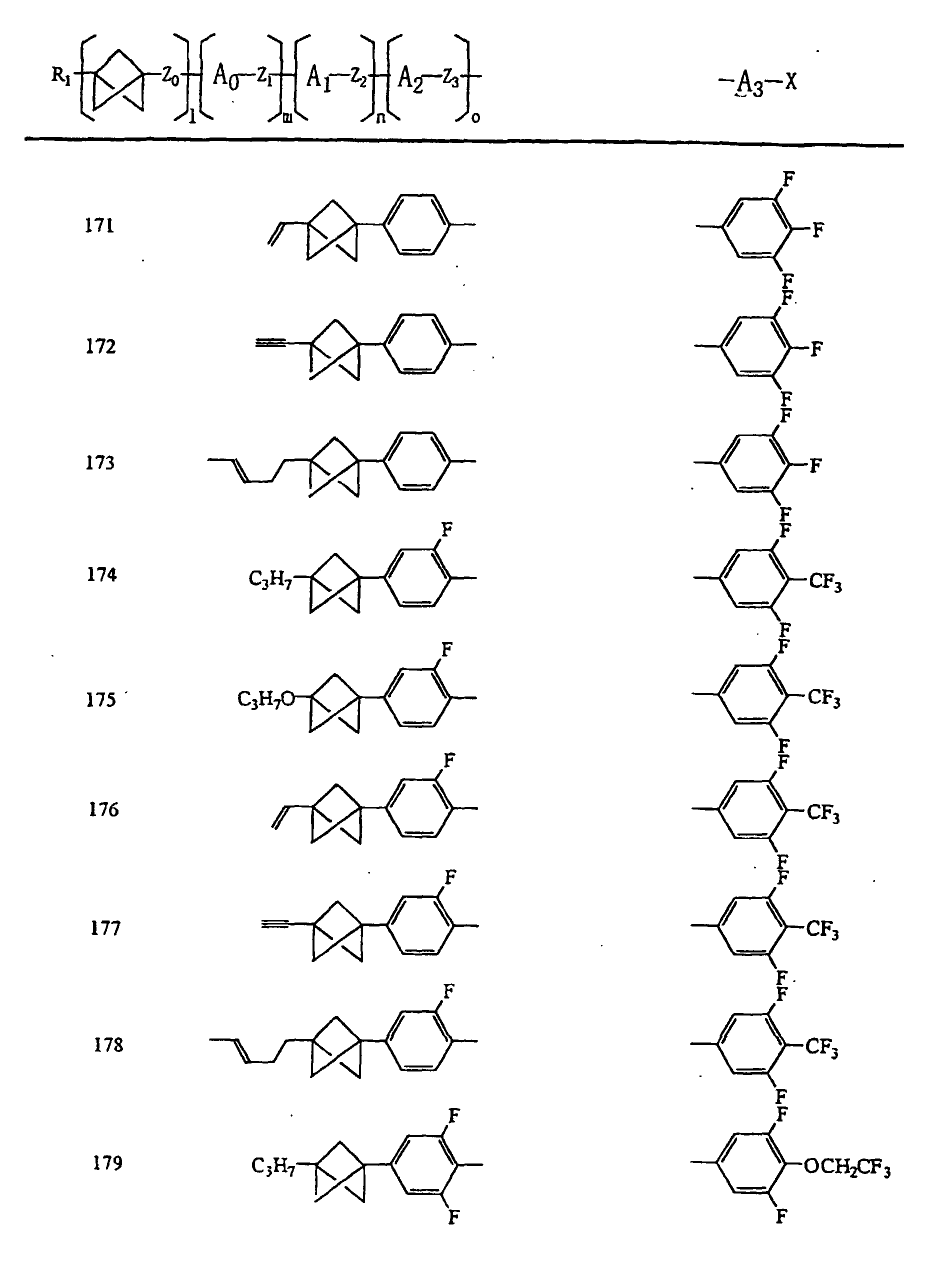

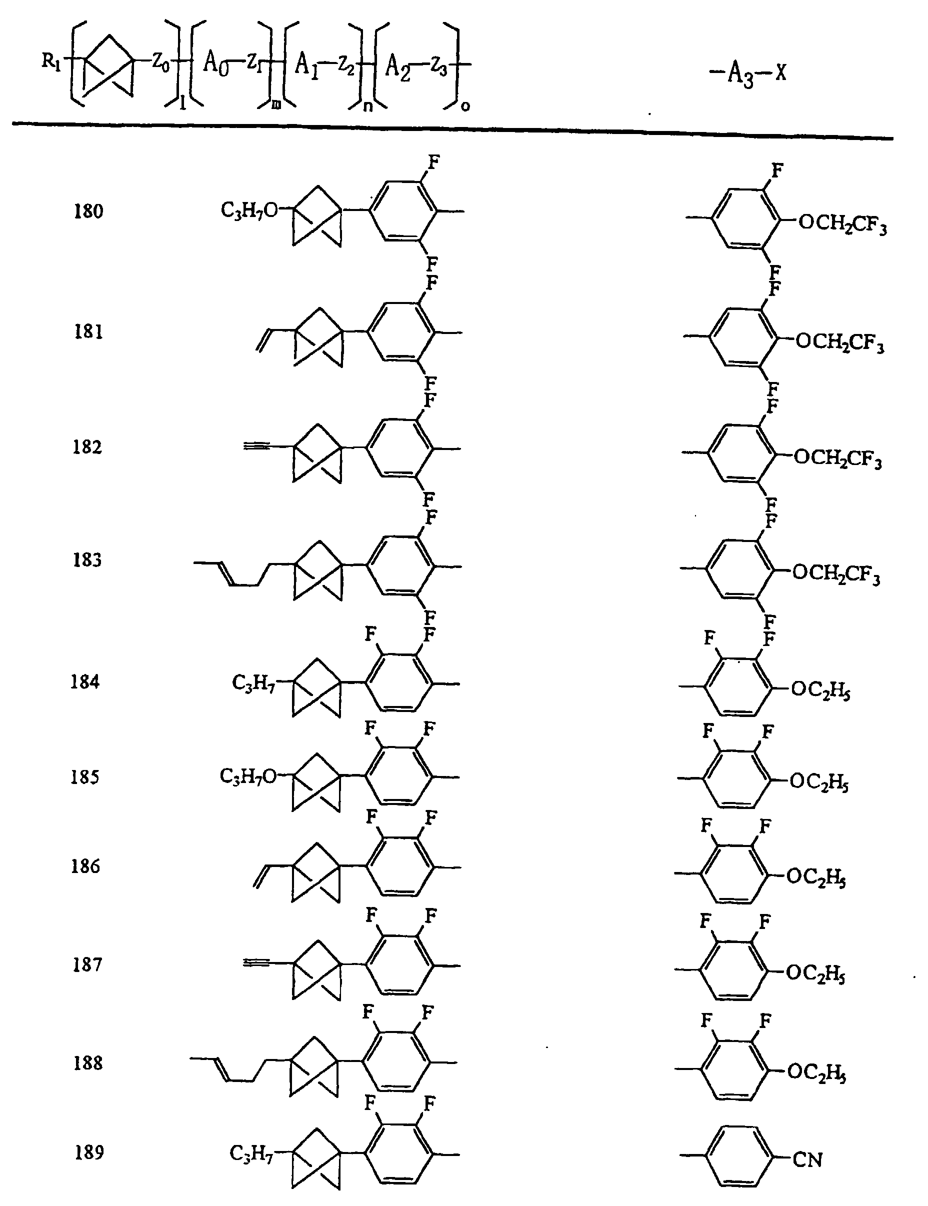

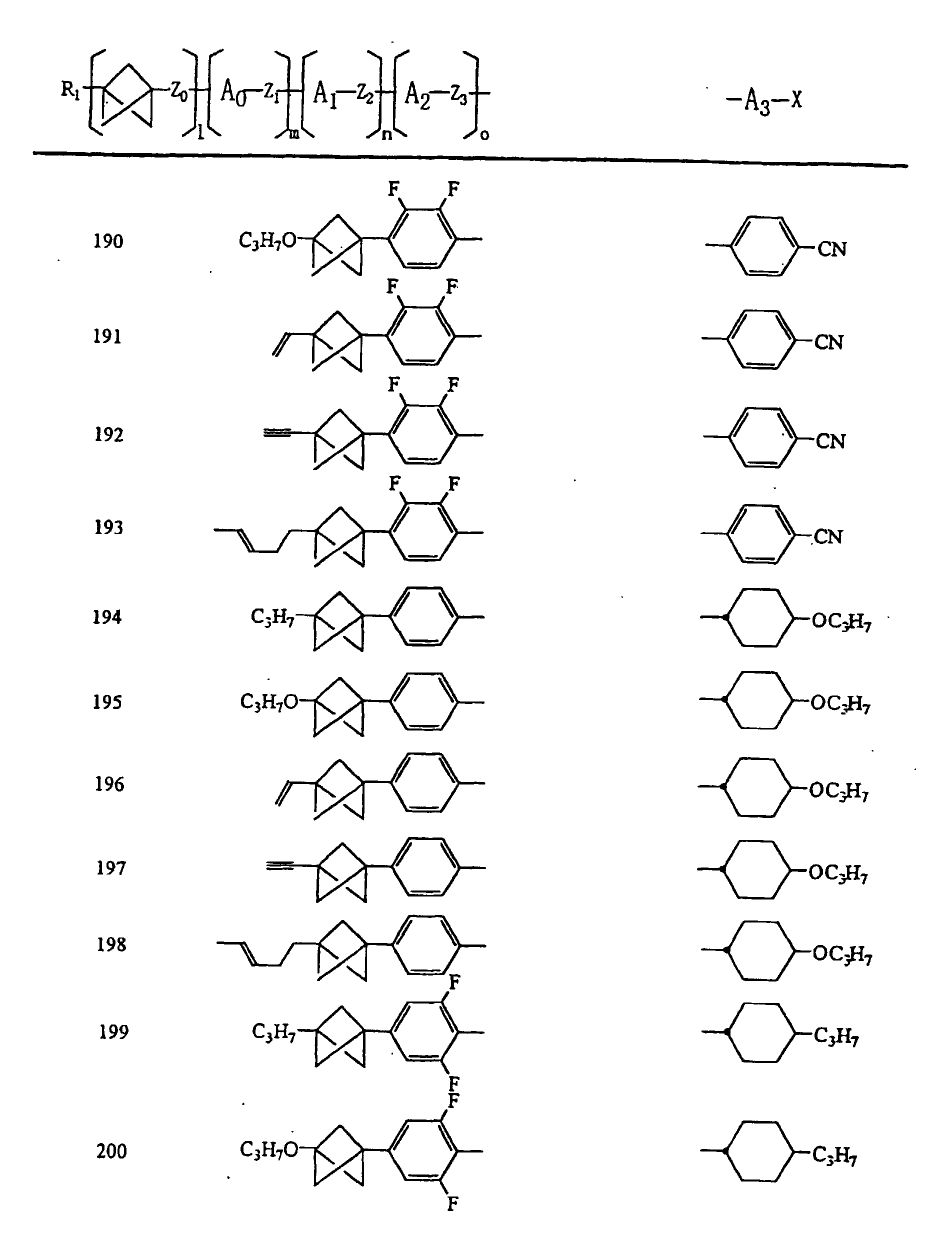

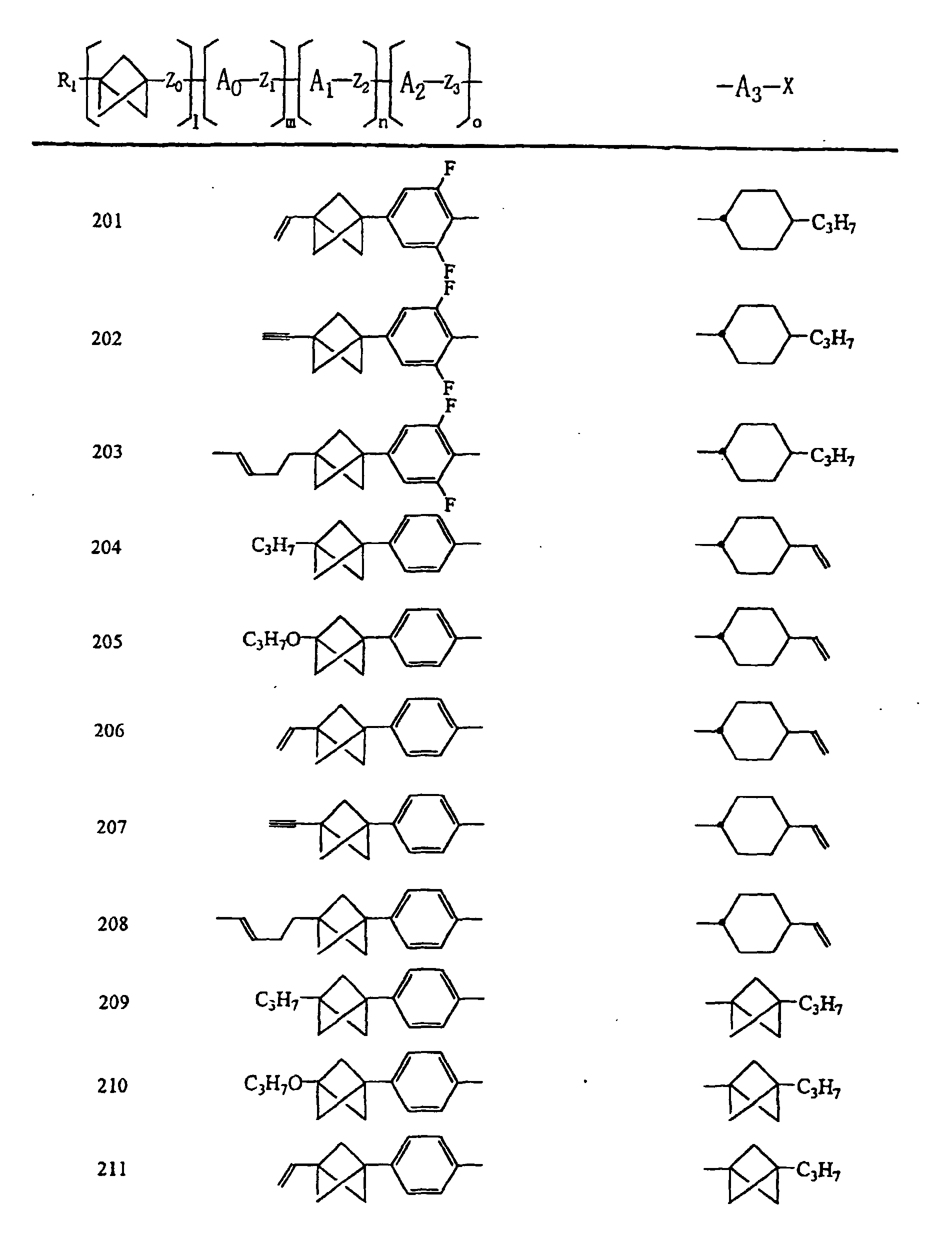

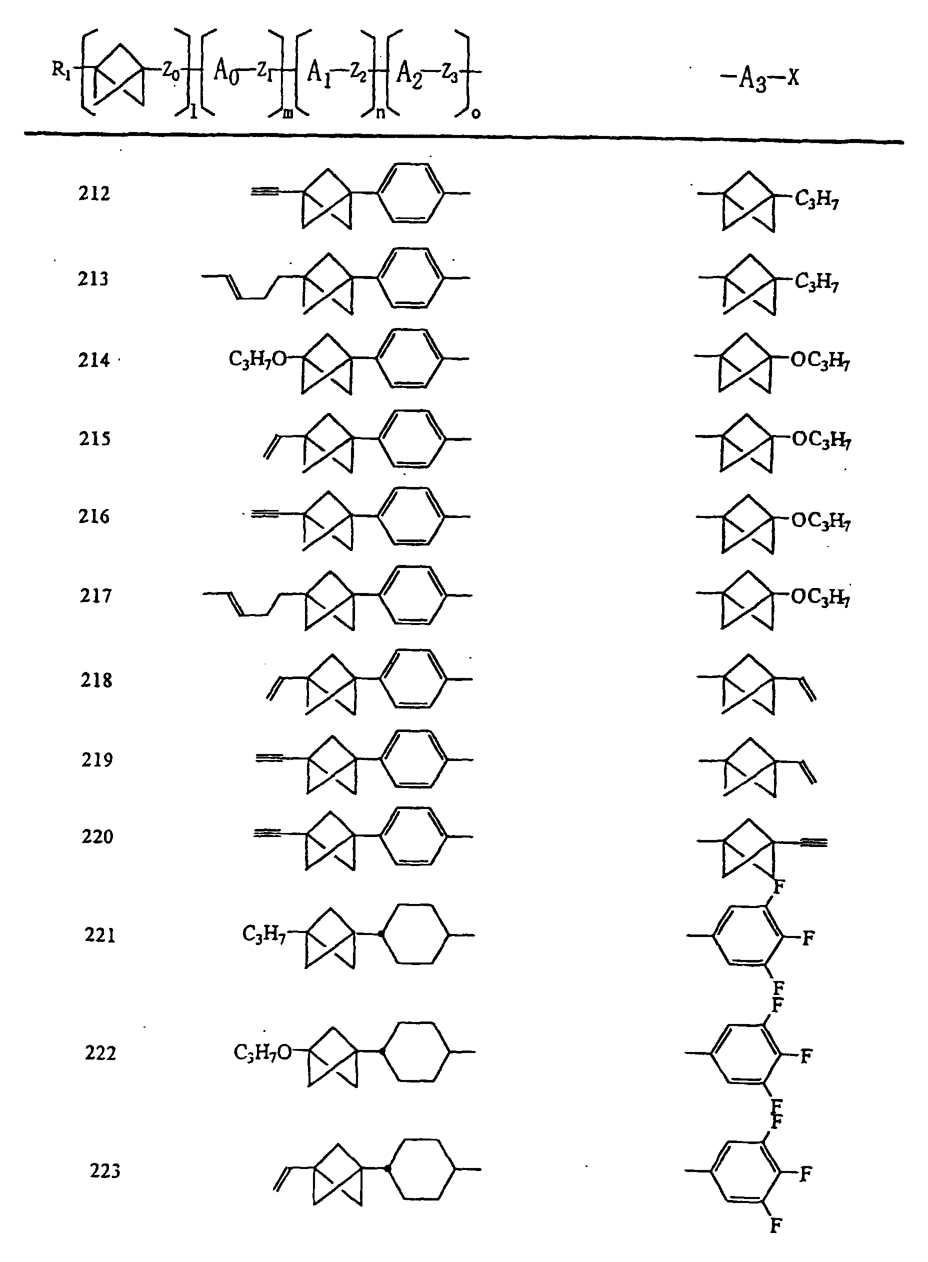

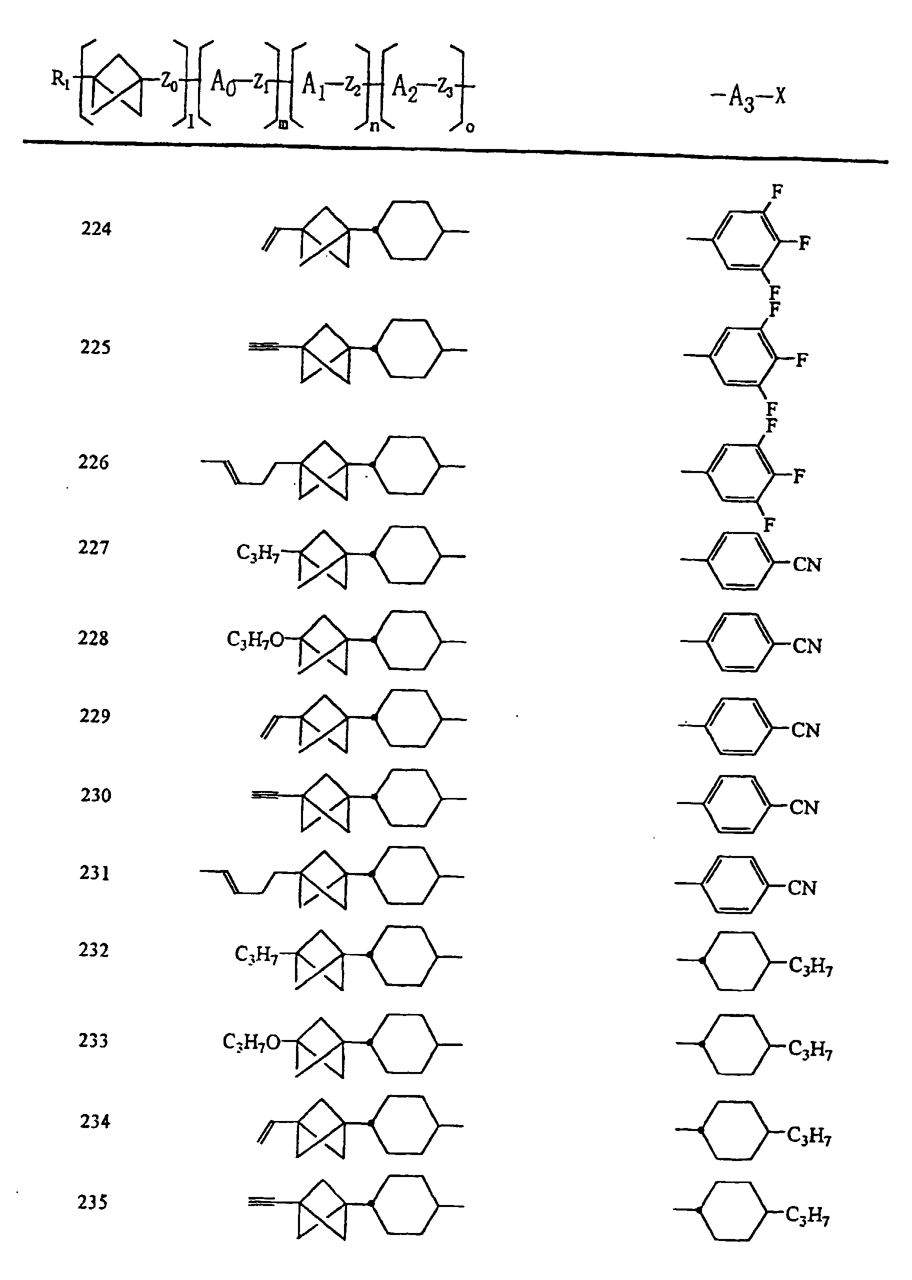

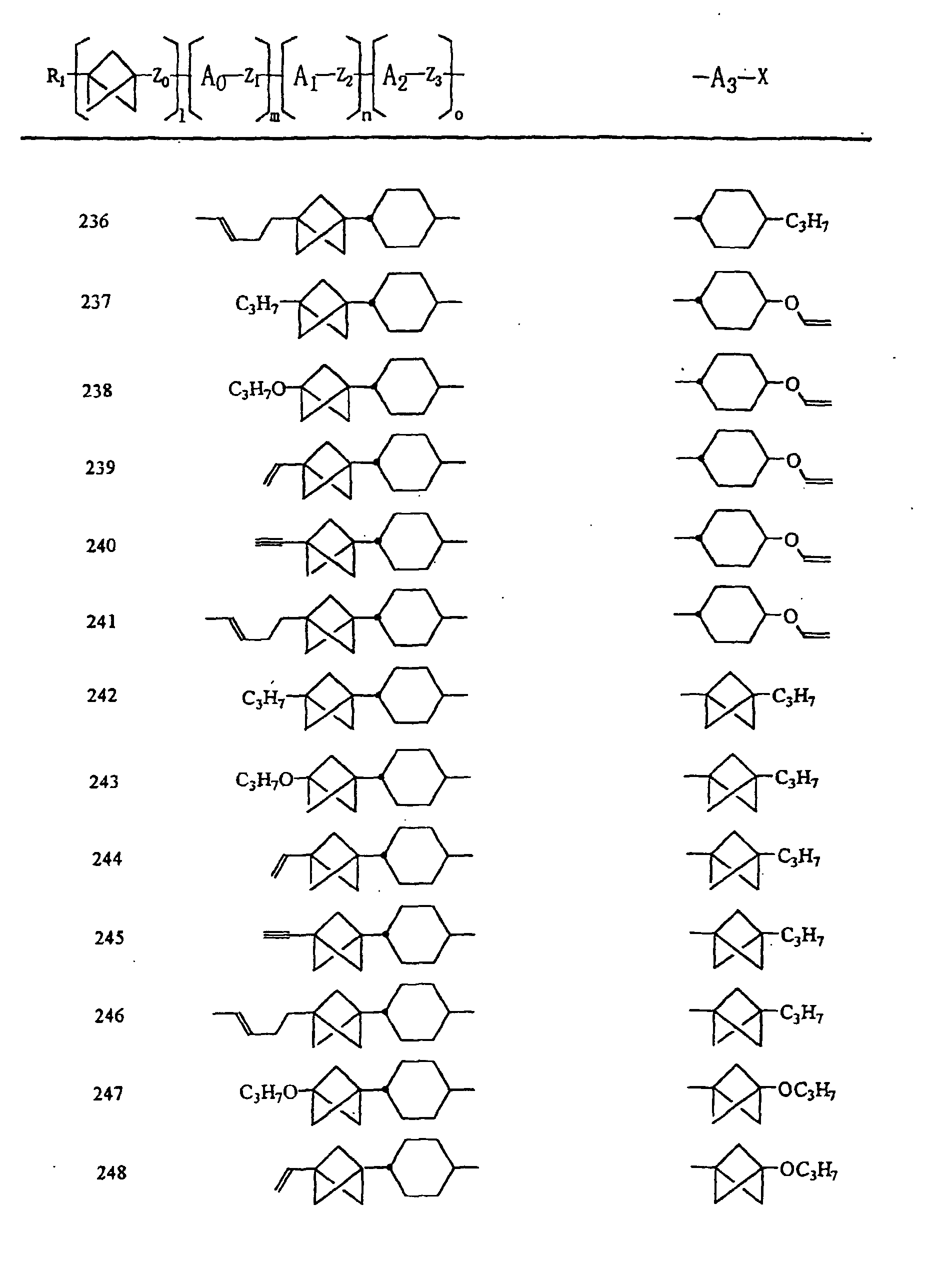

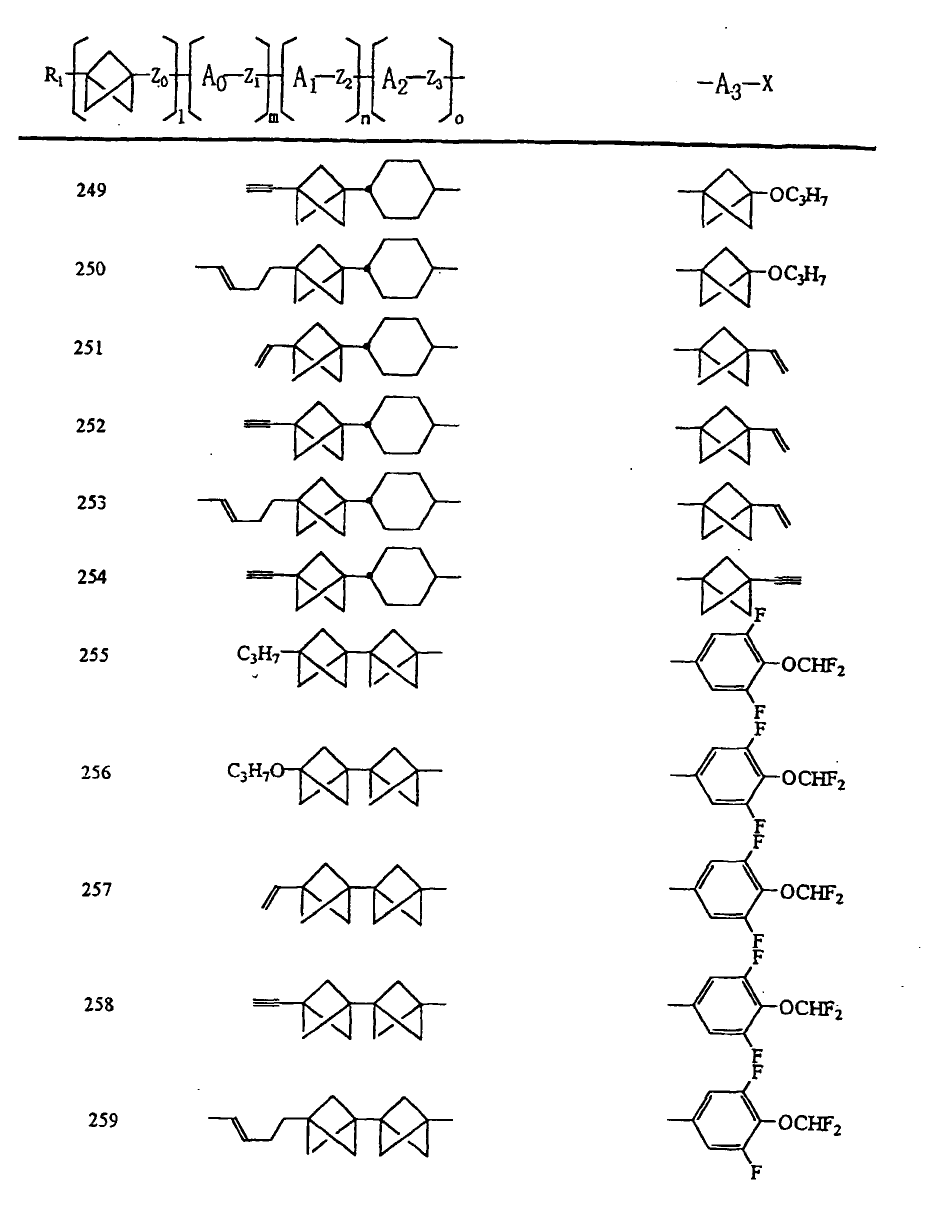

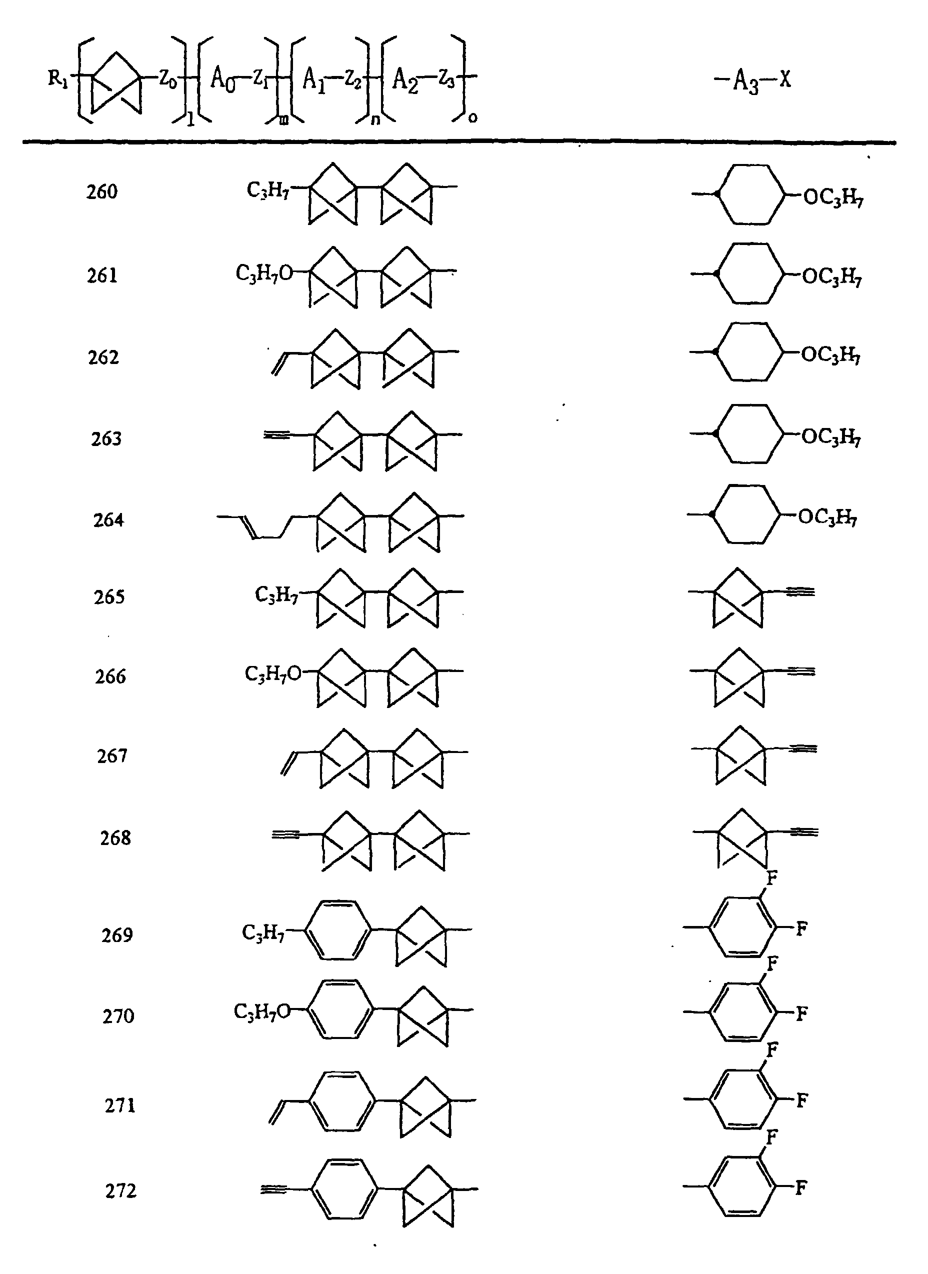

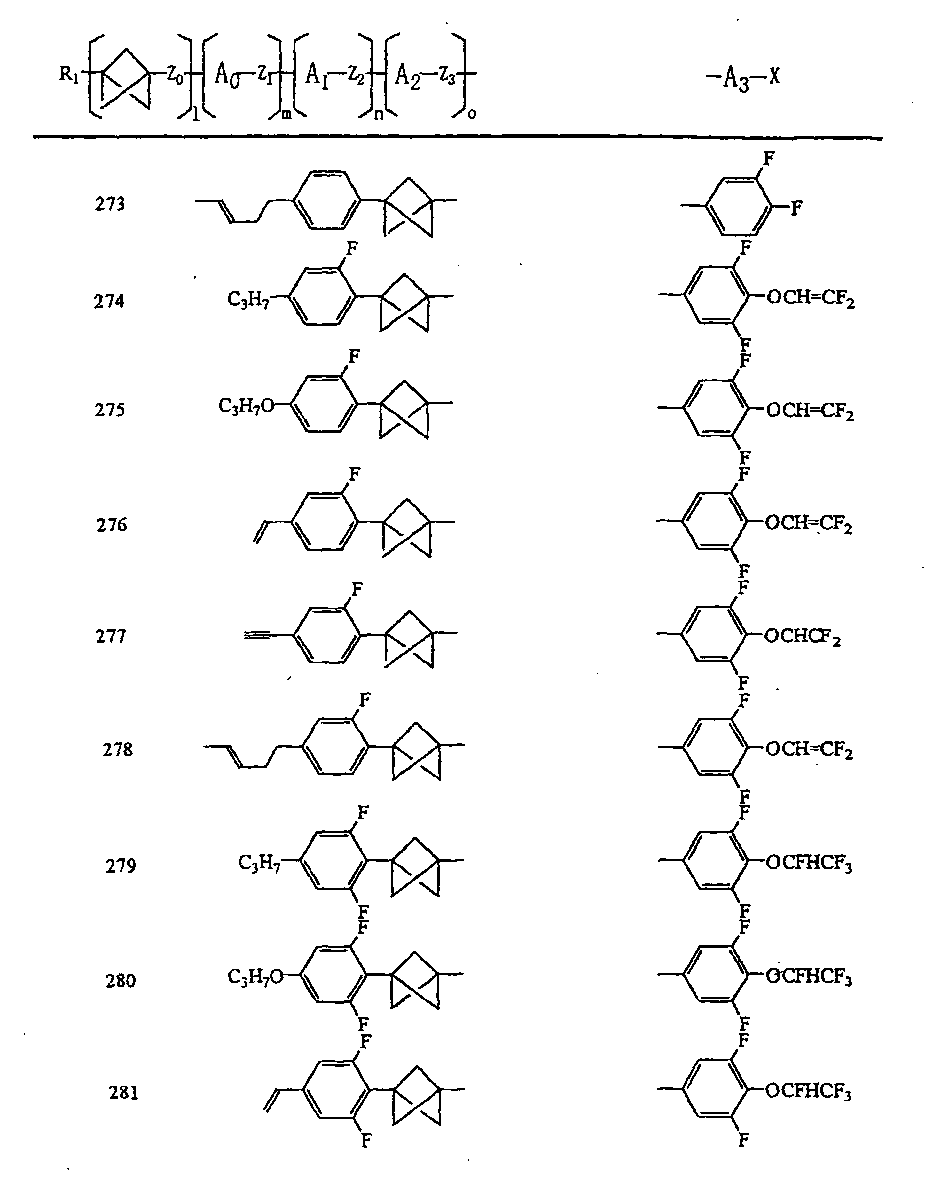

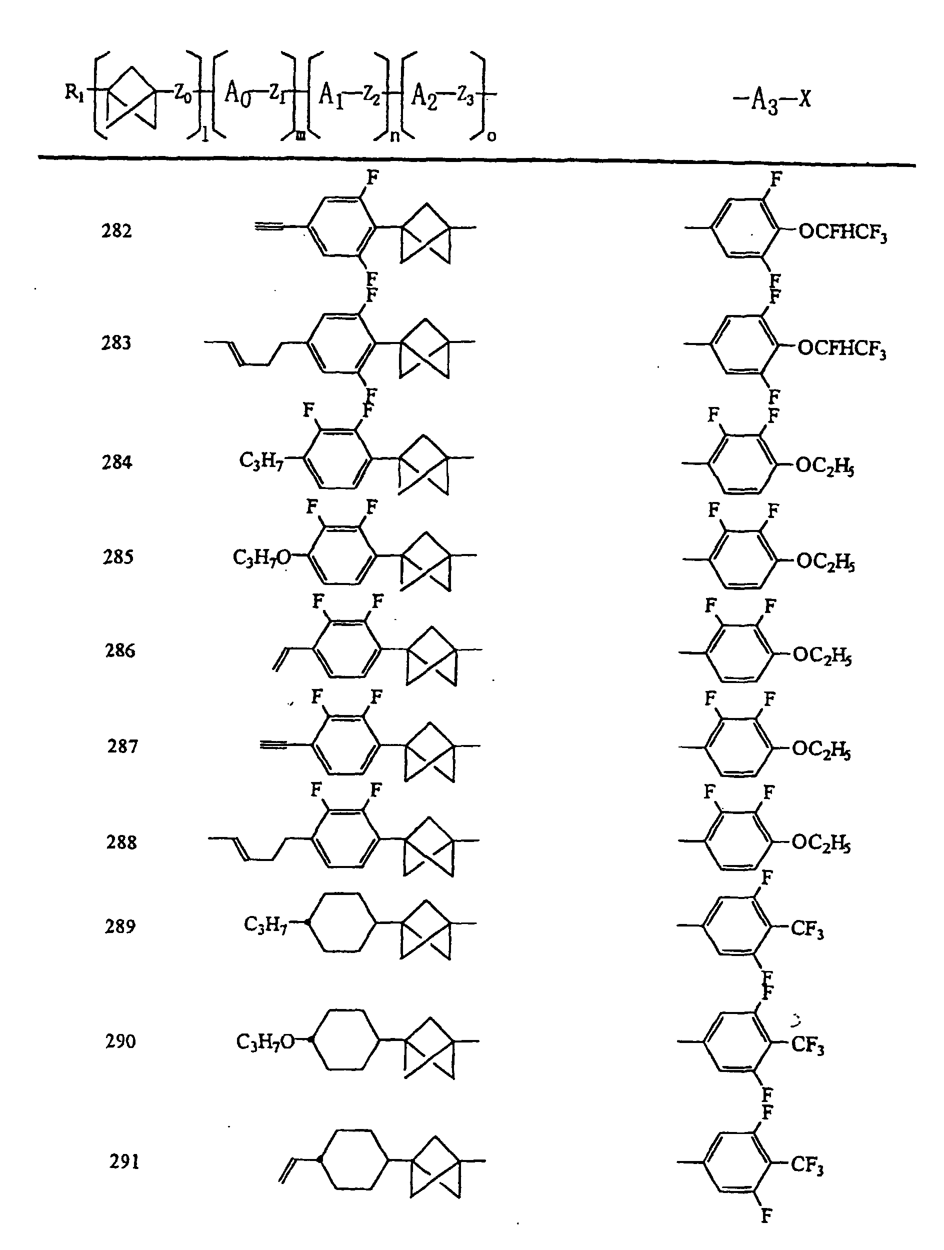

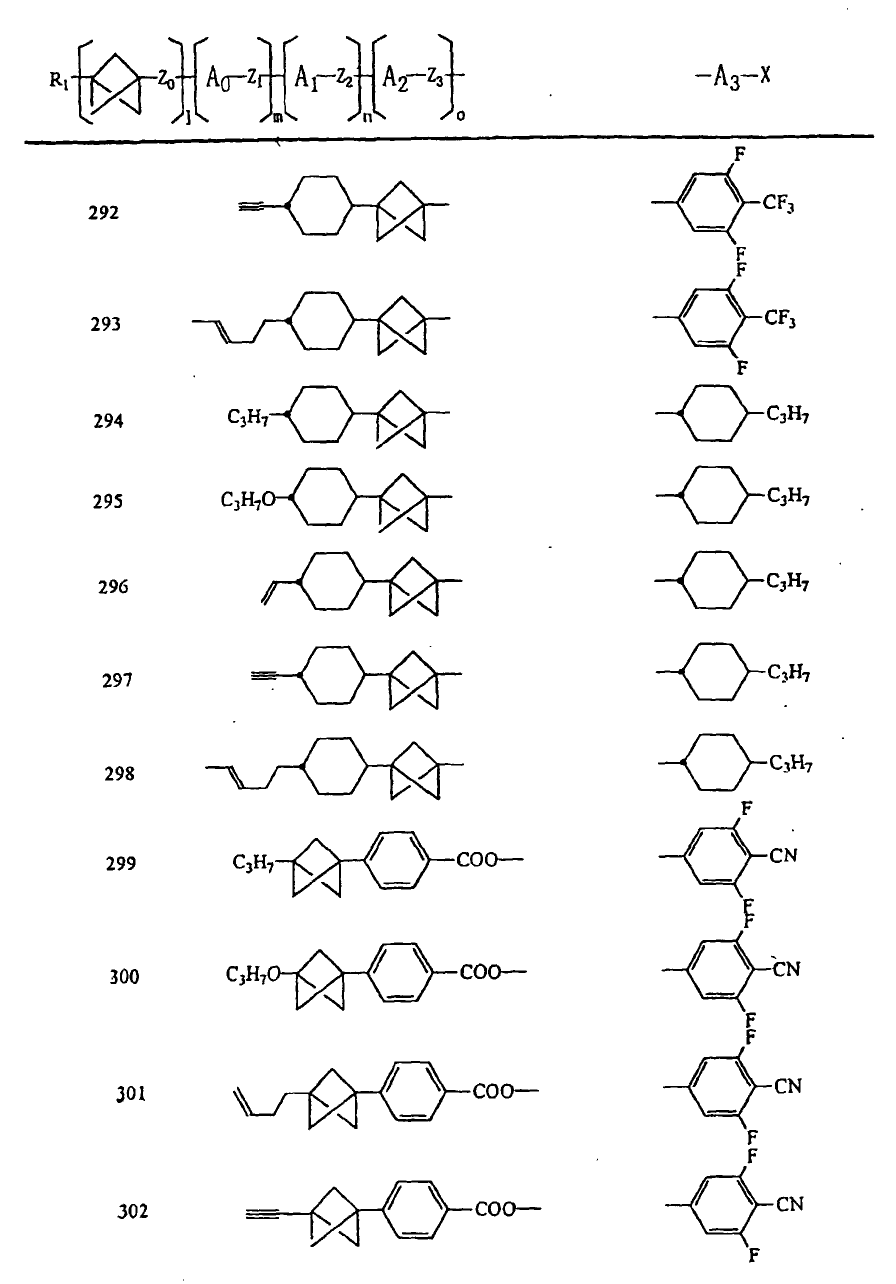

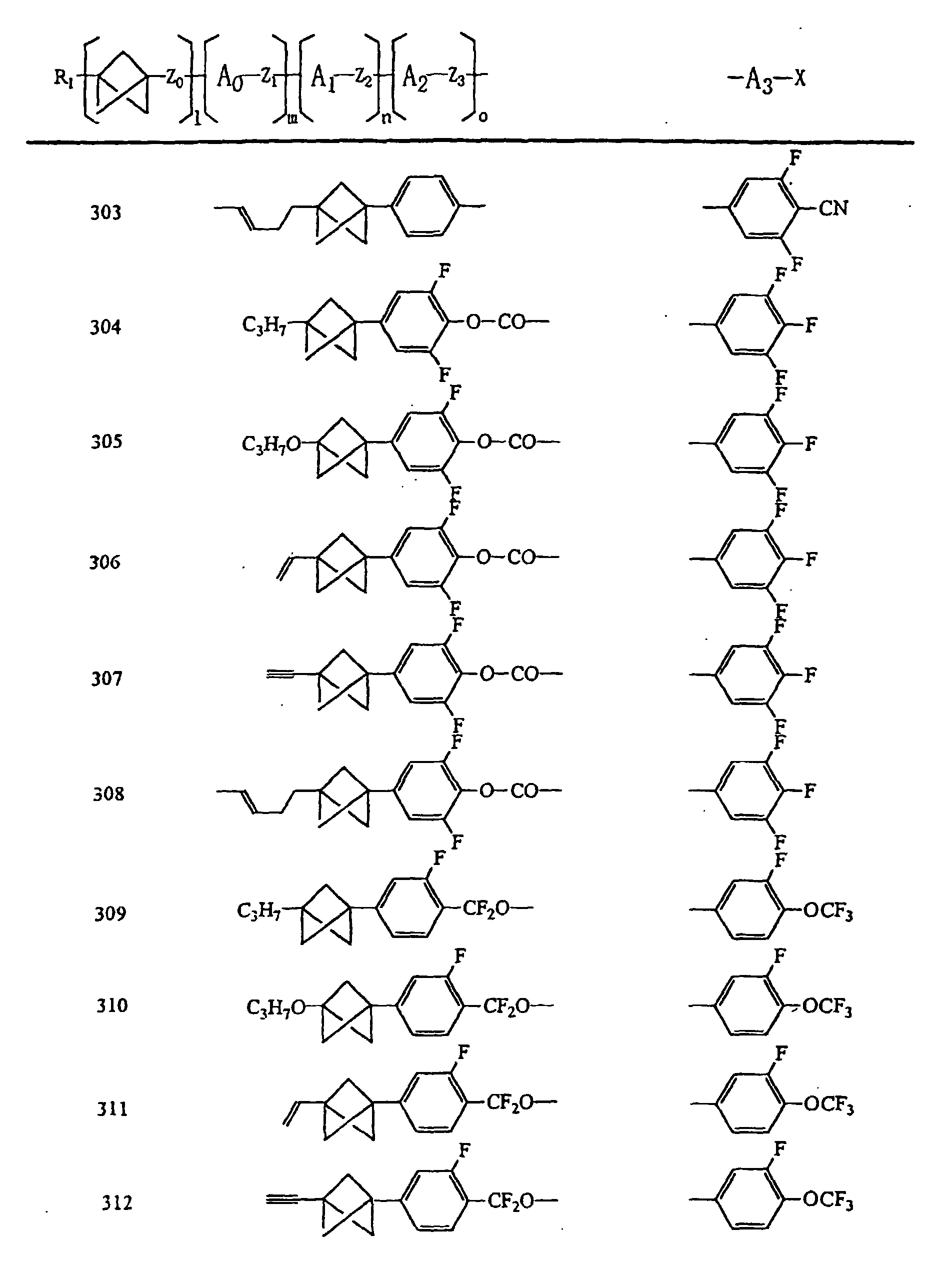

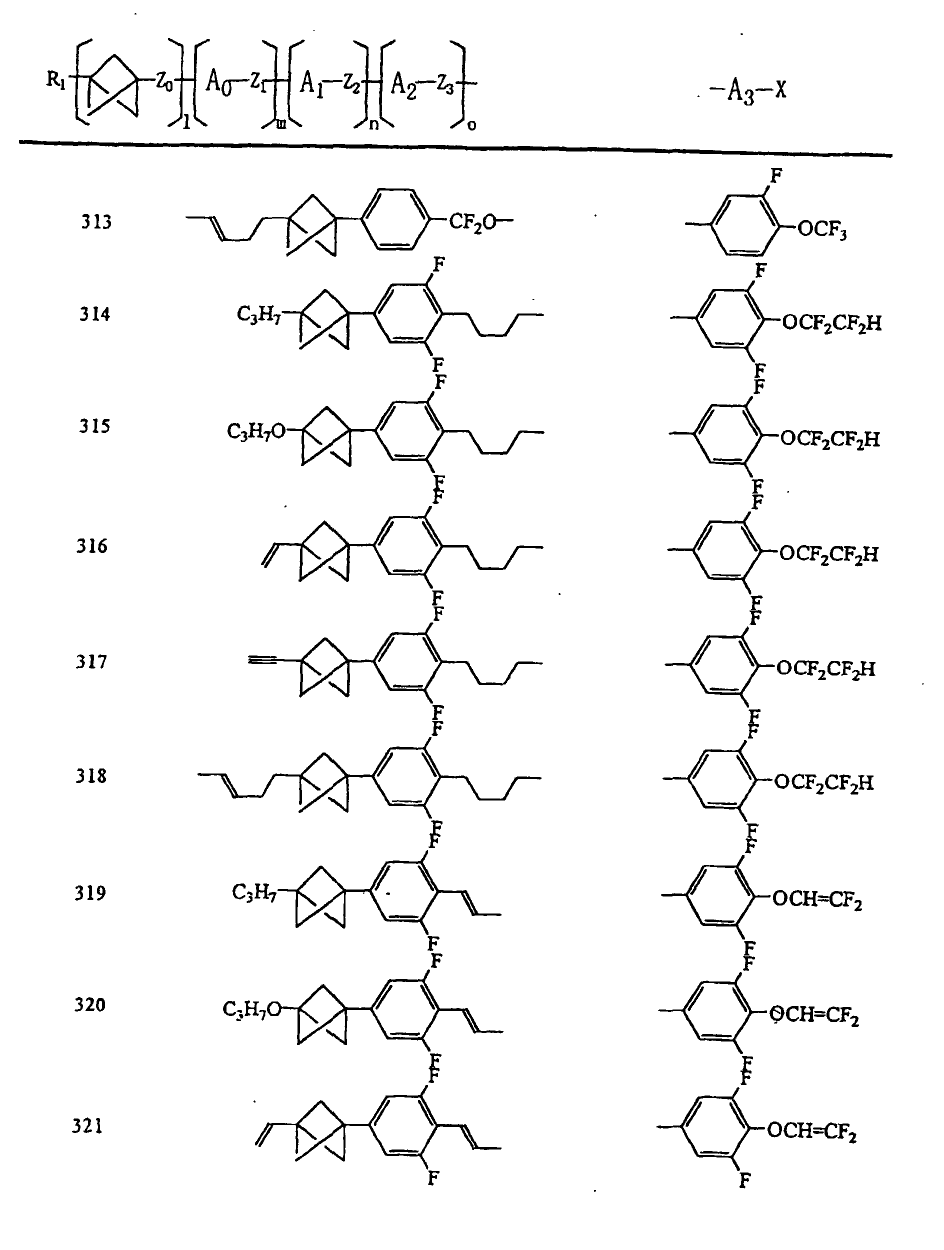

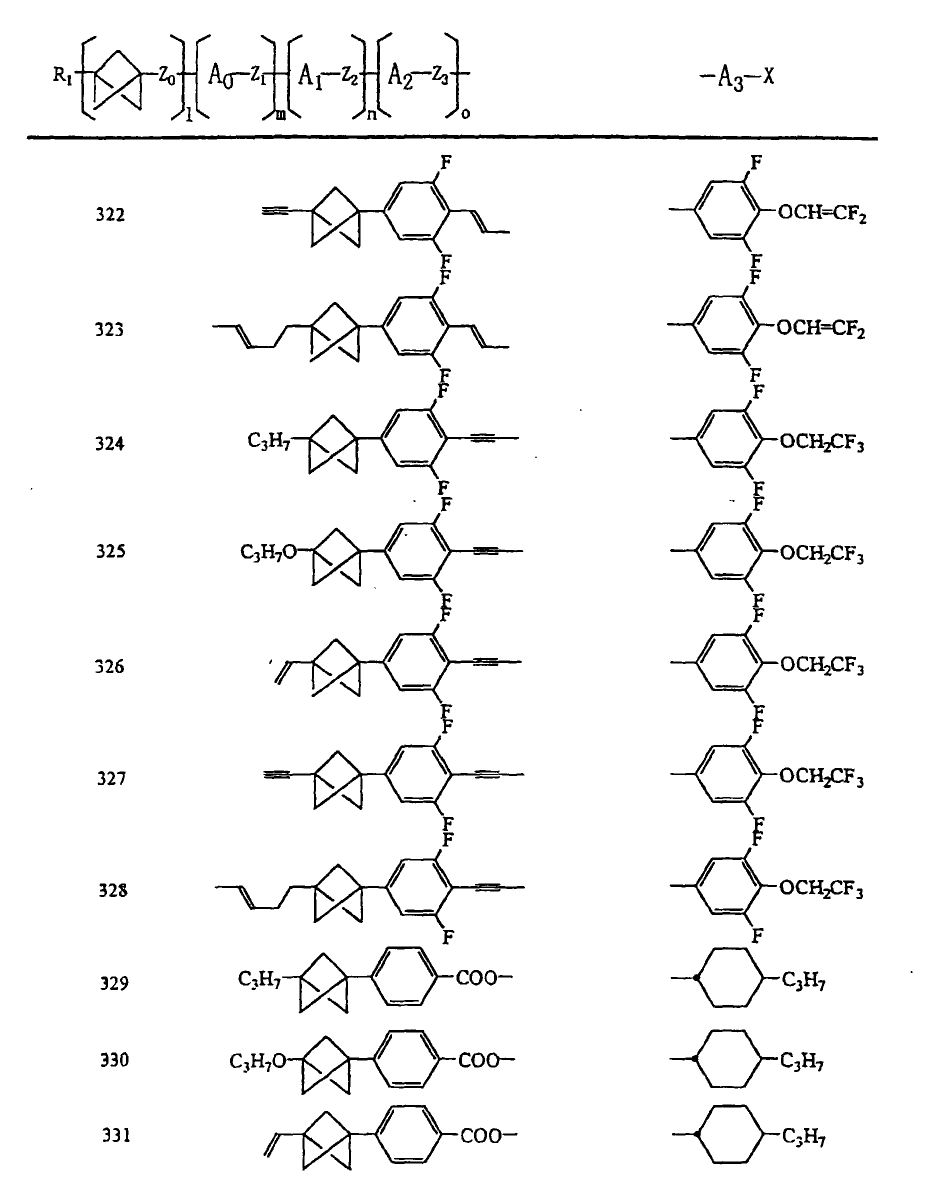

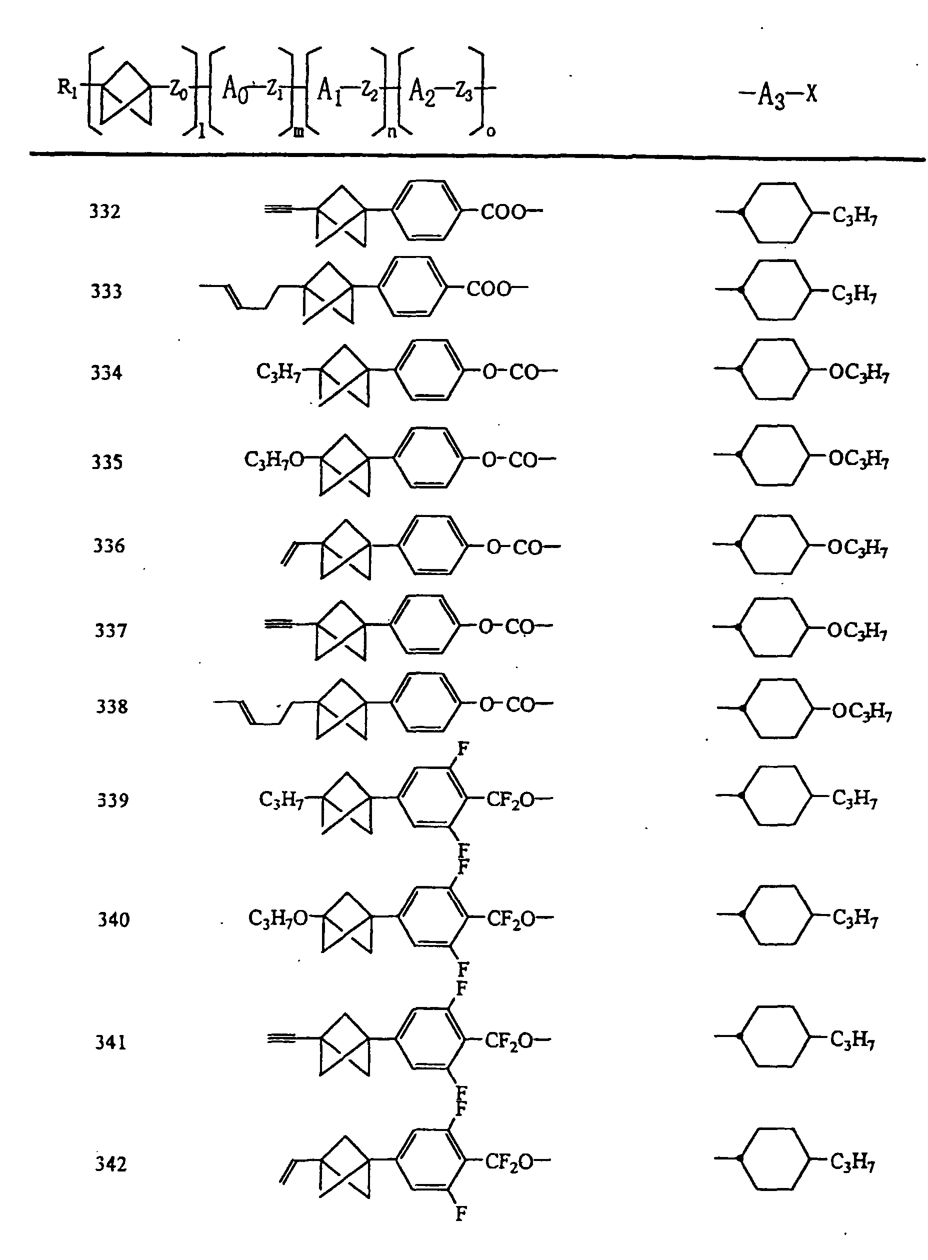

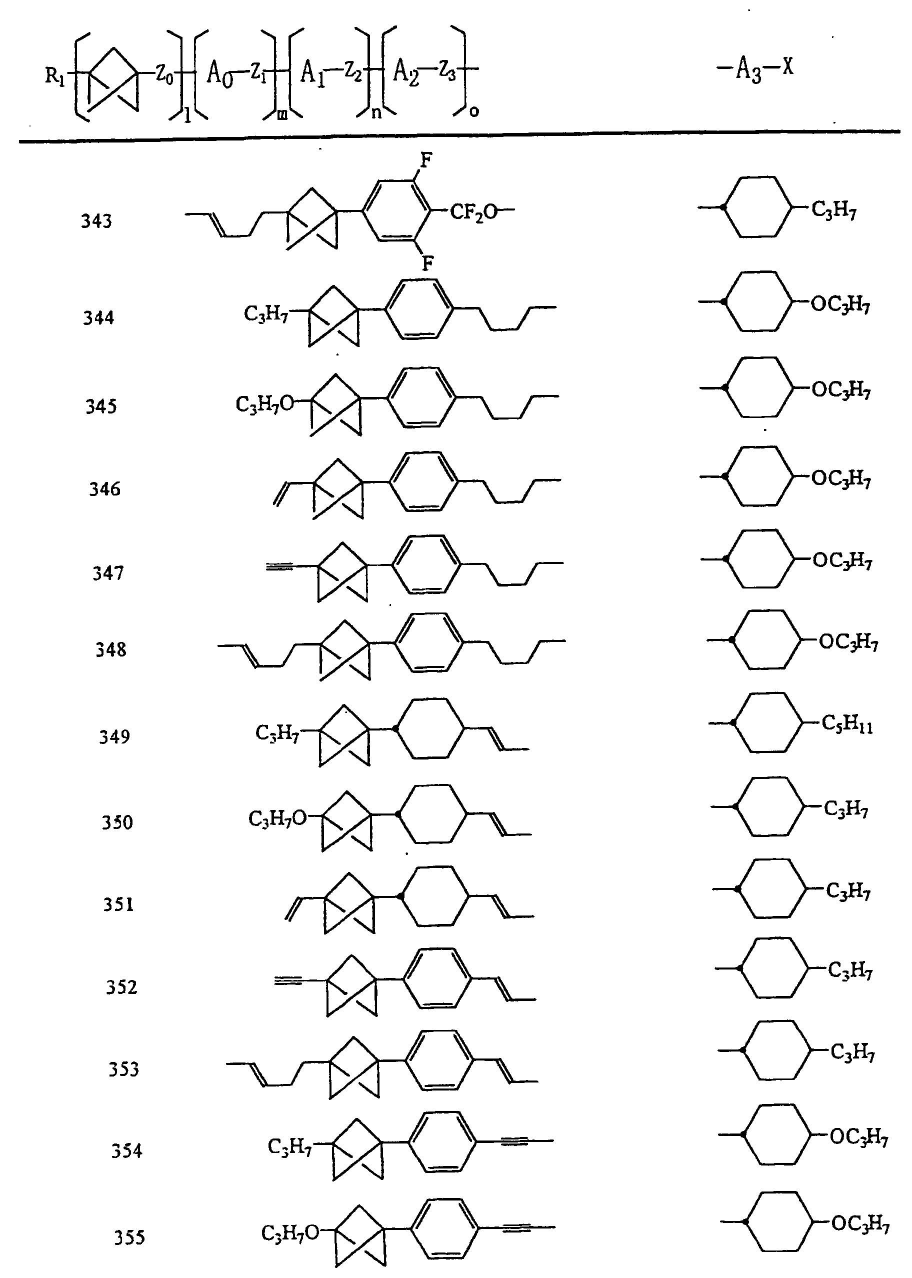

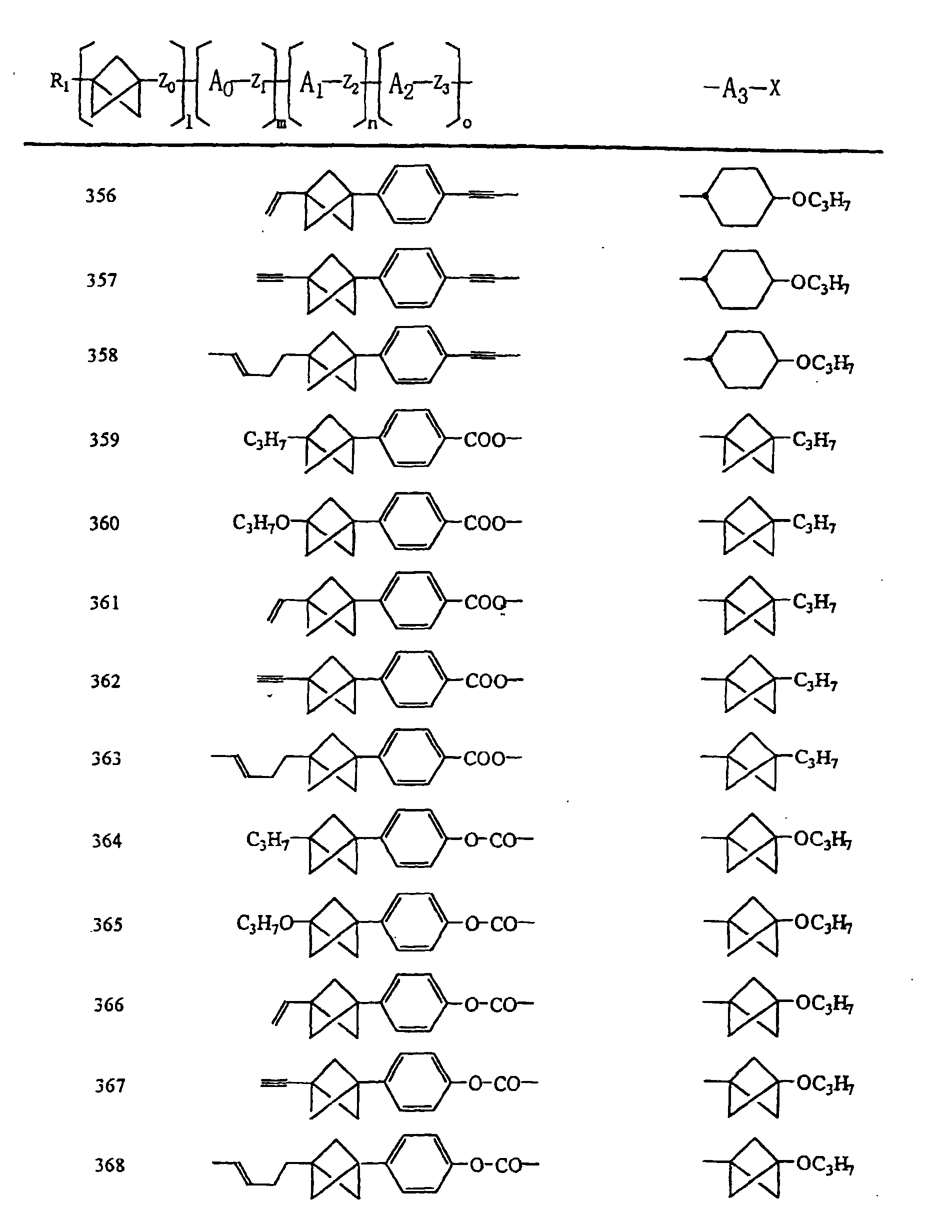

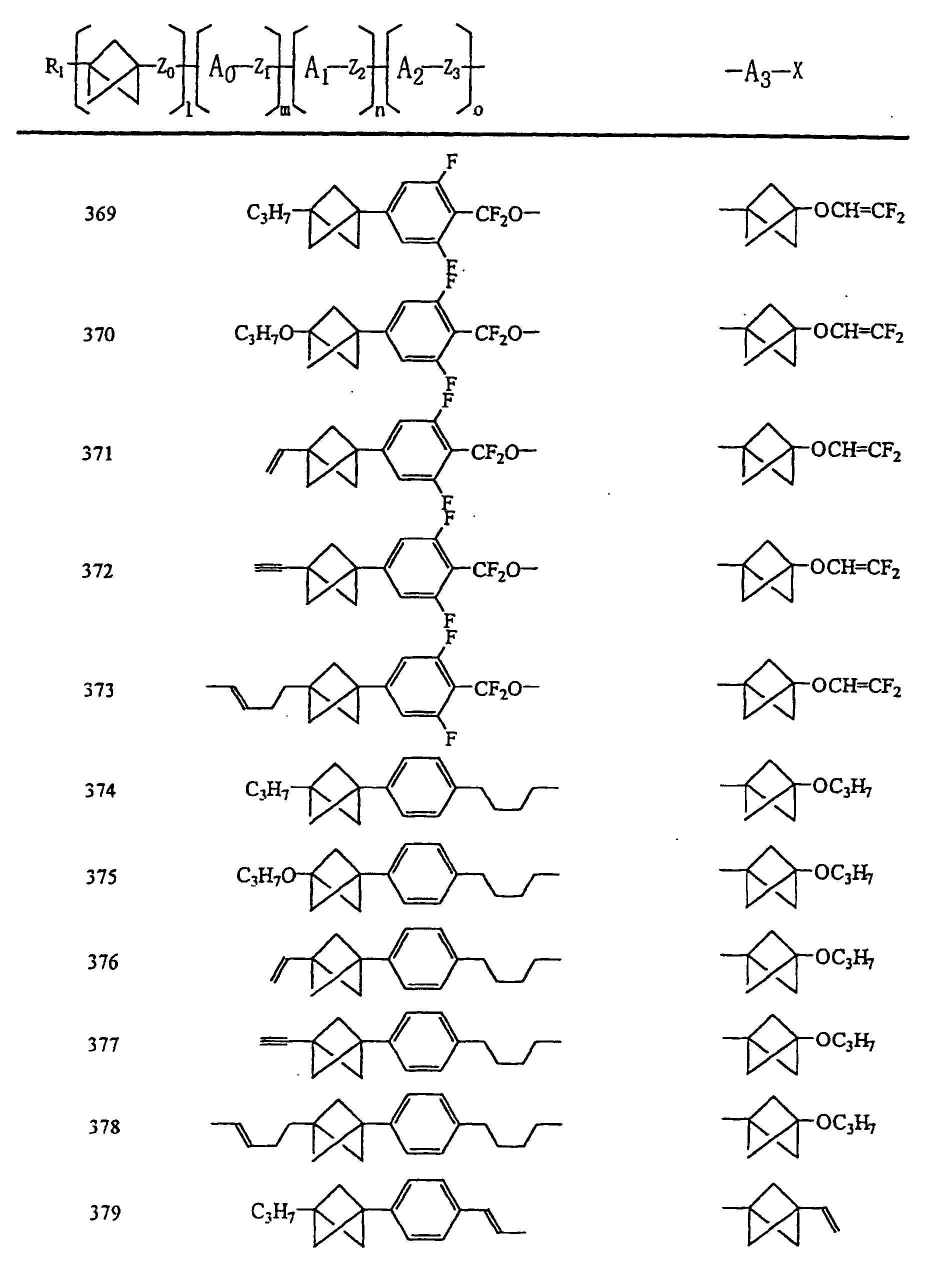

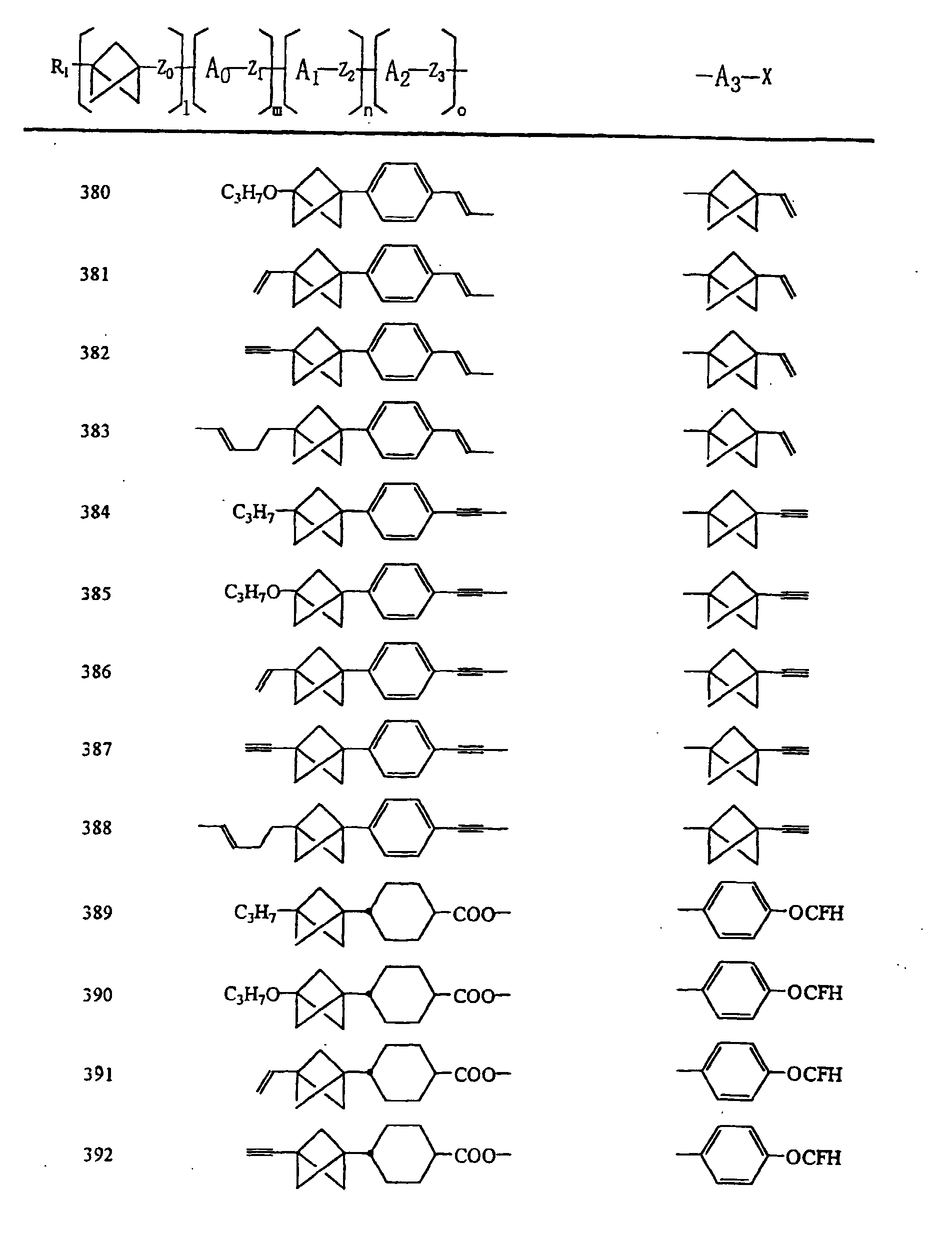

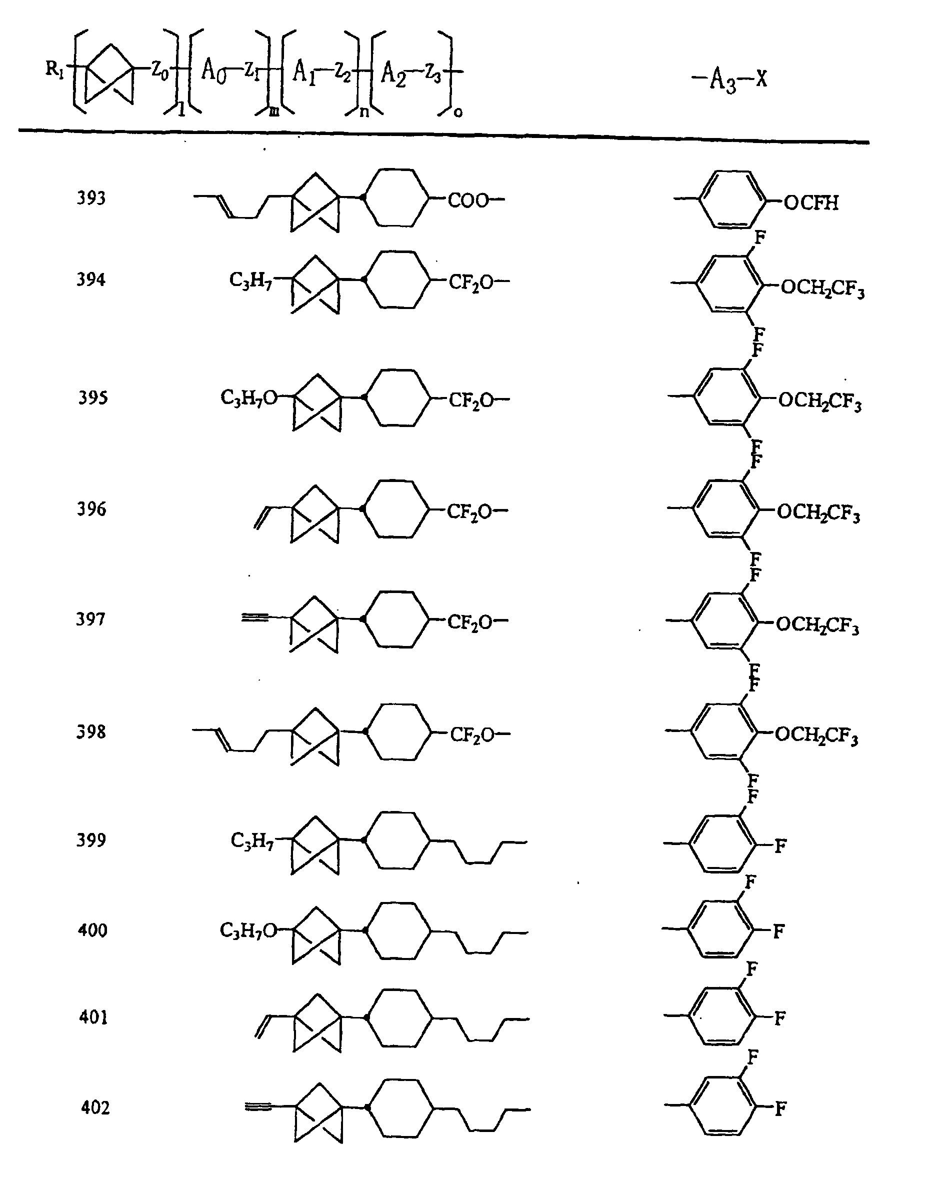

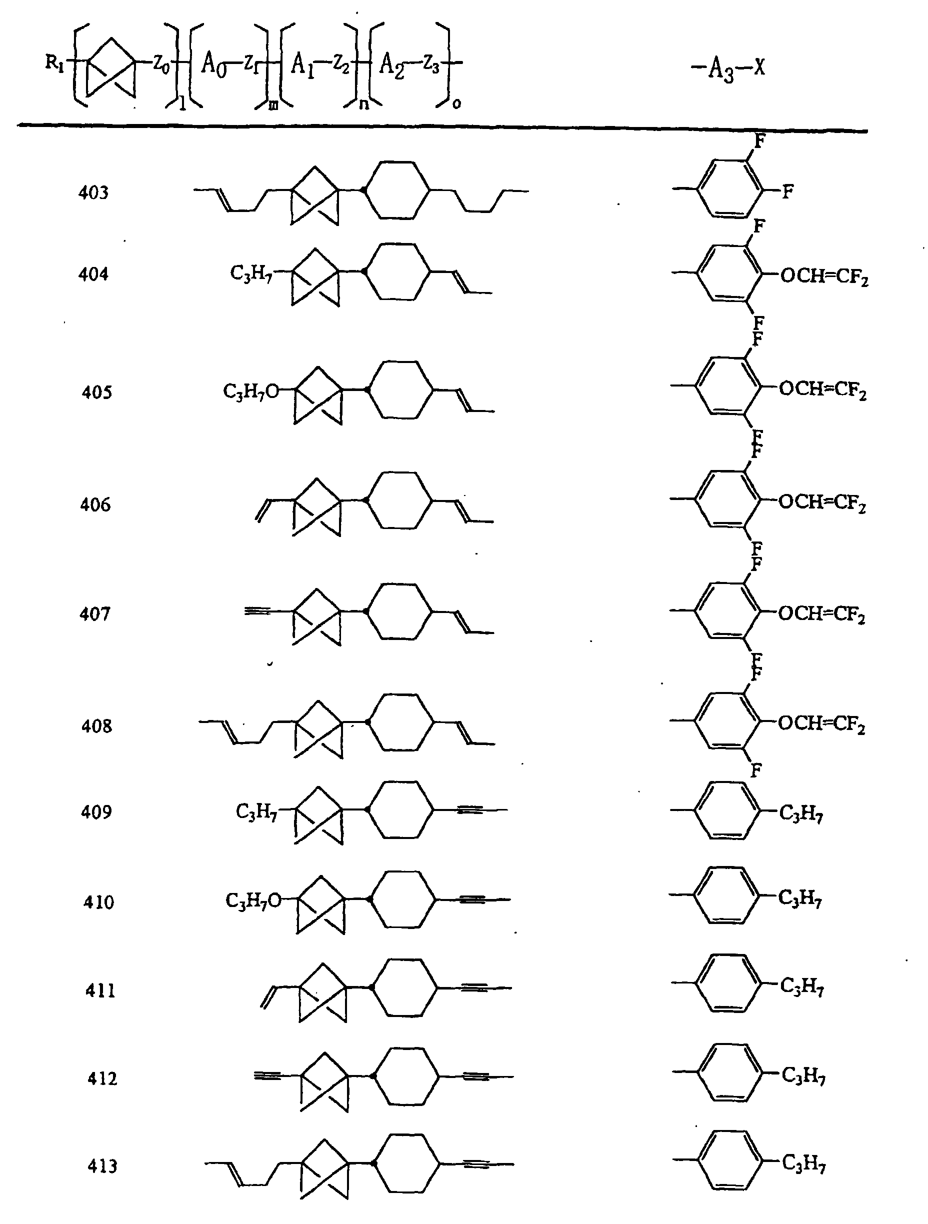

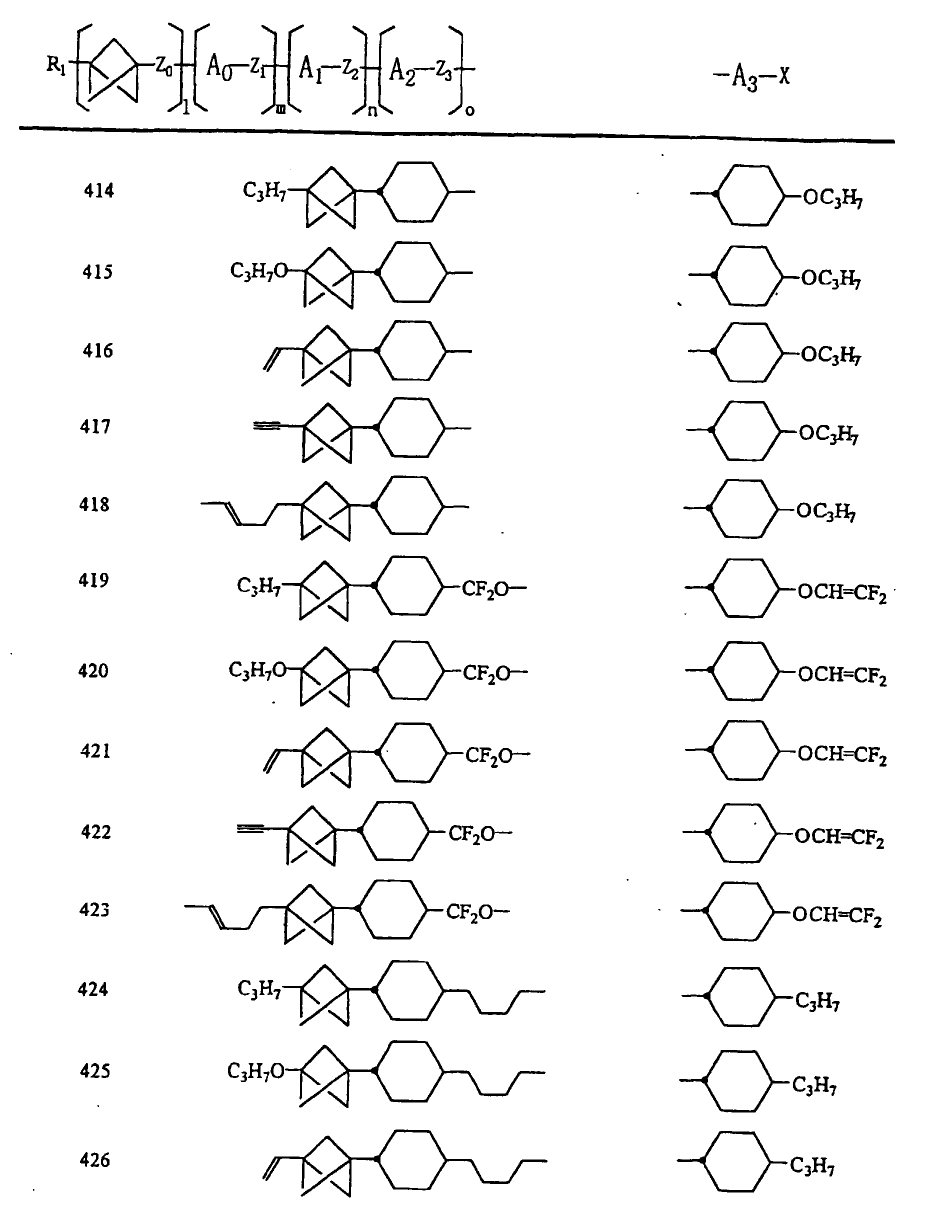

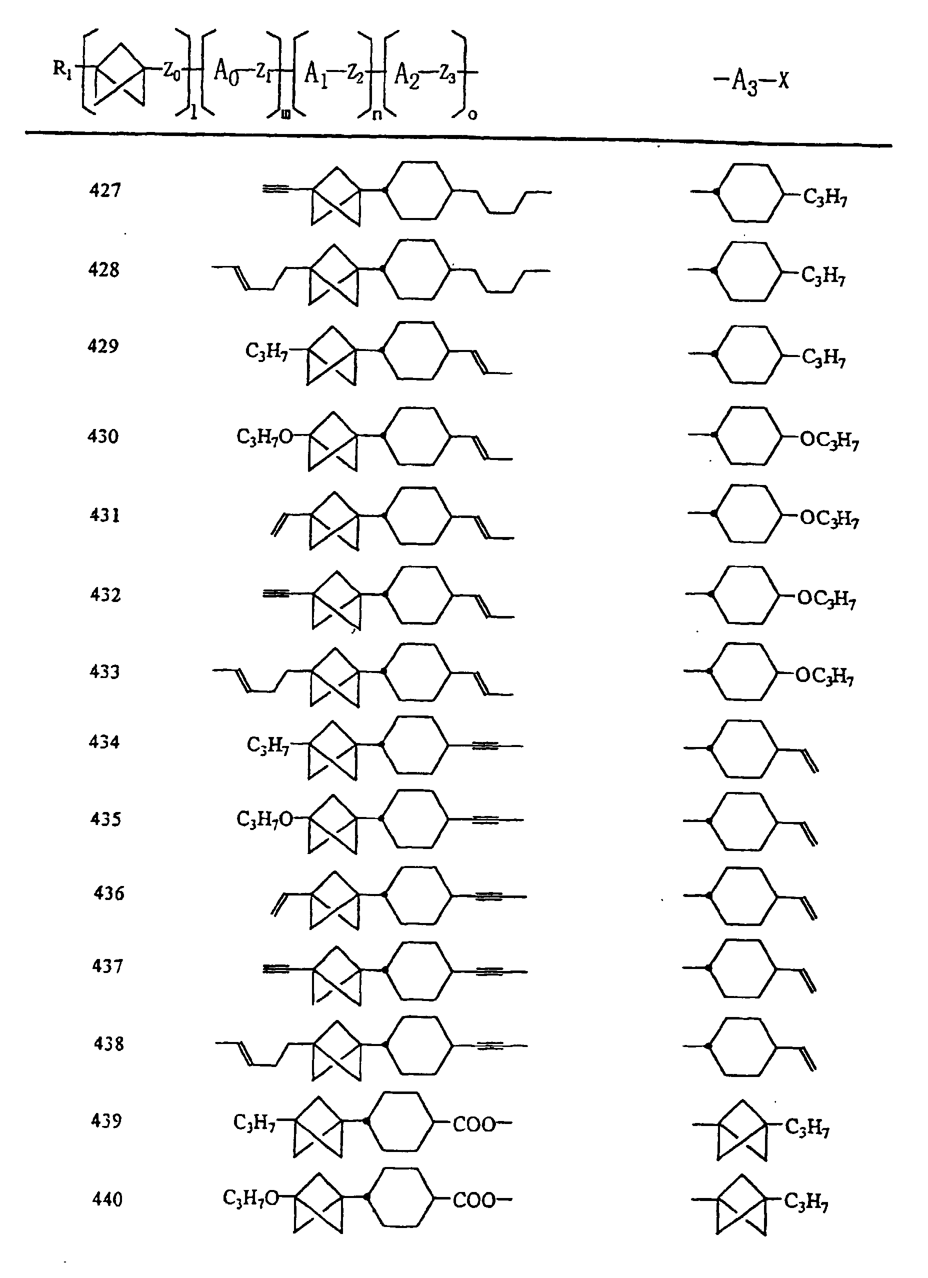

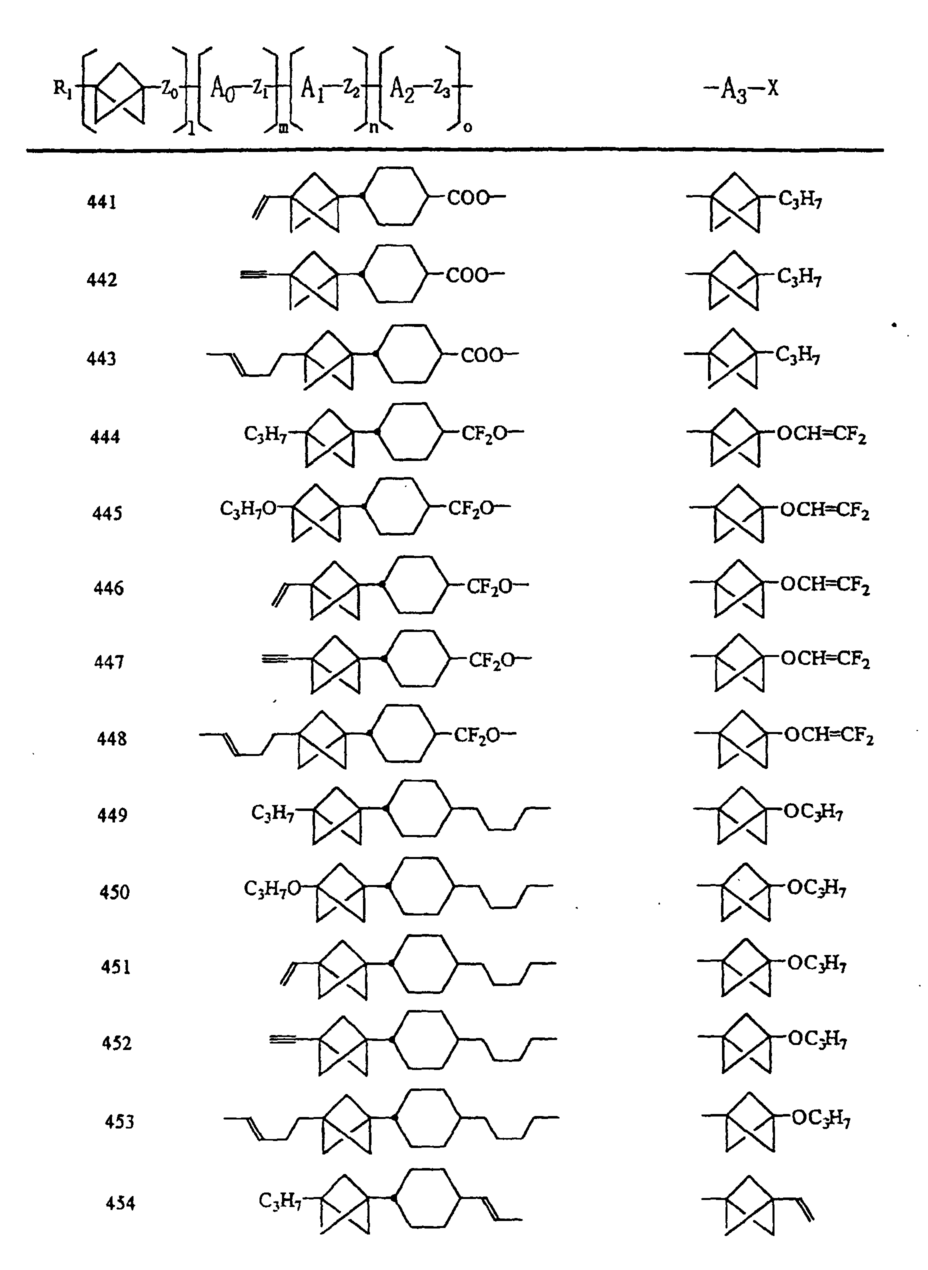

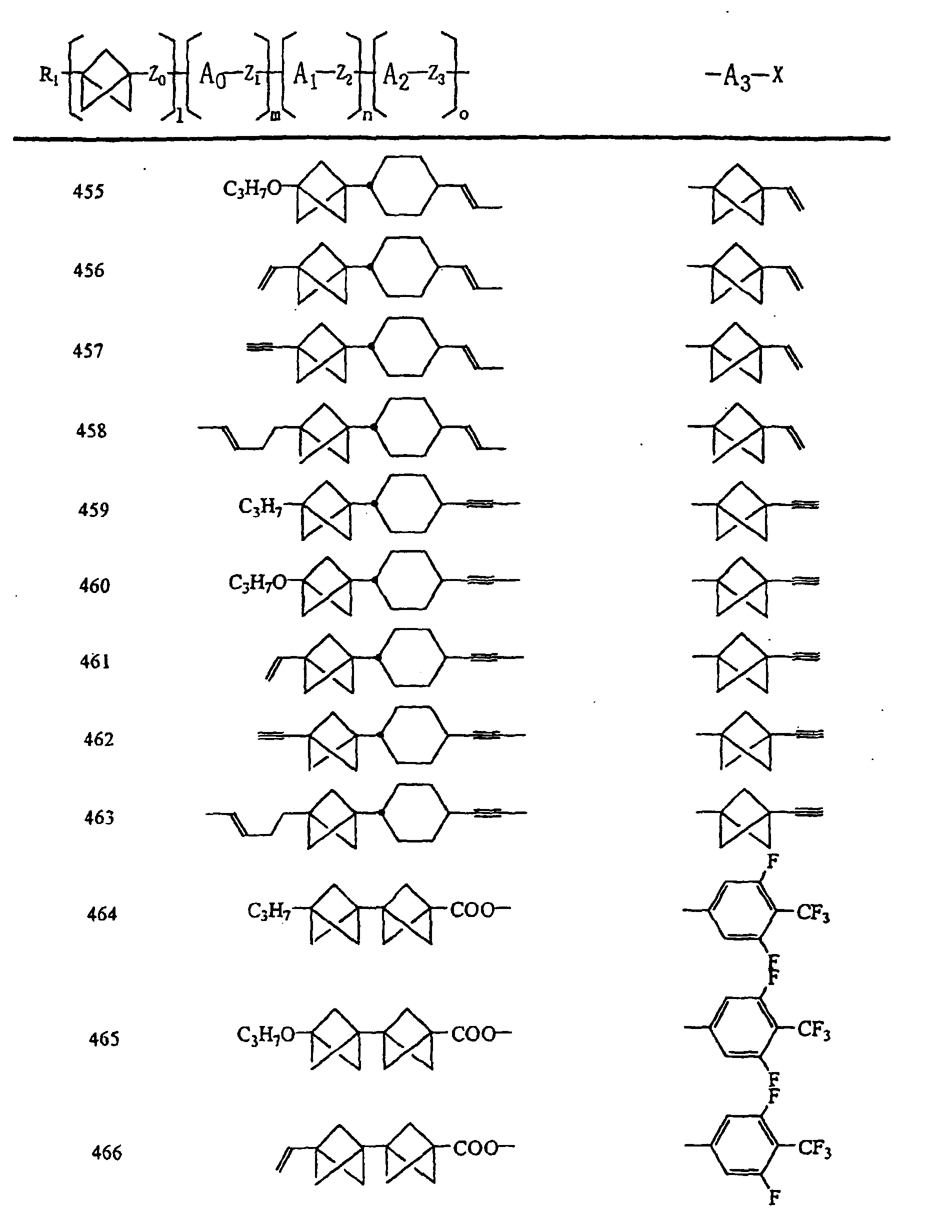

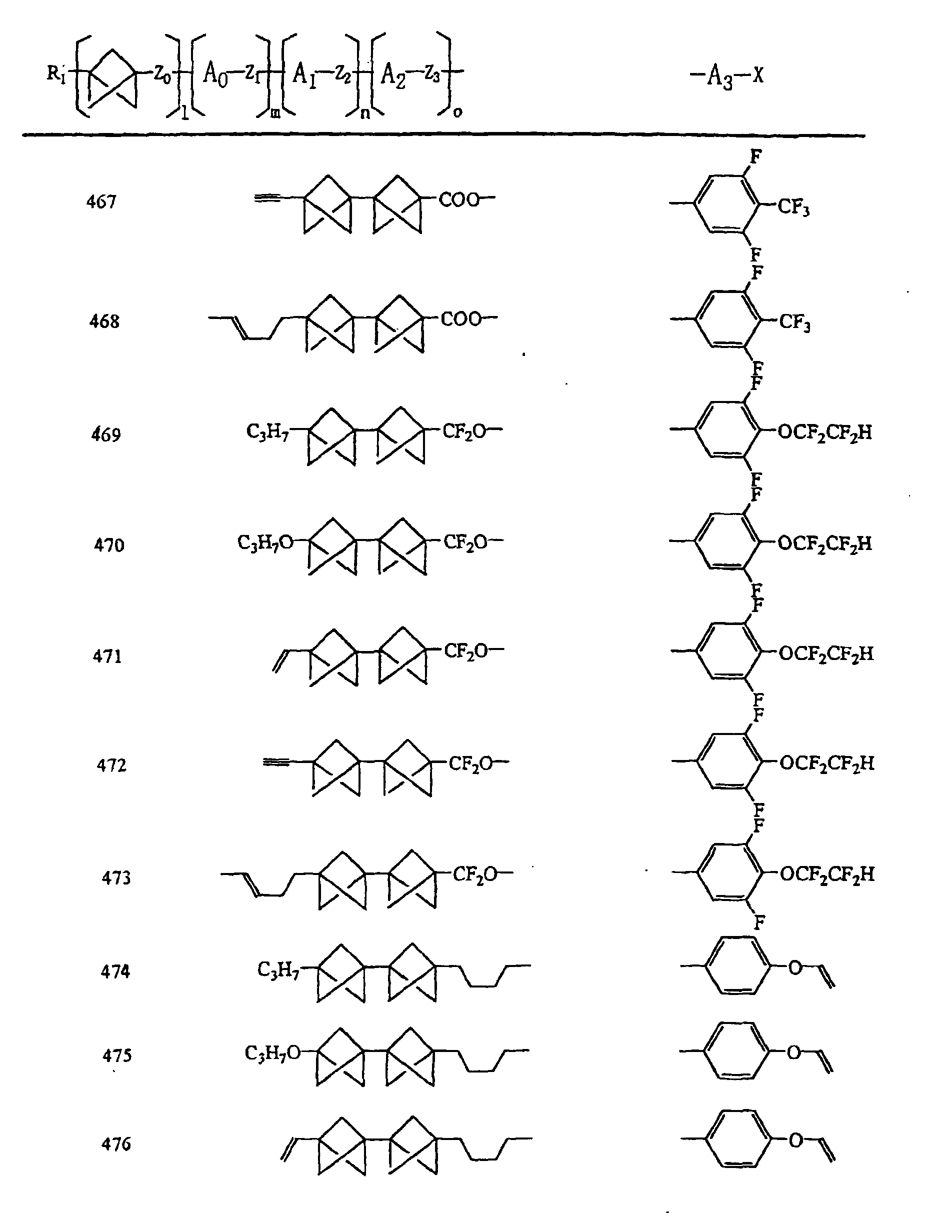

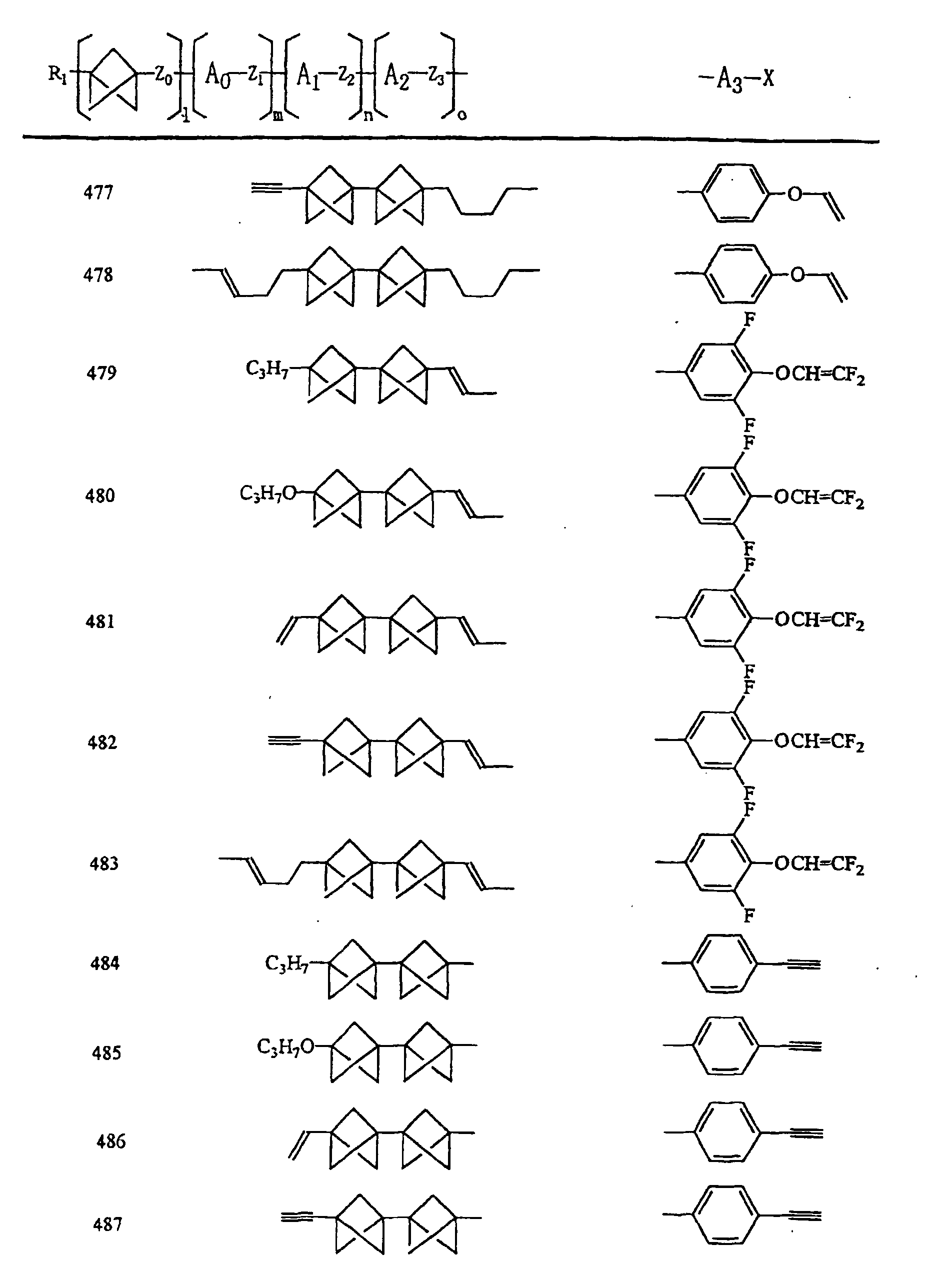

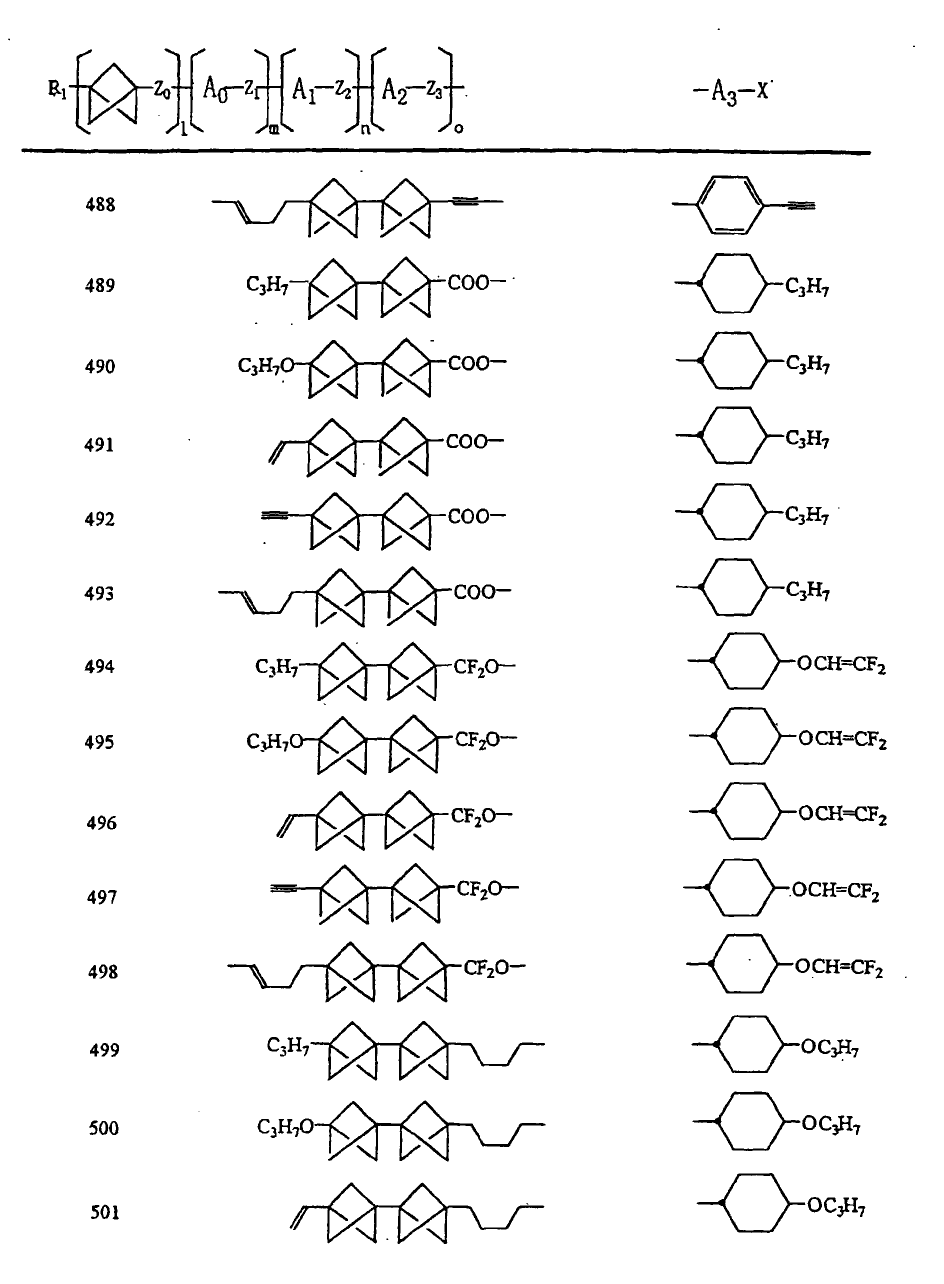

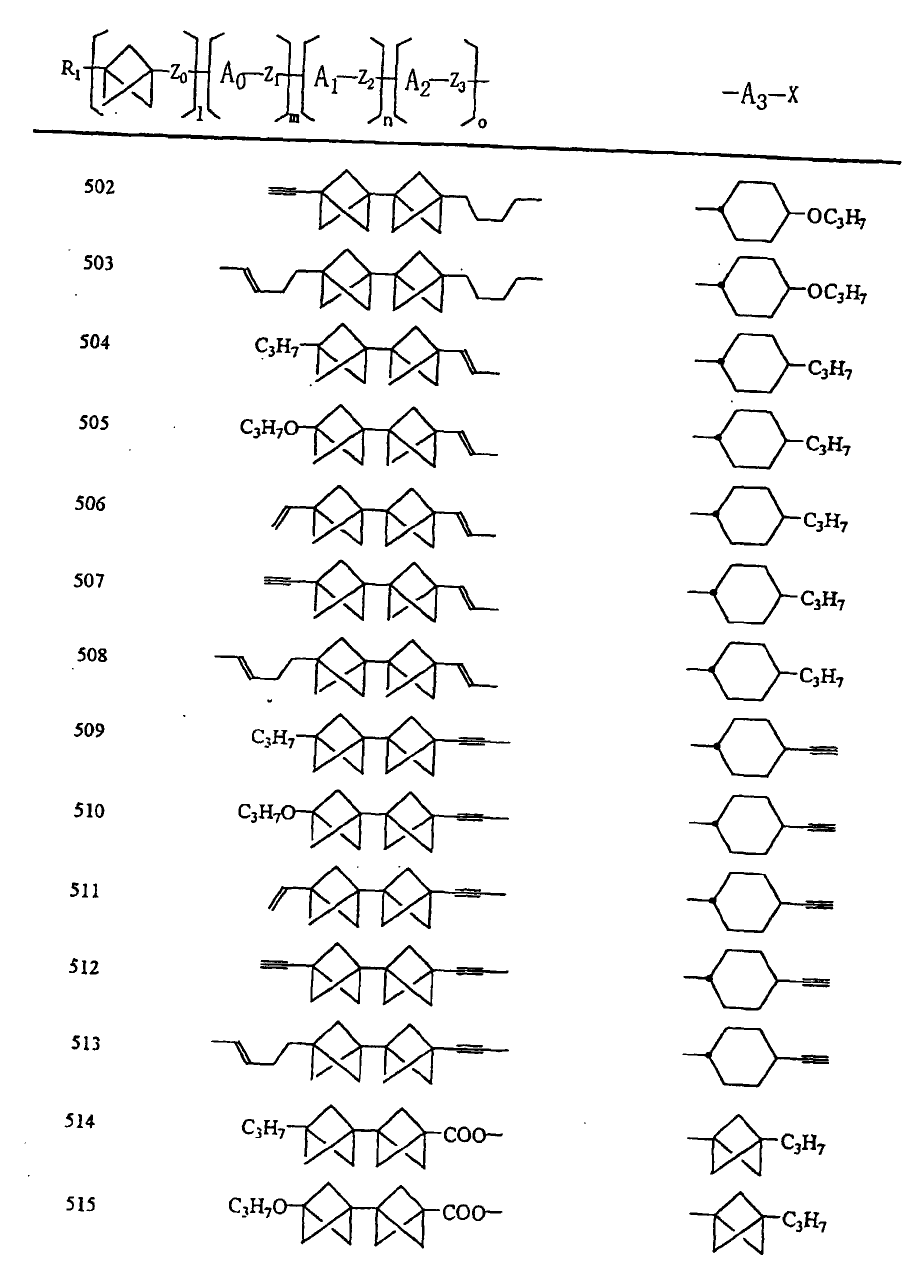

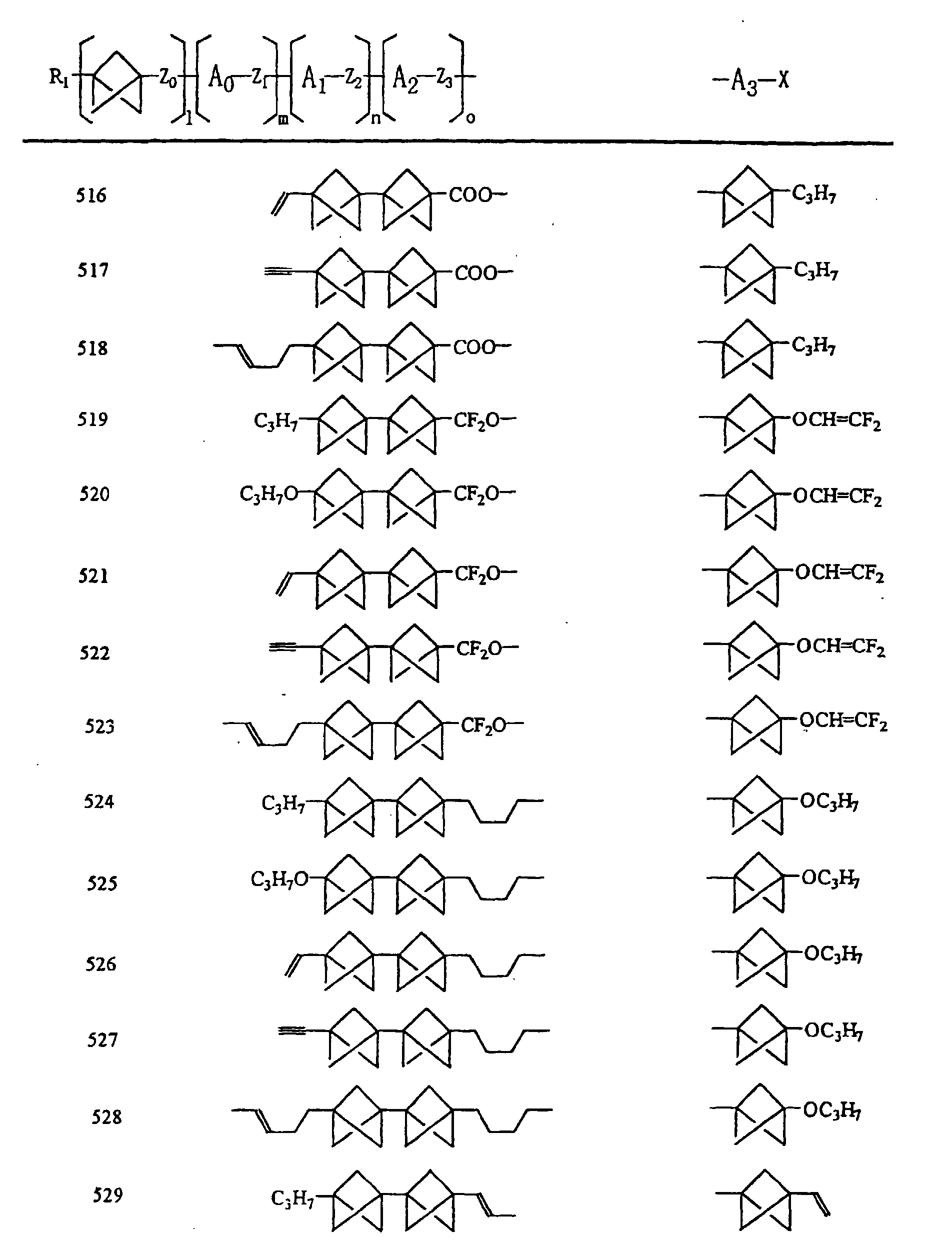

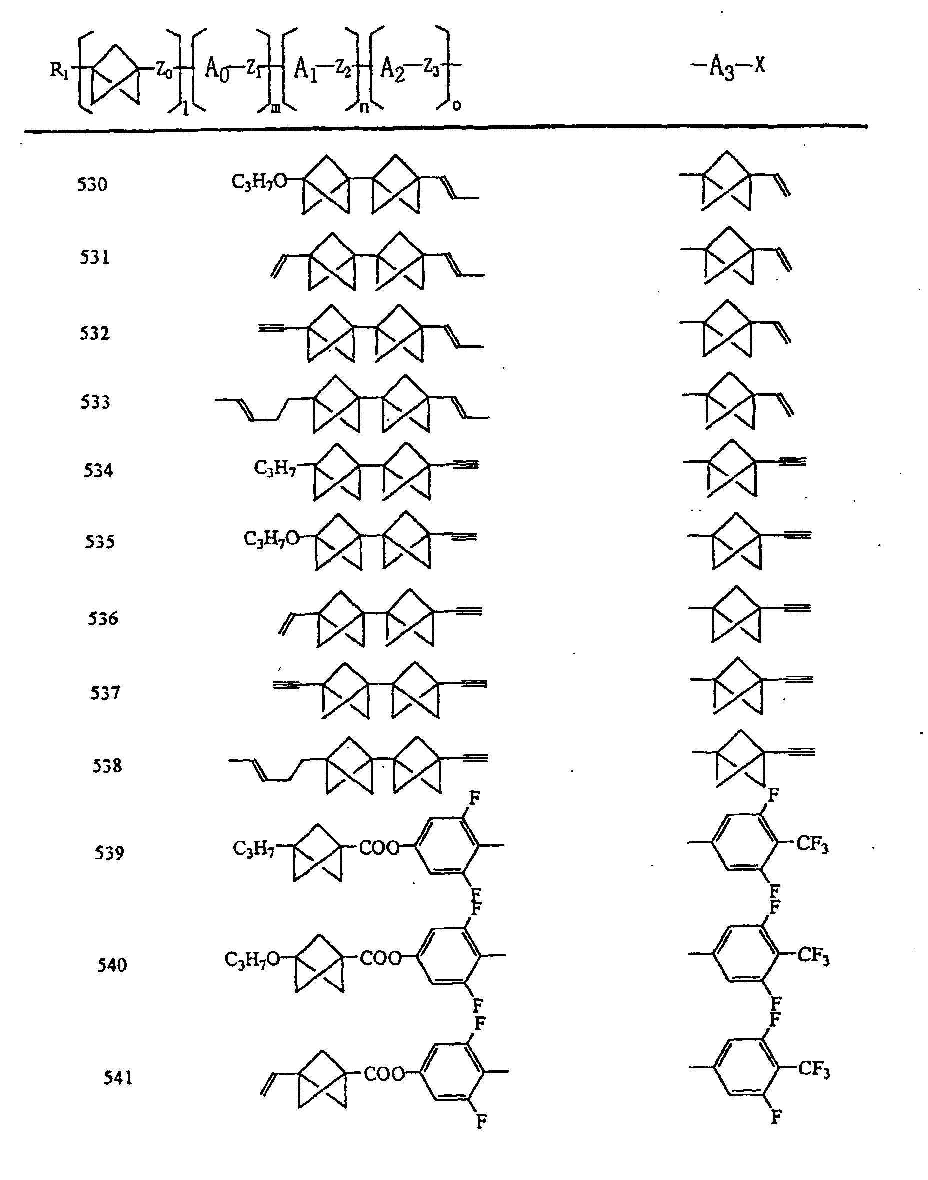

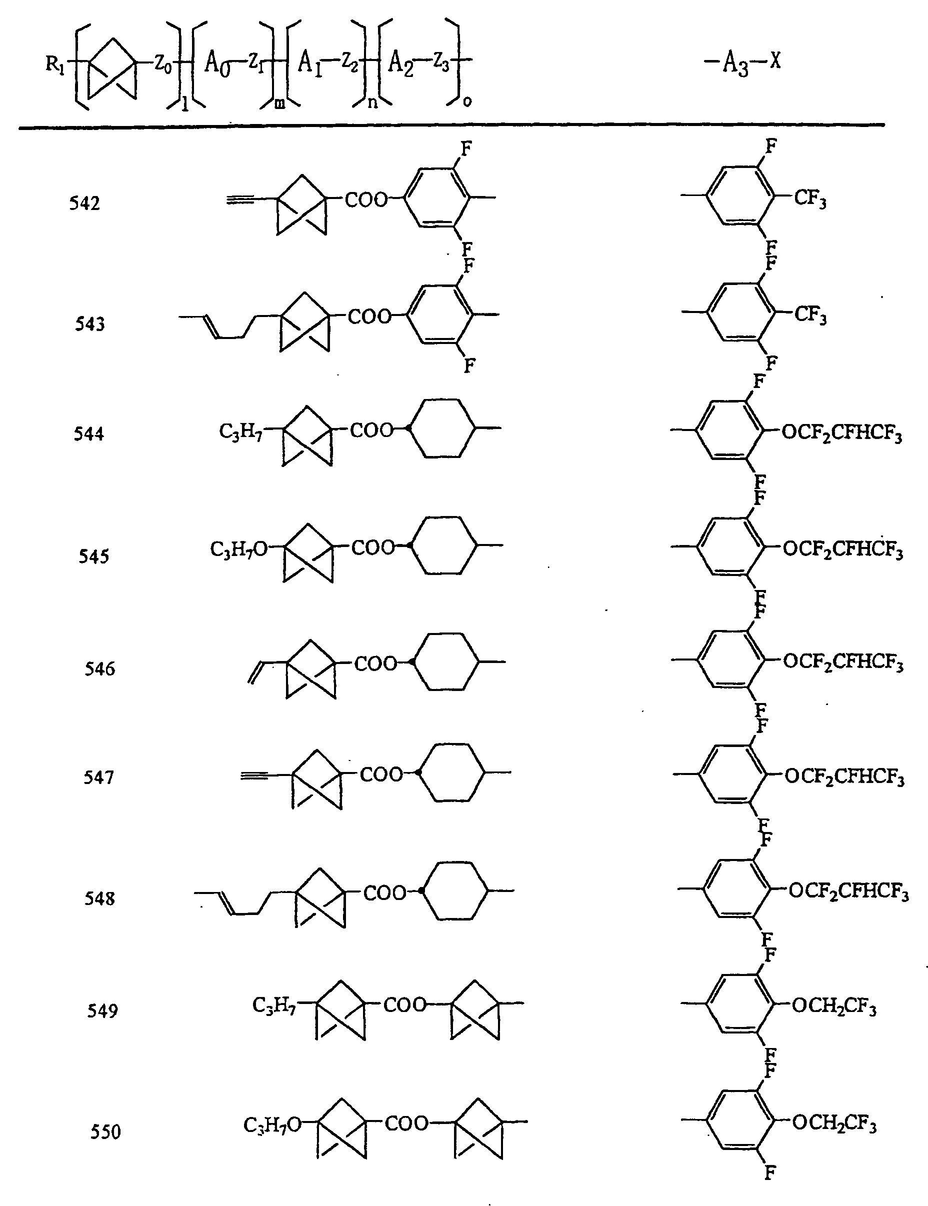

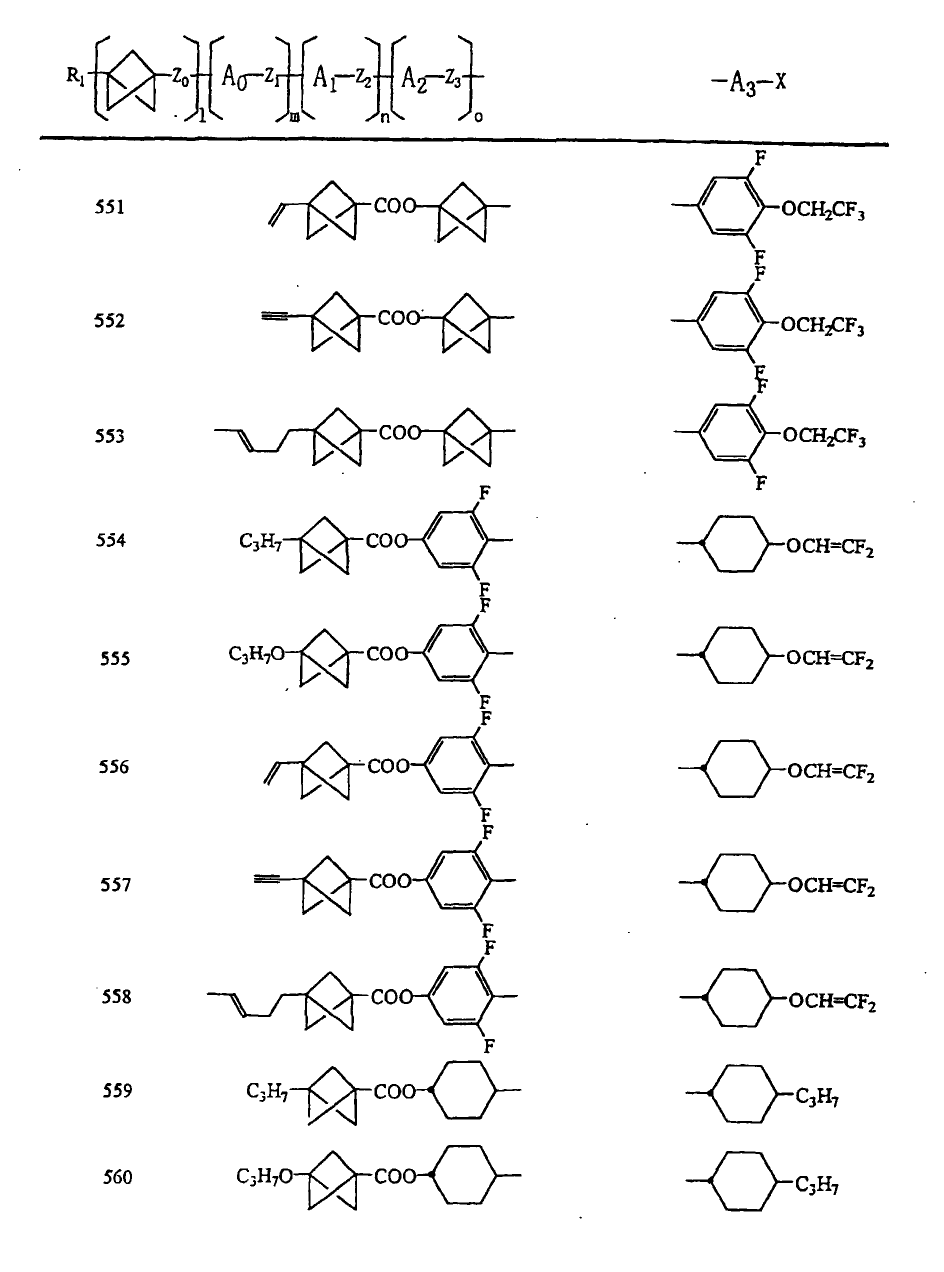

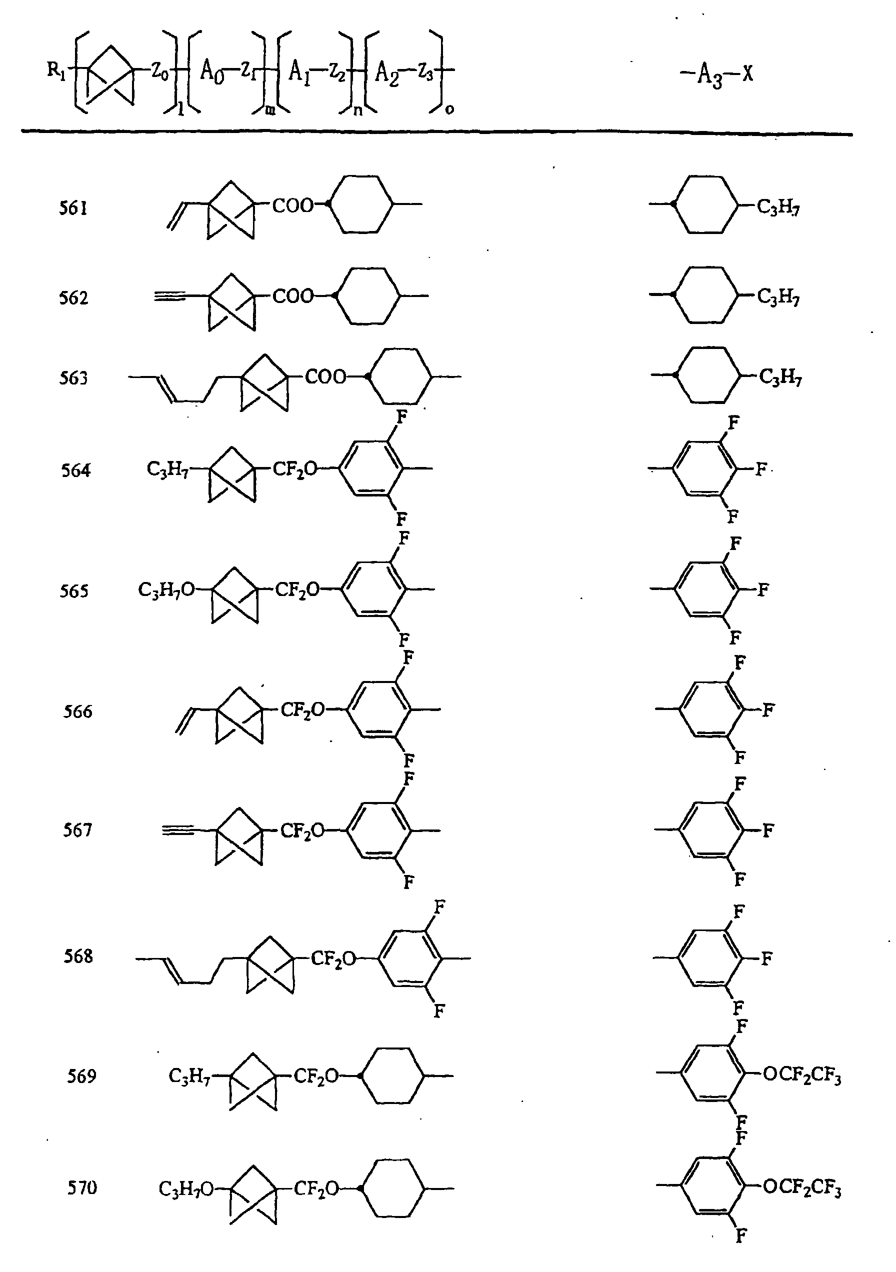

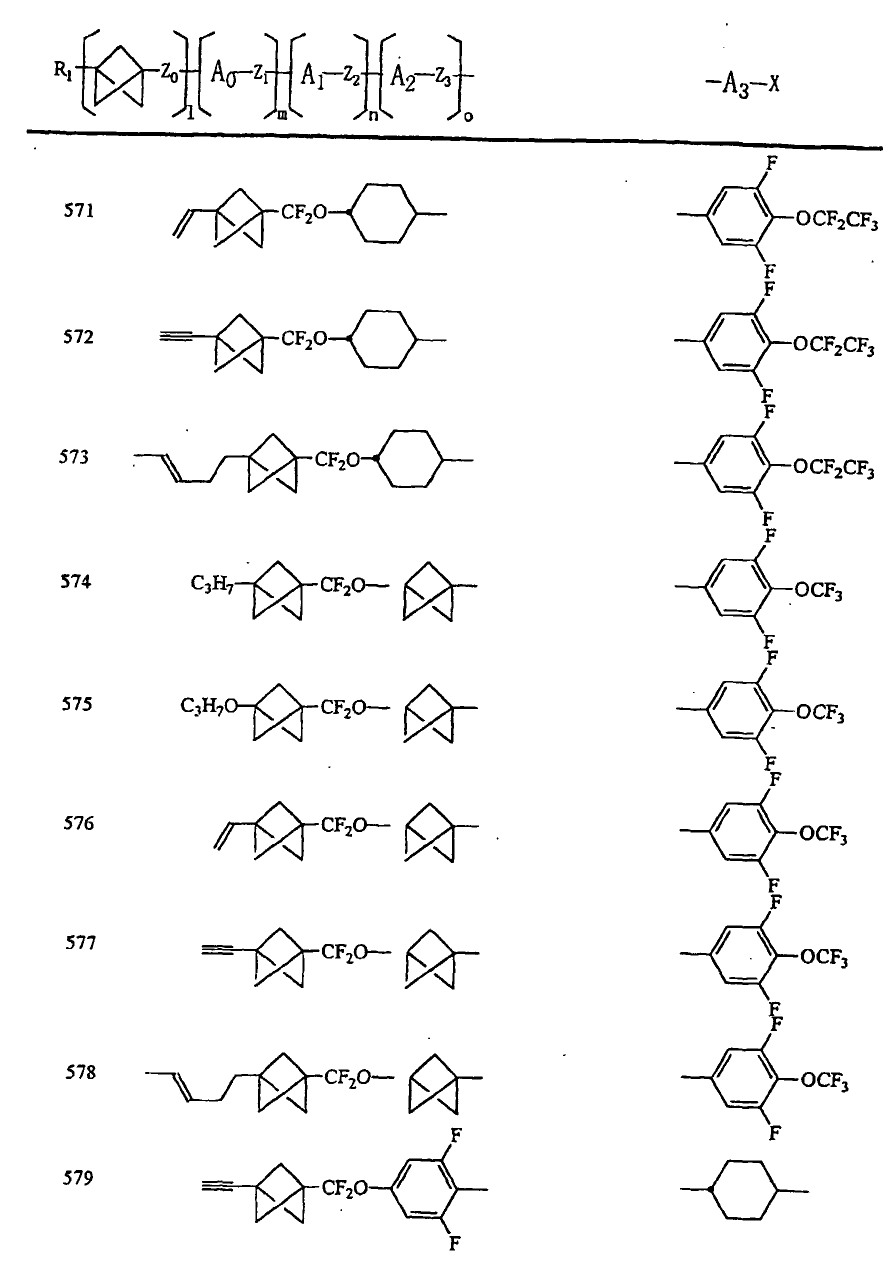

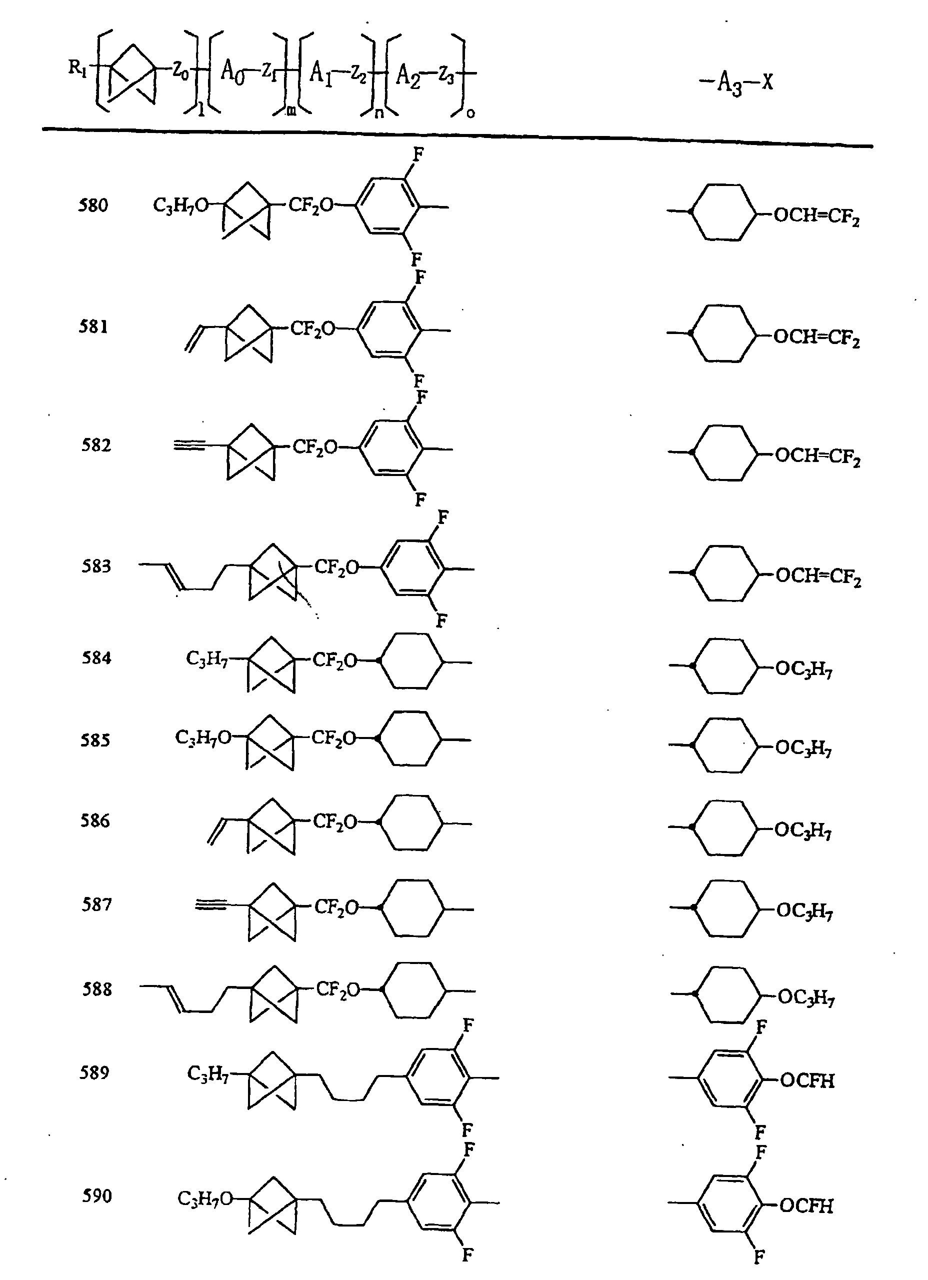

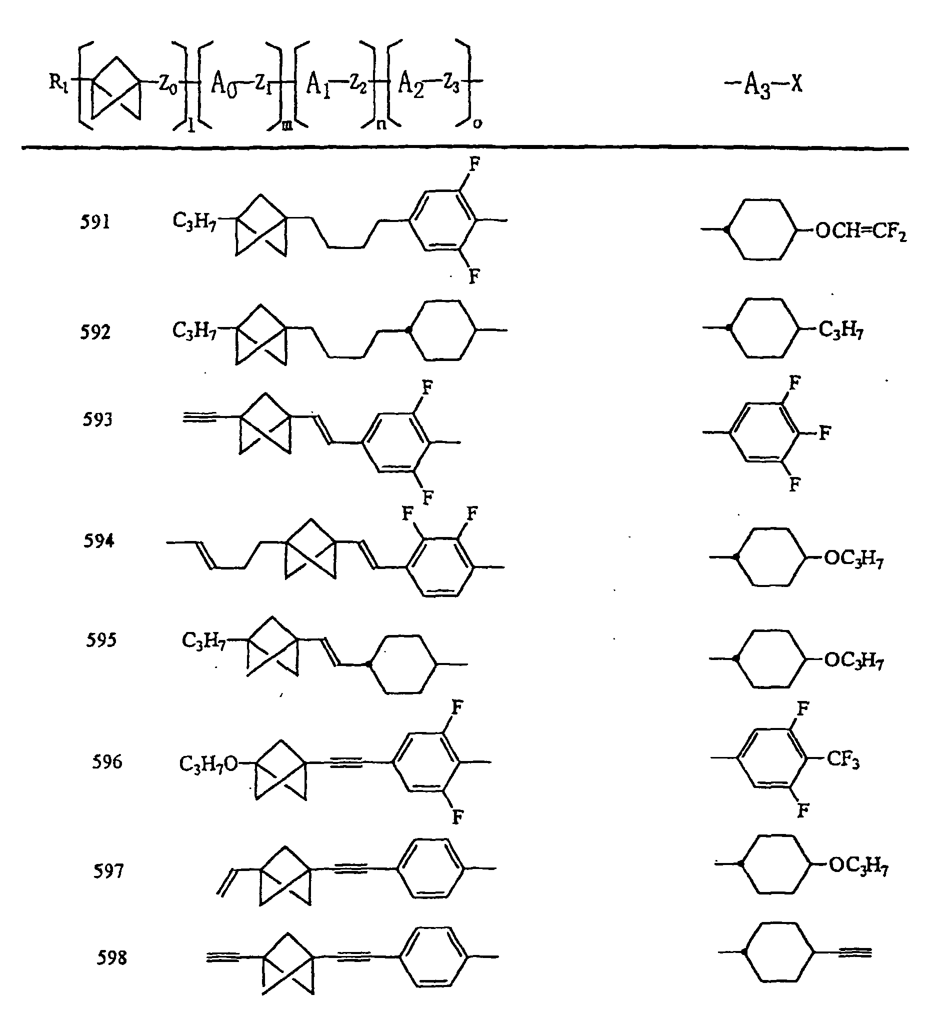

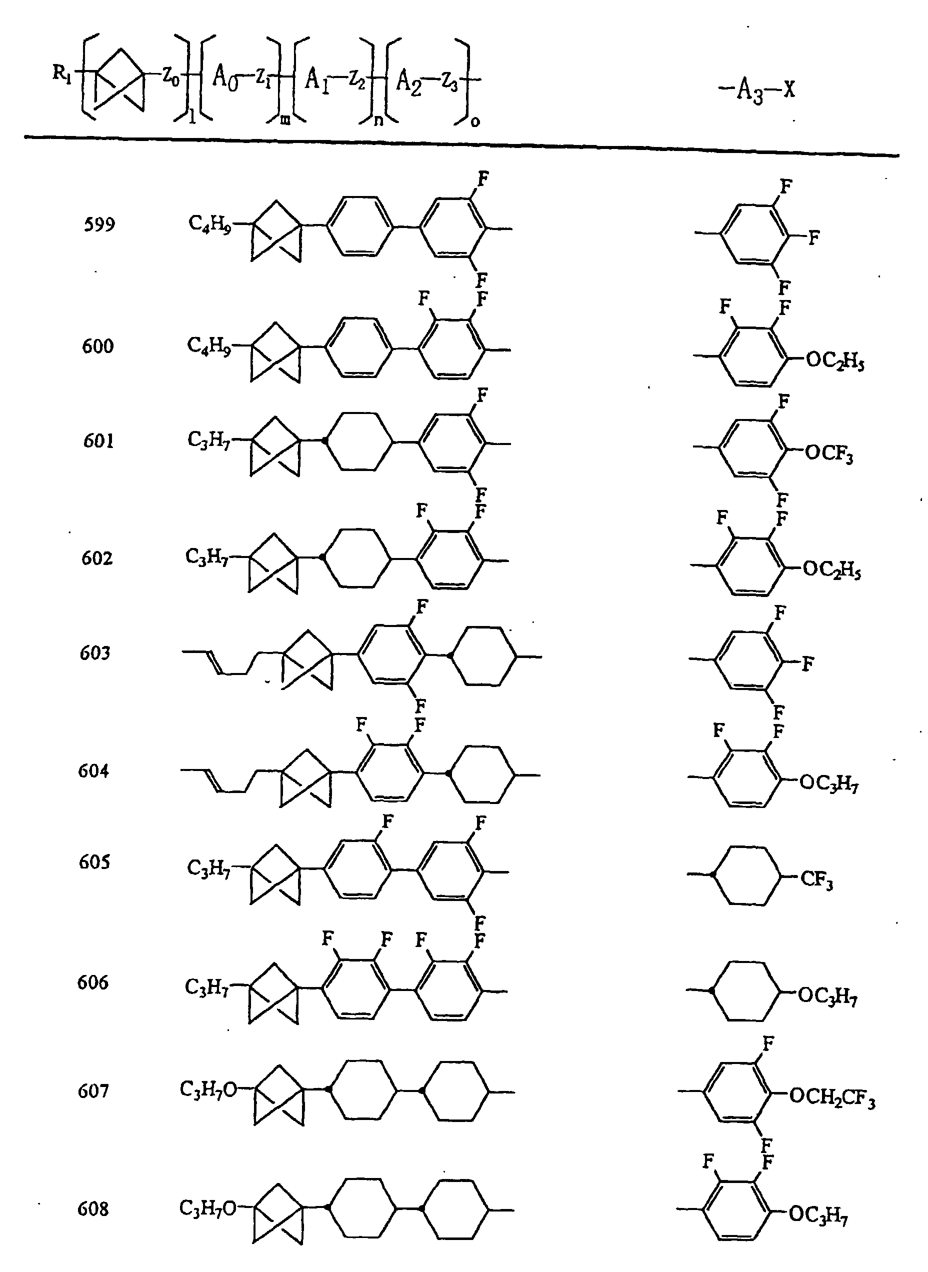

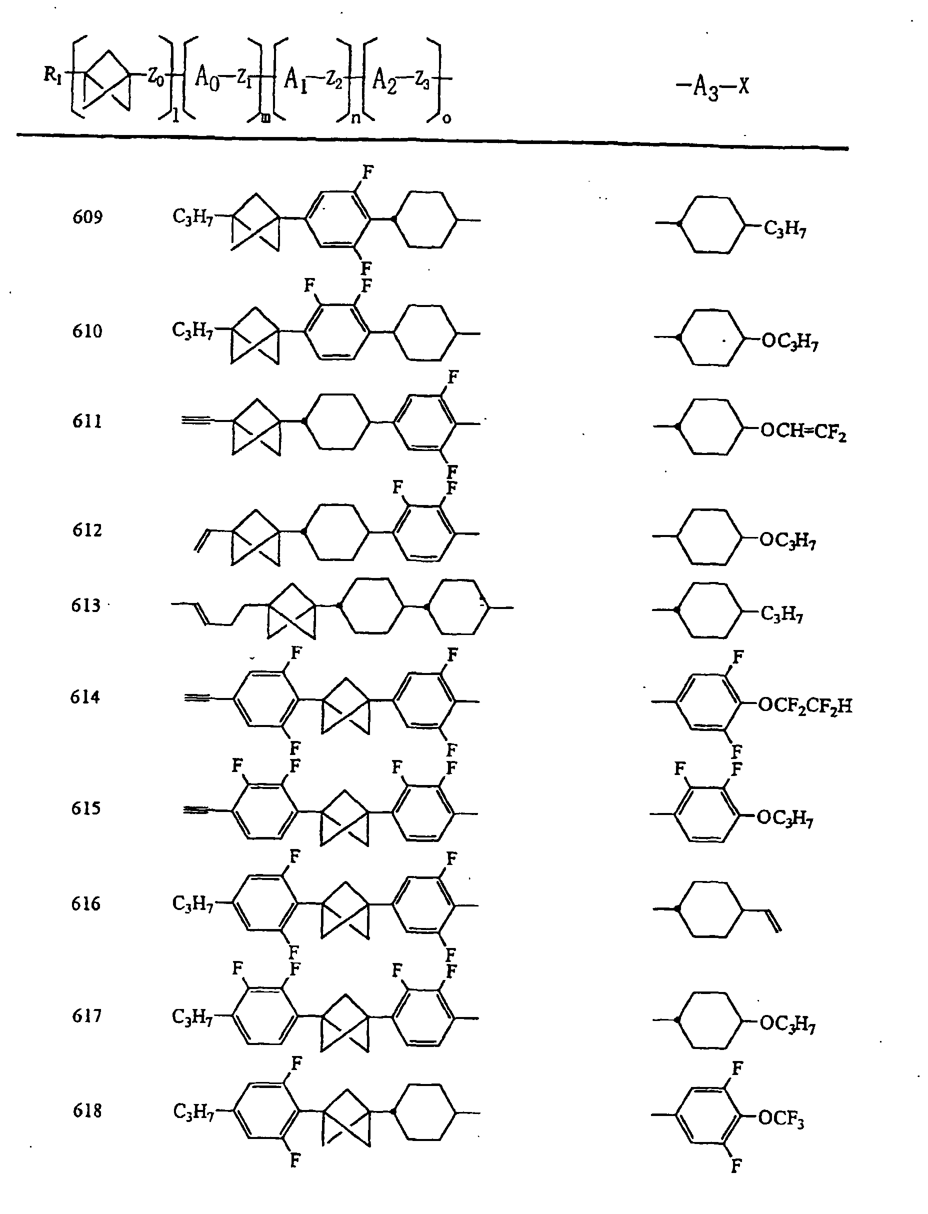

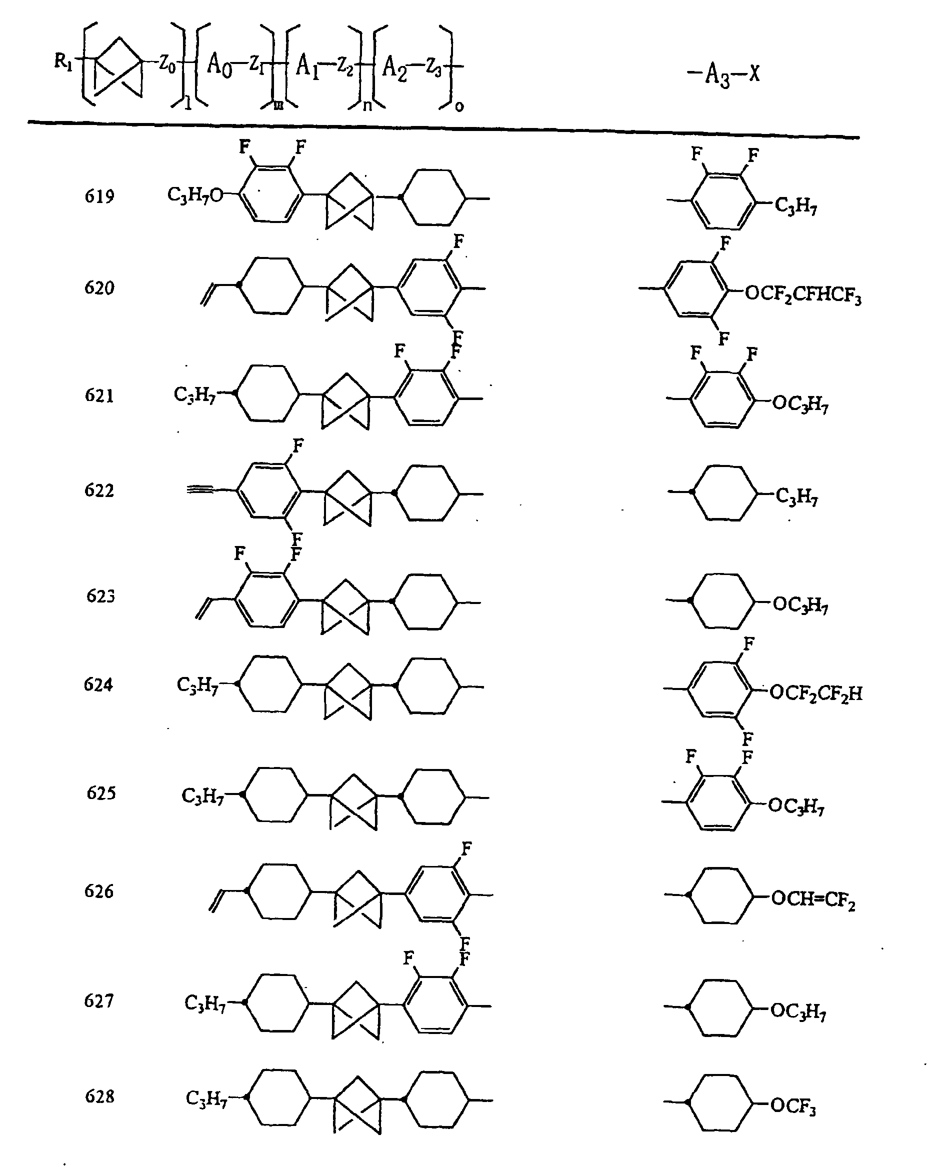

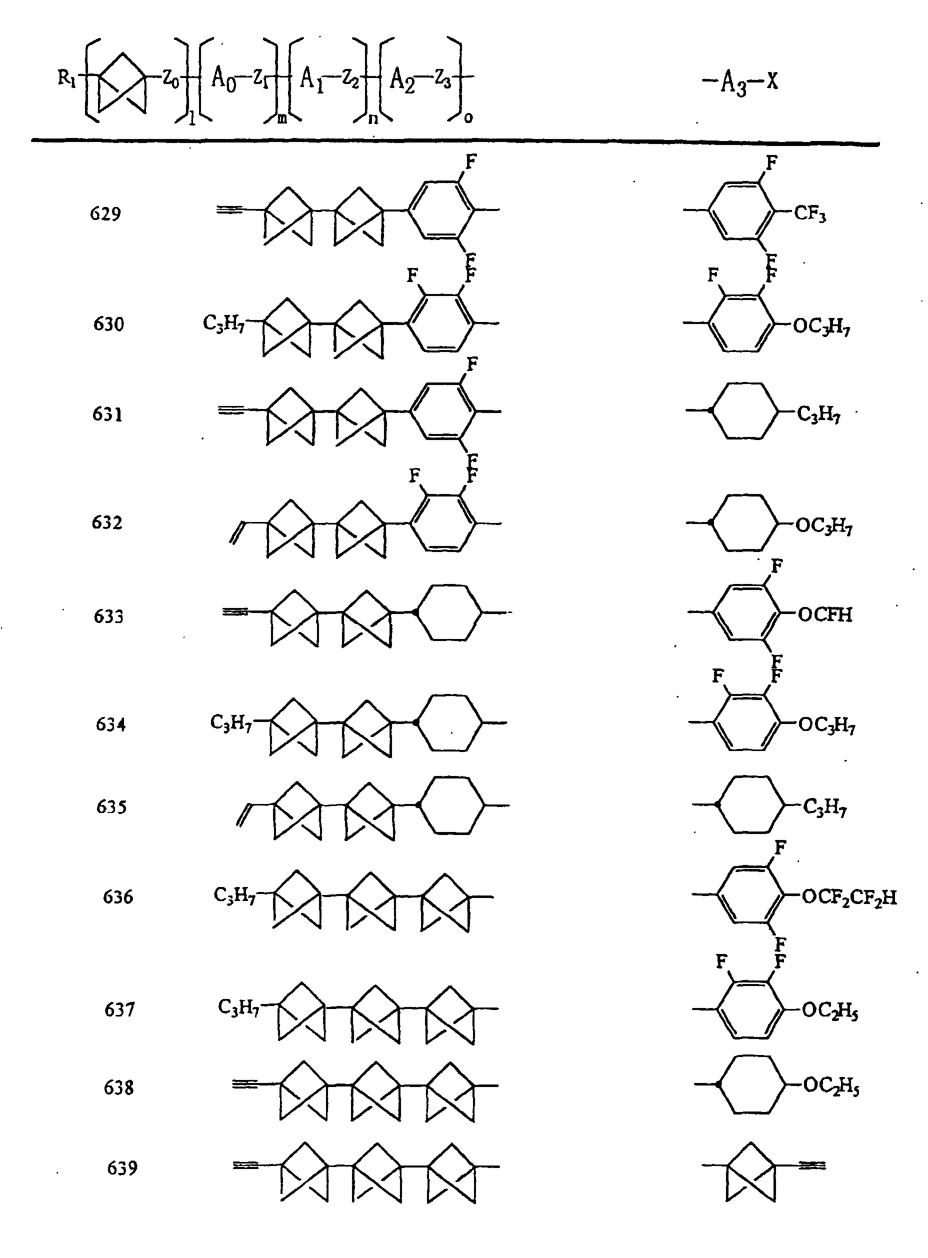

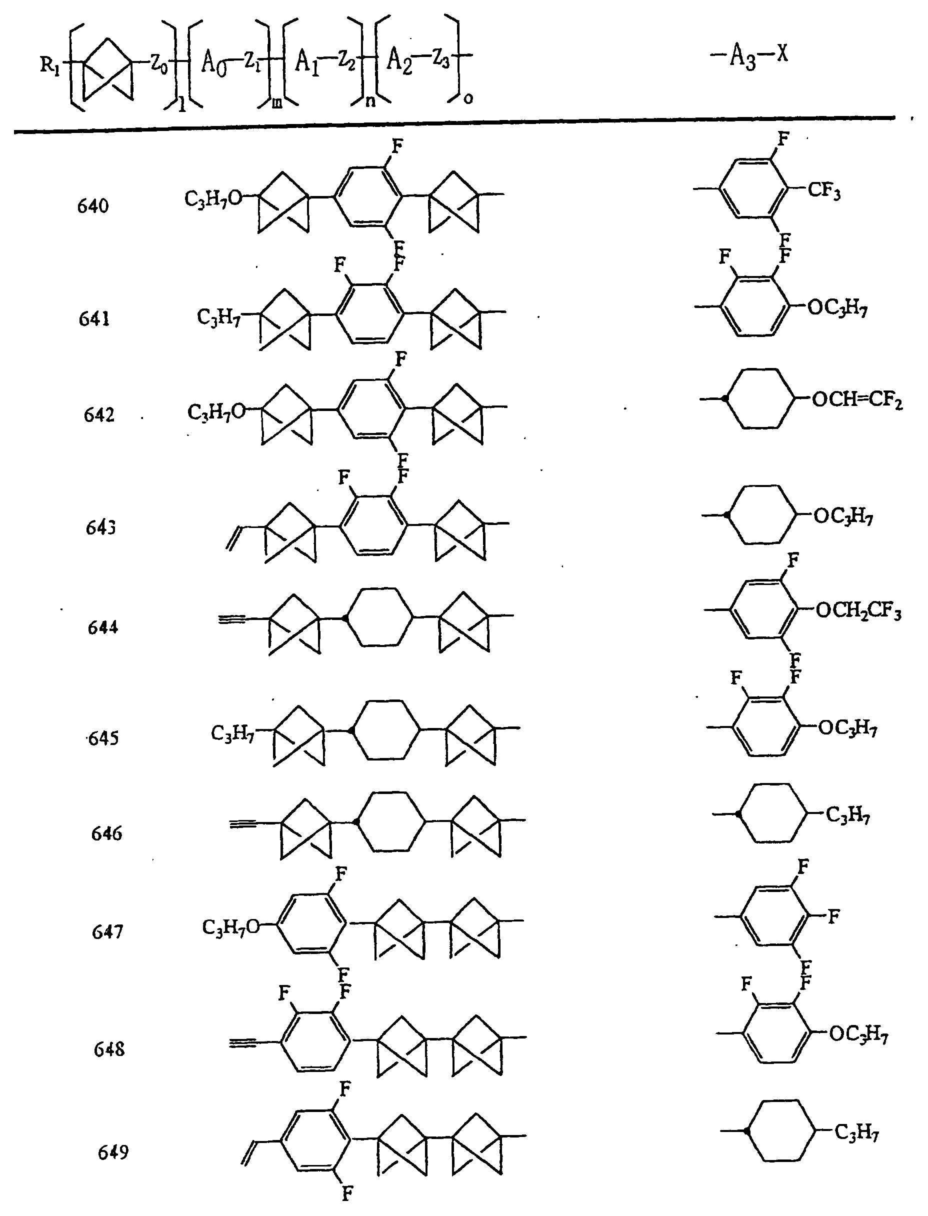

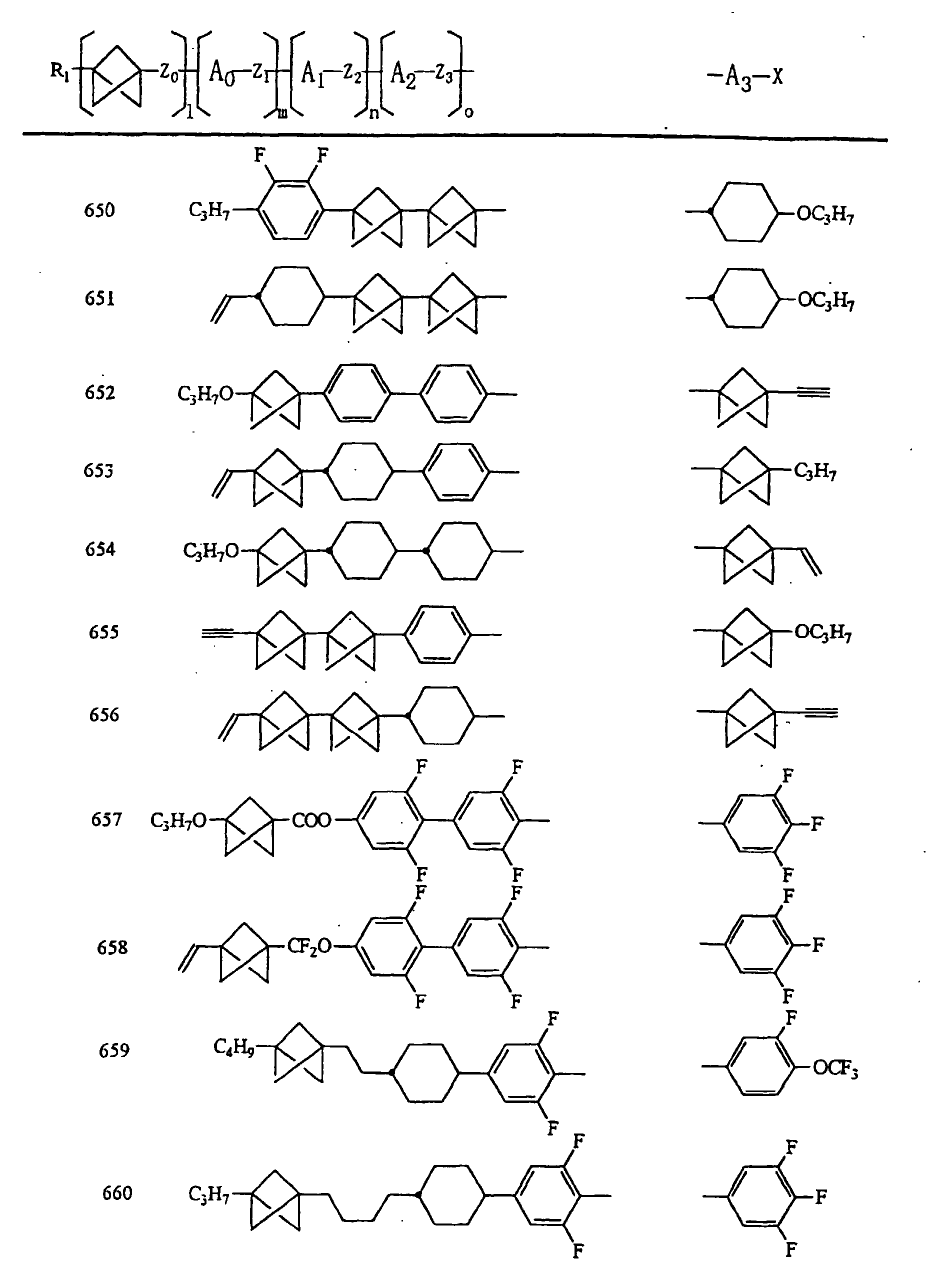

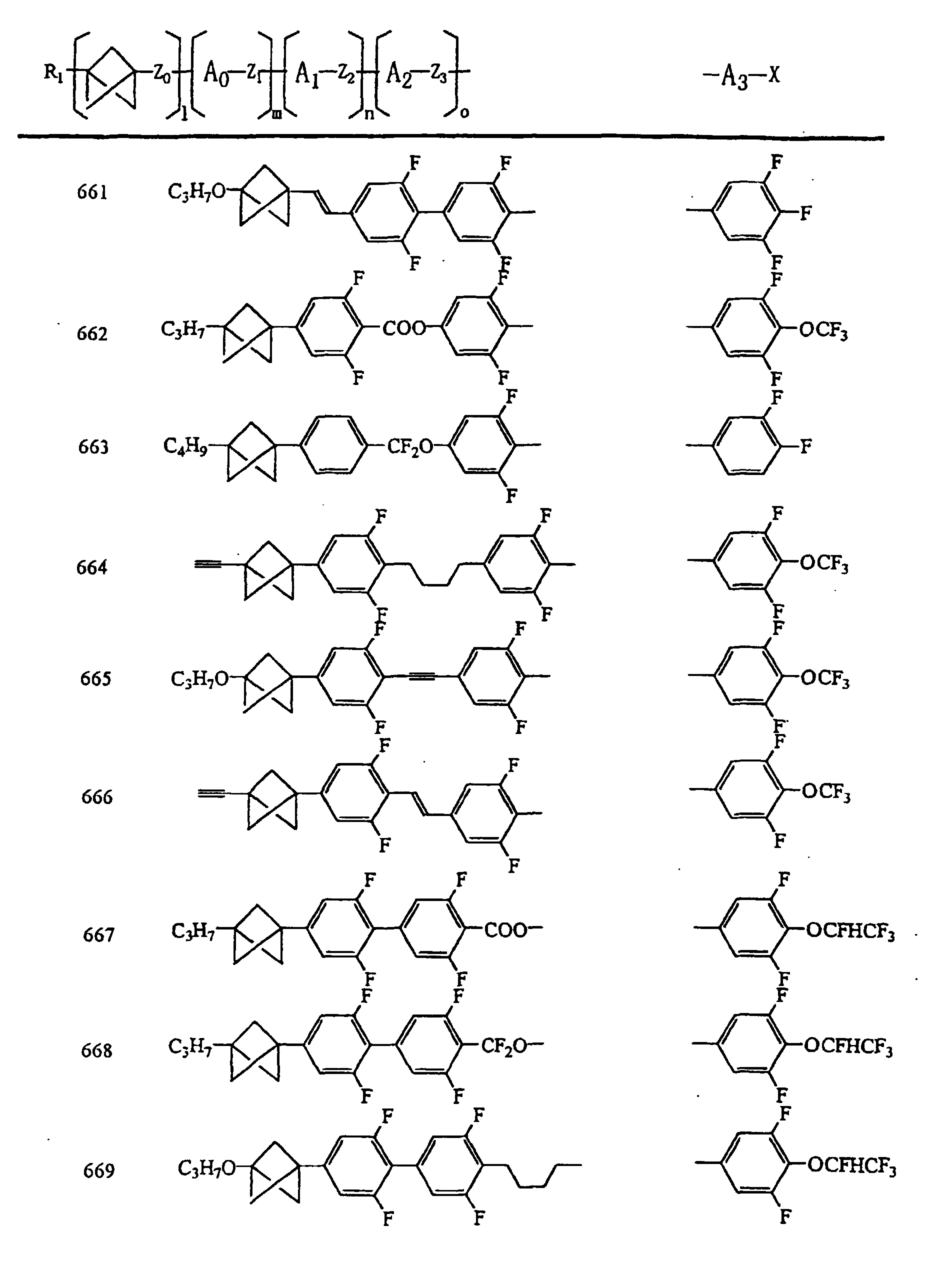

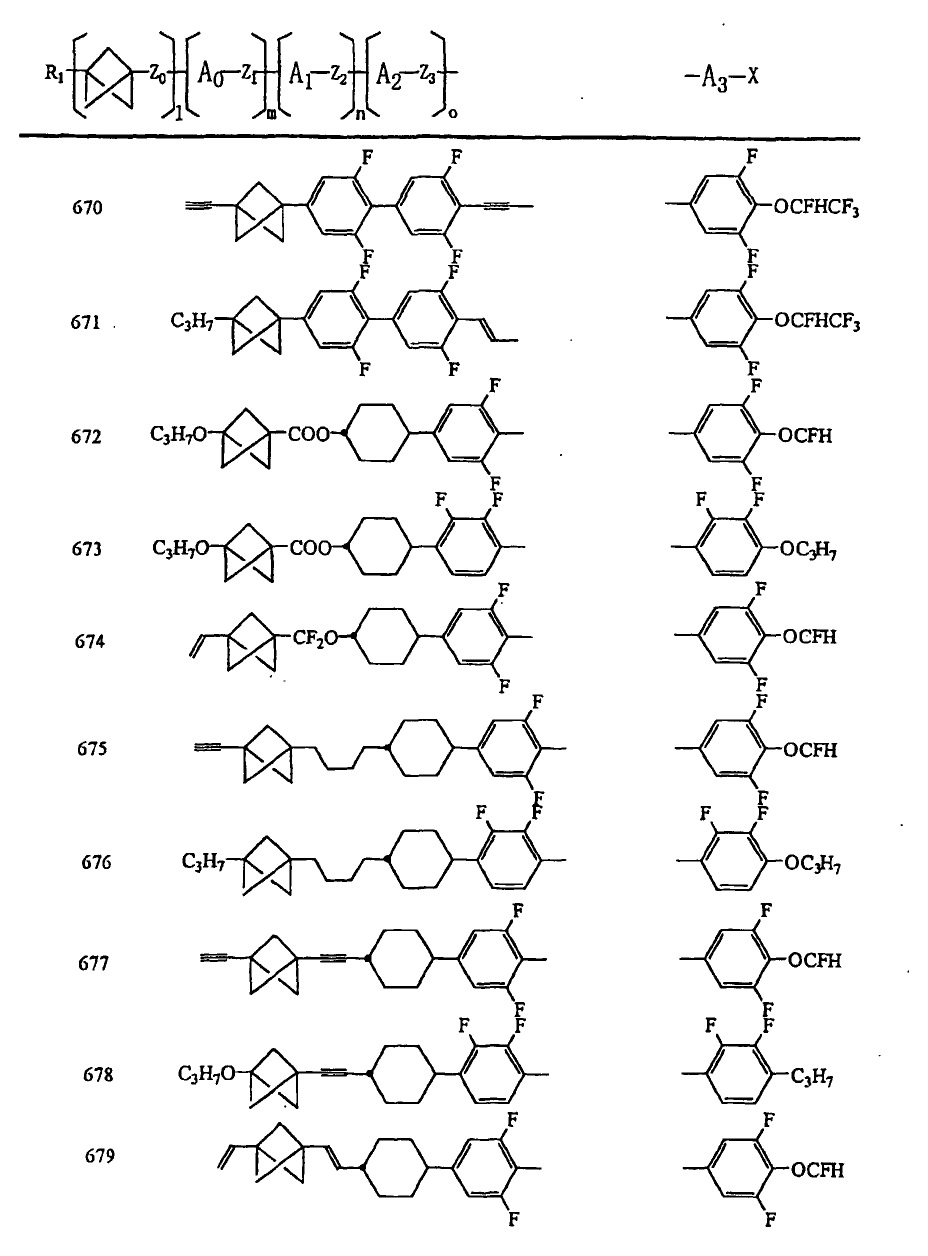

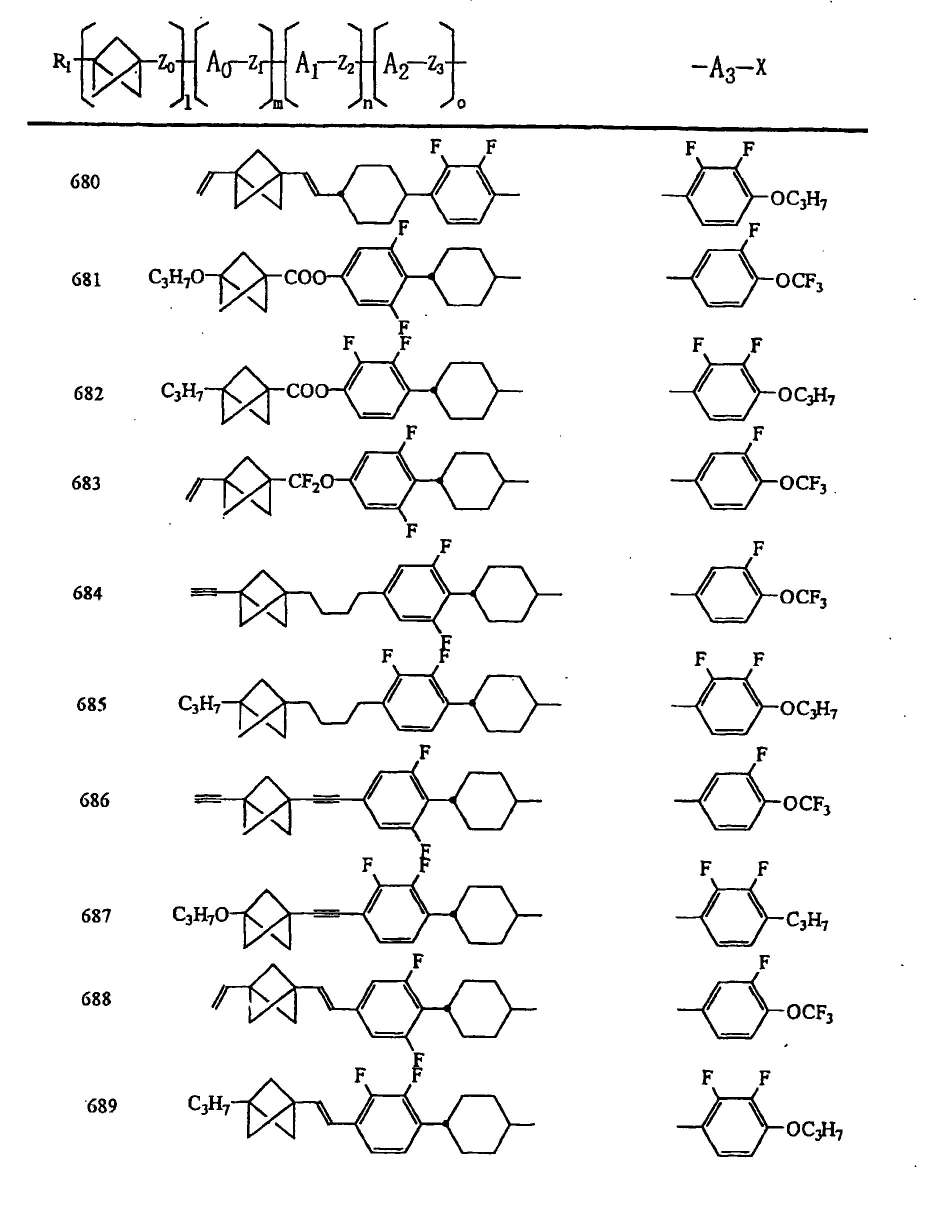

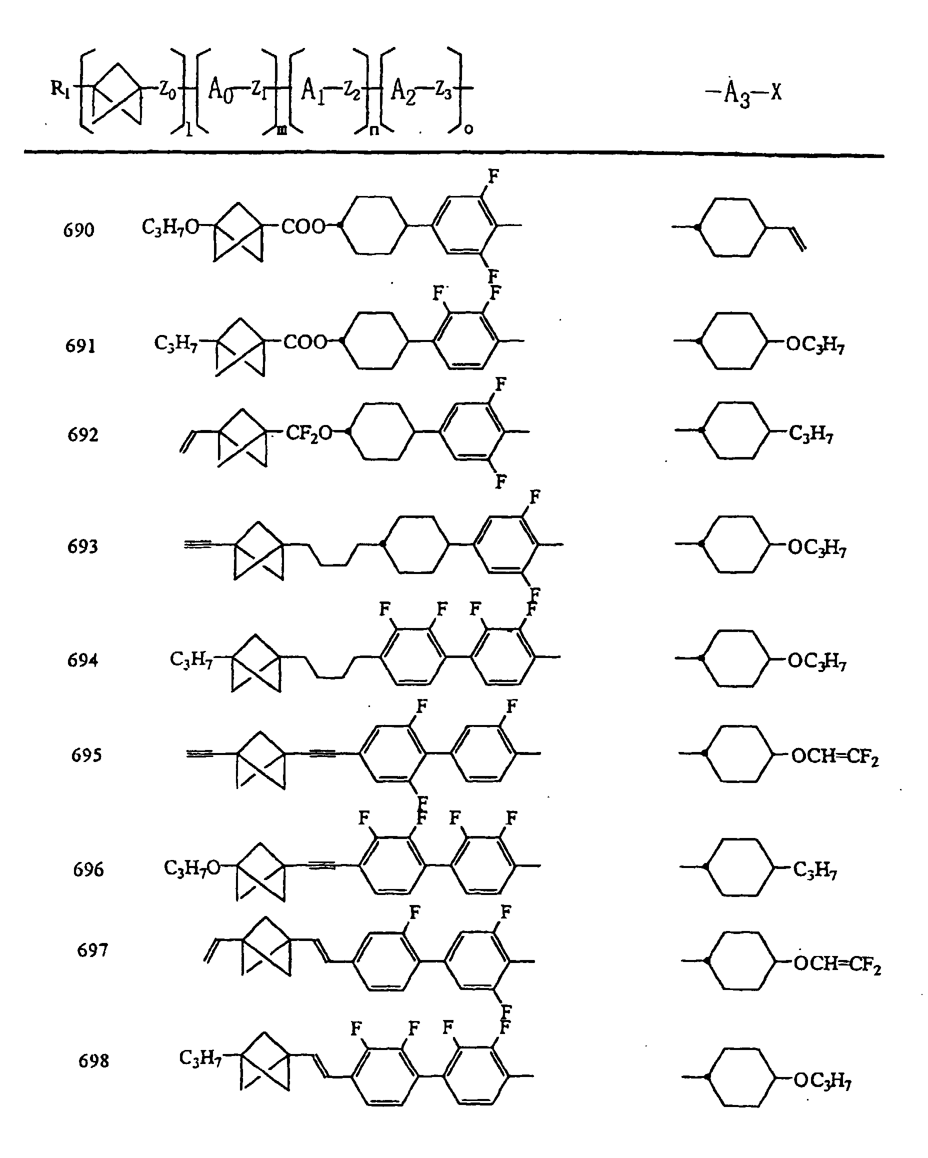

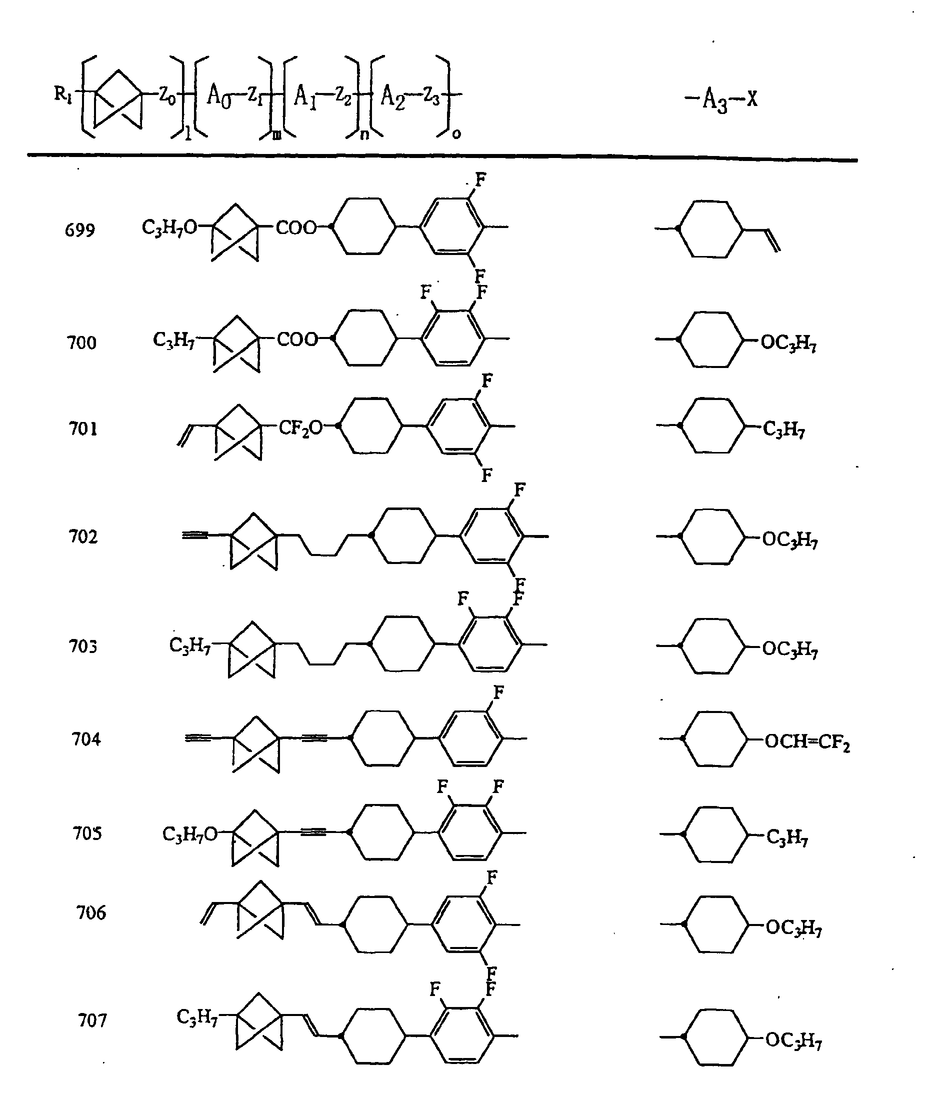

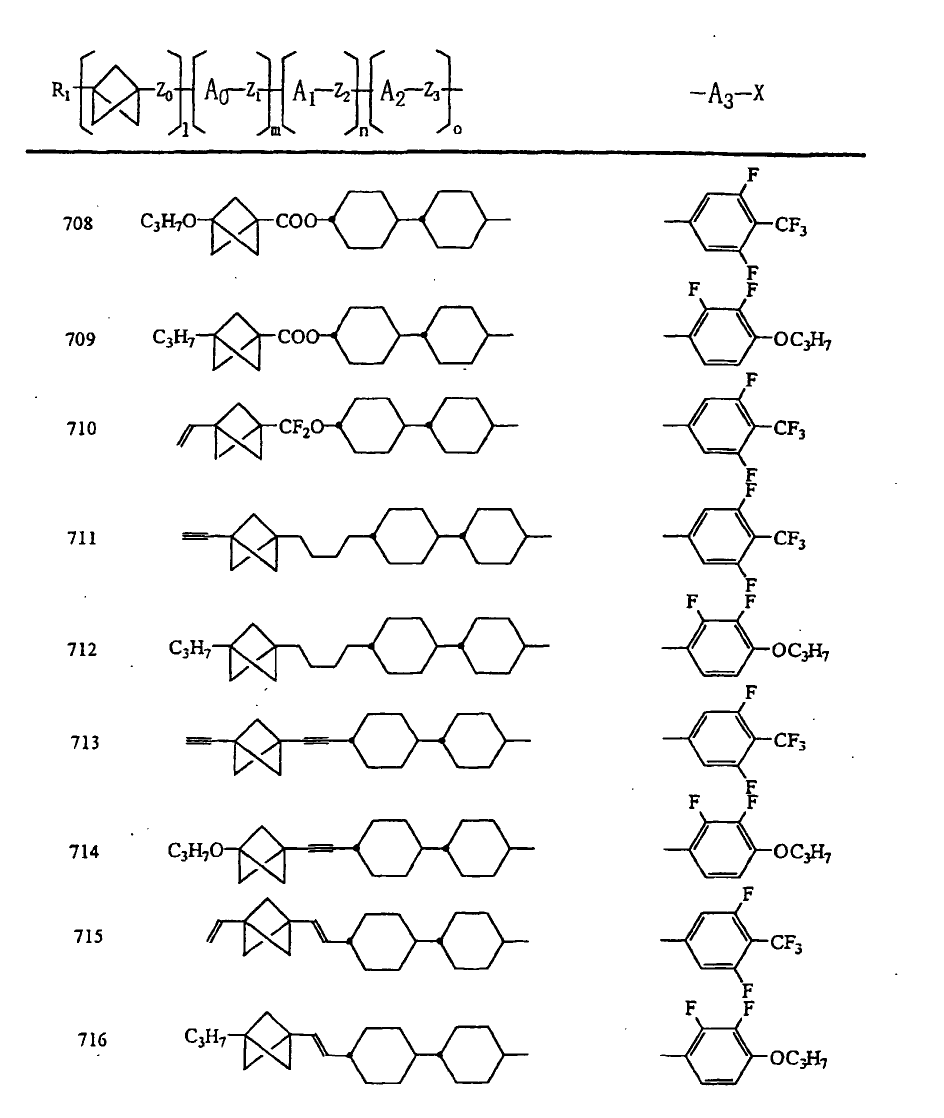

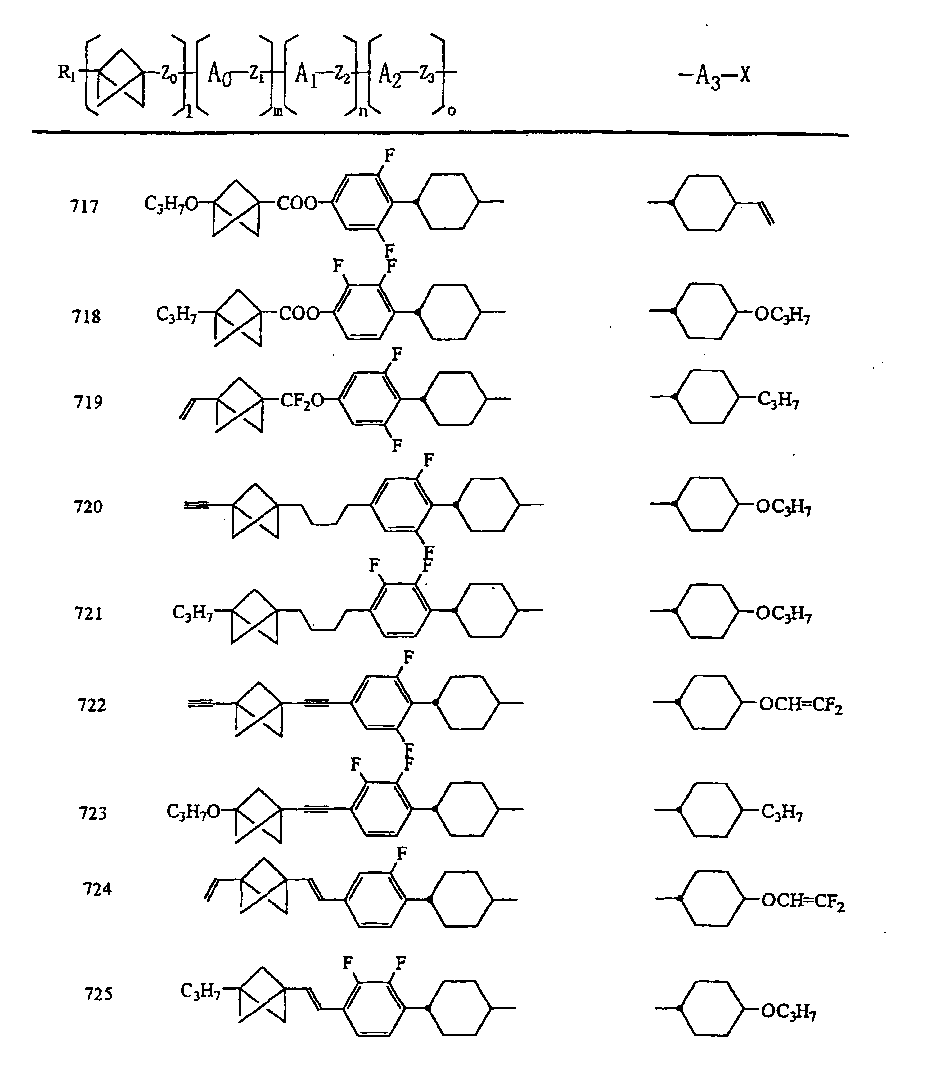

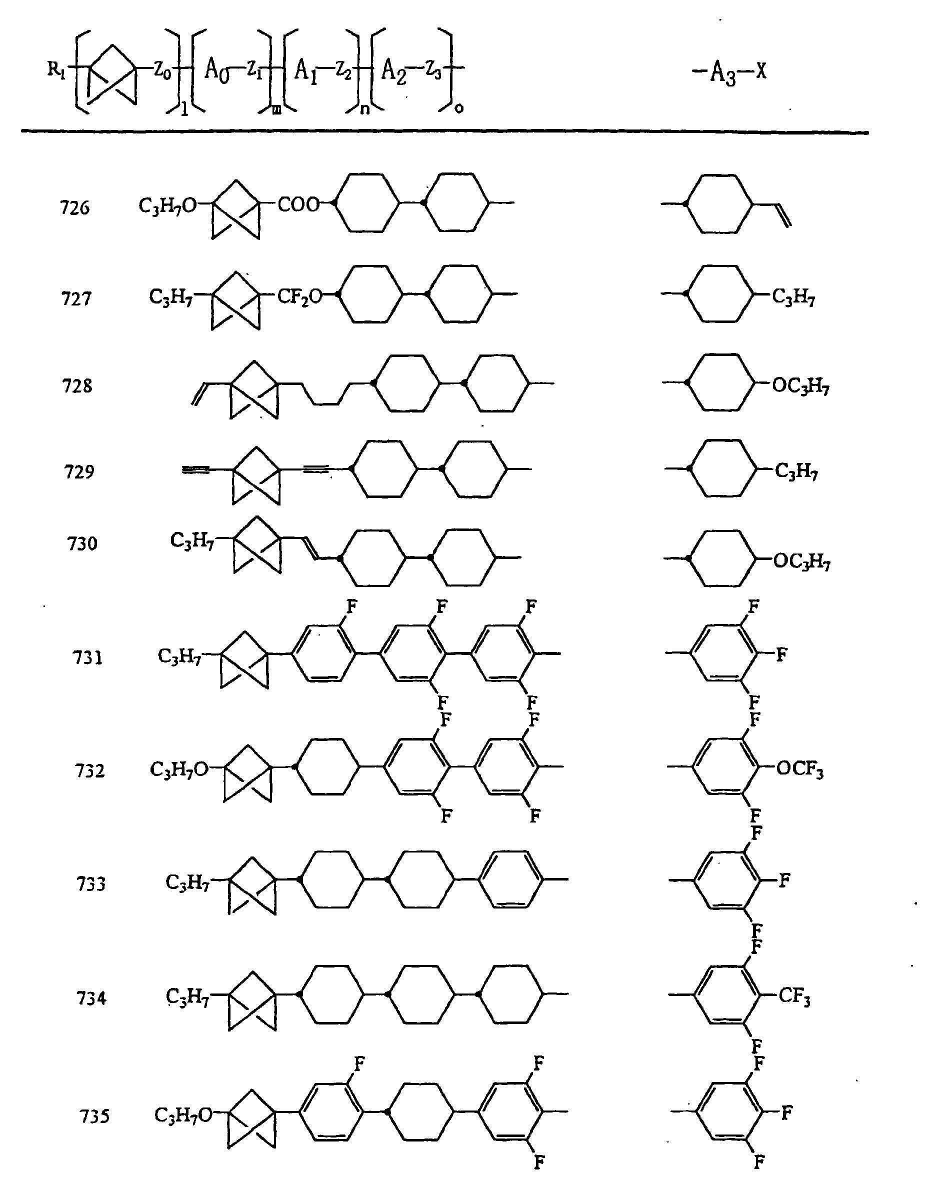

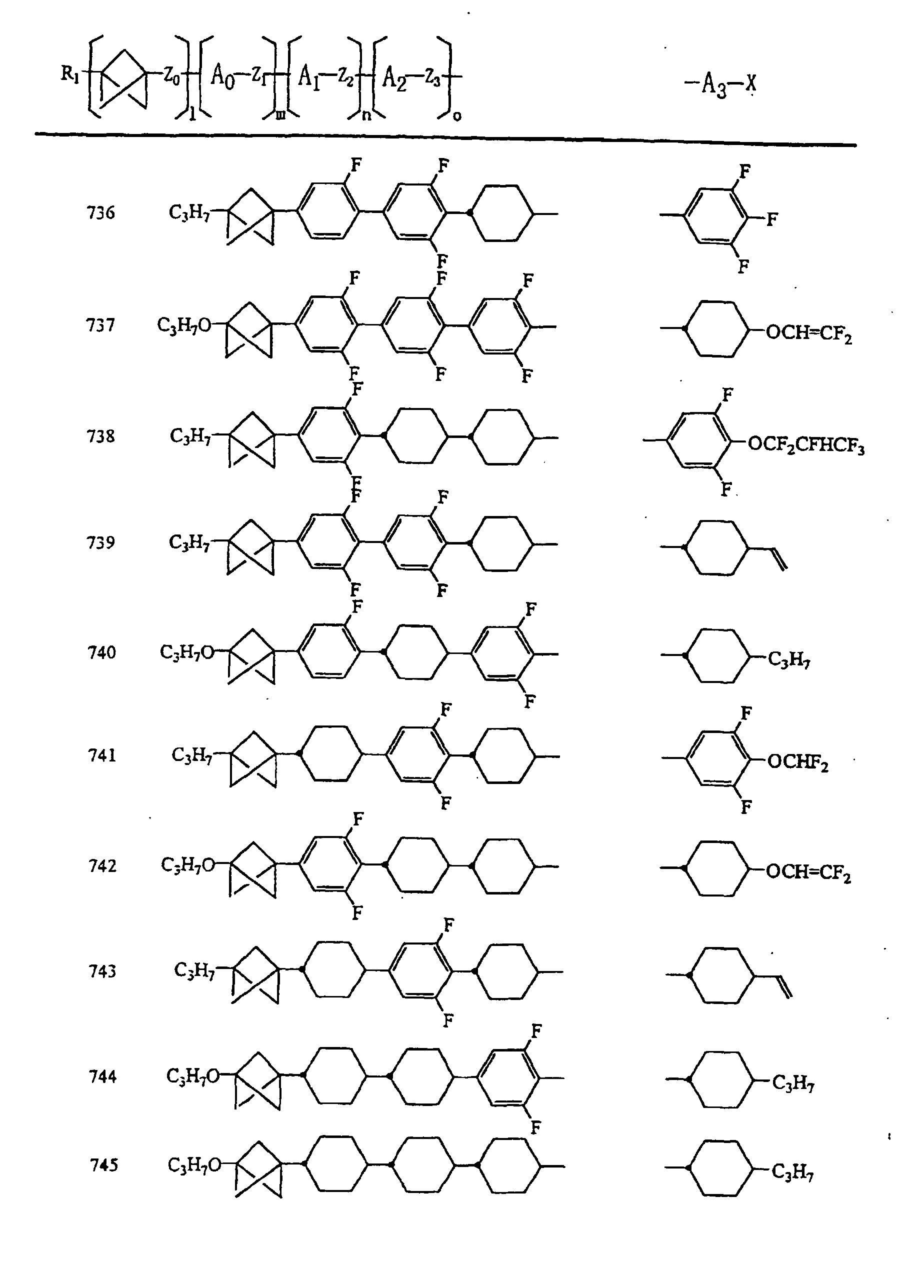

### INDUSTRIAL APPLICABILITY

Compounds of the present invention expressed by the general formula (1) are very stable against the changes in outside environment, exhibit a positive and large dielectric anisotropy value or a negative and large dielectric anisotropy value, and also show a high voltage holding ratio and excellent miscibility with other liquid crystalline compounds by selecting proper substituents, bonding groups, and ring structures. Further, when the compound of the present invention was used as component of liquid crystal compositions, the miscibility of compositions at low temperatures is improved, that is, nematic phase at cryogenic temperatures can be stabilized while maintaining viscosity and voltage holding ratio, elastic constant ratio (K33/K11) of whole composition can efficiently be lowered, and the steepness of "Voltage-Transmittance characteristic" of liquid crystal compositions principally for TN and TFT can be improved in the case, for example, of two ring compounds. Besides, when three to five ring compound was used as component of liquid crystal compositions, widening of the temperature range of liquid crystal phase is possible and increase of voltage holding ratio particularly at high temperatures is possible while maintaining the miscibility at cryogenic temperatures. As described above, it is possible to provide liquid crystal compositions having improved characteristics compared with prior art technology, by using the compound of the present invention as component of liquid crystal compositions.

## Claims

1. A liquid crystalline compound having bicyclo[1.1.1]pentane structure and expressed by the general formula (1) 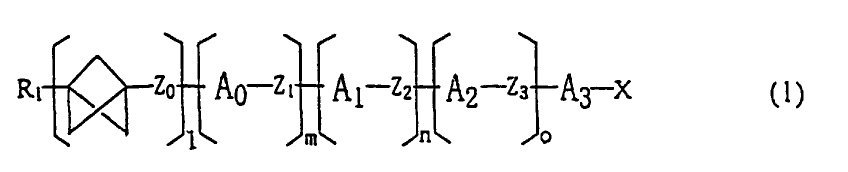 wherein R₁ represents an alkyl group having 1 to 10 carbon atoms in which group not-adjacent any methylene group may be replaced by oxygen atom, sulfur atom, -CH=CH-, -C≡C-, -CO-, -COO-, -OCO-, or SiH₂, and any hydrogen atom in the group may be replaced by a halogen atom; ring A₀, ring A₁, ring A₂, and ring A₃ each independently represent 1,4-cyclohexylene group, 1,3-dioxane-2,5-diyl group, bicyclo[1.1.1]pentane-1,3-diyl group, pyridine-2,5-diyl group, or pyrimidine-2,5-diyl group, or 1,4-phenylene group in which any hydrogen atom on the ring may be replaced by a halogen atom, but at least one of ring A₀, ring A₁, and ring A₂ represents bicyclo[1.1.1]pentane-1,3-diyl group; Z₀, Z₁, Z₂, and Z₃ each independently represent -(CH₂)₂-, -(CH₂)₄-, -CH=CH-, -CH=CH-(CH₂)₂-, -(CH₂)₂-CH=CH-, -C≡C-, -CH₂O-, -OCH₂-, -COO-, -OCO-, -CF₂O-, -OCF₂-, -CF=CF-, or single bond; X represents a halogen atom, cyano group, or an alkyl group having 1 to 10 carbon atoms in which alkyl group not-adjacent any methylene group may be replaced by oxygen atom, sulfur atom, -CH=CH-, -C≡C-, -CO-, -COO-, -OCO-, or -SiH₂-, and any hydrogen atom in this group may be replaced by a halogen atom; ℓ, m, n, and o are each independently an integer of 0 to 4, but ℓ + m + n + o ≦ 4, and when ℓ = 0, then m + n + o ≧ 1; and any atom which constitutes this compound may be replaced by its isotope.

2. The liquid crystalline compound according to claim 1 wherein $\text{ℓ = m = n = o = 1}$ in the general formula (1).

3. The liquid crystalline compound according to claim 1 wherein $\text{ℓ = m = 1}$, $\text{n = o = 0}$, and both ring A₀ and ring A₃ are 1,4-cyclohexylene group in the general formula (1).

4. The liquid crystalline compound according to claim 3 wherein Z₁ is -CH=CH- in the general formula (1).

5. The liquid crystalline compound according to claim 3 wherein X is an alkenyl group in the general formula (1).

6. The liquid crystalline compound according to claim 4 wherein X is an alkenyl group in the general formula (1).

7. The liquid crystalline compound according to claim 1 wherein ℓ = m = 1, $\text{n = o = 0}$, ring A₃ is 1,4-phenylene group in which any hydrogen atom on the ring may be replaced by a halogen atom, and X is a halogen atom, cyano group, -CF₃, -CF₂H, -OCF₃, or -OCHF₂ in the general formula (1).

8. The liquid crystalline compound according to claim 7 wherein Z₁ is -COO- or -CF₂O- in the general formula (1).

9. The liquid crystalline compound according to claim 7 wherein R₁ is an alkenyl group in the general formula (1).

10. The liquid crystalline compound according to claim 1 wherein X is an alkyl group having 3 to 10 carbon atoms in which group any hydrogen atom may be replaced by a halogen atom in the general formula (1).

11. The liquid crystalline compound according to claim 1 wherein at least one of ring A₀, ring A₁, ring A₂, and ring A₃ is 2,3-difluoro-1,4-phenylene group in the general formula (1).

12. The liquid crystalline compound according to claim 1 wherein the compound expressed by the general formula (1) is also expressed by the following general formula (1-1), (1-2), (1-3), or (1-4) 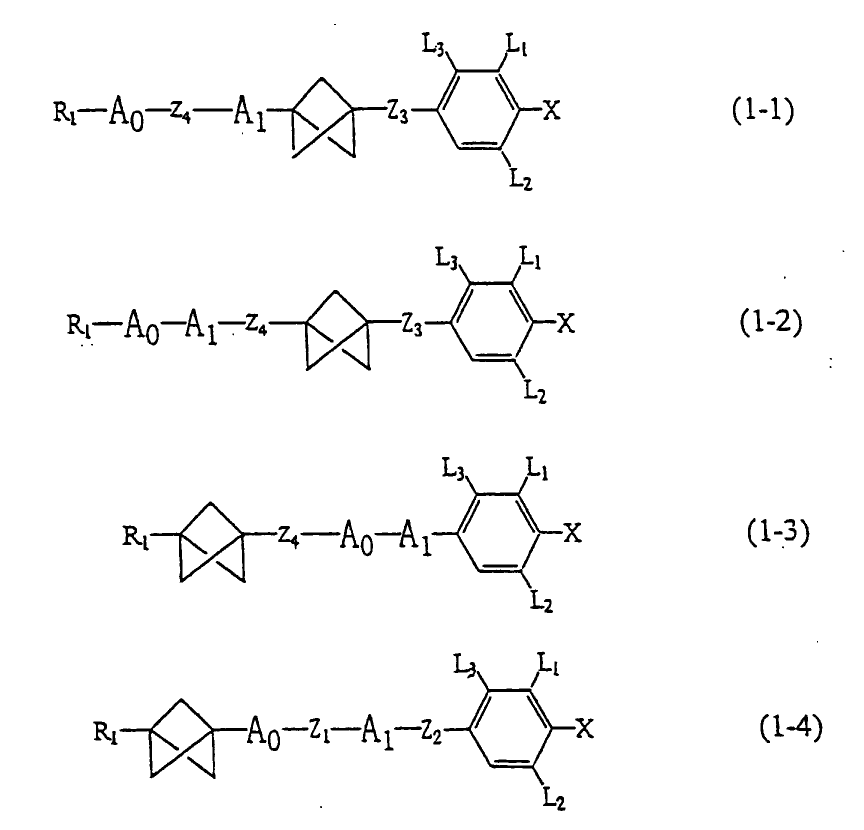 wherein R₁ represents an alkyl group having 1 to 10 carbon atoms in which group not-adjacent any methylene group may be replaced by oxygen atom, sulfur atom, -CH=CH-, -C≡C-, -CO-, -COO-, -OCO-, or -SiH₂-, and any hydrogen atom in this group may be replaced by a halogen atom; ring A₀ and ring A₁ each independently represent 1,4-cyclohexylene group, bicyclo[1.1.1]pentane-1,3-diyl group, 1,3-dioxane-2,5-diyl group, pyridine-2,5-diyl group, or pyrimidine-2,5-diyl group, or 1,4-phenylene group in which any hydrogen atom on the ring may be replaced by a halogen atom; Z₁, Z₂, and Z₃ each independently represent -(CH₂)₂-, -(CH₂)₄-, -CH=CH-, -CH=CH-(CH₂)₂-, -(CH₂)₂CH=CH-, -C≡C-, -CH₂O-, -OCH₂-, -COO-, -OCO-, -CF₂O-, -OCF₂-, -CF=CF-, or single bond; Z₄ represents -CH₂CH₂-, -(CH₂)₄-, -CH=CH-, -CH=CH-(CH₂)₂-, -(CH₂)₂CH=CH-, -C≡C-, -CH₂O-, -OCH₂-, -COO-, -OCO-, -CF₂O-, -OCF₂-, or -CF=CF-; L₁, L₂, and L₃ each independently represent hydrogen atom or a halogen atom; X represents a halogen atom, cyano group, or an alkyl group having 1 to 10 carbon atoms in which alkyl group not-adjacent any methylene group may be replaced by oxygen atom, sulfur atom, -CH=CH-, -C≡C-, -CO-, -COO-, -OCO-, or -SiH₂- and any hydrogen atom in this group may be replaced by a halogen atom; and each atom which constitutes those compounds may be replaced by its isotope.

13. The liquid crystalline compound according to claim 12 wherein X is -CF₃, -CHF₂, -OCF₃, -OCHF₂, or -OCF₂H in the general formula (1-1), (1-2), (1-3), or (1-4).

14. A liquid crystal composition comprising at least two components and comprising at least one liquid crystalline compound expressed by the general formula (1) defined in any one of claims 1 to 13.

15. A liquid crystal composition comprising, as a first component, at least one compound defined in any one of claims 1 to 13, and comprising, as a second component, at least one compound selected from the group consisting of the compounds expressed by any one of the general formulas (2), (3), and (4) 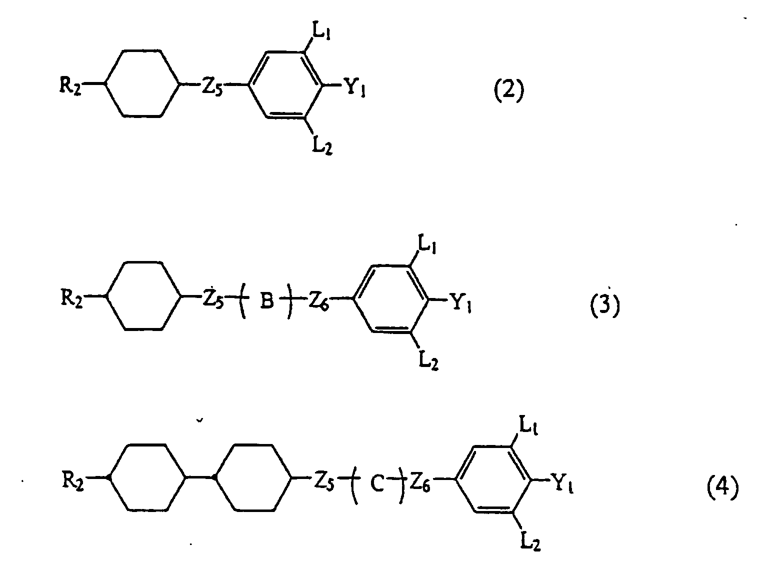 wherein R₂ represents an alkyl group having 1 to 10 carbon atoms in which group not-adjacent any methylene group may be replaced by oxygen atom or -CH=CH-, and any hydrogen atom in the group may be replaced by fluorine atom; Y₁ represents fluorine atom, chlorine atom, -CF₃, -CF₂H, -OCF₃, -OCF₂H, -OCF₂CF₂H, or -OCF₂CFHCF₃; L₁ and L₂ each independently represent hydrogen atom or fluorine atom; Z₅ and Z₆ each independently represent -(CH₂)₂-, -(CH₂)₄-, -CH=CH-, -COO-, -CF₂O-, -OCF₂-, or single bond; ring B represents trans-1,4-cyclohexylene group or 1,3-dioxane-2,5-diyl group, or 1,4-phenylene group in which any hydrogen atom on the ring may be replaced by fluorine atom; ring C represents trans-1,4-cyclohexylene group, or 1,4-phenylene group in which any hydrogen atom on the ring may be replaced by fluorine atom; and any atom which constitutes those compounds may be replaced by its isotope.

16. A liquid crystal composition comprising, as a first component, at least one compound defined in any one of claims 1 to 13, and comprising, as a second component, at least one compound selected from the group consisting of the compounds expressed by the general formula (5) or (6) 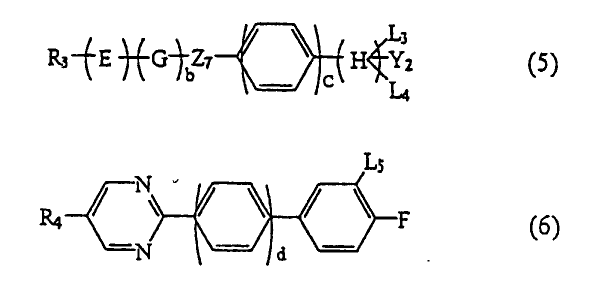 wherein R₃ and R₄ each independently represent an alkyl group having 1 to 10 carbon atoms in which group not-adjacent any methylene group may be replaced by oxygen atom or -CH=CH-, and any hydrogen atom in the group may be replaced by fluorine atom; Y₂ represents cyano group or -C≡C-CN; ring E represents trans-1,4-cyclohexylene group, 1,4-phenylene group, 1,3-dioxane-2,5-diyl group, or pyrimidine-2,5-diyl group; ring G represents trans-1,4-cyclohexylene group, or pyrimidine-2,5-diyl group, or 1,4-phenylene group in which hydrogen atom on the ring may be replaced by fluorine atom; ring H represents trans-1,4-cyclohexylene group or 1,4-phenylene group; Z₇ represents -(CH₂)₂-, -COO-, or single bond; L₃, L₄, and L₅ each independently represent hydrogen atom or fluorine atom; b, c, and d are each independently 0 or 1; and each atom which constitutes those compounds may be replaced by its isotope.

17. A liquid crystal composition comprising, as a first component, at least one compound defined in any one of claims 1 to 13, comprising, as a second component, at least one compound selected from the group consisting of the compounds expressed by any one of the general formulas (2), (3), and (4) described above, and comprising, as a third component, at least one compound selected from the group consisting of the compounds expressed by any one of the general formulas (7), (8), and (9)
R₅(̵I)̵Z₈(̵J)̵Z₉-R₆ (7)
R₅(̵I)̵Z₈(̵J)̵Z₉(̵K)̵R₆ (8) 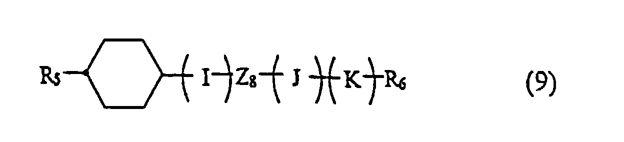
wherein R₅ and R₆ each independently represent an alkyl group having 1 to 10 carbon atoms in which group not-adjacent any methylene group may be replaced by oxygen atom or -CH=CH-, and any hydrogen atom in the group may be replaced by fluorine atom; ring I, ring J, and ring K each independently represent trans-1,4-cyclohexylene group or pyrimidine-2,5-diyl group, or 1,4-phenylene group in which hydrogen atom may be replaced by fluorine atom; Z₈ and Z₉ each independently represent -(CH₂)₂-, -CH=CH-, -C≡C-, -COO-, or single bond; and each atom which constitutes those compounds may be replaced by its isotope.

18. A liquid crystal composition comprising, as a first component, at least one compound defined in any one of claims 1 to 13, and comprising, as a second component, at least one compound selected from the group consisting of the compounds expressed by any one of the general formulas (10), (11), and (12) 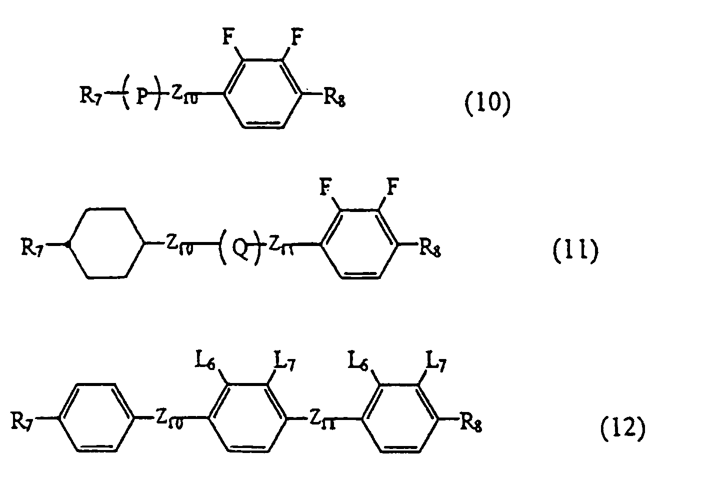 wherein R₇ and R₈ each independently represent an alkyl group having 1 to 10 carbon atoms in which group not-adjacent any methylene group may be replaced by oxygen atom or -CH=CH, and any hydrogen atom in the group may be replaced by fluorine atom; ring P and ring Q each independently represent trans-1,4-cyclohexylene group or 1,4-phenylene group; L₆ and L₇ each independently represent hydrogen atom or fluorine atom but in no case represent L₆ and L₇ simultaneously hydrogen atom; Z₁₀ and Z₁₁ each independently represent -(CH₂)₂-, -COO-, or single bond; and any atom which constitutes those compounds may be replaced by its isotope.

19. A liquid crystal composition comprising, as a first component, at least one compound defined in any one of claims 1 to 13, comprising, as a second component, at least one compound selected from the group consisting of the compounds expressed by any one of the general formulas (7), (8), and (9) described above, and comprising, as a third component, at least one compound selected from the group consisting of the compounds expressed by any one of the general formulas (10), (11), and (12) described above.

20. A liquid crystal composition comprising, as a first component, at least one compound defined in any one of claims 1 to 13, comprising, as a second component, at least one compound selected from the group consisting of the compounds expressed by any one of the general formulas (2), (3), and (4) described above, and comprising, as a third component, at least one component selected from the group consisting of the compounds expressed by any one of the general formulas (7), (8), and (9) described above.

21. A liquid crystal composition comprising, as a first component, at least one compound defined in any one of claims 1 to 13, comprising, as a second component, at least one compound selected from the group consisting of the compounds expressed by the general formula (5) or (6) described above, and comprising, as a third component, at least one compound selected from the group consisting of the compounds expressed by any one of the general formulas (7), (8), and (9) described above.

22. A liquid crystal composition comprising, as a first component, at least one compound defined in any one of claims 1 to 13, comprising, as a second component, at least one compound selected from the group consisting of the compounds expressed by any one of the general formulas (2), (3), and (4) described above, comprising, as a third component, at least one compound selected from the group consisting of the compounds expressed by the general formula (5) or (6) described above, and comprising, as a fourth component, at least one compound selected from the group consisting of the compounds expressed by any one of the general formulas (7), (8), and (9) described above.

23. A liquid crystal composition further comprising an optically active compound in addition to the liquid crystal composition defined in any one of claims 14 to 22.

24. A liquid crystal display device fabricated by using the liquid crystal composition defined in any one of claims 14 to 23.
